(19)

Europäisches
Patentamt
European
Patent Office
Office européen
des brevets

(11) **EP 2 811 036 A2**

(12) **EUROPEAN PATENT APPLICATION**

(43) Date of publication:
**10.12.2014 Bulletin 2014/50**

(51) Int Cl.:
*C12Q 1/68* (2006.01)  *G01N 33/68* (2006.01)

(21) Application number: **14181027.5**

(22) Date of filing: **21.11.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.11.2008  US 117137 P**
**22.11.2008  US 117159 P**
**22.11.2008  US 117142 P**
**22.11.2008  US 117164 P**
**22.11.2008  US 117174 P**
**22.11.2008  US 117170 P**
**22.11.2008  US 117166 P**
**22.11.2008  US 117171 P**
**22.11.2008  US 117165 P**
**22.11.2008  US 117147 P**

(62) Document number(s) of the earlier application(s) in accordance with Art. 76 EPC:
**09828325.2 / 2 364 370**

(71) Applicant: **Astute Medical, Inc.**
**San Diego, CA 92121 (US)**

(72) Inventors:
• **Gray, Jeff**
**Solana Beach, CA 92075 (US)**
• **Anderberg, Joseph**
**Encinitas, CA 92024 (US)**
• **McPherson, Paul**
**Encinitas, CA 92024 (US)**
• **Nakamura, Kevin**
**Cardiff by the Sea, CA 92007 (US)**

(74) Representative: **Schiweck, Weinzierl & Koch**
**European Patent Attorneys**
**Landsberger Straße 98**
**80339 München (DE)**

Remarks:
This application was filed on 14-08-2014 as a divisional application to the application mentioned under INID code 62.

(54) **Methods and compositions for diagnosis and prognosis of renal injury and renal failure**

(57)    The present invention relates to methods and compositions for monitoring, diagnosis, prognosis, and determination of treatment regimens in subjects suffering from or suspected of having a renal injury. In particular, the invention relates to using assays that detect Growth-regulated alpha protein as well as optionally one or more of Epidermal growth factor, Complement C3, Interleukin-4, Interleukin-1 alpha, Tubulointerstitial nephritis antigen, Transforming growth factor bet-1, Bone morphogenetic protein 7, Osteopontin, and Netrin-1, as diagnostic and prognostic biomarkers in renal injuries.

EP 2 811 036 A2

SEQUENCE LISTING

<110> ASTUTE MEDICAL, INC.

<120> METHODS AND COMPOSITIONS FOR DIAGNOSIS AND PROGNOSIS OF RENAL INJURY AND RENAL FAILURE

<130> BLG14168PCTEPD1

<141> 2009-11-21

<150> 61/117,137
<151> 2008-11-22

<150> 61/117,142
<151> 2008-11-22

<150> 61/117,147
<151> 2008-11-22

<150> 61/117,159
<151> 2008-11-22

<150> 61/117,164
<151> 2008-11-22

<150> 61/117,165
<151> 2008-11-22

<150> 61/117,166
<151> 2008-11-22

<150> 61/117,170
<151> 2008-11-22

<150> 61/117,171
<151> 2008-11-22

<150> 61/117,174
<151> 2008-11-22

<160> 10

<170> PatentIn version 3.5

<210> 1
<211> 1207
<212> PRT
<213> Homo sapiens

<400> 1
Met Leu Leu Thr Leu Ile Ile Leu Leu Pro Val Val Ser Lys Phe Ser
1               5                   10                  15


Phe Val Ser Leu Ser Ala Pro Gln His Trp Ser Cys Pro Glu Gly Thr
                20                  25                  30


Leu Ala Gly Asn Gly Asn Ser Thr Cys Val Gly Pro Ala Pro Phe Leu
                35                  40                  45

```
Ile Phe Ser His Gly Asn Ser Ile Phe Arg Ile Asp Thr Glu Gly Thr
    50              55              60

Asn Tyr Glu Gln Leu Val Val Asp Ala Gly Val Ser Val Ile Met Asp
65          70              75                          80

Phe His Tyr Asn Glu Lys Arg Ile Tyr Trp Val Asp Leu Glu Arg Gln
            85              90                          95

Leu Leu Gln Arg Val Phe Leu Asn Gly Ser Arg Gln Glu Arg Val Cys
            100             105             110

Asn Ile Glu Lys Asn Val Ser Gly Met Ala Ile Asn Trp Ile Asn Glu
            115             120             125

Glu Val Ile Trp Ser Asn Gln Gln Glu Gly Ile Ile Thr Val Thr Asp
            130             135             140

Met Lys Gly Asn Asn Ser His Ile Leu Leu Ser Ala Leu Lys Tyr Pro
145             150             155                         160

Ala Asn Val Ala Val Asp Pro Val Glu Arg Phe Ile Phe Trp Ser Ser
            165                 170                     175

Glu Val Ala Gly Ser Leu Tyr Arg Ala Asp Leu Asp Gly Val Gly Val
            180                 185             190

Lys Ala Leu Leu Glu Thr Ser Glu Lys Ile Thr Ala Val Ser Leu Asp
            195             200             205

Val Leu Asp Lys Arg Leu Phe Trp Ile Gln Tyr Asn Arg Glu Gly Ser
210                 215             220

Asn Ser Leu Ile Cys Ser Cys Asp Tyr Asp Gly Gly Ser Val His Ile
225             230             235                         240

Ser Lys His Pro Thr Gln His Asn Leu Phe Ala Met Ser Leu Phe Gly
            245             250             255

Asp Arg Ile Phe Tyr Ser Thr Trp Lys Met Lys Thr Ile Trp Ile Ala
            260             265             270

Asn Lys His Thr Gly Lys Asp Met Val Arg Ile Asn Leu His Ser Ser
            275             280             285

Phe Val Pro Leu Gly Glu Leu Lys Val Val His Pro Leu Ala Gln Pro
290             295             300
```

Lys Ala Glu Asp Asp Thr Trp Glu Pro Glu Gln Lys Leu Cys Lys Leu
305                 310                 315                 320

Arg Lys Gly Asn Cys Ser Ser Thr Val Cys Gly Gln Asp Leu Gln Ser
                325                 330                 335

His Leu Cys Met Cys Ala Glu Gly Tyr Ala Leu Ser Arg Asp Arg Lys
            340                 345                 350

Tyr Cys Glu Asp Val Asn Glu Cys Ala Phe Trp Asn His Gly Cys Thr
    355                 360                 365

Leu Gly Cys Lys Asn Thr Pro Gly Ser Tyr Tyr Cys Thr Cys Pro Val
    370                 375                 380

Gly Phe Val Leu Leu Pro Asp Gly Lys Arg Cys His Gln Leu Val Ser
385                 390                 395                 400

Cys Pro Arg Asn Val Ser Glu Cys Ser His Asp Cys Val Leu Thr Ser
                405                 410                 415

Glu Gly Pro Leu Cys Phe Cys Pro Glu Gly Ser Val Leu Glu Arg Asp
            420                 425                 430

Gly Lys Thr Cys Ser Gly Cys Ser Ser Pro Asp Asn Gly Gly Cys Ser
    435                 440                 445

Gln Leu Cys Val Pro Leu Ser Pro Val Ser Trp Glu Cys Asp Cys Phe
    450                 455                 460

Pro Gly Tyr Asp Leu Gln Leu Asp Glu Lys Ser Cys Ala Ala Ser Gly
465                 470                 475                 480

Pro Gln Pro Phe Leu Leu Phe Ala Asn Ser Gln Asp Ile Arg His Met
            485                 490                 495

His Phe Asp Gly Thr Asp Tyr Gly Thr Leu Leu Ser Gln Gln Met Gly
            500                 505                 510

Met Val Tyr Ala Leu Asp His Asp Pro Val Glu Asn Lys Ile Tyr Phe
        515                 520                 525

Ala His Thr Ala Leu Lys Trp Ile Glu Arg Ala Asn Met Asp Gly Ser
    530                 535                 540

Gln Arg Glu Arg Leu Ile Glu Glu Gly Val Asp Val Pro Glu Gly Leu
545                 550                 555                 560

Ala Val Asp Trp Ile Gly Arg Arg Phe Tyr Trp Thr Asp Arg Gly Lys
565 570 575

Ser Leu Ile Gly Arg Ser Asp Leu Asn Gly Lys Arg Ser Lys Ile Ile
580 585 590

Thr Lys Glu Asn Ile Ser Gln Pro Arg Gly Ile Ala Val His Pro Met
595 600 605

Ala Lys Arg Leu Phe Trp Thr Asp Thr Gly Ile Asn Pro Arg Ile Glu
610 615 620

Ser Ser Ser Leu Gln Gly Leu Gly Arg Leu Val Ile Ala Ser Ser Asp
625 630 635 640

Leu Ile Trp Pro Ser Gly Ile Thr Ile Asp Phe Leu Thr Asp Lys Leu
645 650 655

Tyr Trp Cys Asp Ala Lys Gln Ser Val Ile Glu Met Ala Asn Leu Asp
660 665 670

Gly Ser Lys Arg Arg Arg Leu Thr Gln Asn Asp Val Gly His Pro Phe
675 680 685

Ala Val Ala Val Phe Glu Asp Tyr Val Trp Phe Ser Asp Trp Ala Met
690 695 700

Pro Ser Val Ile Arg Val Asn Lys Arg Thr Gly Lys Asp Arg Val Arg
705 710 715 720

Leu Gln Gly Ser Met Leu Lys Pro Ser Ser Leu Val Val Val His Pro
725 730 735

Leu Ala Lys Pro Gly Ala Asp Pro Cys Leu Tyr Gln Asn Gly Gly Cys
740 745 750

Glu His Ile Cys Lys Lys Arg Leu Gly Thr Ala Trp Cys Ser Cys Arg
755 760 765

Glu Gly Phe Met Lys Ala Ser Asp Gly Lys Thr Cys Leu Ala Leu Asp
770 775 780

Gly His Gln Leu Leu Ala Gly Gly Glu Val Asp Leu Lys Asn Gln Val
785 790 795 800

Thr Pro Leu Asp Ile Leu Ser Lys Thr Arg Val Ser Glu Asp Asn Ile

805                               810                               815

Thr Glu Ser Gln His Met Leu Val Ala Glu Ile Met Val Ser Asp Gln
            820                   825                   830

Asp Asp Cys Ala Pro Val Gly Cys Ser Met Tyr Ala Arg Cys Ile Ser
            835                   840                   845

Glu Gly Glu Asp Ala Thr Cys Gln Cys Leu Lys Gly Phe Ala Gly Asp
            850                   855                   860

Gly Lys Leu Cys Ser Asp Ile Asp Glu Cys Glu Met Gly Val Pro Val
865                   870                   875                   880

Cys Pro Pro Ala Ser Ser Lys Cys Ile Asn Thr Glu Gly Gly Tyr Val
                  885                   890                   895

Cys Arg Cys Ser Glu Gly Tyr Gln Gly Asp Gly Ile His Cys Leu Asp
            900                   905                   910

Ile Asp Glu Cys Gln Leu Gly Val His Ser Cys Gly Glu Asn Ala Ser
            915                   920                   925

Cys Thr Asn Thr Glu Gly Gly Tyr Thr Cys Met Cys Ala Gly Arg Leu
930                   935                   940

Ser Glu Pro Gly Leu Ile Cys Pro Asp Ser Thr Pro Pro Pro His Leu
945                   950                   955                   960

Arg Glu Asp Asp His His Tyr Ser Val Arg Asn Ser Asp Ser Glu Cys
                  965                   970                   975

Pro Leu Ser His Asp Gly Tyr Cys Leu His Asp Gly Val Cys Met Tyr
                  980                   985                   990

Ile Glu Ala Leu Asp Lys Tyr Ala Cys Asn Cys Val Val Gly Tyr Ile
            995                   1000                  1005

Gly Glu Arg Cys Gln Tyr Arg Asp Leu Lys Trp Trp Glu Leu Arg
      1010                  1015                  1020

His Ala Gly His Gly Gln Gln Gln Lys Val Ile Val Val Ala Val
      1025                  1030                  1035

Cys Val Val Val Leu Val Met Leu Leu Leu Leu Ser Leu Trp Gly
      1040                  1045                  1050

```
Ala His  Tyr Tyr Arg Thr Gln  Lys Leu Leu Ser Lys  Asn Pro Lys
    1055                1060                1065


Asn Pro  Tyr Glu Glu Ser Ser  Arg Asp Val Arg Ser  Arg Arg Pro
    1070                1075                1080


Ala Asp  Thr Glu Asp Gly Met  Ser Ser Cys Pro Gln  Pro Trp Phe
    1085                1090                1095


Val Val  Ile Lys Glu His Gln  Asp Leu Lys Asn Gly  Gly Gln Pro
    1100                1105                1110


Val Ala  Gly Glu Asp Gly Gln  Ala Ala Asp Gly Ser  Met Gln Pro
    1115                1120                1125


Thr Ser  Trp Arg Gln Glu Pro  Gln Leu Cys Gly Met  Gly Thr Glu
    1130                1135                1140


Gln Gly  Cys Trp Ile Pro Val  Ser Ser Asp Lys Gly  Ser Cys Pro
    1145                1150                1155


Gln Val  Met Glu Arg Ser Phe  His Met Pro Ser Tyr  Gly Thr Gln
    1160                1165                1170


Thr Leu  Glu Gly Gly Val Glu  Lys Pro His Ser Leu  Leu Ser Ala
    1175                1180                1185


Asn Pro  Leu Trp Gln Gln Arg  Ala Leu Asp Pro Pro  His Gln Met
    1190                1195                1200


Glu Leu  Thr Gln
    1205


<210> 2
<211> 1663
<212> PRT
<213> Homo sapiens

<400> 2
Met Gly Pro Thr Ser Gly Pro Ser Leu Leu Leu Leu Leu Leu Thr His
1                   5                   10                  15


Leu Pro Leu Ala Leu Gly Ser Pro Met Tyr Ser Ile Ile Thr Pro Asn
            20                  25                  30


Ile Leu Arg Leu Glu Ser Glu Glu Thr Met Val Leu Glu Ala His Asp
        35                  40                  45


Ala Gln Gly Asp Val Pro Val Thr Val Thr Val His Asp Phe Pro Gly
```

42

                    50                          55                              60

    Lys Lys Leu Val Leu Ser Ser Glu Lys Thr Val Leu Thr Pro Ala Thr
    65                  70                  75                  80

    Asn His Met Gly Asn Val Thr Phe Thr Ile Pro Ala Asn Arg Glu Phe
                    85                  90                  95

    Lys Ser Glu Lys Gly Arg Asn Lys Phe Val Thr Val Gln Ala Thr Phe
                    100                 105                 110

    Gly Thr Gln Val Val Glu Lys Val Val Leu Val Ser Leu Gln Ser Gly
                    115                 120                 125

    Tyr Leu Phe Ile Gln Thr Asp Lys Thr Ile Tyr Thr Pro Gly Ser Thr
                    130                 135                 140

    Val Leu Tyr Arg Ile Phe Thr Val Asn His Lys Leu Leu Pro Val Gly
    145                 150                 155                 160

    Arg Thr Val Met Val Asn Ile Glu Asn Pro Glu Gly Ile Pro Val Lys
                    165                 170                 175

    Gln Asp Ser Leu Ser Ser Gln Asn Gln Leu Gly Val Leu Pro Leu Ser
                    180                 185                 190

    Trp Asp Ile Pro Glu Leu Val Asn Met Gly Gln Trp Lys Ile Arg Ala
                    195                 200                 205

    Tyr Tyr Glu Asn Ser Pro Gln Gln Val Phe Ser Thr Glu Phe Glu Val
                    210                 215                 220

    Lys Glu Tyr Val Leu Pro Ser Phe Glu Val Ile Val Glu Pro Thr Glu
    225                 230                 235                 240

    Lys Phe Tyr Tyr Ile Tyr Asn Glu Lys Gly Leu Glu Val Thr Ile Thr
                    245                 250                 255

    Ala Arg Phe Leu Tyr Gly Lys Lys Val Glu Gly Thr Ala Phe Val Ile
                    260                 265                 270

    Phe Gly Ile Gln Asp Gly Glu Gln Arg Ile Ser Leu Pro Glu Ser Leu
                    275                 280                 285

    Lys Arg Ile Pro Ile Glu Asp Gly Ser Gly Glu Val Val Leu Ser Arg
                    290                 295                 300

Lys Val Leu Leu Asp Gly Val Gln Asn Pro Arg Ala Glu Asp Leu Val
305 310 315 320

Gly Lys Ser Leu Tyr Val Ser Ala Thr Val Ile Leu His Ser Gly Ser
325 330 335

Asp Met Val Gln Ala Glu Arg Ser Gly Ile Pro Ile Val Thr Ser Pro
340 345 350

Tyr Gln Ile His Phe Thr Lys Thr Pro Lys Tyr Phe Lys Pro Gly Met
355 360 365

Pro Phe Asp Leu Met Val Phe Val Thr Asn Pro Asp Gly Ser Pro Ala
370 375 380

Tyr Arg Val Pro Val Ala Val Gln Gly Glu Asp Thr Val Gln Ser Leu
385 390 395 400

Thr Gln Gly Asp Gly Val Ala Lys Leu Ser Ile Asn Thr His Pro Ser
405 410 415

Gln Lys Pro Leu Ser Ile Thr Val Arg Thr Lys Lys Gln Glu Leu Ser
420 425 430

Glu Ala Glu Gln Ala Thr Arg Thr Met Gln Ala Leu Pro Tyr Ser Thr
435 440 445

Val Gly Asn Ser Asn Asn Tyr Leu His Leu Ser Val Leu Arg Thr Glu
450 455 460

Leu Arg Pro Gly Glu Thr Leu Asn Val Asn Phe Leu Leu Arg Met Asp
465 470 475 480

Arg Ala His Glu Ala Lys Ile Arg Tyr Tyr Thr Tyr Leu Ile Met Asn
485 490 495

Lys Gly Arg Leu Leu Lys Ala Gly Arg Gln Val Arg Glu Pro Gly Gln
500 505 510

Asp Leu Val Val Leu Pro Leu Ser Ile Thr Thr Asp Phe Ile Pro Ser
515 520 525

Phe Arg Leu Val Ala Tyr Tyr Thr Leu Ile Gly Ala Ser Gly Gln Arg
530 535 540

Glu Val Val Ala Asp Ser Val Trp Val Asp Val Lys Asp Ser Cys Val
545 550 555 560

```
Gly Ser Leu Val Val Lys Ser Gly Gln Ser Glu Asp Arg Gln Pro Val
            565             570             575

Pro Gly Gln Gln Met Thr Leu Lys Ile Glu Gly Asp His Gly Ala Arg
            580             585             590

Val Val Leu Val Ala Val Asp Lys Gly Val Phe Val Leu Asn Lys Lys
            595             600             605

Asn Lys Leu Thr Gln Ser Lys Ile Trp Asp Val Val Glu Lys Ala Asp
    610             615             620

Ile Gly Cys Thr Pro Gly Ser Gly Lys Asp Tyr Ala Gly Val Phe Ser
625             630             635             640

Asp Ala Gly Leu Thr Phe Thr Ser Ser Ser Gly Gln Gln Thr Ala Gln
            645             650             655

Arg Ala Glu Leu Gln Cys Pro Gln Pro Ala Ala Arg Arg Arg Arg Ser
            660             665             670

Val Gln Leu Thr Glu Lys Arg Met Asp Lys Val Gly Lys Tyr Pro Lys
            675             680             685

Glu Leu Arg Lys Cys Cys Glu Asp Gly Met Arg Glu Asn Pro Met Arg
    690             695             700

Phe Ser Cys Gln Arg Arg Thr Arg Phe Ile Ser Leu Gly Glu Ala Cys
705             710             715             720

Lys Lys Val Phe Leu Asp Cys Cys Asn Tyr Ile Thr Glu Leu Arg Arg
            725             730             735

Gln His Ala Arg Ala Ser His Leu Gly Leu Ala Arg Ser Asn Leu Asp
            740             745             750

Glu Asp Ile Ile Ala Glu Glu Asn Ile Val Ser Arg Ser Glu Phe Pro
    755             760             765

Glu Ser Trp Leu Trp Asn Val Glu Asp Leu Lys Glu Pro Pro Lys Asn
    770             775             780

Gly Ile Ser Thr Lys Leu Met Asn Ile Phe Leu Lys Asp Ser Ile Thr
785             790             795             800

Thr Trp Glu Ile Leu Ala Val Ser Met Ser Asp Lys Lys Gly Ile Cys
            805             810             815
```

45

```
Val Ala Asp Pro Phe Glu Val Thr Val Met Gln Asp Phe Phe Ile Asp
            820                 825                 830

Leu Arg Leu Pro Tyr Ser Val Val Arg Asn Glu Gln Val Glu Ile Arg
            835                 840                 845

Ala Val Leu Tyr Asn Tyr Arg Gln Asn Gln Glu Leu Lys Val Arg Val
            850                 855                 860

Glu Leu Leu His Asn Pro Ala Phe Cys Ser Leu Ala Thr Thr Lys Arg
865                 870                 875                 880

Arg His Gln Gln Thr Val Thr Ile Pro Pro Lys Ser Ser Leu Ser Val
                885                 890                 895

Pro Tyr Val Ile Val Pro Leu Lys Thr Gly Leu Gln Glu Val Glu Val
            900                 905                 910

Lys Ala Ala Val Tyr His His Phe Ile Ser Asp Gly Val Arg Lys Ser
            915                 920                 925

Leu Lys Val Val Pro Glu Gly Ile Arg Met Asn Lys Thr Val Ala Val
            930                 935                 940

Arg Thr Leu Asp Pro Glu Arg Leu Gly Arg Glu Gly Val Gln Lys Glu
945                 950                 955                 960

Asp Ile Pro Pro Ala Asp Leu Ser Asp Gln Val Pro Asp Thr Glu Ser
                965                 970                 975

Glu Thr Arg Ile Leu Leu Gln Gly Thr Pro Val Ala Gln Met Thr Glu
            980                 985                 990

Asp Ala Val Asp Ala Glu Arg Leu Lys His Leu Ile Val Thr Pro Ser
            995                 1000                1005

Gly Cys Gly Glu Gln Asn Met Ile Gly Met Thr Pro Thr Val Ile
            1010                1015                1020

Ala Val His Tyr Leu Asp Glu Thr Glu Gln Trp Glu Lys Phe Gly
            1025                1030                1035

Leu Glu Lys Arg Gln Gly Ala Leu Glu Leu Ile Lys Lys Gly Tyr
            1040                1045                1050

Thr Gln Gln Leu Ala Phe Arg Gln Pro Ser Ser Ala Phe Ala Ala
```

```
        1055                    1060                    1065


Phe Val Lys Arg Ala Pro Ser  Thr Trp Leu Thr Ala  Tyr Val Val
    1070                1075                1080


Lys Val Phe Ser Leu Ala Val  Asn Leu Ile Ala Ile  Asp Ser Gln
    1085                1090                1095


Val Leu Cys Gly Ala Val Lys  Trp Leu Ile Leu Glu  Lys Gln Lys
    1100                1105                1110


Pro Asp Gly Val Phe Gln Glu  Asp Ala Pro Val Ile  His Gln Glu
    1115                1120                1125


Met Ile Gly Gly Leu Arg Asn  Asn Asn Glu Lys Asp  Met Ala Leu
    1130                1135                1140


Thr Ala Phe Val Leu Ile Ser  Leu Gln Glu Ala Lys  Asp Ile Cys
    1145                1150                1155


Glu Glu Gln Val Asn Ser Leu  Pro Gly Ser Ile Thr  Lys Ala Gly
    1160                1165                1170


Asp Phe Leu Glu Ala Asn Tyr  Met Asn Leu Gln Arg  Ser Tyr Thr
    1175                1180                1185


Val Ala Ile Ala Gly Tyr Ala  Leu Ala Gln Met Gly  Arg Leu Lys
    1190                1195                1200


Gly Pro Leu Leu Asn Lys Phe  Leu Thr Thr Ala Lys  Asp Lys Asn
    1205                1210                1215


Arg Trp Glu Asp Pro Gly Lys  Gln Leu Tyr Asn Val  Glu Ala Thr
    1220                1225                1230


Ser Tyr Ala Leu Leu Ala Leu  Leu Gln Leu Lys Asp  Phe Asp Phe
    1235                1240                1245


Val Pro Pro Val Val Arg Trp  Leu Asn Glu Gln Arg  Tyr Tyr Gly
    1250                1255                1260


Gly Gly Tyr Gly Ser Thr Gln  Ala Thr Phe Met Val  Phe Gln Ala
    1265                1270                1275


Leu Ala Gln Tyr Gln Lys Asp  Ala Pro Asp His Gln  Glu Leu Asn
    1280                1285                1290
```

```
Leu Asp Val Ser Leu Gln Leu Pro Ser Arg Ser Ser Lys Ile Thr
    1295             1300             1305

His Arg Ile His Trp Glu Ser Ala Ser Leu Leu Arg Ser Glu Glu
    1310             1315             1320

Thr Lys Glu Asn Glu Gly Phe Thr Val Thr Ala Glu Gly Lys Gly
    1325             1330             1335

Gln Gly Thr Leu Ser Val Val Thr Met Tyr His Ala Lys Ala Lys
    1340             1345             1350

Asp Gln Leu Thr Cys Asn Lys Phe Asp Leu Lys Val Thr Ile Lys
    1355             1360             1365

Pro Ala Pro Glu Thr Glu Lys Arg Pro Gln Asp Ala Lys Asn Thr
    1370             1375             1380

Met Ile Leu Glu Ile Cys Thr Arg Tyr Arg Gly Asp Gln Asp Ala
    1385             1390             1395

Thr Met Ser Ile Leu Asp Ile Ser Met Met Thr Gly Phe Ala Pro
    1400             1405             1410

Asp Thr Asp Asp Leu Lys Gln Leu Ala Asn Gly Val Asp Arg Tyr
    1415             1420             1425

Ile Ser Lys Tyr Glu Leu Asp Lys Ala Phe Ser Asp Arg Asn Thr
    1430             1435             1440

Leu Ile Ile Tyr Leu Asp Lys Val Ser His Ser Glu Asp Asp Cys
    1445             1450             1455

Leu Ala Phe Lys Val His Gln Tyr Phe Asn Val Glu Leu Ile Gln
    1460             1465             1470

Pro Gly Ala Val Lys Val Tyr Ala Tyr Tyr Asn Leu Glu Glu Ser
    1475             1480             1485

Cys Thr Arg Phe Tyr His Pro Glu Lys Glu Asp Gly Lys Leu Asn
    1490             1495             1500

Lys Leu Cys Arg Asp Glu Leu Cys Arg Cys Ala Glu Glu Asn Cys
    1505             1510             1515

Phe Ile Gln Lys Ser Asp Asp Lys Val Thr Leu Glu Glu Arg Leu
    1520             1525             1530
```

48

```
Asp Lys  Ala Cys Glu Pro Gly  Val Asp Tyr Val Tyr  Lys Thr Arg
    1535             1540             1545

Leu Val  Lys Val Gln Leu Ser  Asn Asp Phe Asp Glu  Tyr Ile Met
    1550             1555             1560

Ala Ile  Glu Gln Thr Ile Lys  Ser Gly Ser Asp Glu  Val Gln Val
    1565             1570             1575

Gly Gln  Gln Arg Thr Phe Ile  Ser Pro Ile Lys Cys  Arg Glu Ala
    1580             1585             1590

Leu Lys  Leu Glu Glu Lys Lys  His Tyr Leu Met Trp  Gly Leu Ser
    1595             1600             1605

Ser Asp  Phe Trp Gly Glu Lys  Pro Asn Leu Ser Tyr  Ile Ile Gly
    1610             1615             1620

Lys Asp  Thr Trp Val Glu His  Trp Pro Glu Glu Asp  Glu Cys Gln
    1625             1630             1635

Asp Glu  Glu Asn Gln Lys Gln  Cys Gln Asp Leu Gly  Ala Phe Thr
    1640             1645             1650

Glu Ser  Met Val Val Phe Gly  Cys Pro Asn
    1655             1660
```

```
<210> 3
<211> 153
<212> PRT
<213> Homo sapiens

<400> 3
Met Gly Leu Thr Ser Gln Leu Leu Pro Pro Leu Phe Phe Leu Leu Ala
1               5               10              15

Cys Ala Gly Asn Phe Val His Gly His Lys Cys Asp Ile Thr Leu Gln
            20              25              30

Glu Ile Ile Lys Thr Leu Asn Ser Leu Thr Glu Gln Lys Thr Leu Cys
        35              40              45

Thr Glu Leu Thr Val Thr Asp Ile Phe Ala Ala Ser Lys Asn Thr Thr
    50              55              60

Glu Lys Glu Thr Phe Cys Arg Ala Ala Thr Val Leu Arg Gln Phe Tyr
65              70              75              80
```

```
Ser His His Glu Lys Asp Thr Arg Cys Leu Gly Ala Thr Ala Gln Gln
                85                  90                  95

Phe His Arg His Lys Gln Leu Ile Arg Phe Leu Lys Arg Leu Asp Arg
            100                 105                 110

Asn Leu Trp Gly Leu Ala Gly Leu Asn Ser Cys Pro Val Lys Glu Ala
            115                 120                 125

Asn Gln Ser Thr Leu Glu Asn Phe Leu Glu Arg Leu Lys Thr Ile Met
    130                 135                 140

Arg Glu Lys Tyr Ser Lys Cys Ser Ser
145                 150


<210> 4
<211> 271
<212> PRT
<213> Homo sapiens

<400> 4
Met Ala Lys Val Pro Asp Met Phe Glu Asp Leu Lys Asn Cys Tyr Ser
1               5                   10                  15

Glu Asn Glu Glu Asp Ser Ser Ser Ile Asp His Leu Ser Leu Asn Gln
            20                  25                  30

Lys Ser Phe Tyr His Val Ser Tyr Gly Pro Leu His Glu Gly Cys Met
            35                  40                  45

Asp Gln Ser Val Ser Leu Ser Ile Ser Glu Thr Ser Lys Thr Ser Lys
    50                  55                  60

Leu Thr Phe Lys Glu Ser Met Val Val Val Ala Thr Asn Gly Lys Val
65                  70                  75                  80

Leu Lys Lys Arg Arg Leu Ser Leu Ser Gln Ser Ile Thr Asp Asp Asp
                85                  90                  95

Leu Glu Ala Ile Ala Asn Asp Ser Glu Glu Glu Ile Ile Lys Pro Arg
            100                 105                 110

Ser Ala Pro Phe Ser Phe Leu Ser Asn Val Lys Tyr Asn Phe Met Arg
            115                 120                 125

Ile Ile Lys Tyr Glu Phe Ile Leu Asn Asp Ala Leu Asn Gln Ser Ile
    130                 135                 140

Ile Arg Ala Asn Asp Gln Tyr Leu Thr Ala Ala Ala Leu His Asn Leu
```

145                150                155                160

Asp Glu Ala Val Lys Phe Asp Met Gly Ala Tyr Lys Ser Ser Lys Asp
             165            170           175

Asp Ala Lys Ile Thr Val Ile Leu Arg Ile Ser Lys Thr Gln Leu Tyr
             180           185           190

Val Thr Ala Gln Asp Glu Asp Gln Pro Val Leu Leu Lys Glu Met Pro
             195           200           205

Glu Ile Pro Lys Thr Ile Thr Gly Ser Glu Thr Asn Leu Leu Phe Phe
             210           215           220

Trp Glu Thr His Gly Thr Lys Asn Tyr Phe Thr Ser Val Ala His Pro
225                230           235          240

Asn Leu Phe Ile Ala Thr Lys Gln Asp Tyr Trp Val Cys Leu Ala Gly
             245           250           255

Gly Pro Pro Ser Ile Thr Asp Phe Gln Ile Leu Glu Asn Gln Ala
             260           265          270


<210> 5
<211> 476
<212> PRT
<213> Homo sapiens

<400> 5
Met Trp Thr Gly Tyr Lys Ile Leu Ile Phe Ser Tyr Leu Thr Thr Glu
1            5                10             15

Ile Trp Met Glu Lys Gln Tyr Leu Ser Gln Arg Glu Val Asp Leu Glu
             20           25           30

Ala Tyr Phe Thr Arg Asn His Thr Val Leu Gln Gly Thr Arg Phe Lys
             35           40           45

Arg Ala Ile Phe Gln Gly Gln Tyr Cys Arg Asn Phe Gly Cys Cys Glu
             50           55           60

Asp Arg Asp Asp Gly Cys Val Thr Glu Phe Tyr Ala Ala Asn Ala Leu
65              70           75          80

Cys Tyr Cys Asp Lys Phe Cys Asp Arg Glu Asn Ser Asp Cys Cys Pro
             85           90          95

Asp Tyr Lys Ser Phe Cys Arg Glu Glu Lys Glu Trp Pro Pro His Thr
             100          105         110

```
Gln Pro Trp Tyr Pro Glu Gly Cys Phe Lys Asp Gly Gln His Tyr Glu
        115                 120                 125

Glu Gly Ser Val Ile Lys Glu Asn Cys Asn Ser Cys Thr Cys Ser Gly
        130                 135                 140

Gln Gln Trp Lys Cys Ser Gln His Val Cys Leu Val Arg Pro Glu Leu
145                 150                 155                 160

Ile Glu Gln Val Asn Lys Gly Asp Tyr Gly Trp Thr Ala Gln Asn Tyr
                165                 170                 175

Ser Gln Phe Trp Gly Met Thr Leu Glu Asp Gly Phe Lys Phe Arg Leu
            180                 185                 190

Gly Thr Leu Pro Pro Ser Pro Met Leu Leu Ser Met Asn Glu Met Thr
        195                 200                 205

Ala Ser Leu Pro Ala Thr Thr Asp Leu Pro Glu Phe Phe Val Ala Ser
    210                 215                 220

Tyr Lys Trp Pro Gly Trp Thr His Gly Pro Leu Asp Gln Lys Asn Cys
225                 230                 235                 240

Ala Ala Ser Trp Ala Phe Ser Thr Ala Ser Val Ala Ala Asp Arg Ile
            245                 250                 255

Ala Ile Gln Ser Lys Gly Arg Tyr Thr Ala Asn Leu Ser Pro Gln Asn
        260                 265                 270

Leu Ile Ser Cys Cys Ala Lys Asn Arg His Gly Cys Asn Ser Gly Ser
        275                 280                 285

Ile Asp Arg Ala Trp Trp Tyr Leu Arg Lys Arg Gly Leu Val Ser His
    290                 295                 300

Ala Cys Tyr Pro Leu Phe Lys Asp Gln Asn Ala Thr Asn Asn Gly Cys
305                 310                 315                 320

Ala Met Ala Ser Arg Ser Asp Gly Arg Gly Lys Arg His Ala Thr Lys
            325                 330                 335

Pro Cys Pro Asn Asn Val Glu Lys Ser Asn Arg Ile Tyr Gln Cys Ser
        340                 345                 350

Pro Pro Tyr Arg Val Ser Ser Asn Glu Thr Glu Ile Met Lys Glu Ile
```

355                    360                    365

Met Gln Asn Gly Pro Val Gln Ala Ile Met Gln Val Arg Glu Asp Phe
    370                375                380

Phe His Tyr Lys Thr Gly Ile Tyr Arg His Val Thr Ser Thr Asn Lys
385                390                395                400

Glu Ser Glu Lys Tyr Arg Lys Leu Gln Thr His Ala Val Lys Leu Thr
                405                410                415

Gly Trp Gly Thr Leu Arg Gly Ala Gln Gly Gln Lys Glu Lys Phe Trp
                420                425                430

Ile Ala Ala Asn Ser Trp Gly Lys Ser Trp Gly Glu Asn Gly Tyr Phe
    435                440                445

Arg Ile Leu Arg Gly Val Asn Glu Ser Asp Ile Glu Lys Leu Ile Ile
    450                455                460

Ala Ala Trp Gly Gln Leu Thr Ser Ser Asp Glu Pro
465                470                475

<210> 6
<211> 390
<212> PRT
<213> Homo sapiens

<400> 6
Met Pro Pro Ser Gly Leu Arg Leu Leu Leu Leu Leu Leu Pro Leu Leu
1                5                10                15

Trp Leu Leu Val Leu Thr Pro Gly Arg Pro Ala Ala Gly Leu Ser Thr
                20                25                30

Cys Lys Thr Ile Asp Met Glu Leu Val Lys Arg Lys Arg Ile Glu Ala
    35                40                45

Ile Arg Gly Gln Ile Leu Ser Lys Leu Arg Leu Ala Ser Pro Pro Ser
    50                55                60

Gln Gly Glu Val Pro Pro Gly Pro Leu Pro Glu Ala Val Leu Ala Leu
65                70                75                80

Tyr Asn Ser Thr Arg Asp Arg Val Ala Gly Glu Ser Ala Glu Pro Glu
                85                90                95

Pro Glu Pro Glu Ala Asp Tyr Tyr Ala Lys Glu Val Thr Arg Val Leu
                100                105                110

```
Met Val Glu Thr His Asn Glu Ile Tyr Asp Lys Phe Lys Gln Ser Thr
        115                 120             125

His Ser Ile Tyr Met Phe Phe Asn Thr Ser Glu Leu Arg Glu Ala Val
        130                 135             140

Pro Glu Pro Val Leu Leu Ser Arg Ala Glu Leu Arg Leu Leu Arg Leu
145                 150                 155                 160

Lys Leu Lys Val Glu Gln His Val Glu Leu Tyr Gln Lys Tyr Ser Asn
                165                 170                 175

Asn Ser Trp Arg Tyr Leu Ser Asn Arg Leu Leu Ala Pro Ser Asp Ser
            180                 185                 190

Pro Glu Trp Leu Ser Phe Asp Val Thr Gly Val Val Arg Gln Trp Leu
            195                 200                 205

Ser Arg Gly Gly Glu Ile Glu Gly Phe Arg Leu Ser Ala His Cys Ser
    210                 215                 220

Cys Asp Ser Arg Asp Asn Thr Leu Gln Val Asp Ile Asn Gly Phe Thr
225                 230                 235                 240

Thr Gly Arg Arg Gly Asp Leu Ala Thr Ile His Gly Met Asn Arg Pro
            245                 250                 255

Phe Leu Leu Leu Met Ala Thr Pro Leu Glu Arg Ala Gln His Leu Gln
            260                 265                 270

Ser Ser Arg His Arg Arg Ala Leu Asp Thr Asn Tyr Cys Phe Ser Ser
    275                 280                 285

Thr Glu Lys Asn Cys Cys Val Arg Gln Leu Tyr Ile Asp Phe Arg Lys
    290                 295                 300

Asp Leu Gly Trp Lys Trp Ile His Glu Pro Lys Gly Tyr His Ala Asn
305                 310                 315                 320

Phe Cys Leu Gly Pro Cys Pro Tyr Ile Trp Ser Leu Asp Thr Gln Tyr
            325                 330                 335

Ser Lys Val Leu Ala Leu Tyr Asn Gln His Asn Pro Gly Ala Ser Ala
            340                 345                 350

Ala Pro Cys Cys Val Pro Gln Ala Leu Glu Pro Leu Pro Ile Val Tyr
```

355                     360                     365

Tyr Val Gly Arg Lys Pro Lys Val Glu Gln Leu Ser Asn Met Ile Val
    370                 375             380

Arg Ser Cys Lys Cys Ser
385                 390

<210> 7
<211> 431
<212> PRT
<213> Homo sapiens

<400> 7
Met His Val Arg Ser Leu Arg Ala Ala Ala Pro His Ser Phe Val Ala
1               5               10              15

Leu Trp Ala Pro Leu Phe Leu Leu Arg Ser Ala Leu Ala Asp Phe Ser
        20              25              30

Leu Asp Asn Glu Val His Ser Ser Phe Ile His Arg Arg Leu Arg Ser
    35              40              45

Gln Glu Arg Arg Glu Met Gln Arg Glu Ile Leu Ser Ile Leu Gly Leu
    50              55              60

Pro His Arg Pro Arg Pro His Leu Gln Gly Lys His Asn Ser Ala Pro
65              70              75              80

Met Phe Met Leu Asp Leu Tyr Asn Ala Met Ala Val Glu Glu Gly Gly
            85              90              95

Gly Pro Gly Gly Gln Gly Phe Ser Tyr Pro Tyr Lys Ala Val Phe Ser
        100             105             110

Thr Gln Gly Pro Pro Leu Ala Ser Leu Gln Asp Ser His Phe Leu Thr
    115             120             125

Asp Ala Asp Met Val Met Ser Phe Val Asn Leu Val Glu His Asp Lys
    130             135             140

Glu Phe Phe His Pro Arg Tyr His His Arg Glu Phe Arg Phe Asp Leu
145             150             155             160

Ser Lys Ile Pro Glu Gly Glu Ala Val Thr Ala Ala Glu Phe Arg Ile
            165             170             175

Tyr Lys Asp Tyr Ile Arg Glu Arg Phe Asp Asn Glu Thr Phe Arg Ile
        180             185             190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
        210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
                260                 265                 270

Lys Gln Pro Phe Met Val Ala Phe Phe Lys Ala Thr Glu Val His Phe
                275                 280                 285

Arg Ser Ile Arg Ser Thr Gly Ser Lys Gln Arg Ser Gln Asn Arg Ser
        290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
                340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
        370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
                420                 425                 430

<210> 8

<211> 314
<212> PRT
<213> Homo sapiens

<400> 8

```
Met Arg Ile Ala Val Ile Cys Phe Cys Leu Leu Gly Ile Thr Cys Ala
1               5                   10                  15

Ile Pro Val Lys Gln Ala Asp Ser Gly Ser Ser Glu Glu Lys Gln Leu
                20                  25                  30

Tyr Asn Lys Tyr Pro Asp Ala Val Ala Thr Trp Leu Asn Pro Asp Pro
            35                  40                  45

Ser Gln Lys Gln Asn Leu Leu Ala Pro Gln Asn Ala Val Ser Ser Glu
        50                  55                  60

Glu Thr Asn Asp Phe Lys Gln Glu Thr Leu Pro Ser Lys Ser Asn Glu
65                  70                  75                  80

Ser His Asp His Met Asp Asp Met Asp Glu Asp Asp Asp Asp His
                85                  90                  95

Val Asp Ser Gln Asp Ser Ile Asp Ser Asn Asp Ser Asp Asp Val Asp
            100                 105                 110

Asp Thr Asp Asp Ser His Gln Ser Asp Glu Ser His His Ser Asp Glu
        115                 120                 125

Ser Asp Glu Leu Val Thr Asp Phe Pro Thr Asp Leu Pro Ala Thr Glu
        130                 135                 140

Val Phe Thr Pro Val Val Pro Thr Val Asp Thr Tyr Asp Gly Arg Gly
145                 150                 155                 160

Asp Ser Val Val Tyr Gly Leu Arg Ser Lys Ser Lys Lys Phe Arg Arg
            165                 170                 175

Pro Asp Ile Gln Tyr Pro Asp Ala Thr Asp Glu Asp Ile Thr Ser His
            180                 185                 190

Met Glu Ser Glu Glu Leu Asn Gly Ala Tyr Lys Ala Ile Pro Val Ala
        195                 200                 205

Gln Asp Leu Asn Ala Pro Ser Asp Trp Asp Ser Arg Gly Lys Asp Ser
        210                 215                 220

Tyr Glu Thr Ser Gln Leu Asp Asp Gln Ser Ala Glu Thr His Ser His
225                 230                 235                 240
```

Lys Gln Ser Arg Leu Tyr Lys Arg Lys Ala Asn Asp Glu Ser Asn Glu
                245                 250                 255

His Ser Asp Val Ile Asp Ser Gln Glu Leu Ser Lys Val Ser Arg Glu
                260                 265                 270

Phe His Ser His Glu Phe His Ser His Glu Asp Met Leu Val Val Asp
                275                 280                 285

Pro Lys Ser Lys Glu Glu Asp Lys His Leu Lys Phe Arg Ile Ser His
    290                 295                 300

Glu Leu Asp Ser Ala Ser Ser Glu Val Asn
305                 310


<210> 9
<211> 107
<212> PRT
<213> Homo sapiens

<400> 9
Met Ala Arg Ala Ala Leu Ser Ala Ala Pro Ser Asn Pro Arg Leu Leu
1                   5                   10                  15

Arg Val Ala Leu Leu Leu Leu Leu Leu Val Ala Ala Gly Arg Arg Ala
                20                  25                  30

Ala Gly Ala Ser Val Ala Thr Glu Leu Arg Cys Gln Cys Leu Gln Thr
                35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile
                85                  90                  95

Ile Glu Lys Met Leu Asn Ser Asp Lys Ser Asn
                100                 105


<210> 10
<211> 604
<212> PRT
<213> Homo sapiens

<400> 10
Met Met Arg Ala Val Trp Glu Ala Leu Ala Ala Leu Ala Ala Val Ala

```
                1                    5                          10                            15


        Cys Leu Val Gly Ala Val Arg Gly Gly Pro Gly Leu Ser Met Phe Ala
                    20                  25                  30


        Gly Gln Ala Ala Gln Pro Asp Pro Cys Ser Asp Glu Asn Gly His Pro
                    35                  40                  45


        Arg Arg Cys Ile Pro Asp Phe Val Asn Ala Ala Phe Gly Lys Asp Val
                    50                  55                  60


        Arg Val Ser Ser Thr Cys Gly Arg Pro Pro Ala Arg Tyr Cys Val Val
        65                  70                  75                  80


        Ser Glu Arg Gly Glu Glu Arg Leu Arg Ser Cys His Leu Cys Asn Ala
                            85                  90                  95


        Ser Asp Pro Lys Lys Ala His Pro Pro Ala Phe Leu Thr Asp Leu Asn
                    100                 105                 110


        Asn Pro His Asn Leu Thr Cys Trp Gln Ser Glu Asn Tyr Leu Gln Phe
                    115                 120                 125


        Pro His Asn Val Thr Leu Thr Leu Ser Leu Gly Lys Lys Phe Glu Val
                    130                 135                 140


        Thr Tyr Val Ser Leu Gln Phe Cys Ser Pro Arg Pro Glu Ser Met Ala
        145                 150                 155                 160


        Ile Tyr Lys Ser Met Asp Tyr Gly Arg Thr Trp Val Pro Phe Gln Phe
                        165                 170                 175


        Tyr Ser Thr Gln Cys Arg Lys Met Tyr Asn Arg Pro His Arg Ala Pro
                    180                 185                 190


        Ile Thr Lys Gln Asn Glu Gln Glu Ala Val Cys Thr Asp Ser His Thr
                    195                 200                 205


        Asp Met Arg Pro Leu Ser Gly Gly Leu Ile Ala Phe Ser Thr Leu Asp
                210                 215                 220


        Gly Arg Pro Ser Ala His Asp Phe Asp Asn Ser Pro Val Leu Gln Asp
        225                 230                 235                 240


        Trp Val Thr Ala Thr Asp Ile Arg Val Ala Phe Ser Arg Leu His Thr
                        245                 250                 255
```

```
Phe Gly Asp Glu Asn Glu Asp Asp Ser Glu Leu Ala Arg Asp Ser Tyr
            260             265             270

Phe Tyr Ala Val Ser Asp Leu Gln Val Gly Gly Arg Cys Lys Cys Asn
        275             280             285

Gly His Ala Ala Arg Cys Val Arg Asp Arg Thr Asp Ser Leu Val Cys
        290             295             300

Asp Cys Arg His Asn Thr Ala Gly Pro Glu Cys Asp Arg Cys Lys Pro
305             310             315             320

Phe His Tyr Asp Arg Pro Trp Gln Arg Ala Thr Ala Arg Glu Ala Asn
            325             330             335

Glu Cys Val Ala Cys Asn Cys Asn Leu His Ala Arg Arg Cys Arg Phe
        340             345             350

Asn Met Glu Leu Tyr Lys Leu Ser Gly Arg Lys Ser Gly Gly Val Cys
        355             360             365

Leu Asn Cys Arg His Asn Thr Ala Gly Arg His Cys His Tyr Cys Lys
        370             375             380

Glu Gly Tyr Tyr Arg Asp Met Gly Lys Pro Ile Thr His Arg Lys Ala
385             390             395             400

Cys Lys Ala Cys Asp Cys His Pro Val Gly Ala Ala Gly Lys Thr Cys
            405             410             415

Asn Gln Thr Thr Gly Gln Cys Pro Cys Lys Asp Gly Val Thr Gly Ile
            420             425             430

Thr Cys Asn Arg Cys Ala Lys Gly Tyr Gln Gln Ser Arg Ser Pro Ile
        435             440             445

Ala Pro Cys Ile Lys Ile Pro Val Ala Pro Pro Thr Thr Ala Ala Ser
    450             455             460

Ser Val Glu Glu Pro Glu Asp Cys Asp Ser Tyr Cys Lys Ala Ser Lys
465             470             475             480

Gly Lys Leu Lys Ile Asn Met Lys Lys Tyr Cys Lys Lys Asp Tyr Ala
            485             490             495

Val Gln Ile His Ile Leu Lys Ala Asp Lys Ala Gly Asp Trp Trp Lys
        500             505             510
```

```
Phe Thr Val Asn Ile Ile Ser Val Tyr Lys Gln Gly Thr Ser Arg Ile
        515                 520                 525


Arg Arg Gly Asp Gln Ser Leu Trp Ile Arg Ser Arg Asp Ile Ala Cys
        530                 535                 540


Lys Cys Pro Lys Ile Lys Pro Leu Lys Lys Tyr Leu Leu Leu Gly Asn
545                 550                 555                 560


Ala Glu Asp Ser Pro Asp Gln Ser Gly Ile Val Ala Asp Lys Ser Ser
                565                 570                 575


Leu Val Ile Gln Trp Arg Asp Thr Trp Ala Arg Arg Leu Arg Lys Phe
                580                 585                 590


Gln Gln Arg Glu Lys Lys Gly Lys Cys Lys Lys Ala
        595                 600
```

**Description**

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830,. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or bums), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulo interstitial nephritis | Drug reaction (eg, β-lactams, NSAIDs, sulfonamides, ciprofloxacin, thiazide diuretics, furosemide, phenytoin, allopurinol, pyelonephritis, papillary necrosis |

(continued)

| Intrinsic Renal | |
|---|---|
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.

More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4): 177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.
Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited. Knowing these things can be of vital importance in managing and treating patients with AKI.
These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein (collectively referred to herein as "kidney injury markers, and individually as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

These kidney injury markers may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.);* for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more kidney injury markers of the present invention in a body fluid sample obtained from the subject. The assay result(s), for example a measured concentration of one or more markers selected from the group consisting of Epidermal growth factor, Complement C3, Interleukin-4, Interleukin-1 alpha, Tubulo interstitial nephritis antigen, Transforming growth factor beta-1, Bone morphogenetic protein 7, Osteopontin, Netrin-1, and Growth-regulated alpha protein is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example a measured concentration of one or more markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example a measured concentration of one or more markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example a measured concentration of one or more markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.),* by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ec1 (electrochemical luminescence) labels, metal chelates, colloidal metal particles, *etc.*) as well as molecules that may be indirectly detected by production of a detectable reaction product (*e.g.*, enzymes such as horseradish peroxidase, alkaline phosphatase, *etc.*) or through the use of a specific binding molecule which itself may be detectable (*e.g.*, a labeled antibody that binds to the second antibody, biotin, digoxigenin, maltose, oligohistidine, 2,4-dintrobenzene, phenylarsenate, ssDNA, dsDNA, *etc.*).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.*, a diffraction grating, electrochemical sensor, etc) for generation of a signal, while in others, a signal is generated by a transducer that is spatially separate from the solid phase antibody (*e.g.*, a fluorometer that employs an excitation light source and an optical detector). This list is not meant to be limiting. Antibody-based biosensors may also be employed to determine the presence or amount of analytes that optionally eliminate the need for a labeled molecule.

BRIEF DESCRIPTION OF THE FIGURES

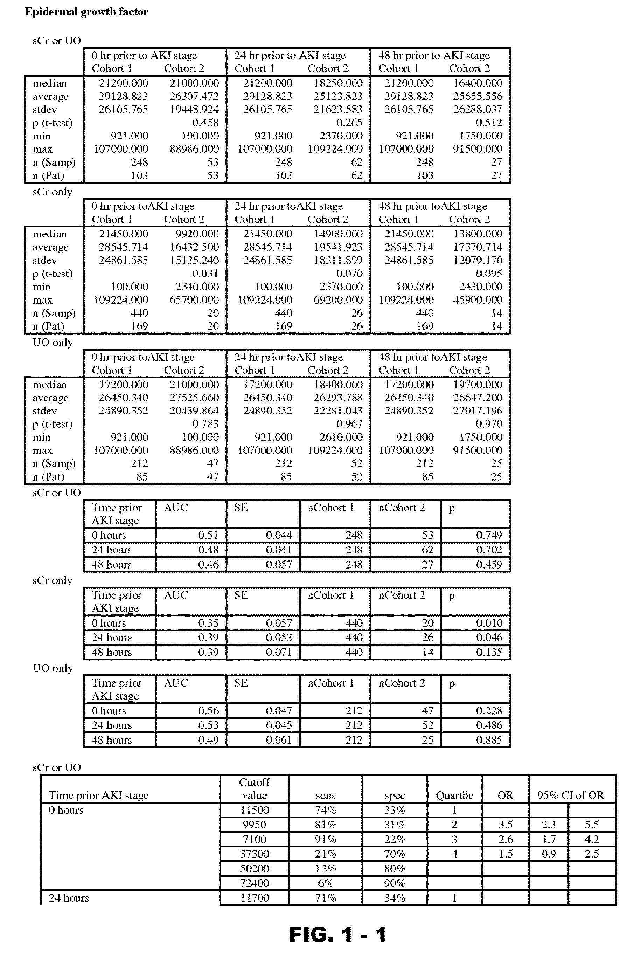

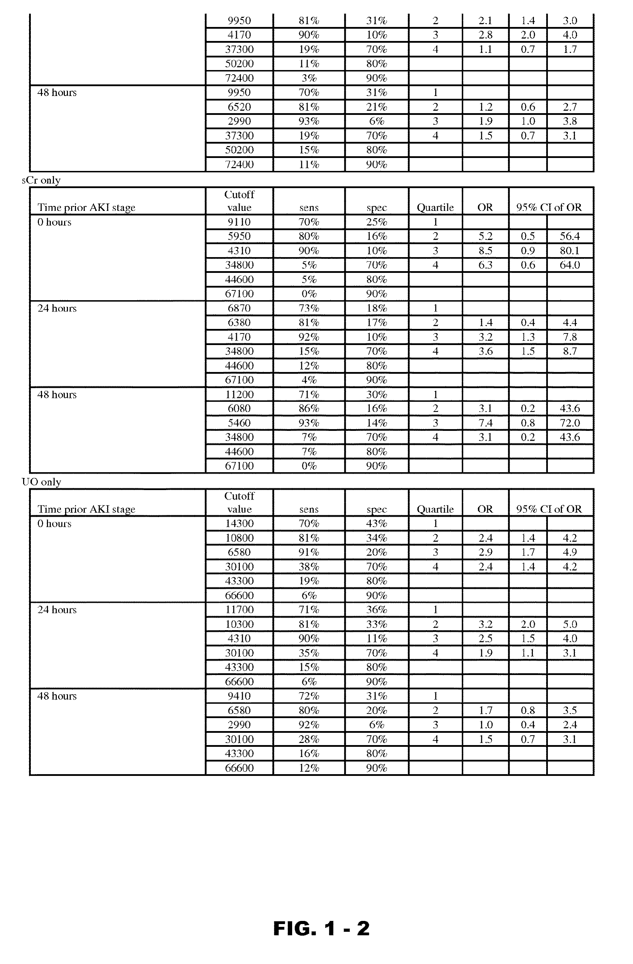

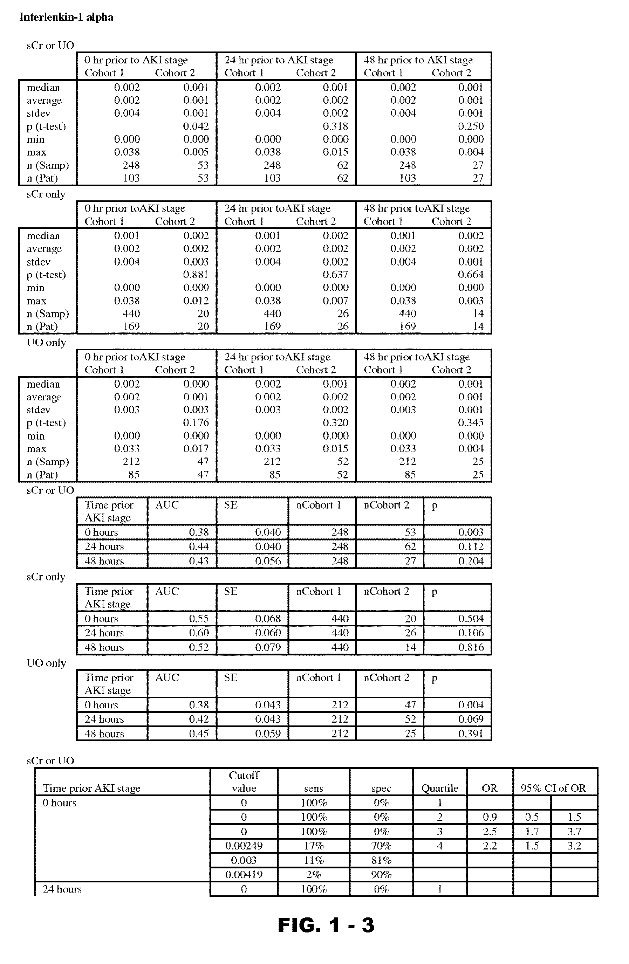

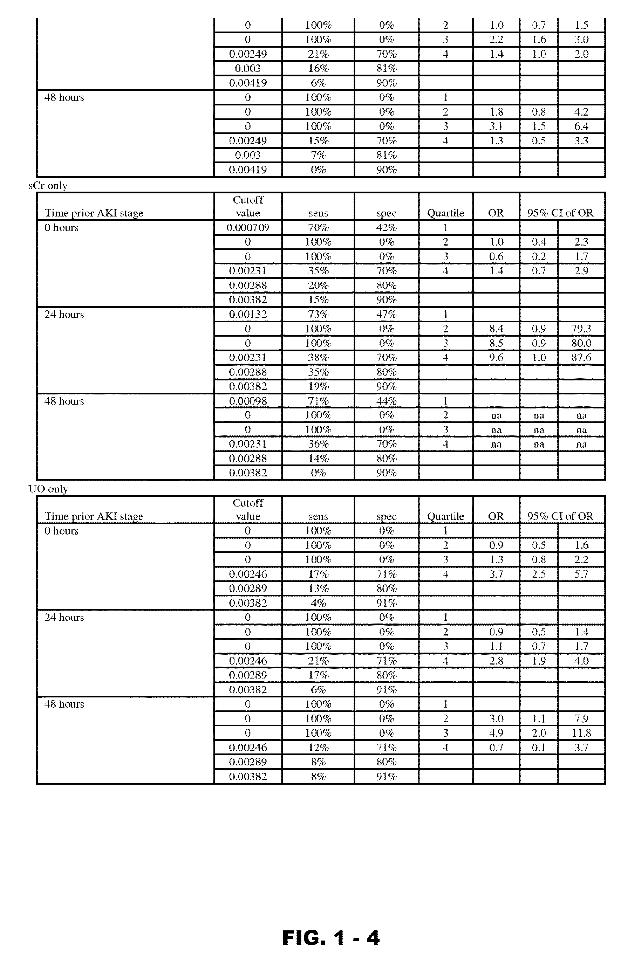

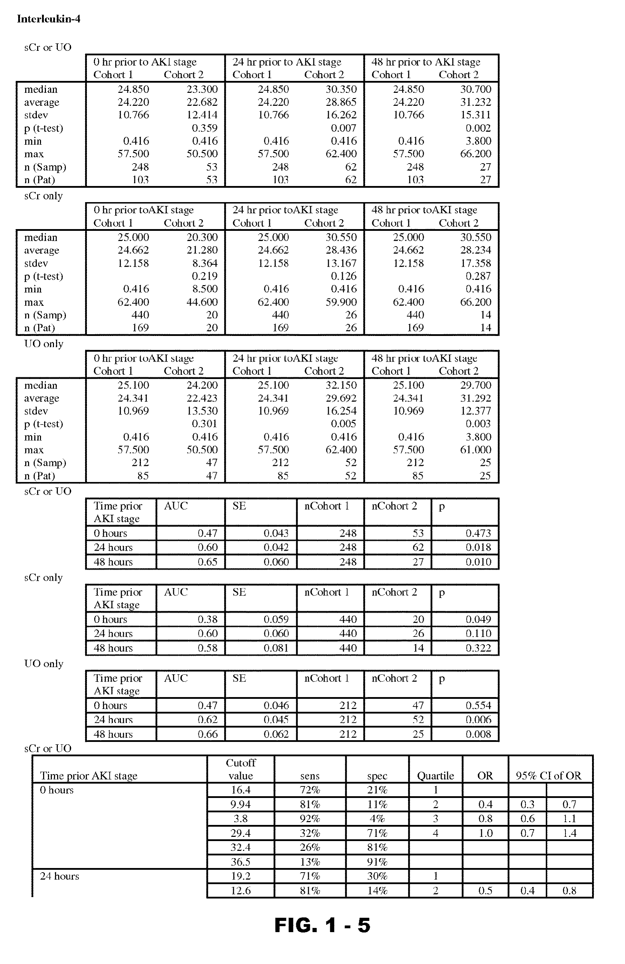

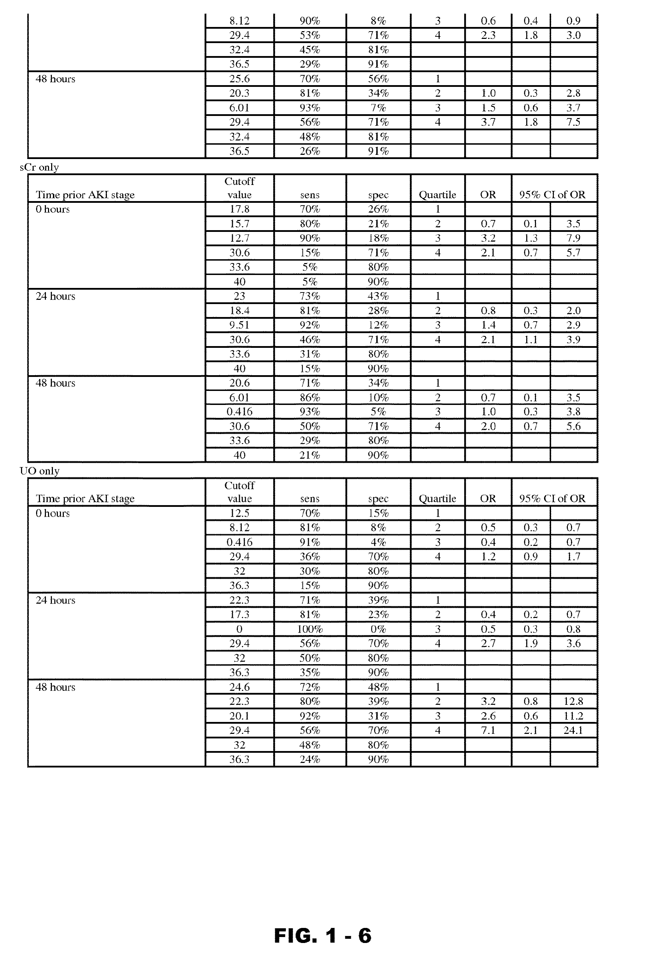

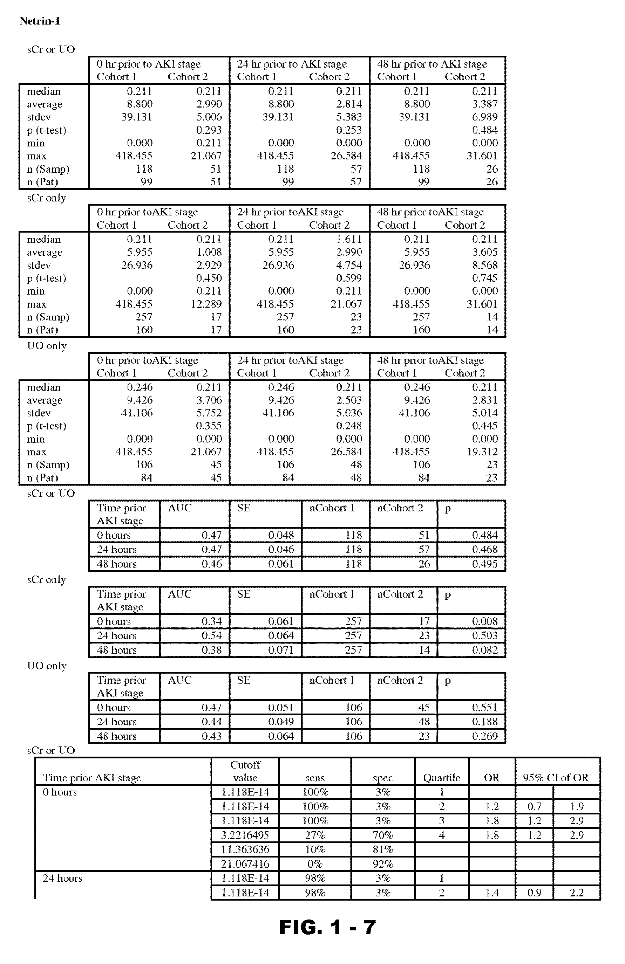

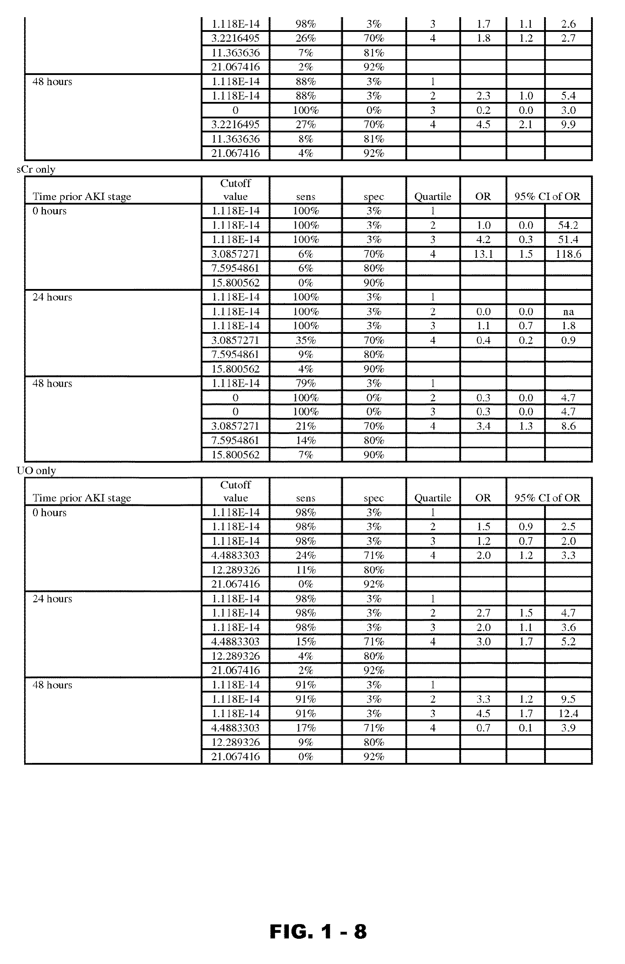

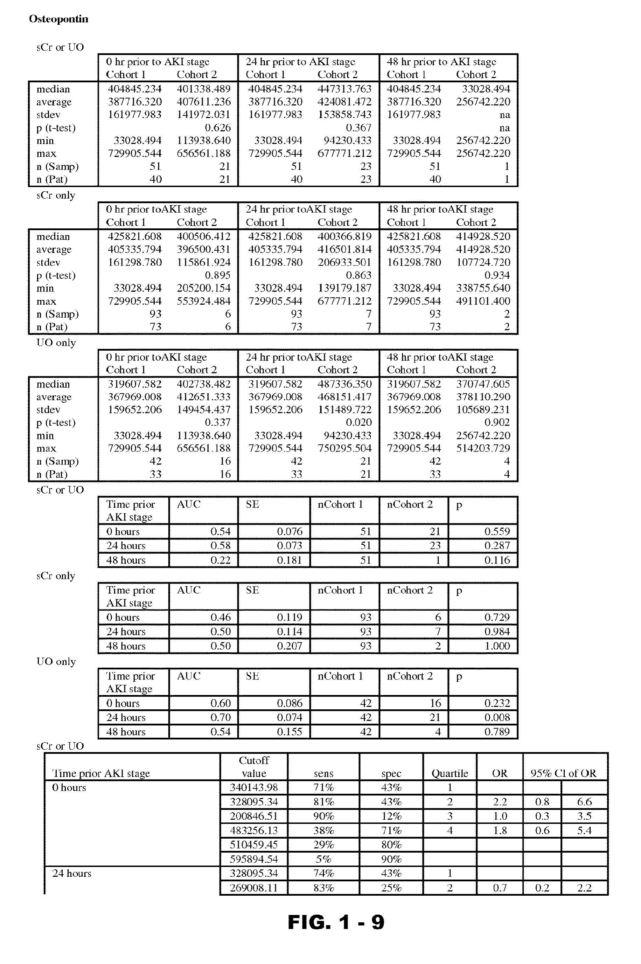

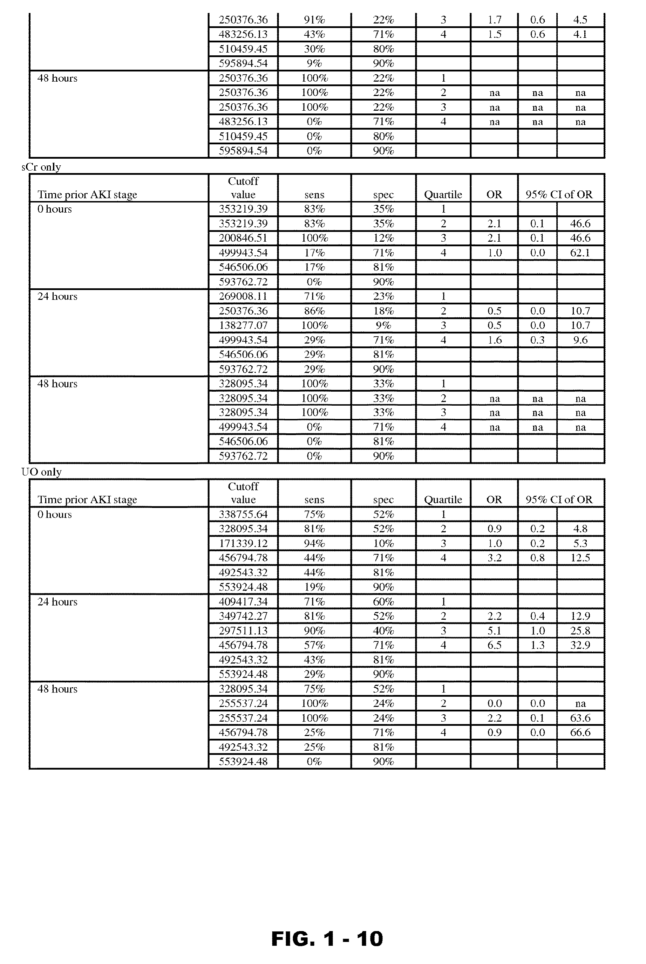

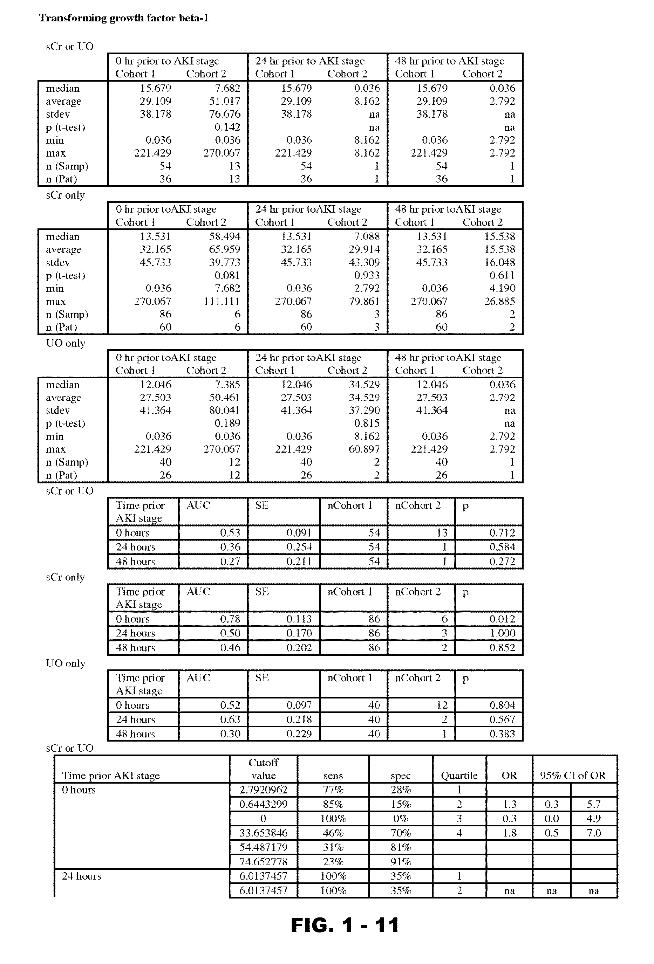

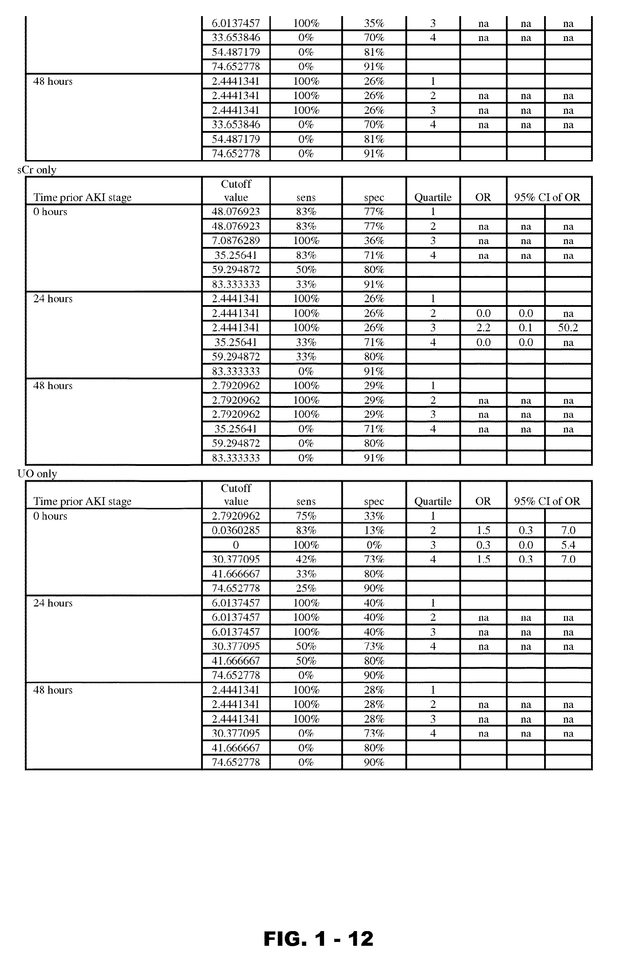

Fig. 1 provides data tables determined in accordance with Example 6 for the comparison of marker levels in urine samples collected for Cohort 1 (patients that did not progress beyond RIFLE stage 0) and in urine samples collected from subjects at 0, 24 hours, and 48 hours prior to reaching stage R, I or F in Cohort 2. Tables provide descriptive statistics, AUC analysis, and sensitivity, specificity and odds ratio calculations at various threshold (cutoff) levels for the various markers.

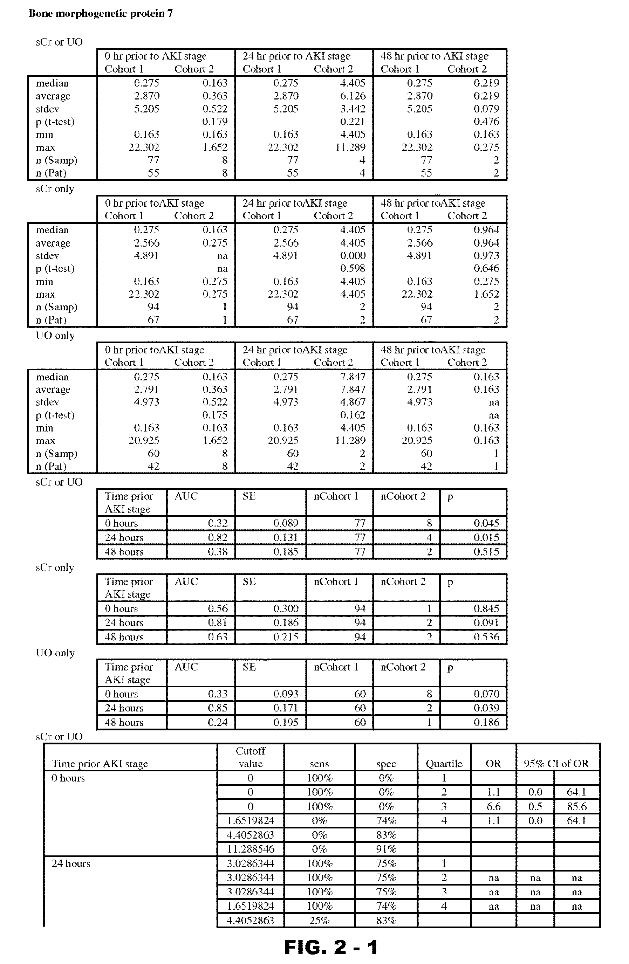

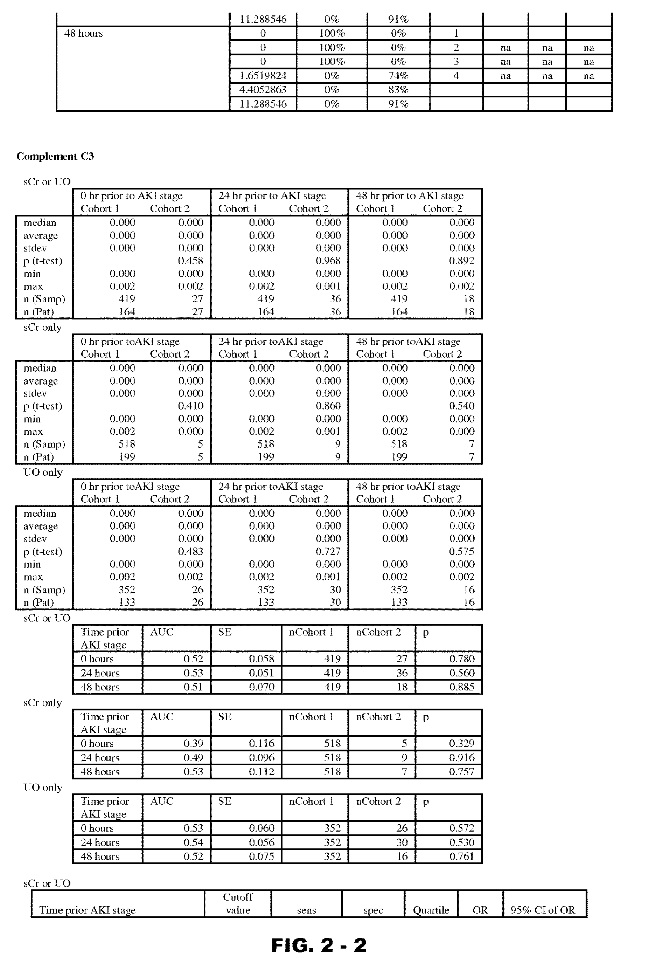

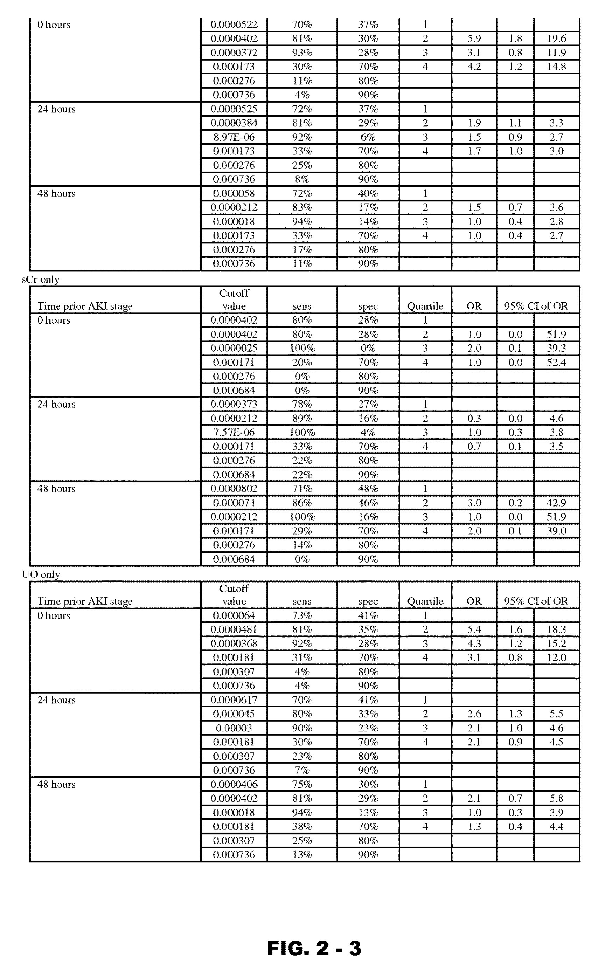

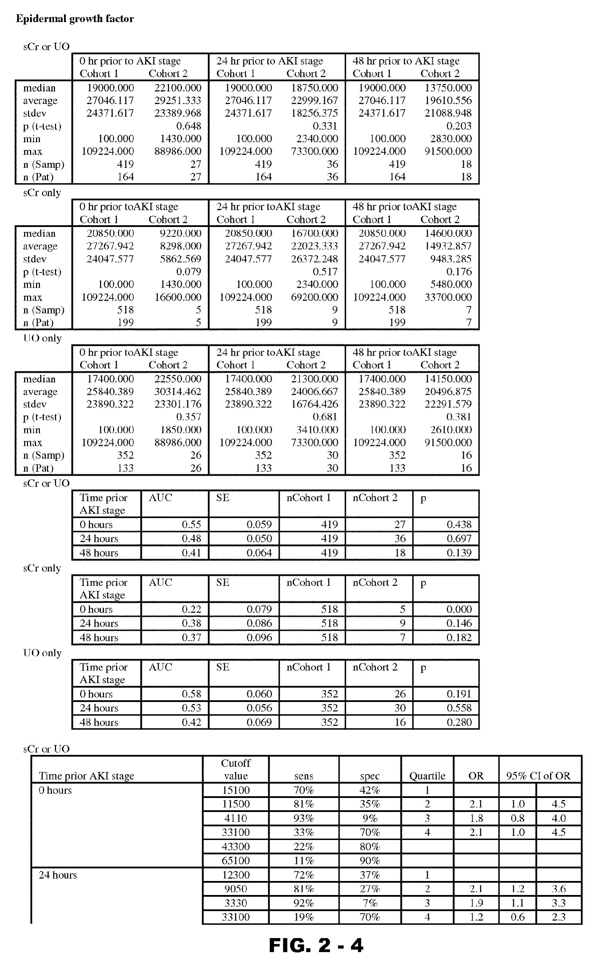

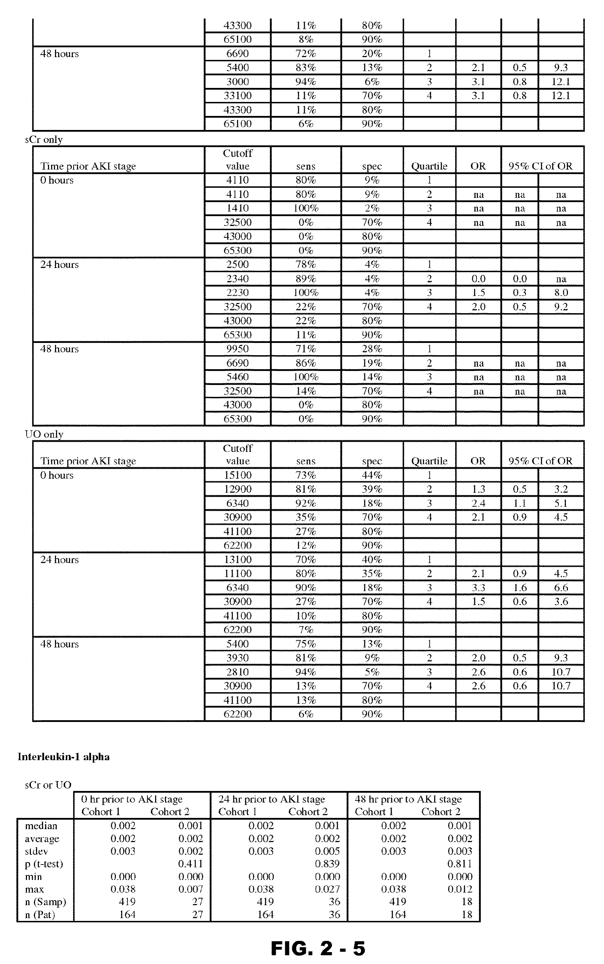

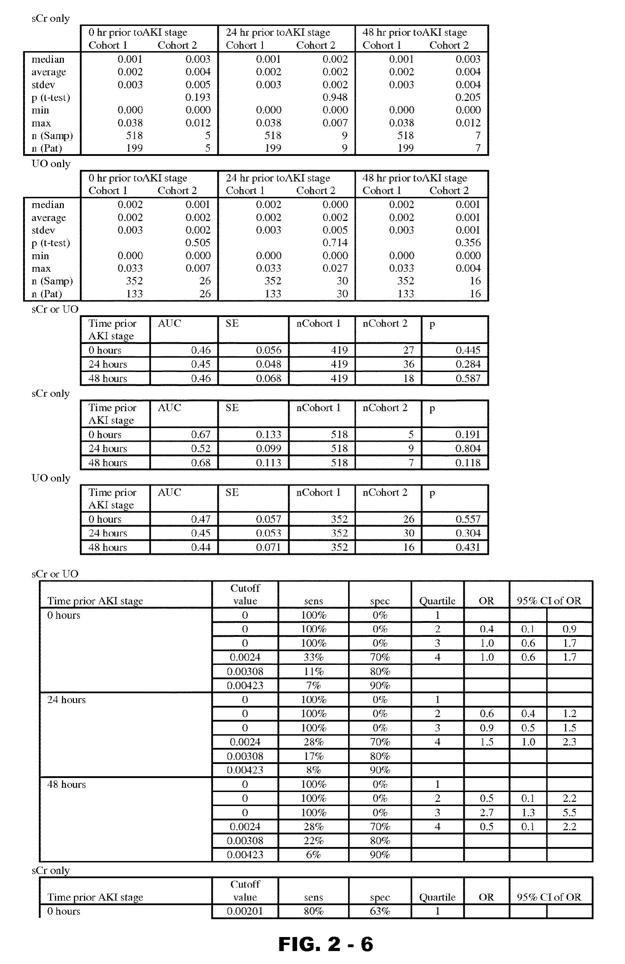

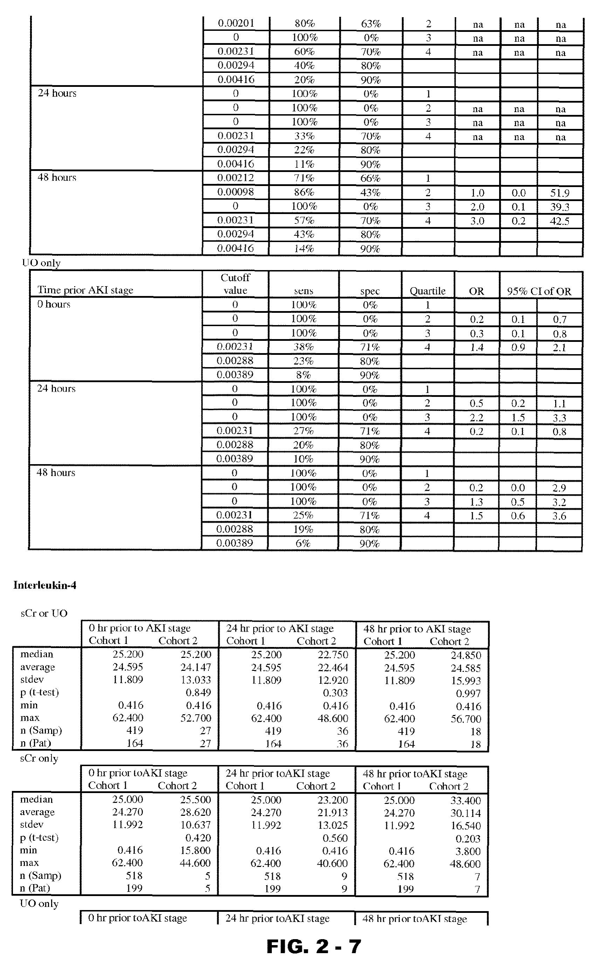

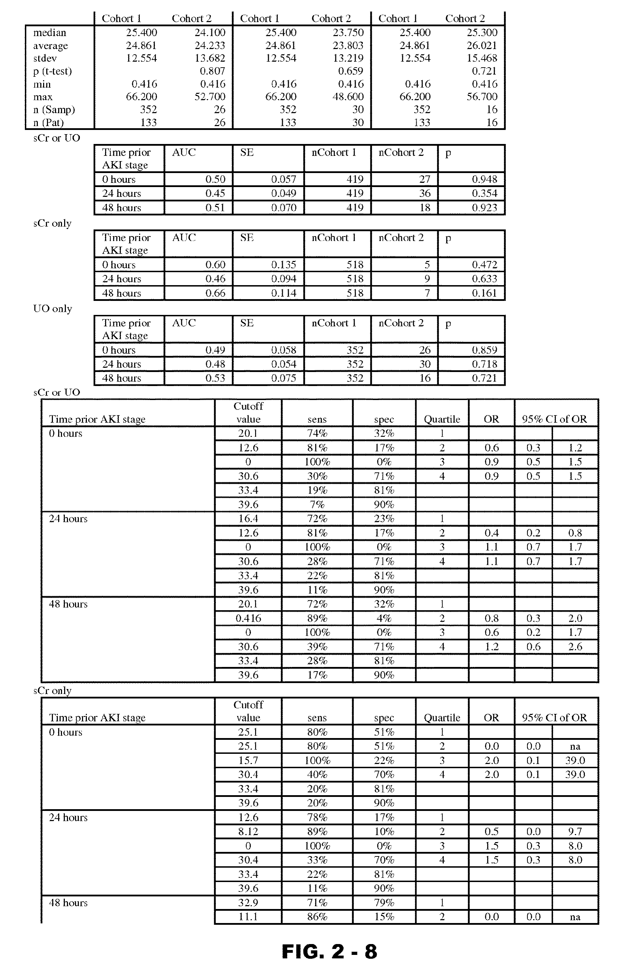

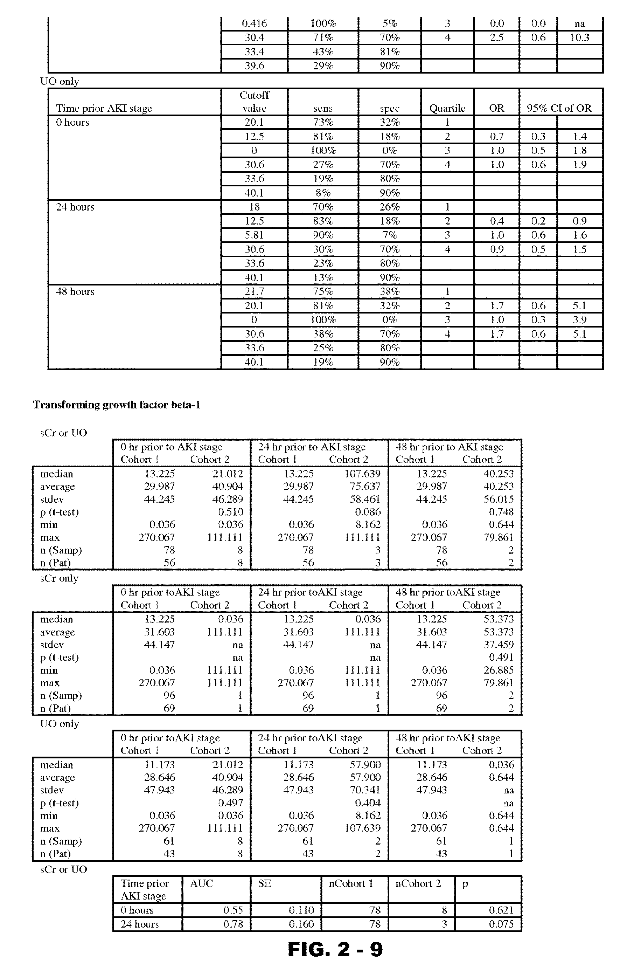

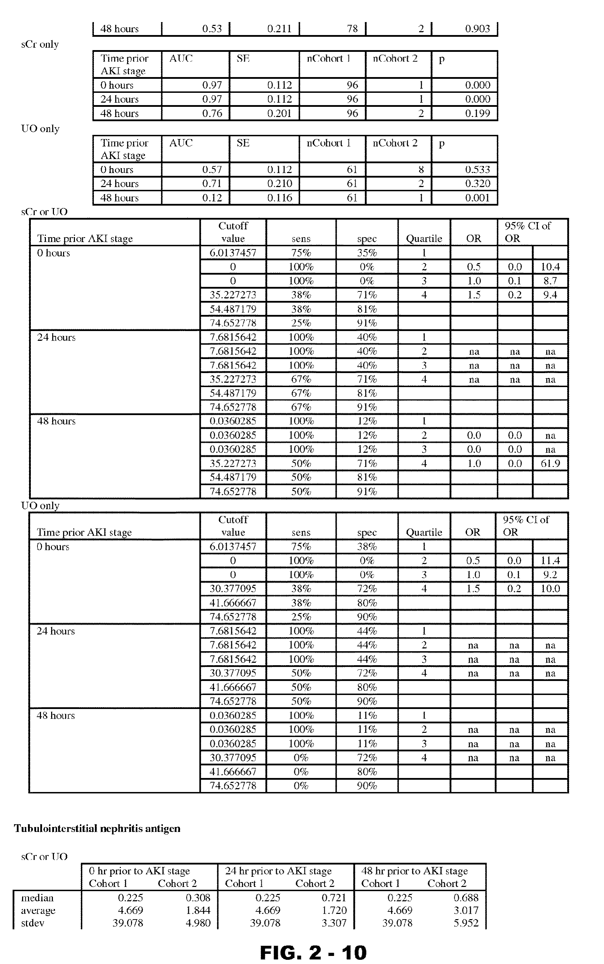

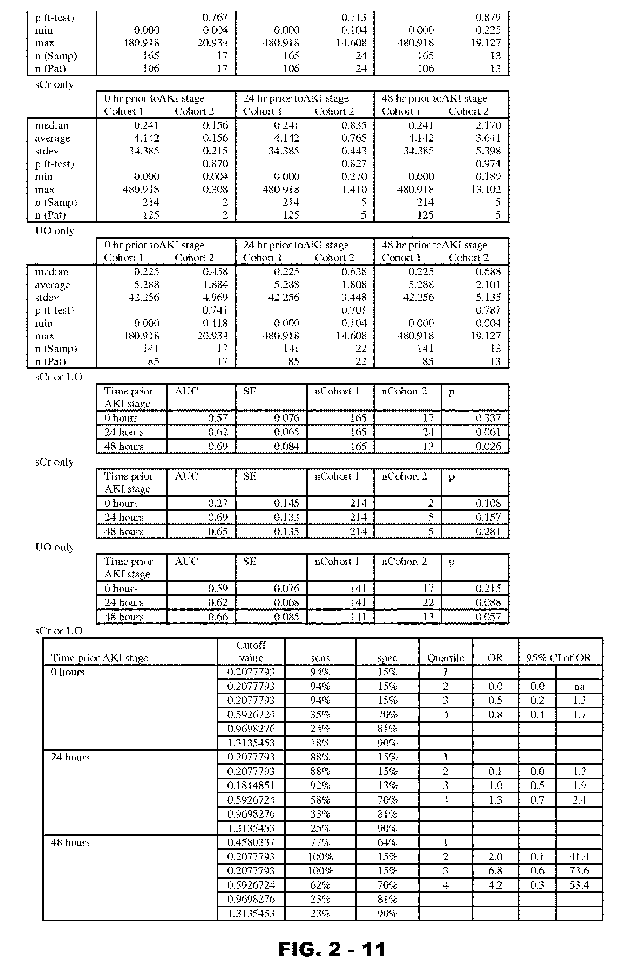

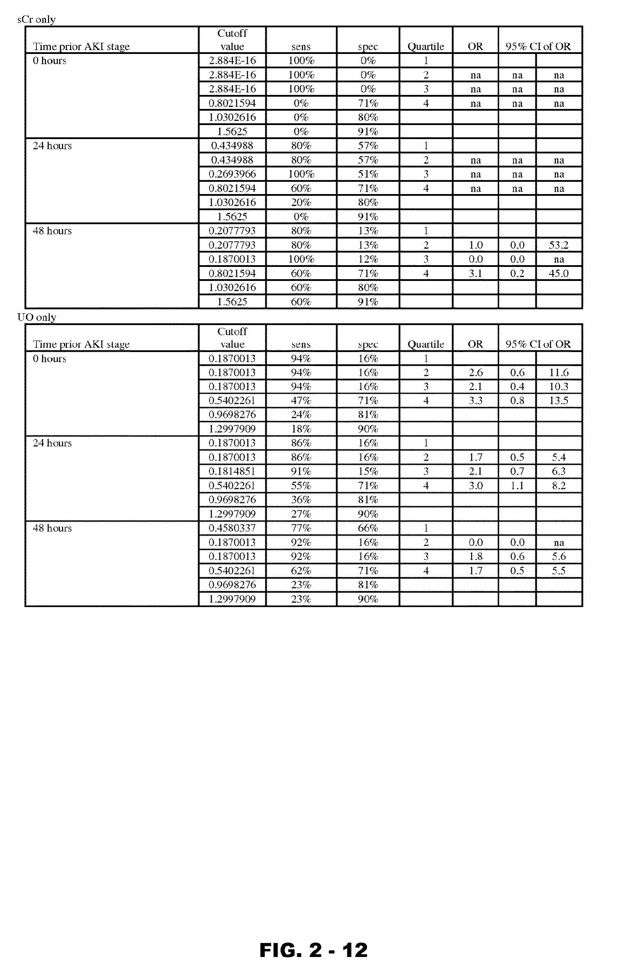

Fig. 2 provides data tables determined in accordance with Example 7 for the comparison of marker levels in urine samples collected for Cohort 1 (patients that did not progress beyond RIFLE stage 0 or R) and in urine samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

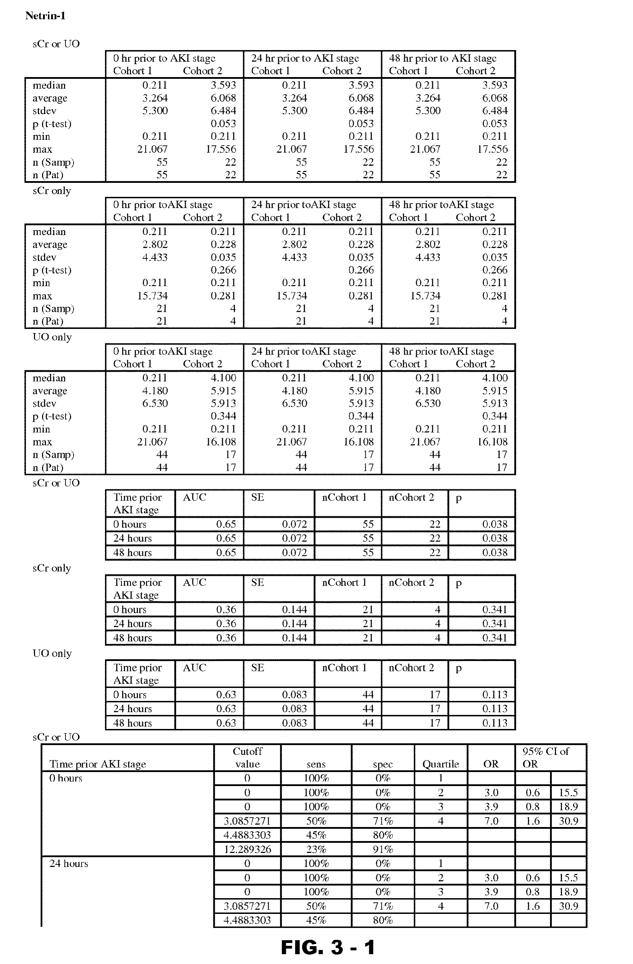

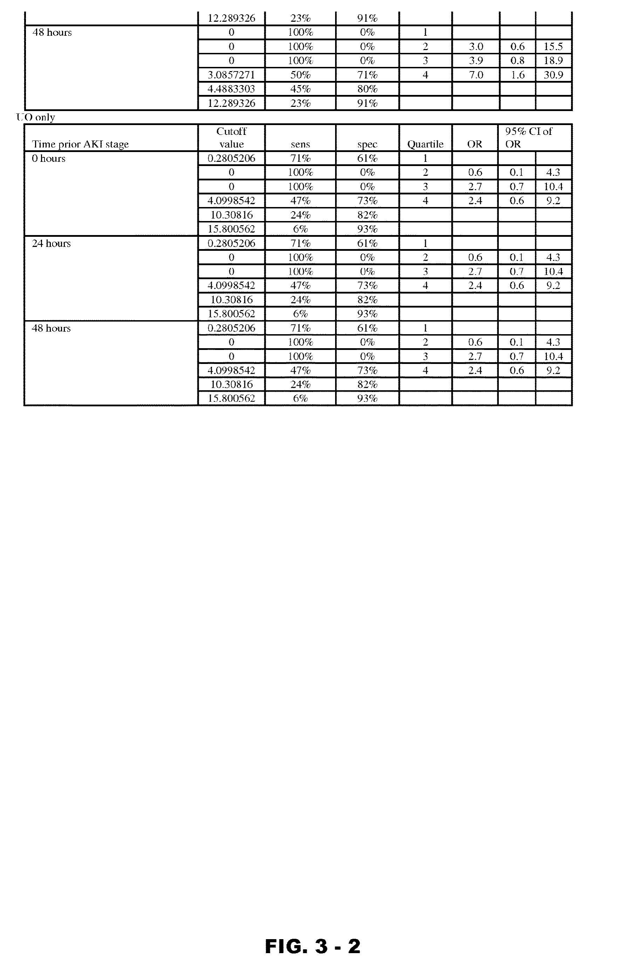

Fig. 3 provides data tables determined in accordance with Example 8 for the comparison of marker levels in urine samples collected for Cohort 1 (patients that reached, but did not progress beyond, RIFLE stage R) and in urine samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

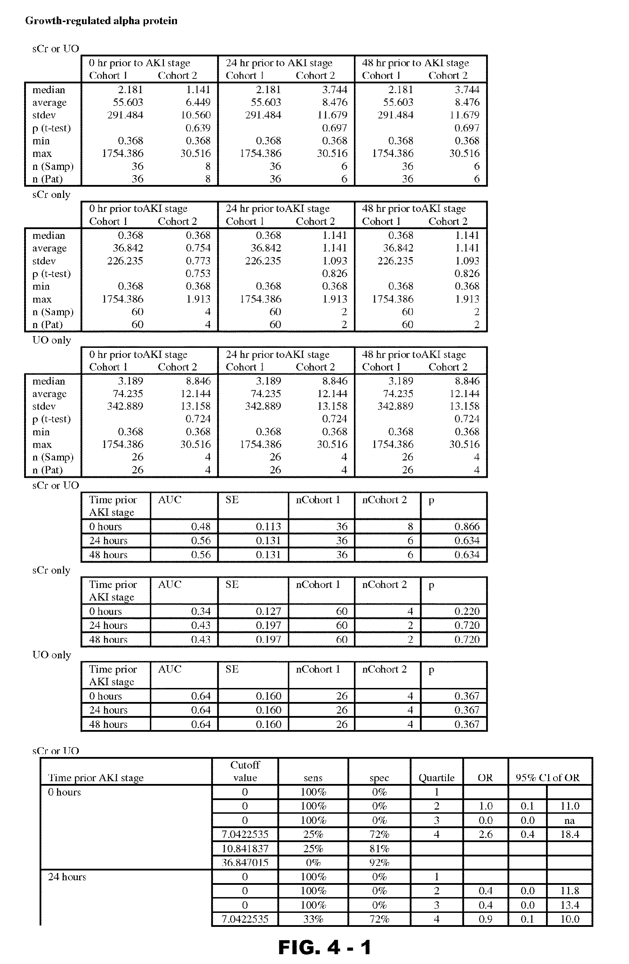

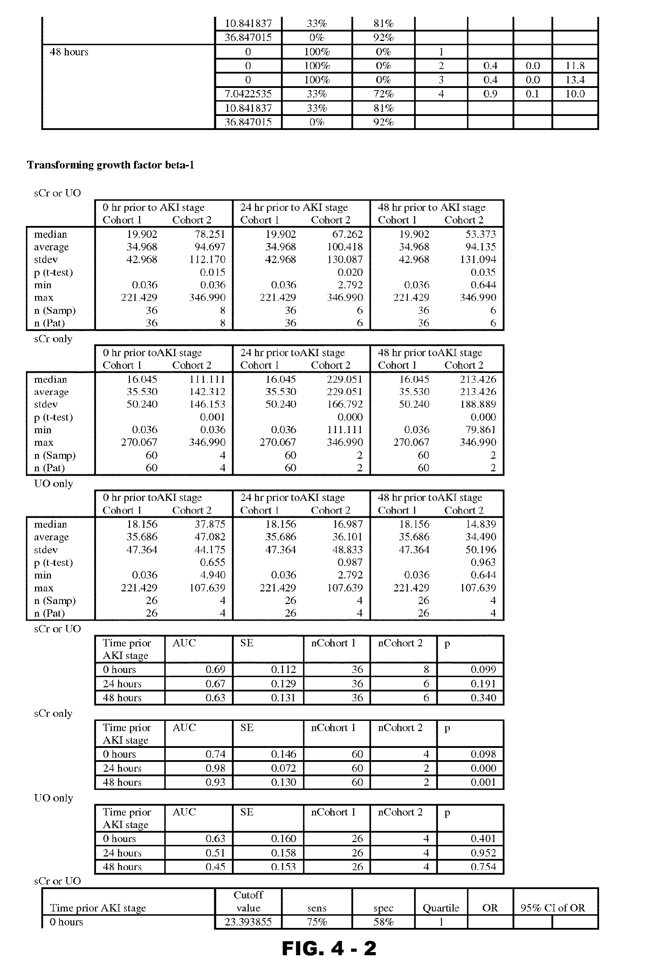

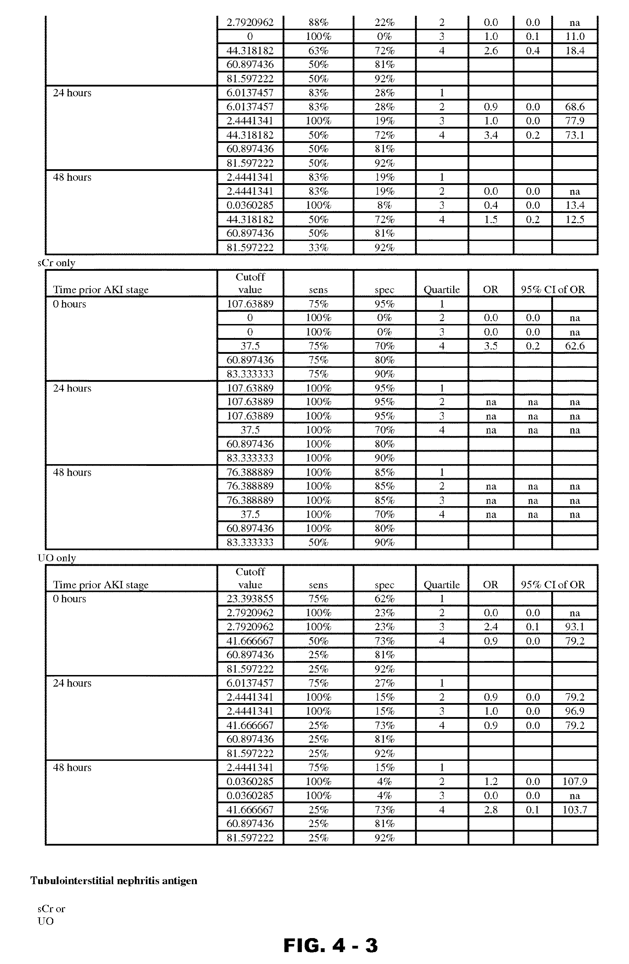

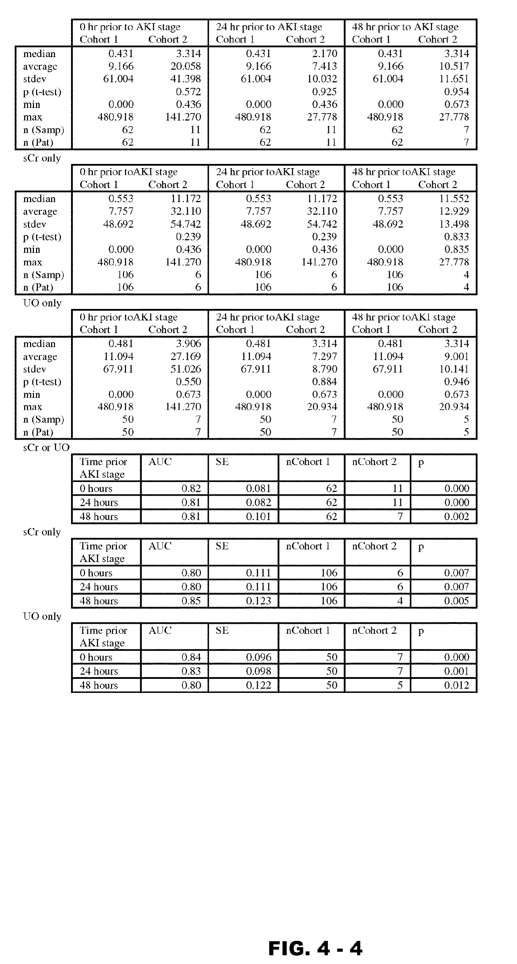

Fig. 4 provides data tables determined in accordance with Example 9 for the comparison of marker levels in urine samples collected for Cohort 1 (patients that did not progress beyond RIFLE stage 0) and in urine samples collected from subjects at 0, 24 hours, and 48 hours prior to reaching stage F in Cohort 2. Tables provide descriptive statistics, AUC analysis, and sensitivity, specificity and odds ratio calculations at various threshold (cutoff) levels for the various markers.

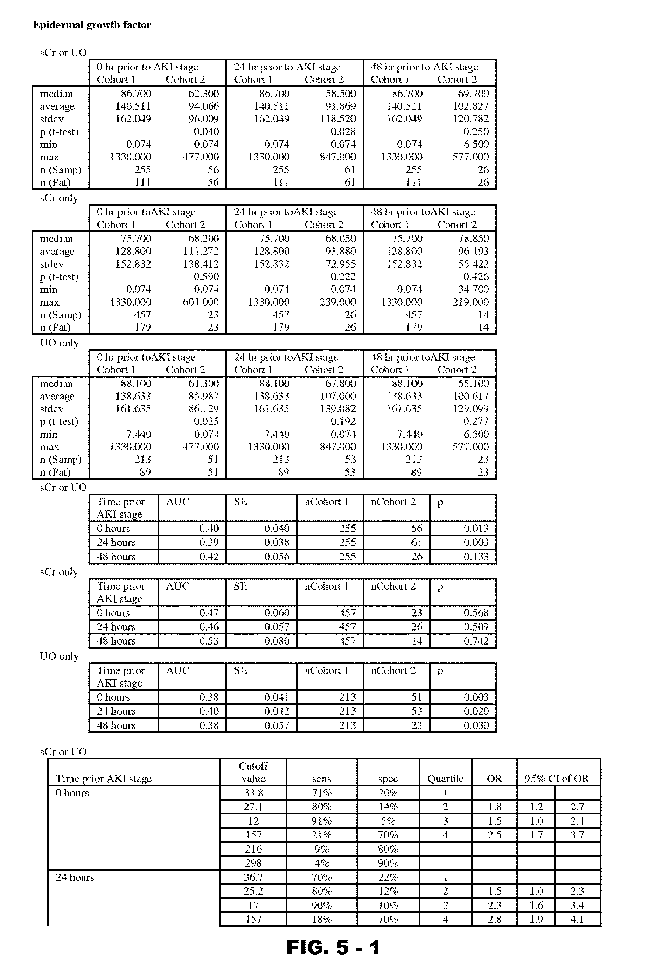

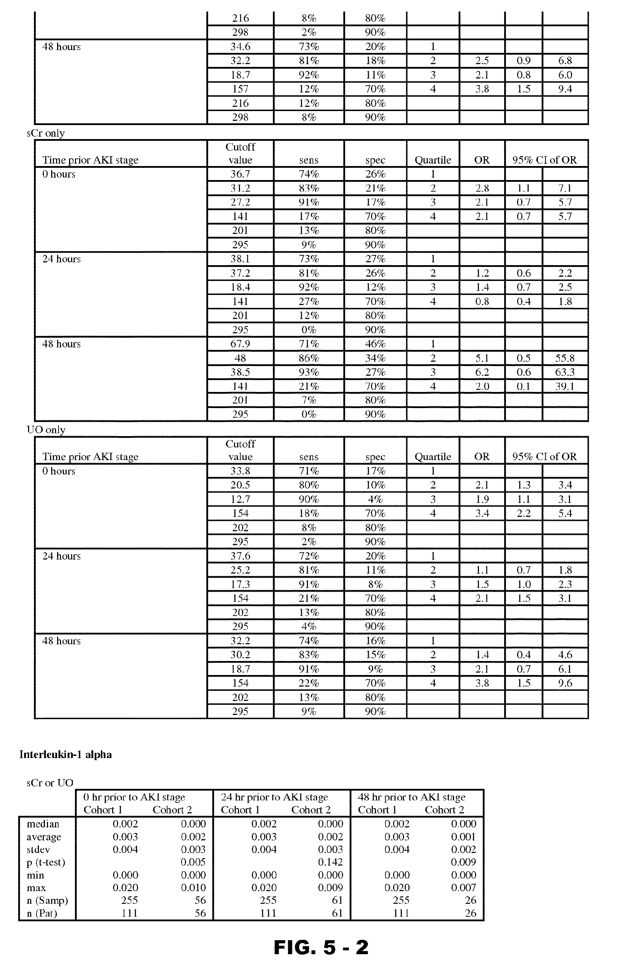

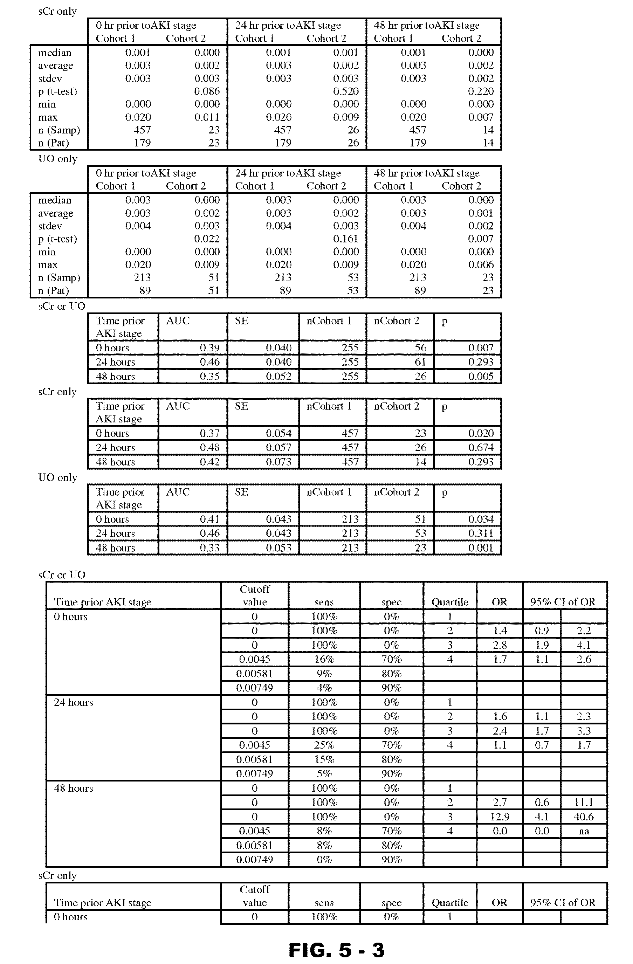

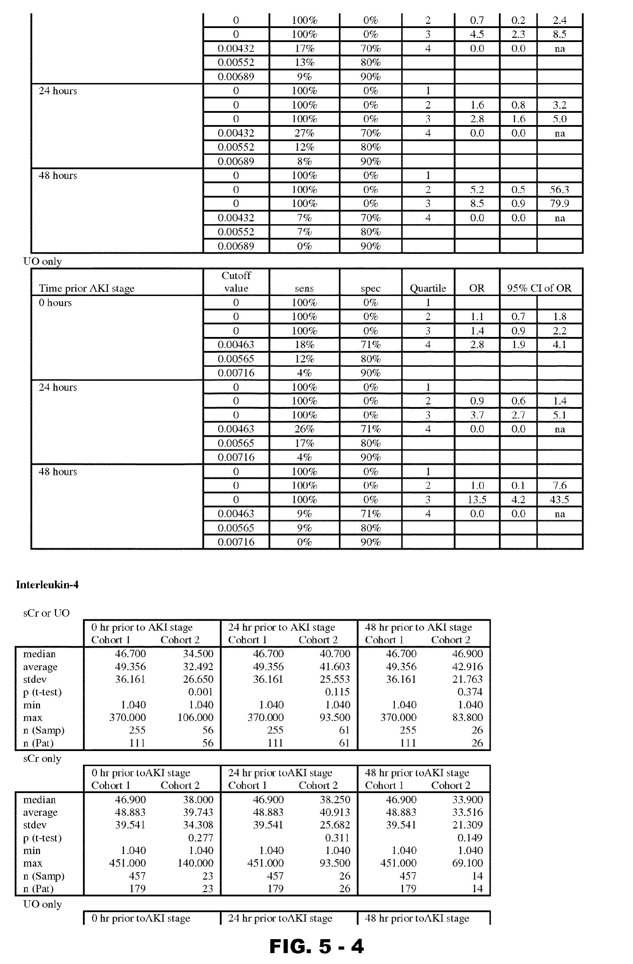

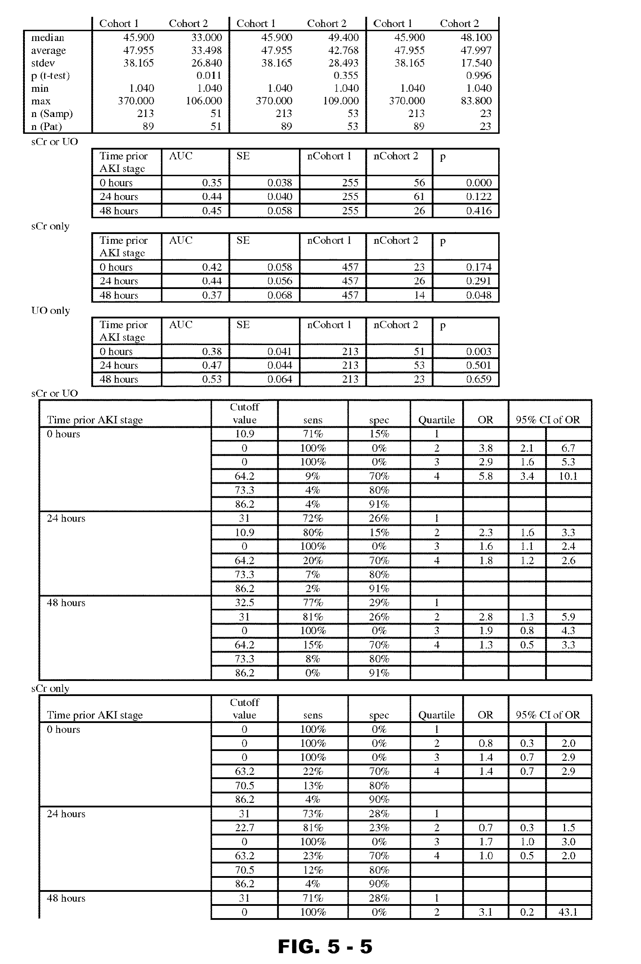

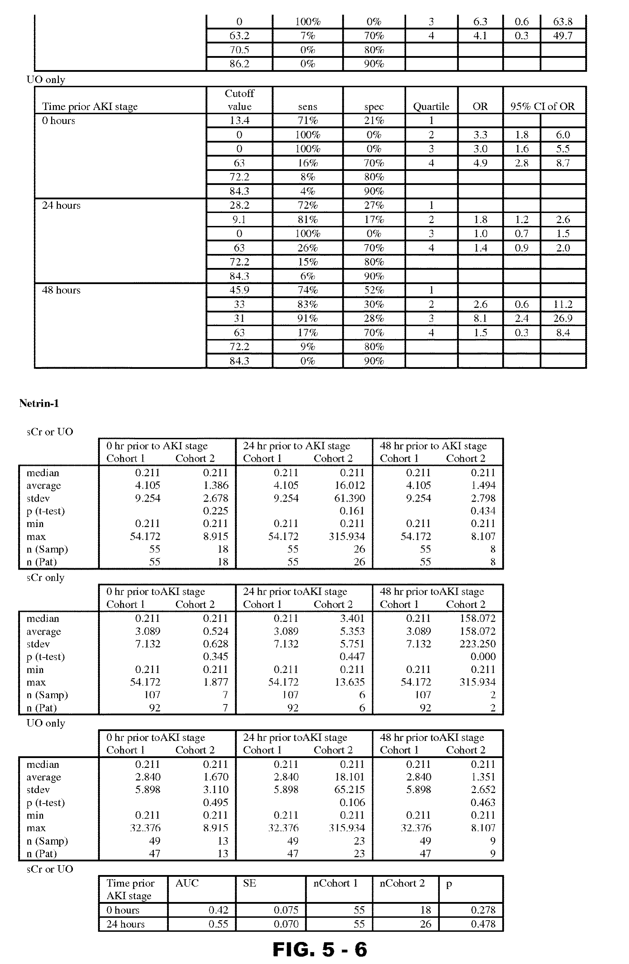

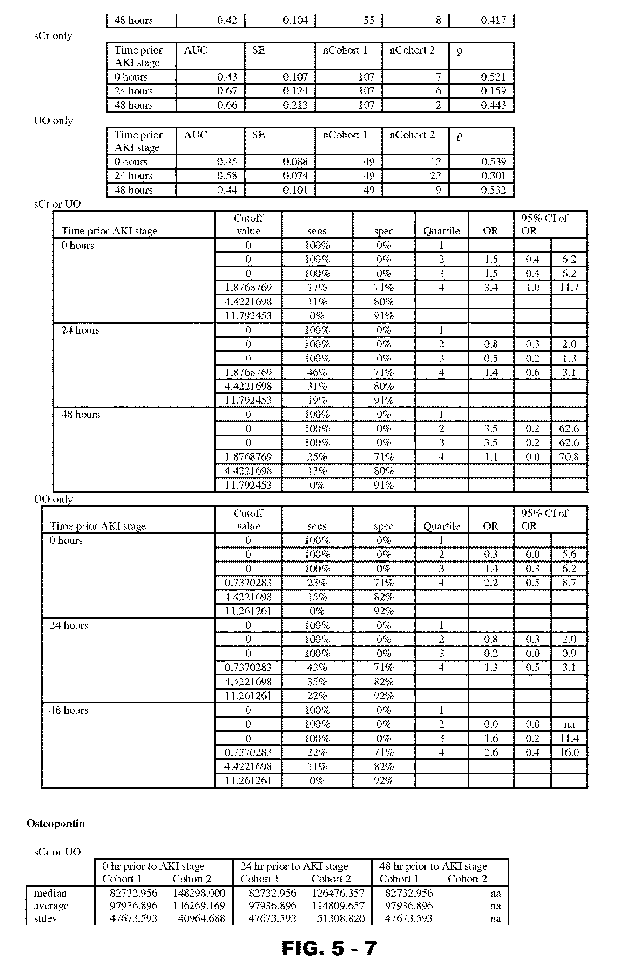

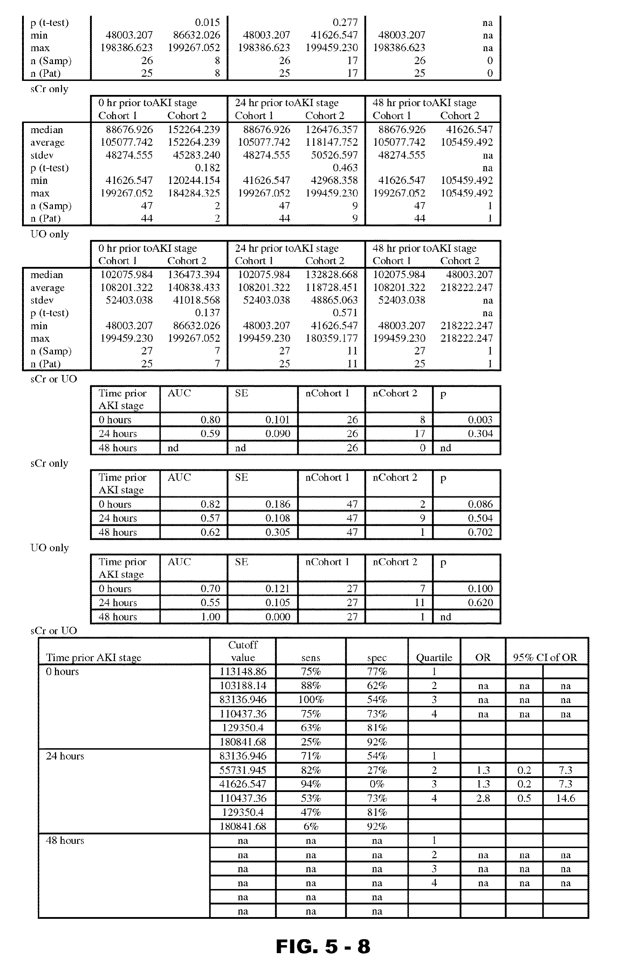

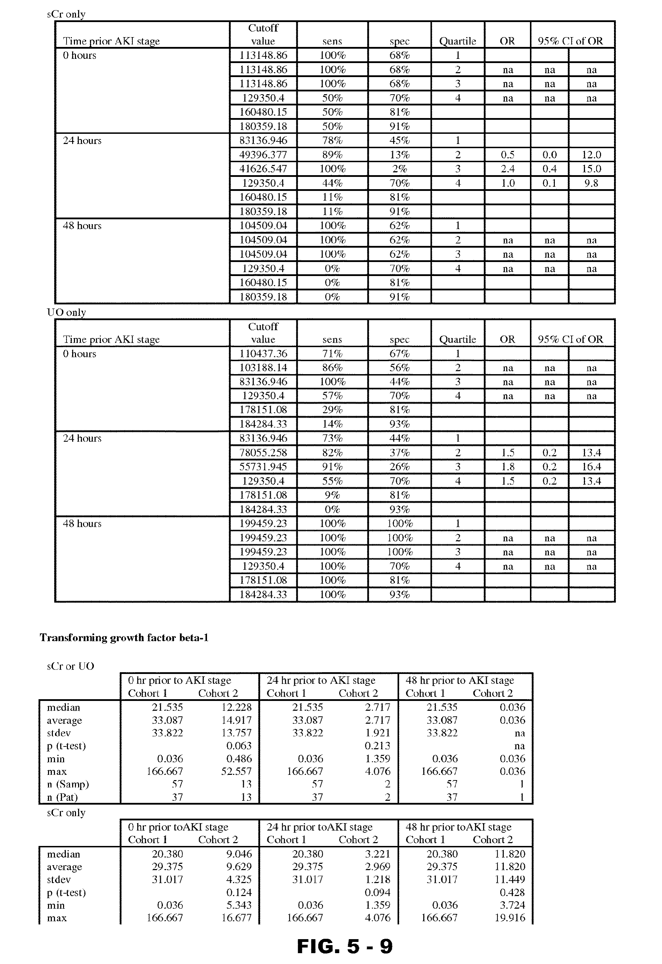

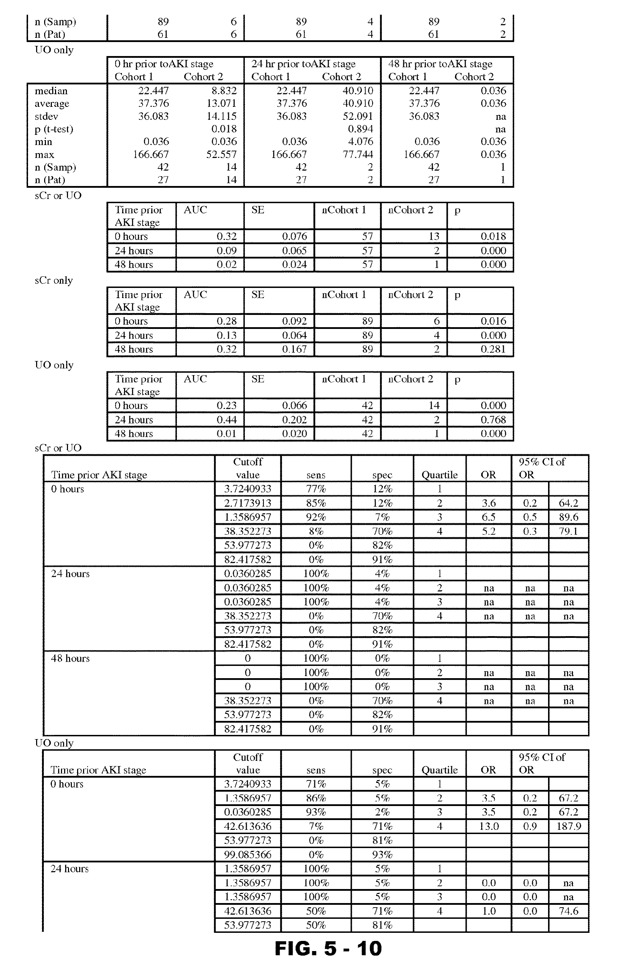

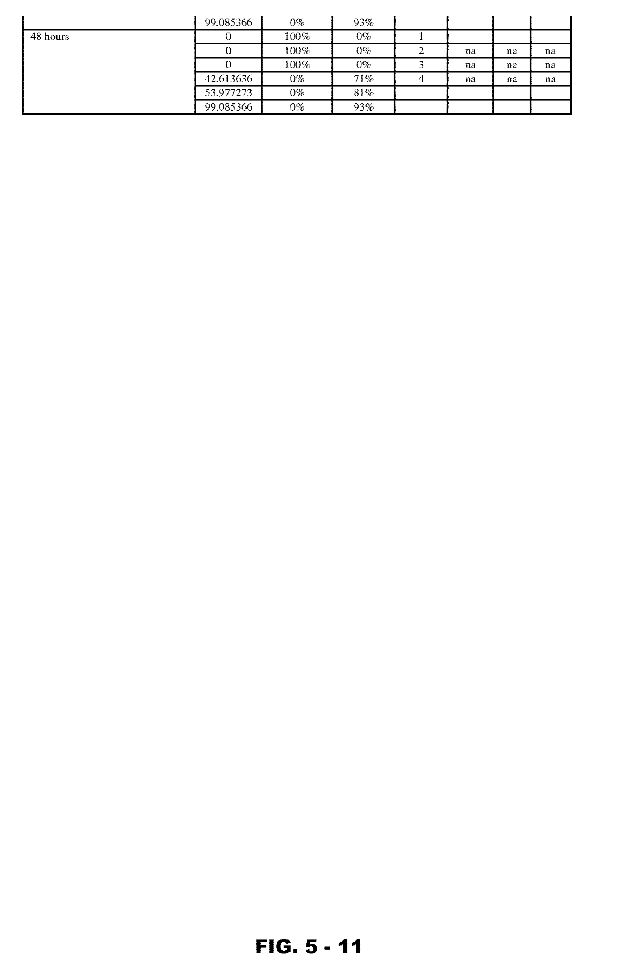

Fig. 5 provides data tables determined in accordance with Example 6 for the comparison of marker levels in plasma samples collected for Cohort 1 (patients that did not progress beyond RIFLE stage 0) and in plasma samples collected from subjects at 0, 24 hours, and 48 hours prior to reaching stage R, I or F in Cohort 2. Tables provide descriptive statistics, AUC analysis, and sensitivity, specificity and odds ratio calculations at various threshold (cutoff) levels for the various markers.

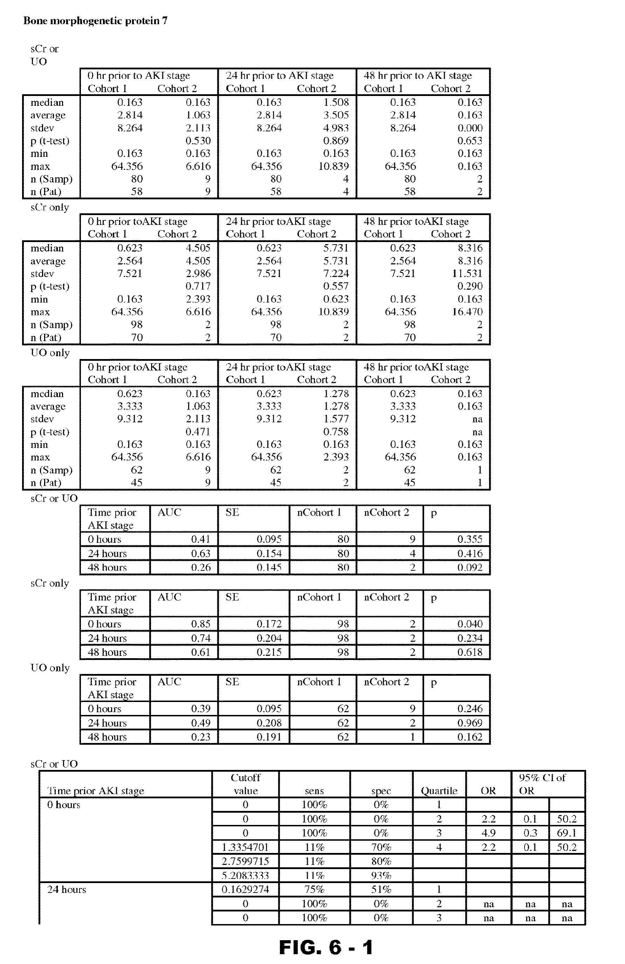

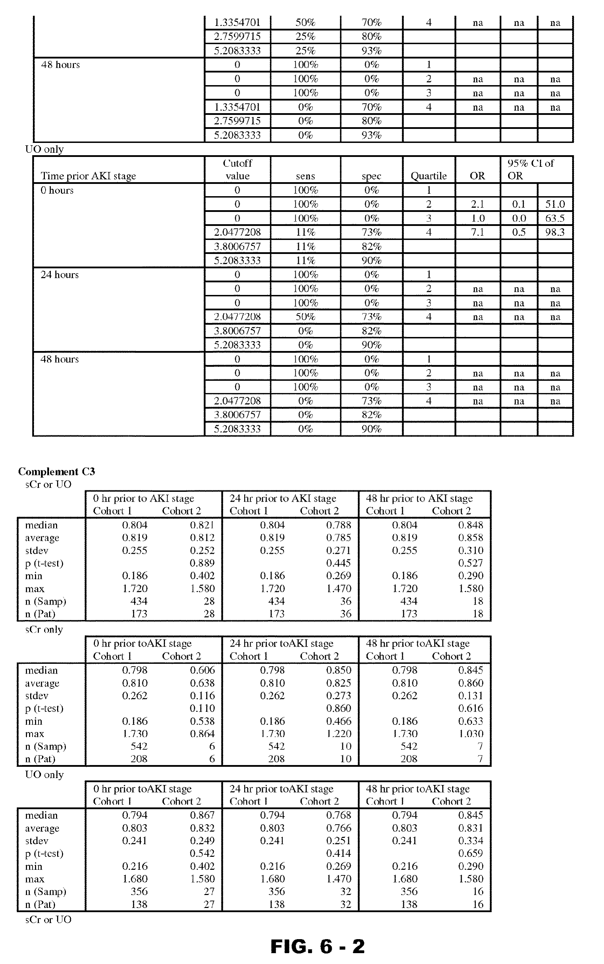

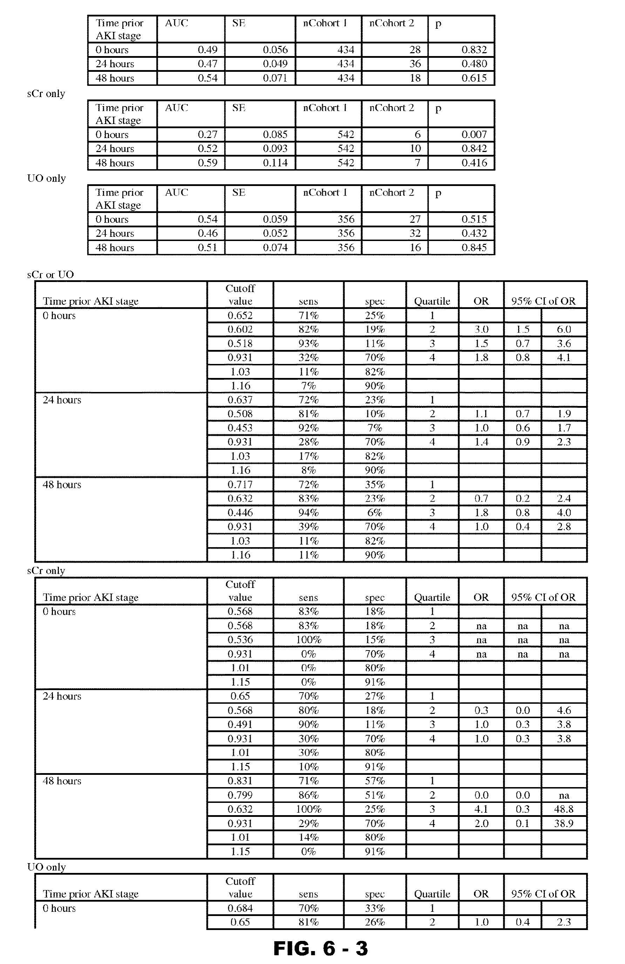

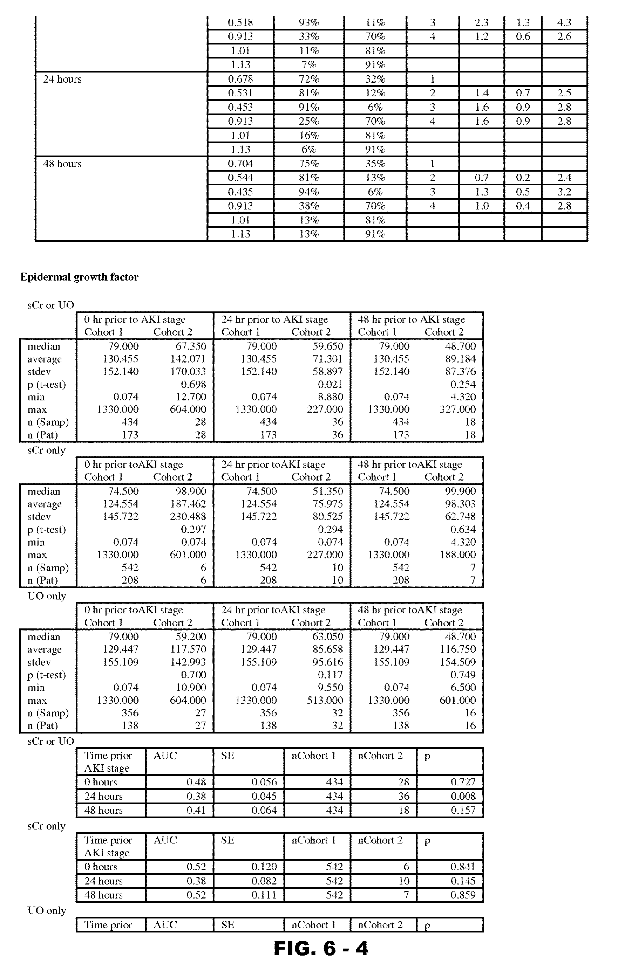

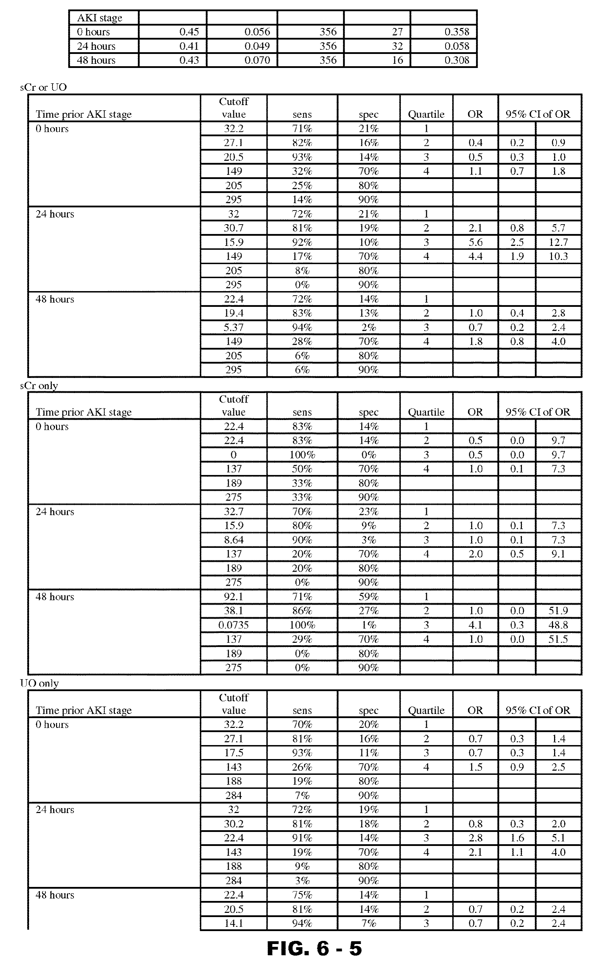

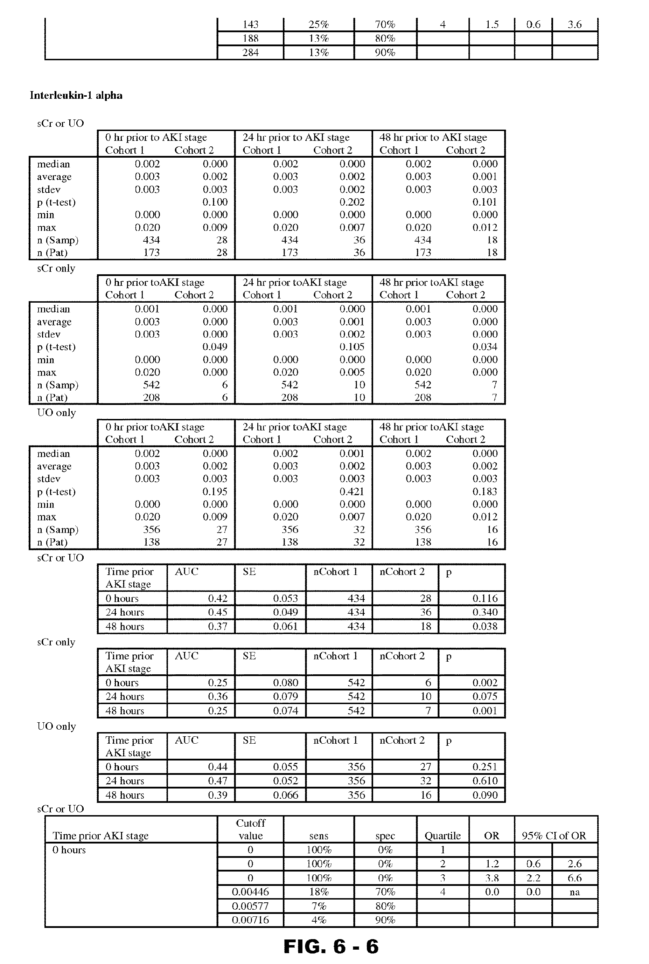

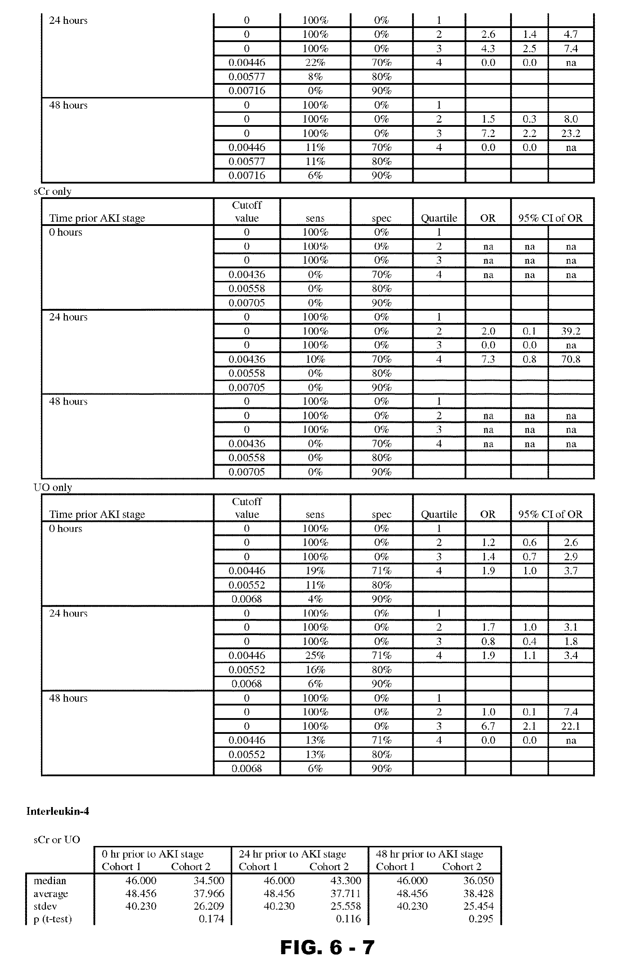

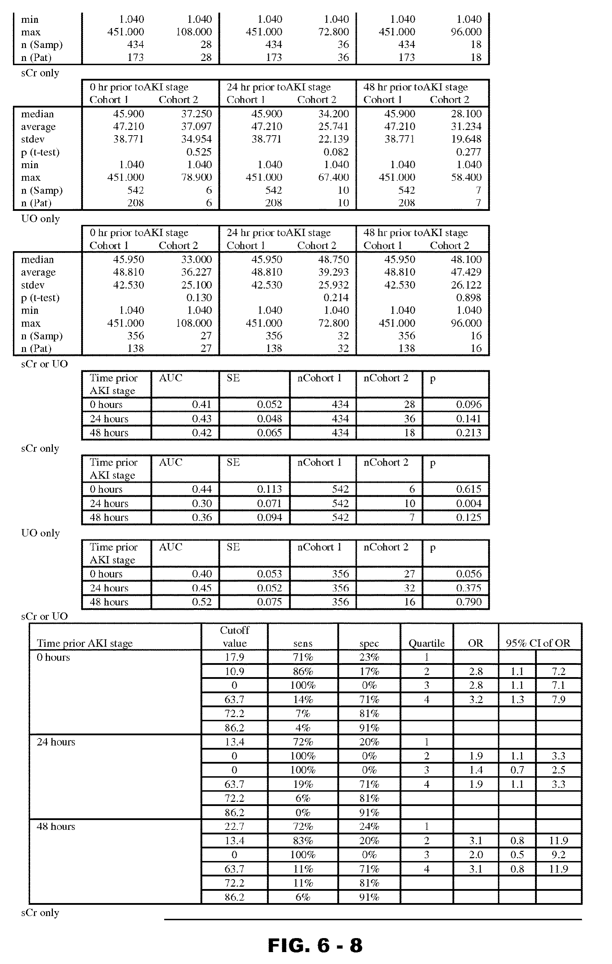

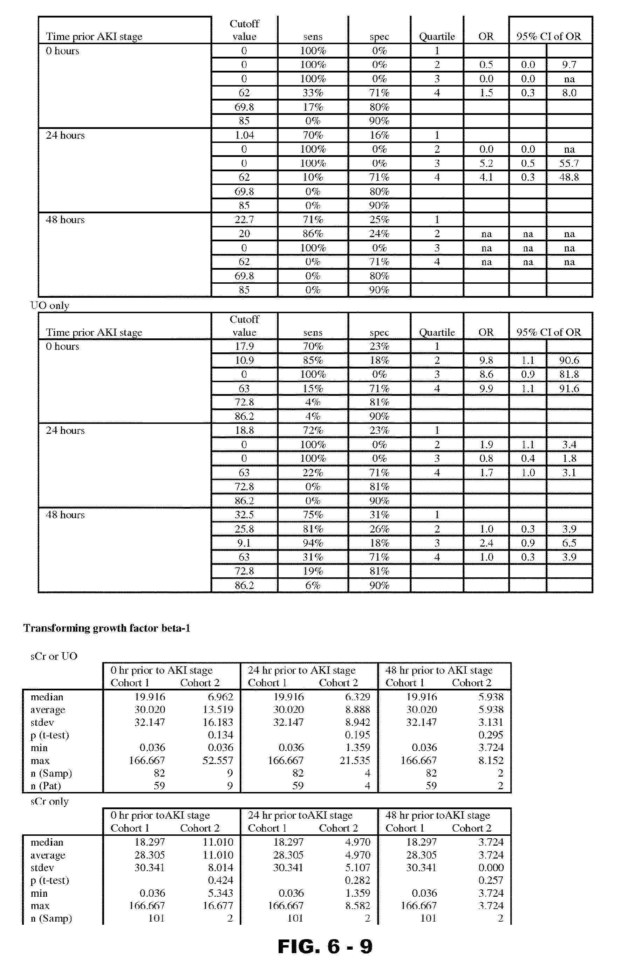

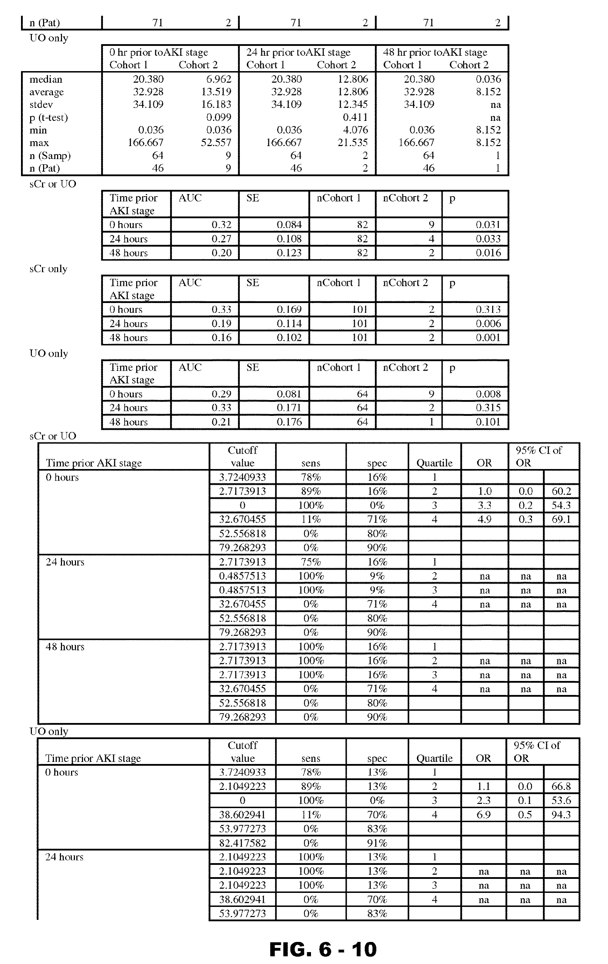

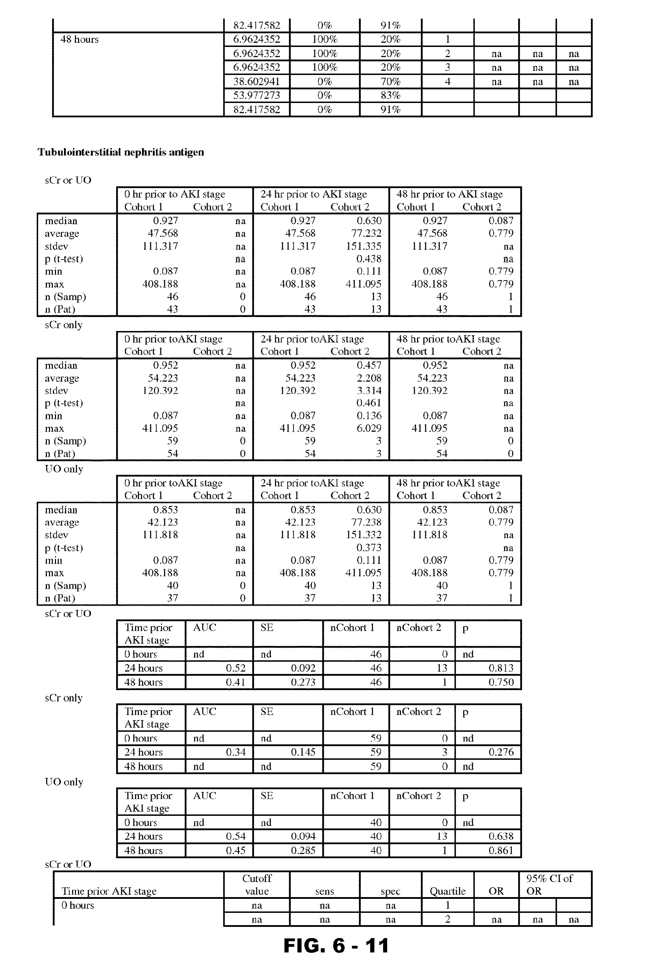

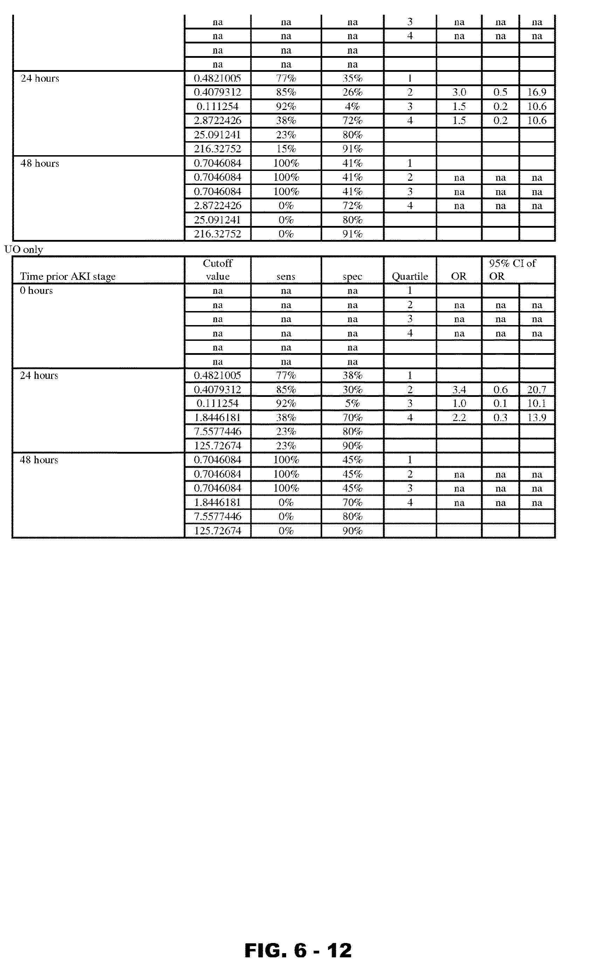

Fig. 6 provides data tables determined in accordance with Example 7 for the comparison of marker levels in plasma samples collected for Cohort 1 (patients that did not progress beyond RIFLE stage 0 or R) and in plasma samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

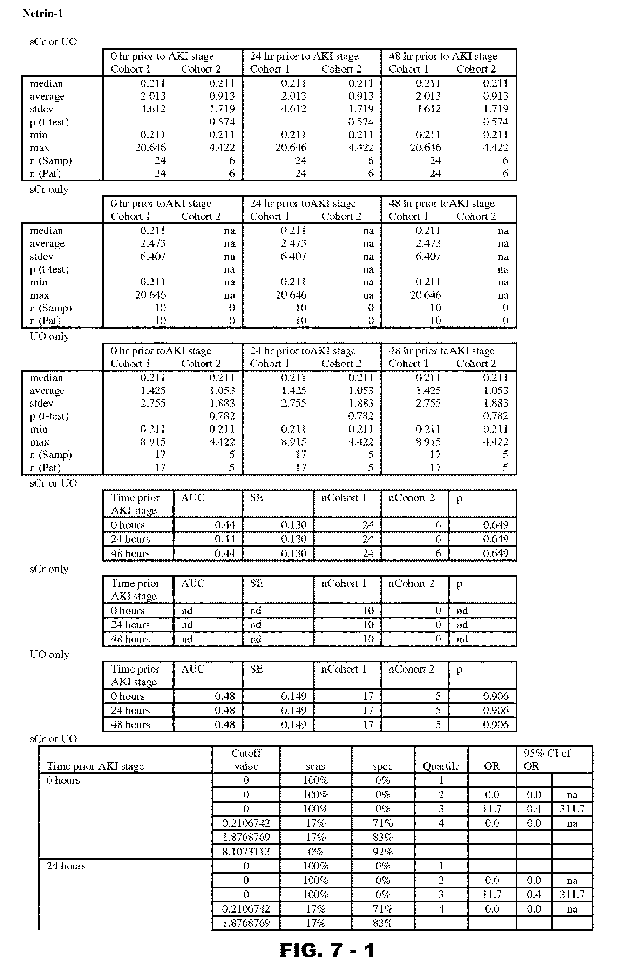

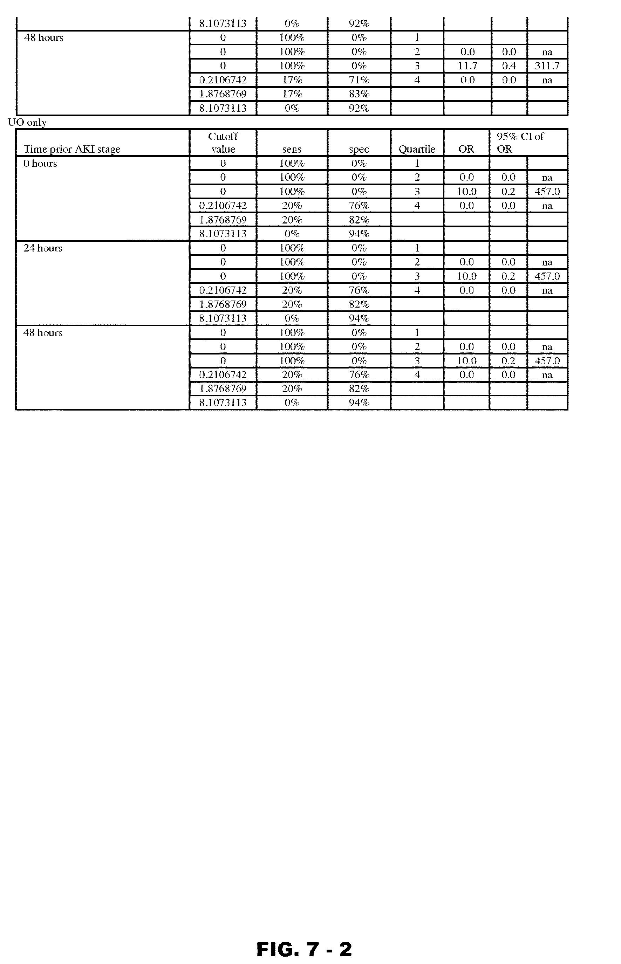

Fig. 7 provides data tables determined in accordance with Example 8 for the comparison of marker levels in plasma samples collected for Cohort 1 (patients that reached, but did not progress beyond, RIFLE stage R) and in plasma samples collected from subjects at 0, 24 hours, and 48 hours prior to reaching stage I or F in Cohort 2. Tables provide descriptive statistics, AUC analysis, and sensitivity, specificity and odds ratio calculations at various threshold (cutoff) levels for the various markers.

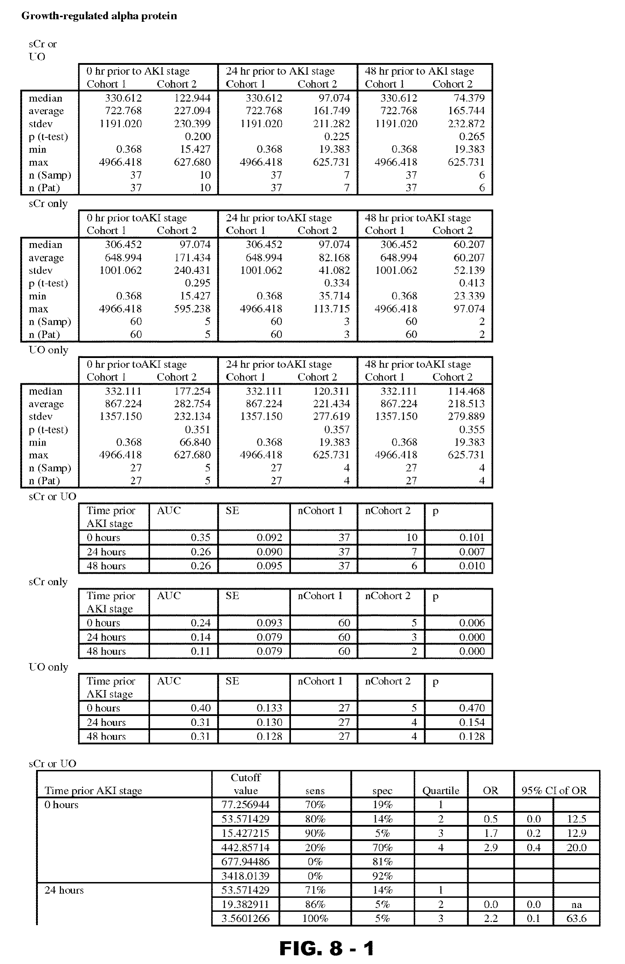

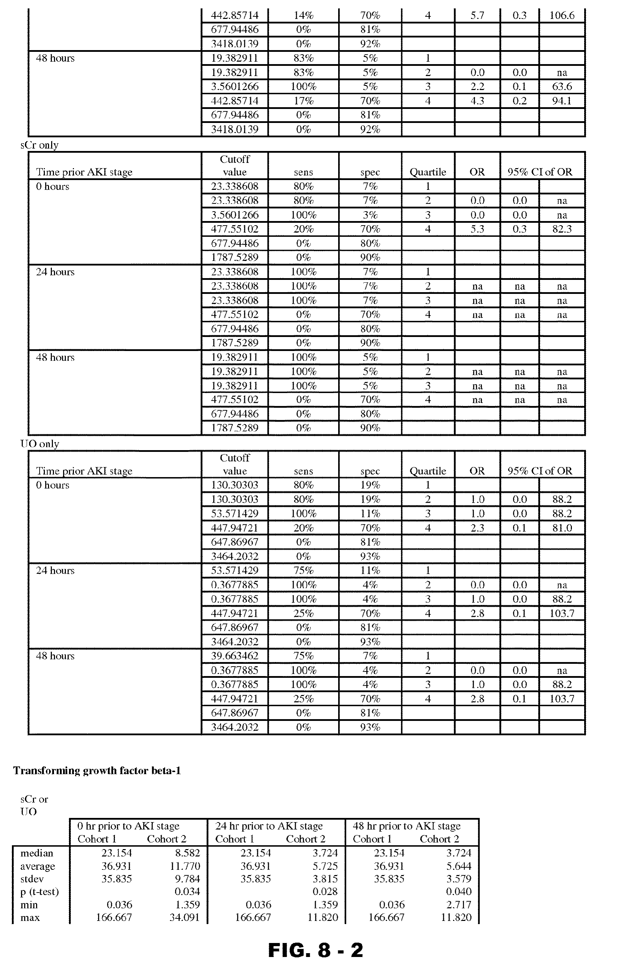

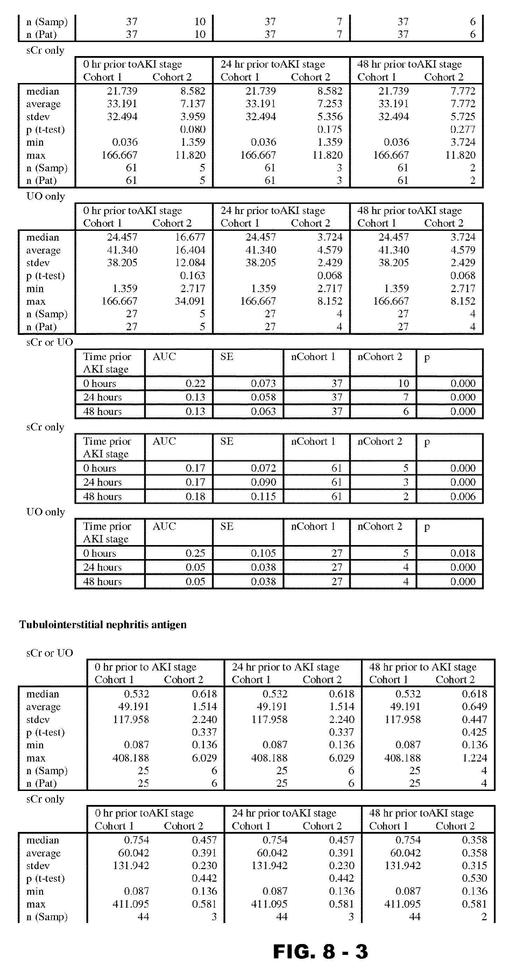

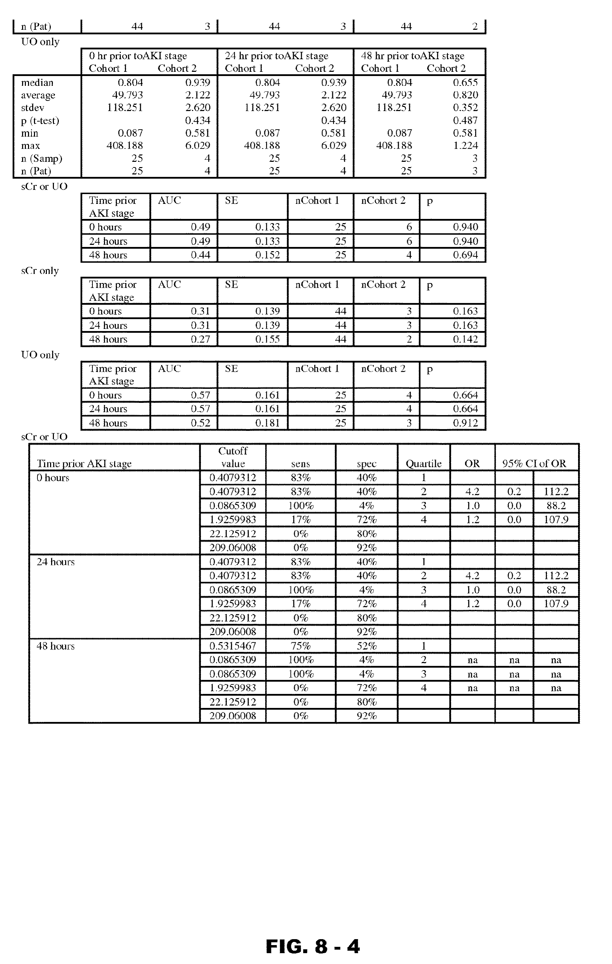

Fig. 8 provides data tables determined in accordance with Example 9 for the comparison of marker levels in plasma samples collected for Cohort 1 (patients that did not progress beyond RIFLE stage 0) and in plasma samples collected from subjects at 0, 24 hours, and 48 hours prior to reaching stage F in Cohort 2. Tables provide descriptive statistics, AUC analysis, and sensitivity, specificity and odds ratio calculations at various threshold (cutoff) levels for the various markers.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein, or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quatitation). This list is not meant to be limiting.
As used herein, the term "Epidermal growth factor" refers to one or more polypeptides present in a biological sample that are derived from the Epidermal growth factor precursor (Swiss-Prot P01133 (SEQ ID NO: 1)).

```
          10         20         30         40         50         60
   MLLTLIILLP VVSKFSFVSL SAPQHWSCPE GTLAGNGNST CVGPAPFLIF SHGNSIFRID

          70         80         90        100        110        120
   TEGTNYEQLV VDAGVSVIMD FHYNEKRIYW VDLERQLLQR VFLNGSRQER VCNIEKNVSG

         130        140        150        160        170        180
   MAINWINEEV IWSNQQEGII TVTDMKGNNS HILLSALKYP ANVAVDPVER FIFWSSEVAG

         190        200        210        220        230        240
   SLYRADLDGV GVKALLETSE KITAVSLDVL DKRLFWIQYN REGSNSLICS CDYDGGSVHI

         250        260        270        280        290        300
   SKHPTQHNLF AMSLFGDRIF YSTWKMKTIW IANKHTGKDM VRINLHSSFV PLGELKVVHP
```

```
      310         320         330         340         350         360
LAQPKAEDDT   WEPEQKLCKL  RKGNCSSTVC  GQDLQSHLCM  CAEGYALSRD  RKYCEDVNEC

      370         380         390         400         410         420
AFWNHGCTLG   CKNTPGSYYC  TCPVGFVLLP  DGKRCHQLVS  CPRNVSECSH  DCVLTSEGPL

      430         440         450         460         470         480
CFCPEGSVLE   RDGKTCSGCS  SPDNGGCSQL  CVPLSPVSWE  CDCFPGYDLQ  LDEKSCAASG

      490         500         510         520         530         540
PQPFLLFANS   QDIRHMHFDG  TDYGTLLSQQ  MGMVYALDHD  PVENKIYFAH  TALKWIERAN

      550         560         570         580         590         600
MDGSQRERLI   EEGVDPEGL   AVDWIGRRFY  WTDRGKSLIG  RSDLNGKRSK  IITKENISQP

      610         620         630         640         650         660
RGIAVHPMAK   RLFWTDTGIN  PRIESSSLQG  LGRLVIASSD  LIWPSGITID  FLTDKLYWCD

      670         680         690         700         710         720
AKQSVIEMAN   LDGSKRRRLT  QNDVGHPFAV  AVFEDYVWFS  DWAMPSVIRV  NKRTGKDRVR

      730         740         750         760         770         780
LQGSMLKPSS   LVVVHPLAKP  GADPCLYQNG  GCEHICKKRL  GTAWCSCREG  FMKASDGKTC

      790         800         810         820         830         840
LALDGHQLLA   GGEVDLKNQV  TPLDILSKTR  VSEDNITESQ  HMLVAEIMVS  DQDDCAPVGC

      850         860         870         880         890         900
SMYARCISEG   EDATCQCLKG  FAGDGKLCSD  IDECEMGVPV  CPPASSKCIN  TEGGYVCRCS

      910         920         930         940         950         960
EGYQGDGIHC   LDIDECQLGV  HSCGENASCT  NTEGGYTCMC  AGRLSEPGLI  CPDSTPPPHL

      970         980         990        1000        1010        1020
REDDHHYSVR   NSDSECPLSH  DGYCLHDGVC  MYIEALDKYA  CNCVVGYIGE  RCQYRDLKWW

     1030        1040        1050        1060        1070        1080
ELRHAGHGQQ   QKVIVVAVCV  VVLVMLLLLS  LWGAHYYRTQ  KLLSKNPKNP  YEESSRDVRS

     1090        1100        1110        1120        1130        1140
RRPADTEDGM   SSCPQPWFVV  IKEHQDLKNG  GQPVAGEDGQ  AADGSMQPTS  WRQEPQLCGM

     1150        1160        1170        1180        1190        1200
GTEQGCWIPV   SSDKGSCPQV  MERSFHMPSY  GTQTLEGGVE  KPHSLLSANP  LWQQRALDPP


HQMELTQ
```

Most preferably, the Epidermal growth factor assay detects one or more soluble forms of Epidermal growth factor. Epidermal growth factor is a single-pass type I membrane protein having a large extracellular domain, some or all of which is present in soluble forms of Epidermal growth factor generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Epidermal growth factor:

| Residues | Length | Domain ID |
|---|---|---|
| 1-22 | 22 | signal sequence |
| 23-1207 | 1185 | Pro-epidermal growth factor |
| 971-1023 | 53 | Epidermal growth factor |
| 23-1032 | 1010 | extracellular |
| 1033-1053 | 21 | transmembrane |
| 1054-1207 | 154 | cytoplasmic |

As used herein, the term "Complement C3" refers to one or more polypeptides present in a biological sample that are derived from the Complement C3 precursor (Swiss-Prot P01024 (SEQ ID NO: 2)).

```
                10         20         30         40         50         60
        MGPTSGPSLL LLLLTHLPLA LGSPMYSIIT PNILRLESEE TMVLEAHDAQ GDVPVTVTVH

                70         80         90        100        110        120
        DFPGKKLVLS SEKTVLTPAT NHMGNVTFTI PANREFKSEK GRNKFVTVQA TFGTQVVEKV

               130        140        150        160        170        180
        VLVSLQSGYL FIQTDKTIYT PGSTVLYRIF TVNHKLLPVG RTVMVNIENP EGIPVKQDSL

               190        200        210        220        230        240
        SSQNQLGVLP LSWDIPELVN MGQWKIRAYY ENSPQQVFST EFEVKEYVLP SFEVIVEPTE

               250        260        270        280        290        300
        KFYYIYNEKG LEVTITARFL YGKKVEGTAF VIFGIQDGEQ RISLPESLKR IPIEDGSGEV

               310        320        330        340        350        360
        VLSRKVLLDG VQNPRAEDLV GKSLYVSATV ILHSGSDMVQ AERSGIPIVT SPYQIHFTKT

               370        380        390        400        410        420
        PKYFKPGMPF DLMVFVTNPD GSPAYRVPVA VQGEDTVQSL TQGDGVAKLS INTHPSQKPL

               430        440        450        460        470        480
        SITVRTKKQE LSEAEQATRT MQALPYSTVG NSNNYLHLSV LRTELRPGET LNVNFLLRMD

               490        500        510        520        530        540
        RAHEAKIRYY TYLIMNKGRL LKAGRQVREP GQDLVVLPLS ITTDFIPSFR LVAYYTLIGA

               550        560        570        580        590        600
        SGQREVVADS VWVDVKDSCV GSLVVKSGQS EDRQPVPGQQ MTLKIEGDHG ARVVLVAVDK
```

```
        610         620         630         640         650         660
GVFVLNKKNK  LTQSKIWDVV  EKADIGCTPG  SGKDYAGVFS  DAGLTFTSSS  GQQTAQRAEL

        670         680         690         700         710         720
QCPQPAARRR  RSVQLTEKRM  DKVGKYPKEL  RKCCEDGMRE  NPMRFSCQRR  TRFISLGEAC

        730         740         750         760         770         780
KKVFLDCCNY  ITELRRQHAR  ASHLGLARSN  LDEDIIAEEN  IVSRSEFPES  WLWNVEDLKE

        790         800         810         820         830         840
PPKNGISTKL  MNIFLKDSIT  TWEILAVSMS  DKKGICVADP  FEVTVMQDFF  IDLRLPYSVV

        850         860         870         880         890         900
RNEQVEIRAV  LYNYRQNQEL  KVRVELLHNP  AFCSLATTKR  RHQQTVTIPP  KSSLSVPYVI

        910         920         930         940         950         960
VPLKTGLQEV  EVKAAVYHHF  ISDGVRKSLK  VVPEGIRMNK  TVAVRTLDPE  RLGREGVQKE

        970         980         990        1000        1010        1020
DIPPADLSDQ  VPDTESETRI  LLQGTPVAQM  TEDAVDAERL  KHLIVTPSGC  GEQNMIGMTP

       1030        1040        1050        1060        1070        1080
TVIAVHYLDE  TEQWEKFGLE  KRQGALELIK  KGYTQQLAFR  QPSSAFAAFV  KRAPSTWLTA

       1090        1100        1110        1120        1130        1140
YVVKVFSLAV  NLIAIDSQVL  CGAVKWLILE  KQKPDGVFQE  DAPVIHQEMI  GGLRNNNEKD

       1150        1160        1170        1180        1190        1200
MALTAFVLIS  LQEAKDICEE  QVNSLPGSIT  KAGDFLEANY  MNLQRSYTVA  IAGYALAQMG

       1210        1220        1230        1240        1250        1260
RLKGPLLNKF  LTTAKDKNRW  EDPGKQLYNV  EATSYALLAL  LQLKDFDFVP  PVVRWLNEQR

       1270        1280        1290        1300        1310        1320
YYGGGYGSTQ  ATFMVFQALA  QYQKDAPDHQ  ELNLDVSLQL  PSRSSKITHR  IHWESASLLR

       1330        1340        1350        1360        1370        1380
SEETKENEGF  TVTAEGKGQG  TLSVVTMYHA  KAKDQLTCNK  FDLKVTIKPA  PETEKRPQDA

       1390        1400        1410        1420        1430        1440
KNTMILEICT  RYRGDQDATM  SILDISMMTG  FAPDTDDLKQ  LANGVDRYIS  KYELDKAFSD

       1450        1460        1470        1480        1490        1500
RNTLIIYLDK  VSHSEDDCLA  FKVHQYFNVE  LIQPGAVKVY  AYYNLEESCT  RFYHPEKEDG

       1510        1520        1530        1540        1550        1560
KLNKLCRDEL  CRCAEENCFI  QKSDDKVTLE  ERLDKACEPG  VDYVYKTRLV  KVQLSNDFDE

       1570        1580        1590        1600        1610        1620
YIMAIEQTIK  SGSDEVQVGQ  QRTFISPIKC  REALKLEEKK  HYLMWGLSSD  FWGEKPNLSY

       1630        1640        1650        1660
IIGKDTWVEH  WPEEDECQDE  ENQKQCQDLG  AFTESMVVFG  CPN
```

The following domains have been identified in Complement C3:

| Residues | Length | Domain ID |
|---|---|---|
| 1-22 | 22 | Signal peptide |
| 23-1663 | 1641 | Complement C3 |
| 23-667 | 645 | Complement C3 beta chain |
| 672-1663 | 992 | Complement C3 alpha chain |
| 672-748 | 77 | Complement C3 anaphylatoxin |
| 749-1663 | 915 | Complement C3b alpha' chain |
| 749-954 | 206 | Complement C3c alpha' chain fragment 1 |
| 955-1303 | 349 | Complement C3dg fragment |
| 955-1001 | 47 | Complement C3g fragment |
| 1002-1303 | 302 | Complement C3d fragment |
| 1304-1320 | 17 | Complement C3f fragment |
| 1321-1663 | 343 | Complement C3c alpha' chain fragment 2 |

As used herein, the term "Interleukin-4" refers to one or more polypeptides present in a biological sample that are derived from the Interleukin-4 precursor (Swiss-Prot P05112 (SEQ ID NO: 3)).

```
        10         20         30         40         50         60
MGLTSQLLPP LFFLLACAGN FVHGHKCDIT LQEIIKTLNS LTEQKTLCTE LTVTDIFAAS

        70         80         90        100        110        120
KNTTEKETFC RAATVLRQFY SHHEKDTRCL GATAQQFHRH KQLIRFLKRL DRNLWGLAGL

       130        140        150
NSCPVKEANQ STLENFLERL KTIMREKYSK CSS
```

The following domains have been identified in Interleukin-4:

| Residues | Length | Domain ID |
|---|---|---|
| 1-24 | 24 | Signal peptide |
| 25-153 | 129 | Interleukin-4 |

As used herein, the term "Interleukin-1 alpha" refers to one or more polypeptides present in a biological sample that are derived from the Interleukin-1 alpha precursor (Swiss-Prot P01583 (SEQ ID NO: 4)).

```
        10         20         30         40         50         60
MAKVPDMFED LKNCYSENEE DSSSIDHLSL NQKSFYHVSY GPLHEGCMDQ SVSLSISETS

        70         80         90        100        110        120
KTSKLTFKES MVVVATNGKV LKKRRLSLSQ SITDDDLEAI ANDSEEEIIK PRSAPFSFLS

       130        140        150        160        170        180
NVKYNFMRII KYEFILNDAL NQSIIRANDQ YLTAAALHNL DEAVKFDMGA YKSSKDDAKI

       190        200        210        220        230        240
TVILRISKTQ LYVTAQDEDQ PVLLKEMPEI PKTITGSETN LLFFWETHGT KNYFTSVAHP

       250        260        270
NLFIATKQDY WVCLAGGPPS ITDFQILENQ A
```

The following domains have been identified in Interleukin-1 alpha:

| Residues | Length | Domain ID |
| --- | --- | --- |
| 1-112 | 112 | Propeptide |
| 113-271 | 159 | Interleukin-1 alpha |

As used herein, the term "Tubulointerstitial nephritis antigen" refers to one or more polypeptides present in a biological sample that are derived from the Tubulointerstitial nephritis antigen precursor (Swiss-Prot Q9UJW2 (SEQ ID NO: 5)).

```
        10         20         30         40         50         60
MWTGYKILIF SYLTTEIWME KQYLSQREVD LEAYFTRNHT VLQGTRFKRA IFQGQYCRNF

        70         80         90        100        110        120
GCCEDRDDGC VTEFYAANAL CYCDKFCDRE NSDCCPDYKS FCREEKEWPP HTQPWYPEGC

       130        140        150        160        170        180
FKDGQHYEEG SVIKENCNSC TCSGQQWKCS QHVCLVRPEL IEQVNKGDYG WTAQNYSQFW

       190        200        210        220        230        240
GMTLEDGFKF RLGTLPPSPM LLSMNEMTAS LPATTDLPEF FVASYKWPGW THGPLDQKNC

       250        260        270        280        290        300
AASWAFSTAS VAADRIAIQS KGRYTANLSP QNLISCCAKN RHGCNSGSID RAWWYLRKRG

       310        320        330        340        350        360
LVSHACYPLF KDQNATNNGC AMASRSDGRG KRHATKPCPN NVEKSNRIYQ CSPPYRVSSN

       370        380        390        400        410        420
ETEIMKEIMQ NGPVQAIMQV REDFFHYKTG IYRHVTSTNK ESEKYRKLQT HAVKLTGWGT

       430        440        450        460        470
LRGAQGQKEK FWIAANSWGK SWGENGYFRI LRGVNESDIE KLIIAAWGQL TSSDEP
```

The following domains have been identified in Tubulointerstitial nephritis antigen:

| Residues | Length | Domain ID |
| --- | --- | --- |
| 1-476 | 112 | Tubulo interstitial nephritis antigen |
| 59-107 | 49 | SMB |

As used herein, the term "Transforming growth factor beta-1" refers to one or more polypeptides present in a biological sample that are derived from the Transforming growth factor beta-1 precursor (Swiss-Prot P01137 (SEQ ID NO: 6)).

```
        10         20         30         40         50         60
MPPSGLRLLL LLLPLLWLLV LTPGRPAAGL STCKTIDMEL VKRKRIEAIR GQILSKLRLA

        70         80         90        100        110        120
SPPSQGEVPP GPLPEAVLAL YNSTRDRVAG ESAEPEPEPE ADYYAKEVTR VLMVETHNEI

       130        140        150        160        170        180
YDKFKQSTHS IYMFFNTSEL REAVPEPVLL SRAELRLLRL KLKVEQHVEL YQKYSNNSWR

       190        200        210        220        230        240
YLSNRLLAPS DSPEWLSFDV TGVVRQWLSR GGEIEGFRLS AHCSCDSRDN TLQVDINGFT

       250        260        270        280        290        300
TGRRGDLATI HGMNRPFLLL MATPLERAQH LQSSRHRRAL DTNYCFSSTE KNCCVRQLYI

       310        320        330        340        350        360
DFRKDLGWKW IHEPKGYHAN FCLGPCPYIW SLDTQYSKVL ALYNQHNPGA SAAPCCVPQA

       370        380        390
LEPLPIVYYV GRKPKVEQLS NMIVRSCKCS
```

The following domains have been identified in Transforming growth factor beta-1:

| Residues | Length | Domain ID |
|---|---|---|
| 1-29 | 29 | Signal peptide |
| 30-278 | 249 | Latency-associated peptide |
| 279-390 | 112 | Transforming growth factor beta-1 |

As used herein, the term "Bone morphogenetic protein 7" refers to one or more polypeptides present in a biological sample that are derived from the Bone morphogenetic protein 7 precursor (Swiss-Prot P18075 (SEQ ID NO: 7)).

```
          10         20         30         40         50         60
   MHVRSLRAAA PHSFVALWAP LFLLRSALAD FSLDNEVHSS FIHRRLRSQE RREMQREILS

          70         80         90        100        110        120
   ILGLPHRPRP HLQGKHNSAP MFMLDLYNAM AVEEGGGPGG QGFSYPYKAV FSTQGPPLAS

         130        140        150        160        170        180
   LQDSHFLTDA DMVMSFVNLV EHDKEFFHPR YHHREFRFDL SKIPEGEAVT AAEFRIYKDY

         190        200        210        220        230        240
   IRERFDNETF RISVYQVLQE HLGRESDLFL LDSRTLWASE EGWLVFDITA TSNHWVVNPR

         250        260        270        280        290        300
   HNLGLQLSVE TLDGQSINPK LAGLIGRHGP QNKQPFMVAF FKATEVHFRS IRSTGSKQRS

         310        320        330        340        350        360
   QNRSKTPKNQ EALRMANVAE NSSSDQRQAC KKHELYVSFR DLGWQDWIIA PEGYAAYYCE

         370        380        390        400        410        420
   GECAFPLNSY MNATNHAIVQ TLVHFINPET VPKPCCAPTQ LNAISVLYFD DSSNVILKKY

         430
   RNMVVRACGC H
```

The following domains have been identified in Bone morphogenetic protein 7:

| Residues | Length | Domain ID |
|---|---|---|
| 1-29 | 29 | Signal peptide |
| 30-292 | 263 | Propeptide |
| 293-431 | 139 | Bone morphogenetic protein 7 |

As used herein, the term "Osteopontin" refers to one or more polypeptides present in a biological sample that are derived from the Osteopontin precursor (Swiss-Prot P10451 (SEQ ID NO: 8)).

```
          10         20         30         40         50         60
   MRIAVICFCL LGITCAIPVK QADSGSSEEK QLYNKYPDAV ATWLNPDPSQ KQNLLAPQNA

          70         80         90        100        110        120
   VSSEETNDFK QETLPSKSNE SHDHMDDMDD EDDDDHVDSQ DSIDSNDSDD VDDTDDSHQS

         130        140        150        160        170        180
   DESHHSDESD ELVTDFPTDL PATEVFTPVV PTVDTYDGRG DSVVYGLRSK SKKFRRPDIQ

         190        200        210        220        230        240
   YPDATDEDIT SHMESEELNG AYKAIPVAQD LNAPSDWDSR GKDSYETSQL DDQSAETHSH

         250        260        270        280        290        300
   KQSRLYKRKA NDESNEHSDV IDSQELSKVS REFHSHEFHS HEDMLVVDPK SKEEDKHLKF

         310
   RISHELDSAS SEVN
```

The following domains have been identified in Osteopontin:

| Residues | Length | Domain ID |
|---|---|---|
| 1-16 | 16 | Signal peptide |
| 17-314 | 427 | Osteopontin |

As used herein, the term "Growth-regulated alpha protein" refers to one or more polypeptides present in a biological sample that are derived from the Growth-regulated alpha protein precursor (Swiss-Prot P09341 (SEQ ID NO: 9)).

```
        10         20         30         40         50         60
MARAALSAAP SNPRLLRVAL LLLLLVAAGR RAAGASVATE LRCQCLQTLQ GIHPKNIQSV

        70         80         90        100
NVKSPGPHCA QTEVIATLKN GRKACLNPAS PIVKKIIEKM LNSDKSN
```

The following domains have been identified in Growth-regulated alpha protein:

| Residues | Length | Domain ID |
|---|---|---|
| 1-34 | 34 | Signal peptide |
| 35-107 | 73 | Growth-regulated alpha protein |
| 38-107 | 70 | Growth-regulated alpha protein (4-73) |
| 39-107 | 69 | Growth-regulated alpha protein (5-73) |
| 40-107 | 68 | Growth-regulated alpha protein (6-73) |

As used herein, the term "Netrin-1" refers to one or more polypeptides present in a biological sample that are derived from the Netrin-1 precursor (Swiss-Prot 095631 (SEQ ID NO: 10)).

```
        10         20         30         40         50         60
MMRAVWEALA ALAAVACLVG AVRGGPGLSM FAGQAAQPDP CSDENGHPRR CIPDFVNAAF

        70         80         90        100        110        120
GKDVRVSSTC GRPPARYCVV SERGEERLRS CHLCNASDPK KAHPPAFLTD LNNPHNLTCW

       130        140        150        160        170        180
QSENYLQFPH NVTLTLSLGK KFEVTYVSLQ FCSPRPESMA IYKSMDYGRT WVPFQFYSTQ

       190        200        210        220        230        240
CRKMYNRPHR APITKQNEQE AVCTDSHTDM RPLSGGLIAF STLDGRPSAH DFDNSPVLQD
```

```
        250        260        270        280        290        300
   WVTATDIRVA FSRLHTFGDE NEDDSELARD SYFYAVSDLQ VGGRCKCNGH AARCVRDRTD

        310        320        330        340        350        360
   SLVCDCRHNT AGPECDRCKP FHYDRPWQRA TAREANECVA CNCNLHARRC RFNMELYKLS

        370        380        390        400        410        420
   GRKSGGVCLN CRHNTAGRHC HYCKEGYYRD MGKPITHRKA CKACDCHPVG AAGKTCNQTT

        430        440        450        460        470        480
   GQCPCKDGVT GITCNRCAKG YQQSRSPIAP CIKIPVAPPT TAASSVEEPE DCDSYCKASK

        490        500        510        520        530        540
   GKLKINMKKY CKKDYAVQIH ILKADKAGDW WKFTVNIISV YKQGTSRIRR GDQSLWIRSR

        550        560        570        580        590        600
   DIACKCPKIK PLKKYLLLGN AEDSPDQSGI VADKSSLVIQ WRDTWARRLR KFQQREKKGK


   CKKA
```

The following domains have been identified in Netrin-1:

| Residues | Length | Domain ID |
|---|---|---|
| 1-24 | 24 | Signal peptide |
| 25-604 | 580 | Netrin-1 |

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.*) as well as molecules that may be indirectly detected by production of a detectable reaction product (*e.g.*, enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non- specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: $r/c = K(n-r)$: where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, *etc.,* assay performance of an antibody may be a more important measure than absolute affinity and specificity of an antibody.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theroy developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, *etc.)* include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Cathepsin B (P07858); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1 alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of F1FO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \; \times \; 24\text{-hour volume}}{P_{Cr} \; \times \; 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \; \times \; 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, *etc.* The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2) ), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients

expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are is measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Concentrations are expressed in the following examples as follows: Epidermal growth factor - pg/mL, Complement C3 - mg/mL, Interleukin-4 - pg/mL, Interleukin-1 alpha - ng/mL, Tubulointerstitial nephritis antigen - $\mu$g/mL, Transforming growth factor beta-1 - pg/mL, Bone morphogenetic protein 7 - pg/mL, Osteopontin - pg/mL, Netrin-1 - ng/mL, and Growth-regulated alpha protein - pg/mL.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Kidney injury markers for evaluating renal status in patients at RIFLE Stage 0

Patients from the intensive care unit (ICU) were classified by kidney status as non-injury (0), risk of injury (R), injury (I), and failure (F) according to the maximum stage reached within 7 days of enrollment as determined by the RIFLE criteria.

Two cohorts were defined as (Cohort 1) patients that did not progress beyond stage 0, and (Cohort 2) patients that reached stage R, I, or F within 10 days. To address normal marker fluctuations that occur within patients at the ICU and thereby assess utility for monitoring AKI status, marker levels were measured in urine samples collected for Cohort 1. Marker concentrations were measured in urine samples collected from a subject at 0, 24 hours, and 48 hours prior to reaching stage R, I or F in Cohort 2. In the following tables, the time "prior max stage" represents the time at which a sample is collected, relative to the time a particular patient reaches the lowest disease stage as defined for that cohort, binned into three groups which are +/- 12 hours. For example, 24 hr prior for this example (0 vs R, I, F) would mean 24 hr (+/- 12 hours) prior to reaching stage R (or I if no sample at R, or F if no sample at R or I).

Each marker was measured by standard immunoassay methods using commercially available assay reagents. A receiver operating characteristic (ROC) curve was generated for each marker and the area under each ROC curve (AUC) was determined. Patients in Cohort 2 were also separated according to the reason for adjudication to stage R, I, or F as being based on serum creatinine measurements (sCr), being based on urine output (UO), or being based on either serum creatinine measurements or urine output. That is, for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, for those patients adjudicated to stage R, I, or F on the basis of serum creatinine measurements or urine output, the adjudication method which yielded the most severe RIFLE stage was used.

The ability to distinguish cohort 1 (subjects remaining in RIFLE 0) from Cohort 2 (subjects progressing to RIFLE R, I or F) was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 1.

Example 7. Kidney injury markers for evaluating renal status in patients at RIFLE Stages 0 and R

Patients were classified and analyzed as described in Example 6. However, patients that reached stage R but did not progress to stage I or F were grouped with patients from non-injury stage 0 in Cohort 1. Cohort 2 in this example included only patients that progressed to stage I or F. Marker concentrations in urine samples were included for Cohort 1. Marker concentrations in urine samples collected within 0, 24, and 48 hours of reaching stage I or F were included for Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 2.

Example 8. Kidney injury markers for evaluating renal status in patients progressing from Stage R to Stages I and F

Patients were classified and analyzed as described in Example 6, but only those patients that reached Stage R were included in this example. Cohort 1 contained patients that reached stage R but did not progress to stage I or F within 10 days, and Cohort 2 included only patients that progressed to stage I or F. Marker concentrations in urine samples collected within 12 hours of reaching stage R were included in the analysis for both Cohort 1 and 2.

The ability to distinguish cohort 1 (subjects remaining in RIFLE R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 3.

Example 9. Kidney injury markers for evaluating renal status in patients at RIFLE Stage 0

Patients were classified and analyzed as described in Example 6. However, patients that reached stage R or I but did not progress to stage F were eliminated from the analysis. Patients from non-injury stage 0 are included in Cohort 1. Cohort 2 in this example included only patients that progressed to stage F. The maximum marker concentrations in urine samples were included for each patient in Cohort 1. The maximum marker concentrations in urine samples collected within 0, 24, and 48 hours of reaching stage F were included for each patient in Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 4.

Example 10. Kidney injury markers for evaluating renal status in patients at RIFLE Stage 0

Patients from the intensive care unit (ICU) were classified by kidney status as non-injury (0), risk of injury (R), injury (I), and failure (F) according to the maximum stage reached within 7 days of enrollment as determined by the RIFLE criteria.

Two cohorts were defined as (Cohort 1) patients that did not progress beyond stage 0, and (Cohort 2) patients that reached stage R, I, or F within 10 days. To address normal marker fluctuations that occur within patients at the ICU and thereby assess utility for monitoring AKI status, marker levels were measured in the plasma component of blood samples collected for Cohort 1. Marker concentrations were measured in the plasma component of blood samples collected from a subject at 0, 24 hours, and 48 hours prior to reaching stage R, I or F in Cohort 2. In the following tables, the time "prior max stage" represents the time at which a sample is collected, relative to the time a particular patient reaches the lowest disease stage as defined for that cohort, binned into three groups which are +/- 12 hours. For example, 24 hr prior for this example (0 vs R, I, F) would mean 24 hr (+/- 12 hours) prior to reaching stage R (or I if no sample at R, or F if no sample at R or I).

Each marker was measured by standard immunoassay methods using commercially available assay reagents. A receiver operating characteristic (ROC) curve was generated for each marker and the area under each ROC curve (AUC) was determined. Patients in Cohort 2 were also separated according to the reason for adjudication to stage R, I, or F as being based on serum creatinine measurements (sCr), being based on urine output (UO), or being based on either serum creatinine measurements or urine output. That is, for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, for those patients adjudicated to stage R, I, or F on the basis of serum creatinine measurements or urine output, the adjudication method which yielded the most severe RIFLE stage was used.

The ability to distinguish cohort 1 (subjects remaining in RIFLE 0) from Cohort 2 (subjects progressing to RIFLE R, I or F) was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 5.

Example 11. Kidney injury markers for evaluating renal status in patients at RIFLE Stages 0 and R

Patients were classified and analyzed as described in Example 10. However, patients that reached stage R but did not progress to stage I or F were grouped with patients from non-injury stage 0 in Cohort 1. Cohort 2 in this example included only patients that progressed to stage I or F. Marker concentrations in the plasma component of blood samples were included for Cohort 1. Marker concentrations in the plasma component of blood samples collected within 0, 24, and 48 hours of reaching stage I or F were included for Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 6.

Example 12. Kidney injury markers for evaluating renal status in patients progressing from Stage R to Stages I and F

Patients were classified and analyzed as described in Example 10, but only those patients that reached Stage R were included in this example. Cohort 1 contained patients that reached stage R but did not progress to stage I or F within 10 days, and Cohort 2 included only patients that progressed to stage I or F. Marker concentrations in the plasma component of blood samples collected within 12 hours of reaching stage R were included in the analysis for both Cohort 1 and 2.

The ability to distinguish cohort 1 (subjects remaining in RIFLE R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 7.

Example 13. Kidney injury markers for evaluating renal status in patients at RIFLE Stage 0

Patients were classified and analyzed as described in Example 10. However, patients that reached stage R or I but did not progress to stage F were eliminated from the analysis. Patients from non-injury stage 0 are included in Cohort 1. Cohort 2 in this example included only patients that progressed to stage F. The maximum marker concentrations in the plasma component of blood samples were included from each patient in Cohort 1. The maximum marker concentrations in the plasma component of blood samples collected within 0, 24, and 48 hours of reaching stage F were included from each patient in Cohort 2.

The ability to distinguish cohort 1 (subjects remaining in RIFLE 0 or R) from Cohort 2 (subjects progressing to RIFLE I or F) was determined using ROC analysis.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

The results of these three analyses for various markers of the present invention are presented in Fig. 8.

The examples provided herein are representative of preferred embodiments, and are exemplary. The following items are furthermore disclosed herein:

1. A method for evaluating renal status in a subject, comprising:

performing one or more assays configured to detect a kidney injury marker selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein on a body fluid sample obtained from the subject to provide one or more assay results; and correlating the assay result(s) to one or more of risk stratification, staging, prognosis, classifying and monitoring of the renal status of the subject.

2. A method according to item 1, wherein said correlating step comprises assigning a likelihood of one or more future changes in renal status to the subject based on the assay result(s).

3. A method according to item 2, wherein said one or more future changes in renal status comprise one or more of a future injury to renal function, future reduced renal function, future improvement in renal function, and future acute renal failure (ARF).

4. A method according to item 3, wherein said assay result(s) comprise one or more of:

(i) a measured concentration of Epidermal growth factor,
(ii) a measured concentration of Complement C3,
(iii) a measured concentration of Interleukin-4,
(iv) a measured concentration of Interleukin-1 alpha,
(v) a measured concentration of Tubulointerstitial nephritis antigen,

(vi) a measured concentration of Transforming growth factor beta-1,

(vii) a measured concentration of Bone morphogenetic protein 7,

(viii) a measured concentration of Osteopontin,

(ix) a measured concentration of Netrin-1, or

(x) a measured concentration of Growth-regulated alpha protein,

and said correlation step comprises, for each assay result, comparing said measured concentration to a threshold concentration, and

for a positive going marker, assigning an increased likelihood of suffering a future injury to renal function, future reduced renal function, future ARF, or a future improvement in renal function to the subject when the measured concentration is above the threshold, relative to a likelihood assigned when the measured concentration is below the threshold or assigning a decreased likelihood of suffering a future injury to renal function, future reduced renal function, future ARF, or a future improvement in renal function to the subject when the measured concentration is below the threshold, relative to a likelihood assigned when the measured concentration is above the threshold, or

for a negative going marker, assigning an increased likelihood of suffering a future injury to renal function, future reduced renal function, future ARF, or a future improvement in renal function to the subject when the measured concentration is below the threshold, relative to a likelihood assigned when the measured concentration is above the threshold or assigning a decreased likelihood of suffering a future injury to renal function, future reduced renal function, future ARF, or a future improvement in renal function to the subject when the measured concentration is above the threshold, relative to a likelihood assigned when the measured concentration is below the threshold.

5. A method according to item 2, wherein said one or more future changes in renal status comprise a clinical outcome related to a renal injury suffered by the subject.

6. A method according to item 1, wherein said assay result(s) comprise one or more of:

(i) a measured concentration of Epidermal growth factor,

(ii) a measured concentration of Complement C3,

(iii) a measured concentration of Interleukin-4,

(iv) a measured concentration of Interleukin-1 alpha,

(v) a measured concentration of Tubulointerstitial nephritis antigen,

(vi) a measured concentration of Transforming growth factor beta-1,

(vii) a measured concentration of Bone morphogenetic protein 7,

(viii) a measured concentration of Osteopontin, Netrin-1, or

(ix) a measured concentration of Netrin-1, or

(x) a measured concentration of Growth-regulated alpha protein,

and said correlation step comprises, for each assay result, comparing said measured concentration to a threshold concentration, and

for a positive going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or

for a negative going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold.

7. A method according to item 2, wherein the likelihood of one or more future changes in renal status is that an event of interest is more or less likely to occur within 30 days of the time at which the body fluid sample is obtained from the subject.

8. A method according to item 7, wherein the likelihood of one or more future changes in renal status is that an event of interest is more or less likely to occur within a period selected from the group consisting of 21 days, 14 days, 7 days, 5 days, 96 hours, 72 hours, 48 hours, 36 hours, 24 hours, and 12 hours.

9. A method according to item 1, wherein the subject is selected for evaluation of renal status based on the pre-existence in the subject of one or more known risk factors for prerenal, intrinsic renal, or postrenal ARF.

10. A method according to item 1, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

11. A method according to item 1, wherein said correlating step comprises assigning a diagnosis of the occurrence or nonoccurrence of one or more of an injury to renal function, reduced renal function, or ARF to the subject based on the assay result(s).

12. A method according to item 1, wherein said correlating step comprises assessing whether or not renal function is improving or worsening in a subject who has suffered from an injury to renal function, reduced renal function, or ARF based on the assay result(s).

13. A method according to item 12, wherein said assay result(s) comprise one or more of:

(i) a measured concentration of Epidermal growth factor,
(ii) a measured concentration of Complement C3,
(iii) a measured concentration of Interleukin-4,
(iv) a measured concentration of Interleukin-1 alpha,
(v) a measured concentration of Tubulointerstitial nephritis antigen,
(vi) a measured concentration of Transforming growth factor beta-1,
(vii) a measured concentration of Bone morphogenetic protein 7,
(viii) a measured concentration of Osteopontin, Netrin-1, or
(ix) a measured concentration of Netrin-1, or
(x) a measured concentration of Growth-regulated alpha protein,

and said correlation step comprises, for each assay result, comparing said measured concentration to a threshold concentration, and

for a positive going marker, assigning a worsening of renal function to the subject when the measured concentration is above the threshold, or assigning an improvement of renal function when the measured concentration is below the threshold, or

for a negative going marker, assigning a worsening of renal function to the subject when the measured concentration is below the threshold, or assigning an improvement of renal function when the measured concentration is above the threshold.

14. A method according to item 1, wherein said method is a method of assigning a risk of the future occurrence or nonoccurrence of an injury to renal function in said subject.

15. A method according to item 1, wherein said method is a method of assigning a risk of the future occurrence or nonoccurrence of reduced renal function in said subject.

16. A method according to item 1, wherein said method is a method of assigning a risk of the future occurrence or nonoccurrence of acute renal failure in said subject.

17. A method according to item 1, wherein said method is a method of assigning a risk of the future occurrence or nonoccurrence of a need for renal replacement therapy in said subject.

18. A method according to item 1, wherein said method is a method of assigning a risk of the future occurrence or nonoccurrence of a need for renal transplantation in said subject.

19. A method according to item 4, wherein said one or more future changes in renal status comprise one or more of a future injury to renal function, future reduced renal function, future improvement in renal function, and future acute renal failure (ARF) within 72 hours of the time at which the body fluid sample is obtained.

20. A method according to item 4, wherein said one or more future changes in renal status comprise one or more of a future injury to renal function, future reduced renal function, future improvement in renal function, and future acute renal failure (ARF) within 48 hours of the time at which the body fluid sample is obtained.

21. A method according to item 4, wherein said one or more future changes in renal status comprise one or more of a future injury to renal function, future reduced renal function, future improvement in renal function, and future acute renal failure (ARF) within 72 hours of the time at which the body fluid sample is obtained.

22. A method according to item 4, wherein said one or more future changes in renal status comprise one or more of a future injury to renal function, future reduced renal function, future improvement in renal function, and future acute renal failure (ARF) within 48 hours of the time at which the body fluid sample is obtained.

23. A method according to item 4, wherein said one or more future changes in renal status comprise one or more of a future injury to renal function, future reduced renal function, future improvement in renal function, and future acute renal failure (ARF) within 24 hours of the time at which the body fluid sample is obtained.

24. Use of one or more kidney injury markers selected from the group consisting of Epidermal growth factor, Complement C3, Interleukin-4, Interleukin-1 alpha, Tubulointerstitial nephritis antigen, Transforming growth factor beta-1, Bone morphogenetic protein 7, Osteopontin, Netrin-1, and Growth-regulated alpha protein for one or more of risk stratification, staging, prognosis, classifying and monitoring of the renal status of a subject.

25. Use of one or more kidney injury markers selected from the group consisting of Epidermal growth factor, Complement C3, Interleukin-4, Interleukin-1 alpha, Tubulo interstitial nephritis antigen, Transforming growth factor beta-1, Bone morphogenetic protein 7, Osteopontin, Netrin-1, and Growth-regulated alpha protein for one or more of risk stratification, staging, prognosis, classifying and monitoring of the renal status of a subject suffering from an acute renal injury.

26. A method according to item 6, wherein the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 30 days of the time at which the body fluid sample is obtained from the subject.

27. A method according to item 6, wherein the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 72 hours of the time at which the body fluid sample is obtained from the subject.

28. A method according to item 6, wherein the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 24 hours of the time at which the body fluid sample is obtained from the subject.

**Claims**

1. A method for evaluating renal status in a subject, comprising:

   performing one or more assays configured to detect one or more kidney injury markers on a body fluid sample obtained from the subject to provide one or more assay results, the one or more kidney injury markers comprising Growth-regulated alpha protein; and
   correlating the assay result(s) to one or more of risk stratification, staging, prognosis, classifying and monitoring of the renal status of the subject.

2. A method according to claim 1, wherein the one or more kidney injury markers further comprise one or more of Epidermal growth factor, Complement C3, Interleukin-4, Interleukin-1 alpha, Tubulointerstitial nephritis antigen, Transforming growth factor beta-1, Bone morphogenetic protein 7, and Osteopontin, Netrin-1.

3. A method according to claim 1 or 2, wherein said correlating step comprises assigning (i) a likelihood of one or more future changes in renal status to the subject based on the assay result(s), or (ii) a diagnosis of the occurrence or nonoccurrence of one or more of an injury to renal function, reduced renal function, or ARF to the subject based on the assay result(s).

4. A method according to claim 3, wherein said one or more future changes in renal status comprise (i) one or more of a future injury to renal function, future reduced renal function, future improvement in renal function, and future acute renal failure (ARF) or (ii) a clinical outcome related to a renal injury suffered by the subject.

5. A method according to claim 4, wherein said assay result(s) comprise a measured concentration of Growth-regulated alpha protein, and optionally one or more of:

   (i) a measured concentration of Epidermal growth factor,
   (ii) a measured concentration of Complement C3,
   (iii) a measured concentration of Interleukin-4,
   (iv) a measured concentration of Interleukin-1 alpha,
   (v) a measured concentration of Tubulo interstitial nephritis antigen,
   (vi) a measured concentration of Transforming growth factor beta-1,
   (vii) a measured concentration of Bone morphogenetic protein 7,
   (viii) a measured concentration of Osteopontin, or
   (ix) a measured concentration of Netrin-1,

and said correlation step comprises, for each assay result, comparing said measured concentration to a threshold concentration, and

for a positive going marker, assigning an increased likelihood of suffering a future injury to renal function, future reduced renal function, future ARF, or a future improvement in renal function to the subject when the measured concentration is above the threshold, relative to a likelihood assigned when the measured concentration is below the threshold or assigning a decreased likelihood of suffering a future injury to renal function, future reduced renal function, future ARF, or a future improvement in renal function to the subject when the measured concentration is below the threshold, relative to a likelihood assigned when the measured concentration is above the threshold, or

for a negative going marker, assigning an increased likelihood of suffering a future injury to renal function, future reduced renal function, future ARF, or a future improvement in renal function to the subject when the measured concentration is below the threshold, relative to a likelihood assigned when the measured concentration is above the threshold or assigning a decreased likelihood of suffering a future injury to renal function, future reduced renal function, future ARF, or a future improvement in renal function to the subject when the measured concentration is above the threshold, relative to a likelihood assigned when the measured concentration is below the threshold.

6. A method according to claim 1, wherein said assay result(s) comprise a measured concentration of Growth-regulated alpha protein, and optionally one or more of:

    (i) a measured concentration of Epidermal growth factor,
    (ii) a measured concentration of Complement C3,
    (iii) a measured concentration of Interleukin-4,
    (iv) a measured concentration of Interleukin-1 alpha,
    (v) a measured concentration of Tubulointerstitial nephritis antigen,
    (vi) a measured concentration of Transforming growth factor beta-1,
    (vii) a measured concentration of Bone morphogenetic protein 7,
    (viii) a measured concentration of Osteopontin, Netrin-1, or
    (ix) a measured concentration of Netrin-1,

and said correlation step comprises, for each assay result, comparing said measured concentration to a threshold concentration, and

for a positive going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or

for a negative going marker, assigning an increased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is below the threshold, relative to a likelihood assigned when the measured concentration is above the threshold, or assigning a decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease to the subject when the measured concentration is above the threshold, relative to a likelihood assigned when the measured concentration is below the threshold.

7. A method according to claim 3, wherein the likelihood of one or more future changes in renal status is that an event of interest is more or less likely to occur within a period selected from the group consisting of 30 days, 21 days, 14 days, 7 days, 5 days, 96 hours, 72 hours, 48 hours, 36 hours, 24 hours, and 12 hours.

8. A method according to claim 1, wherein the subject is selected for evaluation of renal status based on (i) the pre-existence in the subject of one or more known risk factors for prerenal, intrinsic renal, or postrenal ARF, or

(ii) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

9. A method according to claim 1, wherein said correlating step comprises assessing whether or not renal function is improving or worsening in a subject who has suffered from an injury to renal function, reduced renal function, or ARF based on the assay result(s).

10. A method according to claim9, wherein said assay result(s) comprise a measured concentration of Growth-regulated alpha protein, and optionally one or more of:

(i) a measured concentration of Epidermal growth factor,
(ii) a measured concentration of Complement C3,
(iii) a measured concentration of Interleukin-4,
(iv) a measured concentration of Interleukin-1 alpha,
(v) a measured concentration of Tubulointerstitial nephritis antigen,
(vi) a measured concentration of Transforming growth factor beta-1,
(vii) a measured concentration of Bone morphogenetic protein 7,
(viii) a measured concentration of Osteopontin, Netrin-1, or
(ix) a measured concentration of Netrin-1,

and said correlation step comprises, for each assay result, comparing said measure concentration to a threshold concentration, and
for a positive going marker, assigning a worsening of renal function to the subject when the measured concentration is above the threshold, or assigning an improvement of renal function when the measured concentration is below the threshold, or
for a negative going marker, assigning a worsening of renal function to the subject when the measured concentration is below the threshold, or assigning an improvement of renal function when the measured concentration is above the threshold.

11. A method according to claim 1, wherein said method is a method of assigning a risk of

(i) the future occurrence or nonoccurrence of an injury to renal function in said subject,
(ii) the future occurrence or nonoccurrence of reduced renal function in said subject,
(iii) the future occurrence or nonoccurrence of acute renal failure in said subject,
(iv) the future occurrence or nonoccurrence of a need for renal replacement therapy in said subject or
(v) the future occurrence or nonoccurrence of a need for renal transplantation in said subject.

12. The method according to claim 5, wherein said one or more future changes in renal status comprise one or more of a future injury to renal function, future reduced renal function, future improvement in renal function, and future acute renal failure (ARF) within 72 hours, within 48 hours orwithin 24 hours of the time at which the body fluid sample is obtained.

13. Use of Growth-regulated alpha protein, and optionally one or more of Epidermal growth factor, Complement C3, Interleukin-4, Interleukin-1 alpha, Tubulointerstitial nephritis antigen, Transforming growth factor beta-1, Bone morphogenetic protein 7, and Osteopontin, Netrin-1, for one or more of risk stratification, staging, prognosis, classifying and monitoring of the renal status of a subject.

14. The use of claim 13, wherein the subject is suffering from an acute renal injury.

15. A method according to claim 6, wherein the increased or decreased likelihood of subsequent acute kidney injury, worsening stage of AKI, mortality, need for renal replacement therapy, need for withdrawal of renal toxins, end stage renal disease, heart failure, stroke, myocardial infarction, or chronic kidney disease assigned to the subject is a likelihood that an event of interest is more or less likely to occur within 30 days,
within 72 hours or
within 24 hours of the time at which the body fluid sample is obtained from the subject.

**Epidermal growth factor**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 21200.000 | 21000.000 | 21200.000 | 18250.000 | 21200.000 | 16400.000 |
| average | 29128.823 | 26307.472 | 29128.823 | 25123.823 | 29128.823 | 25655.556 |
| stdev | 26105.765 | 19448.924 | 26105.765 | 21623.583 | 26105.765 | 26288.037 |
| p (t-test) |  | 0.458 |  | 0.265 |  | 0.512 |
| min | 921.000 | 100.000 | 921.000 | 2370.000 | 921.000 | 1750.000 |
| max | 107000.000 | 88986.000 | 107000.000 | 109224.000 | 107000.000 | 91500.000 |
| n (Samp) | 248 | 53 | 248 | 62 | 248 | 27 |
| n (Pat) | 103 | 53 | 103 | 62 | 103 | 27 |

sCr only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 21450.000 | 9920.000 | 21450.000 | 14900.000 | 21450.000 | 13800.000 |
| average | 28545.714 | 16432.500 | 28545.714 | 19541.923 | 28545.714 | 17370.714 |
| stdev | 24861.585 | 15135.240 | 24861.585 | 18311.899 | 24861.585 | 12079.170 |
| p (t-test) |  | 0.031 |  | 0.070 |  | 0.095 |
| min | 100.000 | 2340.000 | 100.000 | 2370.000 | 100.000 | 2430.000 |
| max | 109224.000 | 65700.000 | 109224.000 | 69200.000 | 109224.000 | 45900.000 |
| n (Samp) | 440 | 20 | 440 | 26 | 440 | 14 |
| n (Pat) | 169 | 20 | 169 | 26 | 169 | 14 |

UO only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 17200.000 | 21000.000 | 17200.000 | 18400.000 | 17200.000 | 19700.000 |
| average | 26450.340 | 27525.660 | 26450.340 | 26293.788 | 26450.340 | 26647.200 |
| stdev | 24890.352 | 20439.864 | 24890.352 | 22281.043 | 24890.352 | 27017.196 |
| p (t-test) |  | 0.783 |  | 0.967 |  | 0.970 |
| min | 921.000 | 100.000 | 921.000 | 2610.000 | 921.000 | 1750.000 |
| max | 107000.000 | 88986.000 | 107000.000 | 109224.000 | 107000.000 | 91500.000 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.51 | 0.044 | 248 | 53 | 0.749 |
| 24 hours | 0.48 | 0.041 | 248 | 62 | 0.702 |
| 48 hours | 0.46 | 0.057 | 248 | 27 | 0.459 |

sCr only

| Time prior AKI stage | AUC | SE | nCohort 1 | nCohort 2 | p |
|---|---|---|---|---|---|
| 0 hours | 0.35 | 0.057 | 440 | 20 | 0.010 |
| 24 hours | 0.39 | 0.053 | 440 | 26 | 0.046 |
| 48 hours | 0.39 | 0.071 | 440 | 14 | 0.135 |

UO only

| Time prior AKI stage | AUC | SE | nCohort 1 | nCohort 2 | p |
|---|---|---|---|---|---|
| 0 hours | 0.56 | 0.047 | 212 | 47 | 0.228 |
| 24 hours | 0.53 | 0.045 | 212 | 52 | 0.486 |
| 48 hours | 0.49 | 0.061 | 212 | 25 | 0.885 |

sCr or UO

| Time prior AKI stage | Cutoff value | sens | spec | Quartile | OR | 95% CI of OR | |
|---|---|---|---|---|---|---|---|
| 0 hours | 11500 | 74% | 33% | 1 |  |  |  |
|  | 9950 | 81% | 31% | 2 | 3.5 | 2.3 | 5.5 |
|  | 7100 | 91% | 22% | 3 | 2.6 | 1.7 | 4.2 |
|  | 37300 | 21% | 70% | 4 | 1.5 | 0.9 | 2.5 |
|  | 50200 | 13% | 80% |  |  |  |  |
|  | 72400 | 6% | 90% |  |  |  |  |
| 24 hours | 11700 | 71% | 34% | 1 |  |  |  |

# FIG. 1 - 1

| Time prior AKI stage | Cutoff value | sens | spec | Quartile | OR | 95% CI of OR | |
|---|---|---|---|---|---|---|---|
| | 9950 | 81% | 31% | 2 | 2.1 | 1.4 | 3.0 |
| | 4170 | 90% | 10% | 3 | 2.8 | 2.0 | 4.0 |
| | 37300 | 19% | 70% | 4 | 1.1 | 0.7 | 1.7 |
| | 50200 | 11% | 80% | | | | |
| | 72400 | 3% | 90% | | | | |
| 48 hours | 9950 | 70% | 31% | 1 | | | |
| | 6520 | 81% | 21% | 2 | 1.2 | 0.6 | 2.7 |
| | 2990 | 93% | 6% | 3 | 1.9 | 1.0 | 3.8 |
| | 37300 | 19% | 70% | 4 | 1.5 | 0.7 | 3.1 |
| | 50200 | 15% | 80% | | | | |
| | 72400 | 11% | 90% | | | | |

sCr only

| Time prior AKI stage | Cutoff value | sens | spec | Quartile | OR | 95% CI of OR | |
|---|---|---|---|---|---|---|---|
| 0 hours | 9110 | 70% | 25% | 1 | | | |
| | 5950 | 80% | 16% | 2 | 5.2 | 0.5 | 56.4 |
| | 4310 | 90% | 10% | 3 | 8.5 | 0.9 | 80.1 |
| | 34800 | 5% | 70% | 4 | 6.3 | 0.6 | 64.0 |
| | 44600 | 5% | 80% | | | | |
| | 67100 | 0% | 90% | | | | |
| 24 hours | 6870 | 73% | 18% | 1 | | | |
| | 6380 | 81% | 17% | 2 | 1.4 | 0.4 | 4.4 |
| | 4170 | 92% | 10% | 3 | 3.2 | 1.3 | 7.8 |
| | 34800 | 15% | 70% | 4 | 3.6 | 1.5 | 8.7 |
| | 44600 | 12% | 80% | | | | |
| | 67100 | 4% | 90% | | | | |
| 48 hours | 11200 | 71% | 30% | 1 | | | |
| | 6080 | 86% | 16% | 2 | 3.1 | 0.2 | 43.6 |
| | 5460 | 93% | 14% | 3 | 7.4 | 0.8 | 72.0 |
| | 34800 | 7% | 70% | 4 | 3.1 | 0.2 | 43.6 |
| | 44600 | 7% | 80% | | | | |
| | 67100 | 0% | 90% | | | | |

UO only

| Time prior AKI stage | Cutoff value | sens | spec | Quartile | OR | 95% CI of OR | |
|---|---|---|---|---|---|---|---|
| 0 hours | 14300 | 70% | 43% | 1 | | | |
| | 10800 | 81% | 34% | 2 | 2.4 | 1.4 | 4.2 |
| | 6580 | 91% | 20% | 3 | 2.9 | 1.7 | 4.9 |
| | 30100 | 38% | 70% | 4 | 2.4 | 1.4 | 4.2 |
| | 43300 | 19% | 80% | | | | |
| | 66600 | 6% | 90% | | | | |
| 24 hours | 11700 | 71% | 36% | 1 | | | |
| | 10300 | 81% | 33% | 2 | 3.2 | 2.0 | 5.0 |
| | 4310 | 90% | 11% | 3 | 2.5 | 1.5 | 4.0 |
| | 30100 | 35% | 70% | 4 | 1.9 | 1.1 | 3.1 |
| | 43300 | 15% | 80% | | | | |
| | 66600 | 6% | 90% | | | | |
| 48 hours | 9410 | 72% | 31% | 1 | | | |
| | 6580 | 80% | 20% | 2 | 1.7 | 0.8 | 3.5 |
| | 2990 | 92% | 6% | 3 | 1.0 | 0.4 | 2.4 |
| | 30100 | 28% | 70% | 4 | 1.5 | 0.7 | 3.1 |
| | 43300 | 16% | 80% | | | | |
| | 66600 | 12% | 90% | | | | |

**FIG. 1 - 2**

**Interleukin-1 alpha**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.002 | 0.001 | 0.002 | 0.001 | 0.002 | 0.001 |
| average | 0.002 | 0.001 | 0.002 | 0.002 | 0.002 | 0.001 |
| stdev | 0.004 | 0.001 | 0.004 | 0.002 | 0.004 | 0.001 |
| p (t-test) |  | 0.042 |  | 0.318 |  | 0.250 |
| min | 0.000 | 0.000 | 0.000 | 0.000 | 0.000 | 0.000 |
| max | 0.038 | 0.005 | 0.038 | 0.015 | 0.038 | 0.004 |
| n (Samp) | 248 | 53 | 248 | 62 | 248 | 27 |
| n (Pat) | 103 | 53 | 103 | 62 | 103 | 27 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.001 | 0.002 | 0.001 | 0.002 | 0.001 | 0.002 |
| average | 0.002 | 0.002 | 0.002 | 0.002 | 0.002 | 0.002 |
| stdev | 0.004 | 0.003 | 0.004 | 0.002 | 0.004 | 0.001 |
| p (t-test) |  | 0.881 |  | 0.637 |  | 0.664 |
| min | 0.000 | 0.000 | 0.000 | 0.000 | 0.000 | 0.000 |
| max | 0.038 | 0.012 | 0.038 | 0.007 | 0.038 | 0.003 |
| n (Samp) | 440 | 20 | 440 | 26 | 440 | 14 |
| n (Pat) | 169 | 20 | 169 | 26 | 169 | 14 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.002 | 0.000 | 0.002 | 0.001 | 0.002 | 0.001 |
| average | 0.002 | 0.001 | 0.002 | 0.002 | 0.002 | 0.001 |
| stdev | 0.003 | 0.003 | 0.003 | 0.002 | 0.003 | 0.001 |
| p (t-test) |  | 0.176 |  | 0.320 |  | 0.345 |
| min | 0.000 | 0.000 | 0.000 | 0.000 | 0.000 | 0.000 |
| max | 0.033 | 0.017 | 0.033 | 0.015 | 0.033 | 0.004 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
|---|---|---|---|---|---|
| 0 hours | 0.38 | 0.040 | 248 | 53 | 0.003 |
| 24 hours | 0.44 | 0.040 | 248 | 62 | 0.112 |
| 48 hours | 0.43 | 0.056 | 248 | 27 | 0.204 |

sCr only

| Time prior AKI stage | AUC | SE | nCohort 1 | nCohort 2 | p |
|---|---|---|---|---|---|
| 0 hours | 0.55 | 0.068 | 440 | 20 | 0.504 |
| 24 hours | 0.60 | 0.060 | 440 | 26 | 0.106 |
| 48 hours | 0.52 | 0.079 | 440 | 14 | 0.816 |

UO only

| Time prior AKI stage | AUC | SE | nCohort 1 | nCohort 2 | p |
|---|---|---|---|---|---|
| 0 hours | 0.38 | 0.043 | 212 | 47 | 0.004 |
| 24 hours | 0.42 | 0.043 | 212 | 52 | 0.069 |
| 48 hours | 0.45 | 0.059 | 212 | 25 | 0.391 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 0.9 | 0.5 | 1.5 |
|  | 0 | 100% | 0% | 3 | 2.5 | 1.7 | 3.7 |
|  | 0.00249 | 17% | 70% | 4 | 2.2 | 1.5 | 3.2 |
|  | 0.003 | 11% | 81% |  |  |  |  |
|  | 0.00419 | 2% | 90% |  |  |  |  |
| 24 hours | 0 | 100% | 0% | 1 |  |  |  |

**FIG. 1 - 3**

| | Cutoff value | sens | spec | Quartile | OR | 95% CI of OR | |
|---|---|---|---|---|---|---|---|
| | 0 | 100% | 0% | 2 | 1.0 | 0.7 | 1.5 |
| | 0 | 100% | 0% | 3 | 2.2 | 1.6 | 3.0 |
| | 0.00249 | 21% | 70% | 4 | 1.4 | 1.0 | 2.0 |
| | 0.003 | 16% | 81% | | | | |
| | 0.00419 | 6% | 90% | | | | |
| 48 hours | 0 | 100% | 0% | 1 | | | |
| | 0 | 100% | 0% | 2 | 1.8 | 0.8 | 4.2 |
| | 0 | 100% | 0% | 3 | 3.1 | 1.5 | 6.4 |
| | 0.00249 | 15% | 70% | 4 | 1.3 | 0.5 | 3.3 |
| | 0.003 | 7% | 81% | | | | |
| | 0.00419 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0.000709 | 70% | 42% | 1 | | | |
| | 0 | 100% | 0% | 2 | 1.0 | 0.4 | 2.3 |
| | 0 | 100% | 0% | 3 | 0.6 | 0.2 | 1.7 |
| | 0.00231 | 35% | 70% | 4 | 1.4 | 0.7 | 2.9 |
| | 0.00288 | 20% | 80% | | | | |
| | 0.00382 | 15% | 90% | | | | |
| 24 hours | 0.00132 | 73% | 47% | 1 | | | |
| | 0 | 100% | 0% | 2 | 8.4 | 0.9 | 79.3 |
| | 0 | 100% | 0% | 3 | 8.5 | 0.9 | 80.0 |
| | 0.00231 | 38% | 70% | 4 | 9.6 | 1.0 | 87.6 |
| | 0.00288 | 35% | 80% | | | | |
| | 0.00382 | 19% | 90% | | | | |
| 48 hours | 0.00098 | 71% | 44% | 1 | | | |
| | 0 | 100% | 0% | 2 | na | na | na |
| | 0 | 100% | 0% | 3 | na | na | na |
| | 0.00231 | 36% | 70% | 4 | na | na | na |
| | 0.00288 | 14% | 80% | | | | |
| | 0.00382 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.9 | 0.5 | 1.6 |
| | 0 | 100% | 0% | 3 | 1.3 | 0.8 | 2.2 |
| | 0.00246 | 17% | 71% | 4 | 3.7 | 2.5 | 5.7 |
| | 0.00289 | 13% | 80% | | | | |
| | 0.00382 | 4% | 91% | | | | |
| 24 hours | 0 | 100% | 0% | 1 | | | |
| | 0 | 100% | 0% | 2 | 0.9 | 0.5 | 1.4 |
| | 0 | 100% | 0% | 3 | 1.1 | 0.7 | 1.7 |
| | 0.00246 | 21% | 71% | 4 | 2.8 | 1.9 | 4.0 |
| | 0.00289 | 17% | 80% | | | | |
| | 0.00382 | 6% | 91% | | | | |
| 48 hours | 0 | 100% | 0% | 1 | | | |
| | 0 | 100% | 0% | 2 | 3.0 | 1.1 | 7.9 |
| | 0 | 100% | 0% | 3 | 4.9 | 2.0 | 11.8 |
| | 0.00246 | 12% | 71% | 4 | 0.7 | 0.1 | 3.7 |
| | 0.00289 | 8% | 80% | | | | |
| | 0.00382 | 8% | 91% | | | | |

**FIG. 1 - 4**

**Interleukin-4**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 24.850 | 23.300 | 24.850 | 30.350 | 24.850 | 30.700 |
| average | 24.220 | 22.682 | 24.220 | 28.865 | 24.220 | 31.232 |
| stdev | 10.766 | 12.414 | 10.766 | 16.262 | 10.766 | 15.311 |
| p (t-test) | | 0.359 | | 0.007 | | 0.002 |
| min | 0.416 | 0.416 | 0.416 | 0.416 | 0.416 | 3.800 |
| max | 57.500 | 50.500 | 57.500 | 62.400 | 57.500 | 66.200 |
| n (Samp) | 248 | 53 | 248 | 62 | 248 | 27 |
| n (Pat) | 103 | 53 | 103 | 62 | 103 | 27 |

sCr only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 25.000 | 20.300 | 25.000 | 30.550 | 25.000 | 30.550 |
| average | 24.662 | 21.280 | 24.662 | 28.436 | 24.662 | 28.234 |
| stdev | 12.158 | 8.364 | 12.158 | 13.167 | 12.158 | 17.358 |
| p (t-test) | | 0.219 | | 0.126 | | 0.287 |
| min | 0.416 | 8.500 | 0.416 | 0.416 | 0.416 | 0.416 |
| max | 62.400 | 44.600 | 62.400 | 59.900 | 62.400 | 66.200 |
| n (Samp) | 440 | 20 | 440 | 26 | 440 | 14 |
| n (Pat) | 169 | 20 | 169 | 26 | 169 | 14 |

UO only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 25.100 | 24.200 | 25.100 | 32.150 | 25.100 | 29.700 |
| average | 24.341 | 22.423 | 24.341 | 29.692 | 24.341 | 31.292 |
| stdev | 10.969 | 13.530 | 10.969 | 16.254 | 10.969 | 12.377 |
| p (t-test) | | 0.301 | | 0.005 | | 0.003 |
| min | 0.416 | 0.416 | 0.416 | 0.416 | 0.416 | 3.800 |
| max | 57.500 | 50.500 | 57.500 | 62.400 | 57.500 | 61.000 |
| n (Samp) | 212 | 47 | 212 | 52 | 212 | 25 |
| n (Pat) | 85 | 47 | 85 | 52 | 85 | 25 |

sCr or UO

| Time prior AKI stage | AUC | SE | nCohort 1 | nCohort 2 | p |
| --- | --- | --- | --- | --- | --- |
| 0 hours | 0.47 | 0.043 | 248 | 53 | 0.473 |
| 24 hours | 0.60 | 0.042 | 248 | 62 | 0.018 |
| 48 hours | 0.65 | 0.060 | 248 | 27 | 0.010 |

sCr only

| Time prior AKI stage | AUC | SE | nCohort 1 | nCohort 2 | p |
| --- | --- | --- | --- | --- | --- |
| 0 hours | 0.38 | 0.059 | 440 | 20 | 0.049 |
| 24 hours | 0.60 | 0.060 | 440 | 26 | 0.110 |
| 48 hours | 0.58 | 0.081 | 440 | 14 | 0.322 |

UO only

| Time prior AKI stage | AUC | SE | nCohort 1 | nCohort 2 | p |
| --- | --- | --- | --- | --- | --- |
| 0 hours | 0.47 | 0.046 | 212 | 47 | 0.554 |
| 24 hours | 0.62 | 0.045 | 212 | 52 | 0.006 |
| 48 hours | 0.66 | 0.062 | 212 | 25 | 0.008 |

sCr or UO

| Time prior AKI stage | Cutoff value | sens | spec | Quartile | OR | 95% CI of OR | |
| --- | --- | --- | --- | --- | --- | --- | --- |
| 0 hours | 16.4 | 72% | 21% | 1 | | | |
| | 9.94 | 81% | 11% | 2 | 0.4 | 0.3 | 0.7 |
| | 3.8 | 92% | 4% | 3 | 0.8 | 0.6 | 1.1 |
| | 29.4 | 32% | 71% | 4 | 1.0 | 0.7 | 1.4 |
| | 32.4 | 26% | 81% | | | | |
| | 36.5 | 13% | 91% | | | | |
| 24 hours | 19.2 | 71% | 30% | 1 | | | |
| | 12.6 | 81% | 14% | 2 | 0.5 | 0.4 | 0.8 |

# FIG. 1 - 5

| | value | sens | spec | Quartile | OR | 95% CI of OR | |
|---|---|---|---|---|---|---|---|
| | 8.12 | 90% | 8% | 3 | 0.6 | 0.4 | 0.9 |
| | 29.4 | 53% | 71% | 4 | 2.3 | 1.8 | 3.0 |
| | 32.4 | 45% | 81% | | | | |
| | 36.5 | 29% | 91% | | | | |
| 48 hours | 25.6 | 70% | 56% | 1 | | | |
| | 20.3 | 81% | 34% | 2 | 1.0 | 0.3 | 2.8 |
| | 6.01 | 93% | 7% | 3 | 1.5 | 0.6 | 3.7 |
| | 29.4 | 56% | 71% | 4 | 3.7 | 1.8 | 7.5 |
| | 32.4 | 48% | 81% | | | | |
| | 36.5 | 26% | 91% | | | | |

sCr only

| Time prior AKI stage | Cutoff value | sens | spec | Quartile | OR | 95% CI of OR | |
|---|---|---|---|---|---|---|---|
| 0 hours | 17.8 | 70% | 26% | 1 | | | |
| | 15.7 | 80% | 21% | 2 | 0.7 | 0.1 | 3.5 |
| | 12.7 | 90% | 18% | 3 | 3.2 | 1.3 | 7.9 |
| | 30.6 | 15% | 71% | 4 | 2.1 | 0.7 | 5.7 |
| | 33.6 | 5% | 80% | | | | |
| | 40 | 5% | 90% | | | | |
| 24 hours | 23 | 73% | 43% | 1 | | | |
| | 18.4 | 81% | 28% | 2 | 0.8 | 0.3 | 2.0 |
| | 9.51 | 92% | 12% | 3 | 1.4 | 0.7 | 2.9 |
| | 30.6 | 46% | 71% | 4 | 2.1 | 1.1 | 3.9 |
| | 33.6 | 31% | 80% | | | | |
| | 40 | 15% | 90% | | | | |
| 48 hours | 20.6 | 71% | 34% | 1 | | | |
| | 6.01 | 86% | 10% | 2 | 0.7 | 0.1 | 3.5 |
| | 0.416 | 93% | 5% | 3 | 1.0 | 0.3 | 3.8 |
| | 30.6 | 50% | 71% | 4 | 2.0 | 0.7 | 5.6 |
| | 33.6 | 29% | 80% | | | | |
| | 40 | 21% | 90% | | | | |

UO only

| Time prior AKI stage | Cutoff value | sens | spec | Quartile | OR | 95% CI of OR | |
|---|---|---|---|---|---|---|---|
| 0 hours | 12.5 | 70% | 15% | 1 | | | |
| | 8.12 | 81% | 8% | 2 | 0.5 | 0.3 | 0.7 |
| | 0.416 | 91% | 4% | 3 | 0.4 | 0.2 | 0.7 |
| | 29.4 | 36% | 70% | 4 | 1.2 | 0.9 | 1.7 |
| | 32 | 30% | 80% | | | | |
| | 36.3 | 15% | 90% | | | | |
| 24 hours | 22.3 | 71% | 39% | 1 | | | |
| | 17.3 | 81% | 23% | 2 | 0.4 | 0.2 | 0.7 |
| | 0 | 100% | 0% | 3 | 0.5 | 0.3 | 0.8 |
| | 29.4 | 56% | 70% | 4 | 2.7 | 1.9 | 3.6 |
| | 32 | 50% | 80% | | | | |
| | 36.3 | 35% | 90% | | | | |
| 48 hours | 24.6 | 72% | 48% | 1 | | | |
| | 22.3 | 80% | 39% | 2 | 3.2 | 0.8 | 12.8 |
| | 20.1 | 92% | 31% | 3 | 2.6 | 0.6 | 11.2 |
| | 29.4 | 56% | 70% | 4 | 7.1 | 2.1 | 24.1 |
| | 32 | 48% | 80% | | | | |
| | 36.3 | 24% | 90% | | | | |

# FIG. 1 - 6

**Netrin-1**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 8.800 | 2.990 | 8.800 | 2.814 | 8.800 | 3.387 |
| stdev | 39.131 | 5.006 | 39.131 | 5.383 | 39.131 | 6.989 |
| p (t-test) |  | 0.293 |  | 0.253 |  | 0.484 |
| min | 0.000 | 0.211 | 0.000 | 0.000 | 0.000 | 0.000 |
| max | 418.455 | 21.067 | 418.455 | 26.584 | 418.455 | 31.601 |
| n (Samp) | 118 | 51 | 118 | 57 | 118 | 26 |
| n (Pat) | 99 | 51 | 99 | 57 | 99 | 26 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.211 | 0.211 | 0.211 | 1.611 | 0.211 | 0.211 |
| average | 5.955 | 1.008 | 5.955 | 2.990 | 5.955 | 3.605 |
| stdev | 26.936 | 2.929 | 26.936 | 4.754 | 26.936 | 8.568 |
| p (t-test) |  | 0.450 |  | 0.599 |  | 0.745 |
| min | 0.000 | 0.211 | 0.000 | 0.211 | 0.000 | 0.000 |
| max | 418.455 | 12.289 | 418.455 | 21.067 | 418.455 | 31.601 |
| n (Samp) | 257 | 17 | 257 | 23 | 257 | 14 |
| n (Pat) | 160 | 17 | 160 | 23 | 160 | 14 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.246 | 0.211 | 0.246 | 0.211 | 0.246 | 0.211 |
| average | 9.426 | 3.706 | 9.426 | 2.503 | 9.426 | 2.831 |
| stdev | 41.106 | 5.752 | 41.106 | 5.036 | 41.106 | 5.014 |
| p (t-test) |  | 0.355 |  | 0.248 |  | 0.445 |
| min | 0.000 | 0.000 | 0.000 | 0.000 | 0.000 | 0.000 |
| max | 418.455 | 21.067 | 418.455 | 26.584 | 418.455 | 19.312 |
| n (Samp) | 106 | 45 | 106 | 48 | 106 | 23 |
| n (Pat) | 84 | 45 | 84 | 48 | 84 | 23 |

sCr or UO

| Time prior AKI stage | AUC | SE | nCohort 1 | nCohort 2 | p |
|---|---|---|---|---|---|
| 0 hours | 0.47 | 0.048 | 118 | 51 | 0.484 |
| 24 hours | 0.47 | 0.046 | 118 | 57 | 0.468 |
| 48 hours | 0.46 | 0.061 | 118 | 26 | 0.495 |

sCr only

| Time prior AKI stage | AUC | SE | nCohort 1 | nCohort 2 | p |
|---|---|---|---|---|---|
| 0 hours | 0.34 | 0.061 | 257 | 17 | 0.008 |
| 24 hours | 0.54 | 0.064 | 257 | 23 | 0.503 |
| 48 hours | 0.38 | 0.071 | 257 | 14 | 0.082 |

UO only

| Time prior AKI stage | AUC | SE | nCohort 1 | nCohort 2 | p |
|---|---|---|---|---|---|
| 0 hours | 0.47 | 0.051 | 106 | 45 | 0.551 |
| 24 hours | 0.44 | 0.049 | 106 | 48 | 0.188 |
| 48 hours | 0.43 | 0.064 | 106 | 23 | 0.269 |

sCr or UO

| Time prior AKI stage | Cutoff value | sens | spec | Quartile | OR | 95% CI of OR | |
|---|---|---|---|---|---|---|---|
| 0 hours | 1.118E-14 | 100% | 3% | 1 |  |  |  |
|  | 1.118E-14 | 100% | 3% | 2 | 1.2 | 0.7 | 1.9 |
|  | 1.118E-14 | 100% | 3% | 3 | 1.8 | 1.2 | 2.9 |
|  | 3.2216495 | 27% | 70% | 4 | 1.8 | 1.2 | 2.9 |
|  | 11.363636 | 10% | 81% |  |  |  |  |
|  | 21.067416 | 0% | 92% |  |  |  |  |
| 24 hours | 1.118E-14 | 98% | 3% | 1 |  |  |  |
|  | 1.118E-14 | 98% | 3% | 2 | 1.4 | 0.9 | 2.2 |

## FIG. 1 - 7

70

| Time prior AKI stage | Cutoff value | sens | spec | Quartile | OR | 95% CI of OR | |
|---|---|---|---|---|---|---|---|
| | 1.118E-14 | 98% | 3% | 3 | 1.7 | 1.1 | 2.6 |
| | 3.2216495 | 26% | 70% | 4 | 1.8 | 1.2 | 2.7 |
| | 11.363636 | 7% | 81% | | | | |
| | 21.067416 | 2% | 92% | | | | |
| 48 hours | 1.118E-14 | 88% | 3% | 1 | | | |
| | 1.118E-14 | 88% | 3% | 2 | 2.3 | 1.0 | 5.4 |
| | 0 | 100% | 0% | 3 | 0.2 | 0.0 | 3.0 |
| | 3.2216495 | 27% | 70% | 4 | 4.5 | 2.1 | 9.9 |
| | 11.363636 | 8% | 81% | | | | |
| | 21.067416 | 4% | 92% | | | | |

sCr only

| Time prior AKI stage | Cutoff value | sens | spec | Quartile | OR | 95% CI of OR | |
|---|---|---|---|---|---|---|---|
| 0 hours | 1.118E-14 | 100% | 3% | 1 | | | |
| | 1.118E-14 | 100% | 3% | 2 | 1.0 | 0.0 | 54.2 |
| | 1.118E-14 | 100% | 3% | 3 | 4.2 | 0.3 | 51.4 |
| | 3.0857271 | 6% | 70% | 4 | 13.1 | 1.5 | 118.6 |
| | 7.5954861 | 6% | 80% | | | | |
| | 15.800562 | 0% | 90% | | | | |
| 24 hours | 1.118E-14 | 100% | 3% | 1 | | | |
| | 1.118E-14 | 100% | 3% | 2 | 0.0 | 0.0 | na |
| | 1.118E-14 | 100% | 3% | 3 | 1.1 | 0.7 | 1.8 |
| | 3.0857271 | 35% | 70% | 4 | 0.4 | 0.2 | 0.9 |
| | 7.5954861 | 9% | 80% | | | | |
| | 15.800562 | 4% | 90% | | | | |
| 48 hours | 1.118E-14 | 79% | 3% | 1 | | | |
| | 0 | 100% | 0% | 2 | 0.3 | 0.0 | 4.7 |
| | 0 | 100% | 0% | 3 | 0.3 | 0.0 | 4.7 |
| | 3.0857271 | 21% | 70% | 4 | 3.4 | 1.3 | 8.6 |
| | 7.5954861 | 14% | 80% | | | | |
| | 15.800562 | 7% | 90% | | | | |

UO only

| Time prior AKI stage | Cutoff value | sens | spec | Quartile | OR | 95% CI of OR | |
|---|---|---|---|---|---|---|---|
| 0 hours | 1.118E-14 | 98% | 3% | 1 | | | |
| | 1.118E-14 | 98% | 3% | 2 | 1.5 | 0.9 | 2.5 |
| | 1.118E-14 | 98% | 3% | 3 | 1.2 | 0.7 | 2.0 |
| | 4.4883303 | 24% | 71% | 4 | 2.0 | 1.2 | 3.3 |
| | 12.289326 | 11% | 80% | | | | |
| | 21.067416 | 0% | 92% | | | | |
| 24 hours | 1.118E-14 | 98% | 3% | 1 | | | |
| | 1.118E-14 | 98% | 3% | 2 | 2.7 | 1.5 | 4.7 |
| | 1.118E-14 | 98% | 3% | 3 | 2.0 | 1.1 | 3.6 |
| | 4.4883303 | 15% | 71% | 4 | 3.0 | 1.7 | 5.2 |
| | 12.289326 | 4% | 80% | | | | |
| | 21.067416 | 2% | 92% | | | | |
| 48 hours | 1.118E-14 | 91% | 3% | 1 | | | |
| | 1.118E-14 | 91% | 3% | 2 | 3.3 | 1.2 | 9.5 |
| | 1.118E-14 | 91% | 3% | 3 | 4.5 | 1.7 | 12.4 |
| | 4.4883303 | 17% | 71% | 4 | 0.7 | 0.1 | 3.9 |
| | 12.289326 | 9% | 80% | | | | |
| | 21.067416 | 0% | 92% | | | | |

# FIG. 1 - 8

**Osteopontin**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 404845.234 | 401338.489 | 404845.234 | 447313.763 | 404845.234 | 33028.494 |
| average | 387716.320 | 407611.236 | 387716.320 | 424081.472 | 387716.320 | 256742.220 |
| stdev | 161977.983 | 141972.031 | 161977.983 | 153858.743 | 161977.983 | na |
| p (t-test) | | 0.626 | | 0.367 | | na |
| min | 33028.494 | 113938.640 | 33028.494 | 94230.433 | 33028.494 | 256742.220 |
| max | 729905.544 | 656561.188 | 729905.544 | 677771.212 | 729905.544 | 256742.220 |
| n (Samp) | 51 | 21 | 51 | 23 | 51 | 1 |
| n (Pat) | 40 | 21 | 40 | 23 | 40 | 1 |

sCr only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 425821.608 | 400506.412 | 425821.608 | 400366.819 | 425821.608 | 414928.520 |
| average | 405335.794 | 396500.431 | 405335.794 | 416501.814 | 405335.794 | 414928.520 |
| stdev | 161298.780 | 115861.924 | 161298.780 | 206933.501 | 161298.780 | 107724.720 |
| p (t-test) | | 0.895 | | 0.863 | | 0.934 |
| min | 33028.494 | 205200.154 | 33028.494 | 139179.187 | 33028.494 | 338755.640 |
| max | 729905.544 | 553924.484 | 729905.544 | 677771.212 | 729905.544 | 491101.400 |
| n (Samp) | 93 | 6 | 93 | 7 | 93 | 2 |
| n (Pat) | 73 | 6 | 73 | 7 | 73 | 2 |

UO only

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 319607.582 | 402738.482 | 319607.582 | 487336.350 | 319607.582 | 370747.605 |
| average | 367969.008 | 412651.333 | 367969.008 | 468151.417 | 367969.008 | 378110.290 |
| stdev | 159652.206 | 149454.437 | 159652.206 | 151489.722 | 159652.206 | 105689.231 |
| p (t-test) | | 0.337 | | 0.020 | | 0.902 |
| min | 33028.494 | 113938.640 | 33028.494 | 94230.433 | 33028.494 | 256742.220 |
| max | 729905.544 | 656561.188 | 729905.544 | 750295.504 | 729905.544 | 514203.729 |
| n (Samp) | 42 | 16 | 42 | 21 | 42 | 4 |
| n (Pat) | 33 | 16 | 33 | 21 | 33 | 4 |

sCr or UO

| Time prior AKI stage | AUC | SE | nCohort 1 | nCohort 2 | p |
| --- | --- | --- | --- | --- | --- |
| 0 hours | 0.54 | 0.076 | 51 | 21 | 0.559 |
| 24 hours | 0.58 | 0.073 | 51 | 23 | 0.287 |
| 48 hours | 0.22 | 0.181 | 51 | 1 | 0.116 |

sCr only

| Time prior AKI stage | AUC | SE | nCohort 1 | nCohort 2 | p |
| --- | --- | --- | --- | --- | --- |
| 0 hours | 0.46 | 0.119 | 93 | 6 | 0.729 |
| 24 hours | 0.50 | 0.114 | 93 | 7 | 0.984 |
| 48 hours | 0.50 | 0.207 | 93 | 2 | 1.000 |

UO only

| Time prior AKI stage | AUC | SE | nCohort 1 | nCohort 2 | p |
| --- | --- | --- | --- | --- | --- |
| 0 hours | 0.60 | 0.086 | 42 | 16 | 0.232 |
| 24 hours | 0.70 | 0.074 | 42 | 21 | 0.008 |
| 48 hours | 0.54 | 0.155 | 42 | 4 | 0.789 |

sCr or UO

| Time prior AKI stage | Cutoff value | sens | spec | Quartile | OR | 95% CI of OR | |
| --- | --- | --- | --- | --- | --- | --- | --- |
| 0 hours | 340143.98 | 71% | 43% | 1 | | | |
| | 328095.34 | 81% | 43% | 2 | 2.2 | 0.8 | 6.6 |
| | 200846.51 | 90% | 12% | 3 | 1.0 | 0.3 | 3.5 |
| | 483256.13 | 38% | 71% | 4 | 1.8 | 0.6 | 5.4 |
| | 510459.45 | 29% | 80% | | | | |
| | 595894.54 | 5% | 90% | | | | |
| 24 hours | 328095.34 | 74% | 43% | 1 | | | |
| | 269008.11 | 83% | 25% | 2 | 0.7 | 0.2 | 2.2 |

## FIG. 1 - 9

| Time prior AKI stage | Cutoff value | sens | spec | Quartile | OR | 95% CI of OR | |
|---|---|---|---|---|---|---|---|
| | 250376.36 | 91% | 22% | 3 | 1.7 | 0.6 | 4.5 |
| | 483256.13 | 43% | 71% | 4 | 1.5 | 0.6 | 4.1 |
| | 510459.45 | 30% | 80% | | | | |
| | 595894.54 | 9% | 90% | | | | |
| 48 hours | 250376.36 | 100% | 22% | 1 | | | |
| | 250376.36 | 100% | 22% | 2 | na | na | na |
| | 250376.36 | 100% | 22% | 3 | na | na | na |
| | 483256.13 | 0% | 71% | 4 | na | na | na |
| | 510459.45 | 0% | 80% | | | | |
| | 595894.54 | 0% | 90% | | | | |

sCr only

| Time prior AKI stage | Cutoff value | sens | spec | Quartile | OR | 95% CI of OR | |
|---|---|---|---|---|---|---|---|
| 0 hours | 353219.39 | 83% | 35% | 1 | | | |
| | 353219.39 | 83% | 35% | 2 | 2.1 | 0.1 | 46.6 |
| | 200846.51 | 100% | 12% | 3 | 2.1 | 0.1 | 46.6 |
| | 499943.54 | 17% | 71% | 4 | 1.0 | 0.0 | 62.1 |
| | 546506.06 | 17% | 81% | | | | |
| | 593762.72 | 0% | 90% | | | | |
| 24 hours | 269008.11 | 71% | 23% | 1 | | | |
| | 250376.36 | 86% | 18% | 2 | 0.5 | 0.0 | 10.7 |
| | 138277.07 | 100% | 9% | 3 | 0.5 | 0.0 | 10.7 |
| | 499943.54 | 29% | 71% | 4 | 1.6 | 0.3 | 9.6 |
| | 546506.06 | 29% | 81% | | | | |
| | 593762.72 | 29% | 90% | | | | |
| 48 hours | 328095.34 | 100% | 33% | 1 | | | |
| | 328095.34 | 100% | 33% | 2 | na | na | na |
| | 328095.34 | 100% | 33% | 3 | na | na | na |
| | 499943.54 | 0% | 71% | 4 | na | na | na |
| | 546506.06 | 0% | 81% | | | | |
| | 593762.72 | 0% | 90% | | | | |

UO only

| Time prior AKI stage | Cutoff value | sens | spec | Quartile | OR | 95% CI of OR | |
|---|---|---|---|---|---|---|---|
| 0 hours | 338755.64 | 75% | 52% | 1 | | | |
| | 328095.34 | 81% | 52% | 2 | 0.9 | 0.2 | 4.8 |
| | 171339.12 | 94% | 10% | 3 | 1.0 | 0.2 | 5.3 |
| | 456794.78 | 44% | 71% | 4 | 3.2 | 0.8 | 12.5 |
| | 492543.32 | 44% | 81% | | | | |
| | 553924.48 | 19% | 90% | | | | |
| 24 hours | 409417.34 | 71% | 60% | 1 | | | |
| | 349742.27 | 81% | 52% | 2 | 2.2 | 0.4 | 12.9 |
| | 297511.13 | 90% | 40% | 3 | 5.1 | 1.0 | 25.8 |
| | 456794.78 | 57% | 71% | 4 | 6.5 | 1.3 | 32.9 |
| | 492543.32 | 43% | 81% | | | | |
| | 553924.48 | 29% | 90% | | | | |
| 48 hours | 328095.34 | 75% | 52% | 1 | | | |
| | 255537.24 | 100% | 24% | 2 | 0.0 | 0.0 | na |
| | 255537.24 | 100% | 24% | 3 | 2.2 | 0.1 | 63.6 |
| | 456794.78 | 25% | 71% | 4 | 0.9 | 0.0 | 66.6 |
| | 492543.32 | 25% | 81% | | | | |
| | 553924.48 | 0% | 90% | | | | |

# FIG. 1 - 10

**Transforming growth factor beta-1**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 15.679 | 7.682 | 15.679 | 0.036 | 15.679 | 0.036 |
| average | 29.109 | 51.017 | 29.109 | 8.162 | 29.109 | 2.792 |
| stdev | 38.178 | 76.676 | 38.178 | na | 38.178 | na |
| p (t-test) | | 0.142 | | na | | na |
| min | 0.036 | 0.036 | 0.036 | 8.162 | 0.036 | 2.792 |
| max | 221.429 | 270.067 | 221.429 | 8.162 | 221.429 | 2.792 |
| n (Samp) | 54 | 13 | 54 | 1 | 54 | 1 |
| n (Pat) | 36 | 13 | 36 | 1 | 36 | 1 |

sCr only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 13.531 | 58.494 | 13.531 | 7.088 | 13.531 | 15.538 |
| average | 32.165 | 65.959 | 32.165 | 29.914 | 32.165 | 15.538 |
| stdev | 45.733 | 39.773 | 45.733 | 43.309 | 45.733 | 16.048 |
| p (t-test) | | 0.081 | | 0.933 | | 0.611 |
| min | 0.036 | 7.682 | 0.036 | 2.792 | 0.036 | 4.190 |
| max | 270.067 | 111.111 | 270.067 | 79.861 | 270.067 | 26.885 |
| n (Samp) | 86 | 6 | 86 | 3 | 86 | 2 |
| n (Pat) | 60 | 6 | 60 | 3 | 60 | 2 |

UO only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 12.046 | 7.385 | 12.046 | 34.529 | 12.046 | 0.036 |
| average | 27.503 | 50.461 | 27.503 | 34.529 | 27.503 | 2.792 |
| stdev | 41.364 | 80.041 | 41.364 | 37.290 | 41.364 | na |
| p (t-test) | | 0.189 | | 0.815 | | na |
| min | 0.036 | 0.036 | 0.036 | 8.162 | 0.036 | 2.792 |
| max | 221.429 | 270.067 | 221.429 | 60.897 | 221.429 | 2.792 |
| n (Samp) | 40 | 12 | 40 | 2 | 40 | 1 |
| n (Pat) | 26 | 12 | 26 | 2 | 26 | 1 |

sCr or UO

| Time prior AKI stage | AUC | SE | nCohort 1 | nCohort 2 | p |
| --- | --- | --- | --- | --- | --- |
| 0 hours | 0.53 | 0.091 | 54 | 13 | 0.712 |
| 24 hours | 0.36 | 0.254 | 54 | 1 | 0.584 |
| 48 hours | 0.27 | 0.211 | 54 | 1 | 0.272 |

sCr only

| Time prior AKI stage | AUC | SE | nCohort 1 | nCohort 2 | p |
| --- | --- | --- | --- | --- | --- |
| 0 hours | 0.78 | 0.113 | 86 | 6 | 0.012 |
| 24 hours | 0.50 | 0.170 | 86 | 3 | 1.000 |
| 48 hours | 0.46 | 0.202 | 86 | 2 | 0.852 |

UO only

| Time prior AKI stage | AUC | SE | nCohort 1 | nCohort 2 | p |
| --- | --- | --- | --- | --- | --- |
| 0 hours | 0.52 | 0.097 | 40 | 12 | 0.804 |
| 24 hours | 0.63 | 0.218 | 40 | 2 | 0.567 |
| 48 hours | 0.30 | 0.229 | 40 | 1 | 0.383 |

sCr or UO

| Time prior AKI stage | Cutoff value | sens | spec | Quartile | OR | 95% CI of OR | |
| --- | --- | --- | --- | --- | --- | --- | --- |
| 0 hours | 2.7920962 | 77% | 28% | 1 | | | |
| | 0.6443299 | 85% | 15% | 2 | 1.3 | 0.3 | 5.7 |
| | 0 | 100% | 0% | 3 | 0.3 | 0.0 | 4.9 |
| | 33.653846 | 46% | 70% | 4 | 1.8 | 0.5 | 7.0 |
| | 54.487179 | 31% | 81% | | | | |
| | 74.652778 | 23% | 91% | | | | |
| 24 hours | 6.0137457 | 100% | 35% | 1 | | | |
| | 6.0137457 | 100% | 35% | 2 | na | na | na |

# FIG. 1 - 11

| Time prior AKI stage | Cutoff value | sens | spec | Quartile | OR | 95% CI of OR | |
|---|---|---|---|---|---|---|---|
| | 6.0137457 | 100% | 35% | 3 | na | na | na |
| | 33.653846 | 0% | 70% | 4 | na | na | na |
| | 54.487179 | 0% | 81% | | | | |
| | 74.652778 | 0% | 91% | | | | |
| 48 hours | 2.4441341 | 100% | 26% | 1 | | | |
| | 2.4441341 | 100% | 26% | 2 | na | na | na |
| | 2.4441341 | 100% | 26% | 3 | na | na | na |
| | 33.653846 | 0% | 70% | 4 | na | na | na |
| | 54.487179 | 0% | 81% | | | | |
| | 74.652778 | 0% | 91% | | | | |

sCr only

| Time prior AKI stage | Cutoff value | sens | spec | Quartile | OR | 95% CI of OR | |
|---|---|---|---|---|---|---|---|
| 0 hours | 48.076923 | 83% | 77% | 1 | | | |
| | 48.076923 | 83% | 77% | 2 | na | na | na |
| | 7.0876289 | 100% | 36% | 3 | na | na | na |
| | 35.25641 | 83% | 71% | 4 | na | na | na |
| | 59.294872 | 50% | 80% | | | | |
| | 83.333333 | 33% | 91% | | | | |
| 24 hours | 2.4441341 | 100% | 26% | 1 | | | |
| | 2.4441341 | 100% | 26% | 2 | 0.0 | 0.0 | na |
| | 2.4441341 | 100% | 26% | 3 | 2.2 | 0.1 | 50.2 |
| | 35.25641 | 33% | 71% | 4 | 0.0 | 0.0 | na |
| | 59.294872 | 33% | 80% | | | | |
| | 83.333333 | 0% | 91% | | | | |
| 48 hours | 2.7920962 | 100% | 29% | 1 | | | |
| | 2.7920962 | 100% | 29% | 2 | na | na | na |
| | 2.7920962 | 100% | 29% | 3 | na | na | na |
| | 35.25641 | 0% | 71% | 4 | na | na | na |
| | 59.294872 | 0% | 80% | | | | |
| | 83.333333 | 0% | 91% | | | | |

UO only

| Time prior AKI stage | Cutoff value | sens | spec | Quartile | OR | 95% CI of OR | |
|---|---|---|---|---|---|---|---|
| 0 hours | 2.7920962 | 75% | 33% | 1 | | | |
| | 0.0360285 | 83% | 13% | 2 | 1.5 | 0.3 | 7.0 |
| | 0 | 100% | 0% | 3 | 0.3 | 0.0 | 5.4 |
| | 30.377095 | 42% | 73% | 4 | 1.5 | 0.3 | 7.0 |
| | 41.666667 | 33% | 80% | | | | |
| | 74.652778 | 25% | 90% | | | | |
| 24 hours | 6.0137457 | 100% | 40% | 1 | | | |
| | 6.0137457 | 100% | 40% | 2 | na | na | na |
| | 6.0137457 | 100% | 40% | 3 | na | na | na |
| | 30.377095 | 50% | 73% | 4 | na | na | na |
| | 41.666667 | 50% | 80% | | | | |
| | 74.652778 | 0% | 90% | | | | |
| 48 hours | 2.4441341 | 100% | 28% | 1 | | | |
| | 2.4441341 | 100% | 28% | 2 | na | na | na |
| | 2.4441341 | 100% | 28% | 3 | na | na | na |
| | 30.377095 | 0% | 73% | 4 | na | na | na |
| | 41.666667 | 0% | 80% | | | | |
| | 74.652778 | 0% | 90% | | | | |

# FIG. 1 - 12

**Bone morphogenetic protein 7**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.275 | 0.163 | 0.275 | 4.405 | 0.275 | 0.219 |
| average | 2.870 | 0.363 | 2.870 | 6.126 | 2.870 | 0.219 |
| stdev | 5.205 | 0.522 | 5.205 | 3.442 | 5.205 | 0.079 |
| p (t-test) |  | 0.179 |  | 0.221 |  | 0.476 |
| min | 0.163 | 0.163 | 0.163 | 4.405 | 0.163 | 0.163 |
| max | 22.302 | 1.652 | 22.302 | 11.289 | 22.302 | 0.275 |
| n (Samp) | 77 | 8 | 77 | 4 | 77 | 2 |
| n (Pat) | 55 | 8 | 55 | 4 | 55 | 2 |

sCr only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.275 | 0.163 | 0.275 | 4.405 | 0.275 | 0.964 |
| average | 2.566 | 0.275 | 2.566 | 4.405 | 2.566 | 0.964 |
| stdev | 4.891 | na | 4.891 | 0.000 | 4.891 | 0.973 |
| p (t-test) |  | na |  | 0.598 |  | 0.646 |
| min | 0.163 | 0.275 | 0.163 | 4.405 | 0.163 | 0.275 |
| max | 22.302 | 0.275 | 22.302 | 4.405 | 22.302 | 1.652 |
| n (Samp) | 94 | 1 | 94 | 2 | 94 | 2 |
| n (Pat) | 67 | 1 | 67 | 2 | 67 | 2 |

UO only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.275 | 0.163 | 0.275 | 7.847 | 0.275 | 0.163 |
| average | 2.791 | 0.363 | 2.791 | 7.847 | 2.791 | 0.163 |
| stdev | 4.973 | 0.522 | 4.973 | 4.867 | 4.973 | na |
| p (t-test) |  | 0.175 |  | 0.162 |  | na |
| min | 0.163 | 0.163 | 0.163 | 4.405 | 0.163 | 0.163 |
| max | 20.925 | 1.652 | 20.925 | 11.289 | 20.925 | 0.163 |
| n (Samp) | 60 | 8 | 60 | 2 | 60 | 1 |
| n (Pat) | 42 | 8 | 42 | 2 | 42 | 1 |

sCr or UO

| Time prior AKI stage | AUC | SE | nCohort 1 | nCohort 2 | p |
|---|---|---|---|---|---|
| 0 hours | 0.32 | 0.089 | 77 | 8 | 0.045 |
| 24 hours | 0.82 | 0.131 | 77 | 4 | 0.015 |
| 48 hours | 0.38 | 0.185 | 77 | 2 | 0.515 |

sCr only

| Time prior AKI stage | AUC | SE | nCohort 1 | nCohort 2 | p |
|---|---|---|---|---|---|
| 0 hours | 0.56 | 0.300 | 94 | 1 | 0.845 |
| 24 hours | 0.81 | 0.186 | 94 | 2 | 0.091 |
| 48 hours | 0.63 | 0.215 | 94 | 2 | 0.536 |

UO only

| Time prior AKI stage | AUC | SE | nCohort 1 | nCohort 2 | p |
|---|---|---|---|---|---|
| 0 hours | 0.33 | 0.093 | 60 | 8 | 0.070 |
| 24 hours | 0.85 | 0.171 | 60 | 2 | 0.039 |
| 48 hours | 0.24 | 0.195 | 60 | 1 | 0.186 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 1.1 | 0.0 | 64.1 |
|  | 0 | 100% | 0% | 3 | 6.6 | 0.5 | 85.6 |
|  | 1.6519824 | 0% | 74% | 4 | 1.1 | 0.0 | 64.1 |
|  | 4.4052863 | 0% | 83% |  |  |  |  |
|  | 11.288546 | 0% | 91% |  |  |  |  |
| 24 hours | 3.0286344 | 100% | 75% | 1 |  |  |  |
|  | 3.0286344 | 100% | 75% | 2 | na | na | na |
|  | 3.0286344 | 100% | 75% | 3 | na | na | na |
|  | 1.6519824 | 100% | 74% | 4 | na | na | na |
|  | 4.4052863 | 25% | 83% |  |  |  |  |

## FIG. 2 - 1

| | 11.288546 | 0% | 91% | | | | |
|---|---|---|---|---|---|---|---|
| 48 hours | 0 | 100% | 0% | 1 | | | |
| | 0 | 100% | 0% | 2 | na | na | na |
| | 0 | 100% | 0% | 3 | na | na | na |
| | 1.6519824 | 0% | 74% | 4 | na | na | na |
| | 4.4052863 | 0% | 83% | | | | |
| | 11.288546 | 0% | 91% | | | | |

**Complement C3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.000 | 0.000 | 0.000 | 0.000 | 0.000 | 0.000 |
| average | 0.000 | 0.000 | 0.000 | 0.000 | 0.000 | 0.000 |
| stdev | 0.000 | 0.000 | 0.000 | 0.000 | 0.000 | 0.000 |
| p (t-test) | | 0.458 | | 0.968 | | 0.892 |
| min | 0.000 | 0.000 | 0.000 | 0.000 | 0.000 | 0.000 |
| max | 0.002 | 0.002 | 0.002 | 0.001 | 0.002 | 0.002 |
| n (Samp) | 419 | 27 | 419 | 36 | 419 | 18 |
| n (Pat) | 164 | 27 | 164 | 36 | 164 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.000 | 0.000 | 0.000 | 0.000 | 0.000 | 0.000 |
| average | 0.000 | 0.000 | 0.000 | 0.000 | 0.000 | 0.000 |
| stdev | 0.000 | 0.000 | 0.000 | 0.000 | 0.000 | 0.000 |
| p (t-test) | | 0.410 | | 0.860 | | 0.540 |
| min | 0.000 | 0.000 | 0.000 | 0.000 | 0.000 | 0.000 |
| max | 0.002 | 0.000 | 0.002 | 0.001 | 0.002 | 0.000 |
| n (Samp) | 518 | 5 | 518 | 9 | 518 | 7 |
| n (Pat) | 199 | 5 | 199 | 9 | 199 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.000 | 0.000 | 0.000 | 0.000 | 0.000 | 0.000 |
| average | 0.000 | 0.000 | 0.000 | 0.000 | 0.000 | 0.000 |
| stdev | 0.000 | 0.000 | 0.000 | 0.000 | 0.000 | 0.000 |
| p (t-test) | | 0.483 | | 0.727 | | 0.575 |
| min | 0.000 | 0.000 | 0.000 | 0.000 | 0.000 | 0.000 |
| max | 0.002 | 0.002 | 0.002 | 0.001 | 0.002 | 0.002 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.52 | 0.058 | 419 | 27 | 0.780 |
| 24 hours | 0.53 | 0.051 | 419 | 36 | 0.560 |
| 48 hours | 0.51 | 0.070 | 419 | 18 | 0.885 |

sCr only

| Time prior AKI stage | AUC | SE | nCohort 1 | nCohort 2 | p |
|---|---|---|---|---|---|
| 0 hours | 0.39 | 0.116 | 518 | 5 | 0.329 |
| 24 hours | 0.49 | 0.096 | 518 | 9 | 0.916 |
| 48 hours | 0.53 | 0.112 | 518 | 7 | 0.757 |

UO only

| Time prior AKI stage | AUC | SE | nCohort 1 | nCohort 2 | p |
|---|---|---|---|---|---|
| 0 hours | 0.53 | 0.060 | 352 | 26 | 0.572 |
| 24 hours | 0.54 | 0.056 | 352 | 30 | 0.530 |
| 48 hours | 0.52 | 0.075 | 352 | 16 | 0.761 |

sCr or UO

| Time prior AKI stage | Cutoff value | sens | spec | Quartile | OR | 95% CI of OR |
|---|---|---|---|---|---|---|

**FIG. 2 - 2**

| | Cutoff value | sens | spec | Quartile | OR | 95% CI of OR | |
|---|---|---|---|---|---|---|---|
| 0 hours | 0.0000522 | 70% | 37% | 1 | | | |
| | 0.0000402 | 81% | 30% | 2 | 5.9 | 1.8 | 19.6 |
| | 0.0000372 | 93% | 28% | 3 | 3.1 | 0.8 | 11.9 |
| | 0.000173 | 30% | 70% | 4 | 4.2 | 1.2 | 14.8 |
| | 0.000276 | 11% | 80% | | | | |
| | 0.000736 | 4% | 90% | | | | |
| 24 hours | 0.0000525 | 72% | 37% | 1 | | | |
| | 0.0000384 | 81% | 29% | 2 | 1.9 | 1.1 | 3.3 |
| | 8.97E-06 | 92% | 6% | 3 | 1.5 | 0.9 | 2.7 |
| | 0.000173 | 33% | 70% | 4 | 1.7 | 1.0 | 3.0 |
| | 0.000276 | 25% | 80% | | | | |
| | 0.000736 | 8% | 90% | | | | |
| 48 hours | 0.000058 | 72% | 40% | 1 | | | |
| | 0.0000212 | 83% | 17% | 2 | 1.5 | 0.7 | 3.6 |
| | 0.000018 | 94% | 14% | 3 | 1.0 | 0.4 | 2.8 |
| | 0.000173 | 33% | 70% | 4 | 1.0 | 0.4 | 2.7 |
| | 0.000276 | 17% | 80% | | | | |
| | 0.000736 | 11% | 90% | | | | |

sCr only

| Time prior AKI stage | Cutoff value | sens | spec | Quartile | OR | 95% CI of OR | |
|---|---|---|---|---|---|---|---|
| 0 hours | 0.0000402 | 80% | 28% | 1 | | | |
| | 0.0000402 | 80% | 28% | 2 | 1.0 | 0.0 | 51.9 |
| | 0.0000025 | 100% | 0% | 3 | 2.0 | 0.1 | 39.3 |
| | 0.000171 | 20% | 70% | 4 | 1.0 | 0.0 | 52.4 |
| | 0.000276 | 0% | 80% | | | | |
| | 0.000684 | 0% | 90% | | | | |
| 24 hours | 0.0000373 | 78% | 27% | 1 | | | |
| | 0.0000212 | 89% | 16% | 2 | 0.3 | 0.0 | 4.6 |
| | 7.57E-06 | 100% | 4% | 3 | 1.0 | 0.3 | 3.8 |
| | 0.000171 | 33% | 70% | 4 | 0.7 | 0.1 | 3.5 |
| | 0.000276 | 22% | 80% | | | | |
| | 0.000684 | 22% | 90% | | | | |
| 48 hours | 0.0000802 | 71% | 48% | 1 | | | |
| | 0.000074 | 86% | 46% | 2 | 3.0 | 0.2 | 42.9 |
| | 0.0000212 | 100% | 16% | 3 | 1.0 | 0.0 | 51.9 |
| | 0.000171 | 29% | 70% | 4 | 2.0 | 0.1 | 39.0 |
| | 0.000276 | 14% | 80% | | | | |
| | 0.000684 | 0% | 90% | | | | |

UO only

| Time prior AKI stage | Cutoff value | sens | spec | Quartile | OR | 95% CI of OR | |
|---|---|---|---|---|---|---|---|
| 0 hours | 0.000064 | 73% | 41% | 1 | | | |
| | 0.0000481 | 81% | 35% | 2 | 5.4 | 1.6 | 18.3 |
| | 0.0000368 | 92% | 28% | 3 | 4.3 | 1.2 | 15.2 |
| | 0.000181 | 31% | 70% | 4 | 3.1 | 0.8 | 12.0 |
| | 0.000307 | 4% | 80% | | | | |
| | 0.000736 | 4% | 90% | | | | |
| 24 hours | 0.0000617 | 70% | 41% | 1 | | | |
| | 0.000045 | 80% | 33% | 2 | 2.6 | 1.3 | 5.5 |
| | 0.00003 | 90% | 23% | 3 | 2.1 | 1.0 | 4.6 |
| | 0.000181 | 30% | 70% | 4 | 2.1 | 0.9 | 4.5 |
| | 0.000307 | 23% | 80% | | | | |
| | 0.000736 | 7% | 90% | | | | |
| 48 hours | 0.0000406 | 75% | 30% | 1 | | | |
| | 0.0000402 | 81% | 29% | 2 | 2.1 | 0.7 | 5.8 |
| | 0.000018 | 94% | 13% | 3 | 1.0 | 0.3 | 3.9 |
| | 0.000181 | 38% | 70% | 4 | 1.3 | 0.4 | 4.4 |
| | 0.000307 | 25% | 80% | | | | |
| | 0.000736 | 13% | 90% | | | | |

# FIG. 2 - 3

**Epidermal growth factor**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 19000.000 | 22100.000 | 19000.000 | 18750.000 | 19000.000 | 13750.000 |
| average | 27046.117 | 29251.333 | 27046.117 | 22999.167 | 27046.117 | 19610.556 |
| stdev | 24371.617 | 23389.968 | 24371.617 | 18256.375 | 24371.617 | 21088.948 |
| p (t-test) | | 0.648 | | 0.331 | | 0.203 |
| min | 100.000 | 1430.000 | 100.000 | 2340.000 | 100.000 | 2830.000 |
| max | 109224.000 | 88986.000 | 109224.000 | 73300.000 | 109224.000 | 91500.000 |
| n (Samp) | 419 | 27 | 419 | 36 | 419 | 18 |
| n (Pat) | 164 | 27 | 164 | 36 | 164 | 18 |

sCr only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 20850.000 | 9220.000 | 20850.000 | 16700.000 | 20850.000 | 14600.000 |
| average | 27267.942 | 8298.000 | 27267.942 | 22023.333 | 27267.942 | 14932.857 |
| stdev | 24047.577 | 5862.569 | 24047.577 | 26372.248 | 24047.577 | 9483.285 |
| p (t-test) | | 0.079 | | 0.517 | | 0.176 |
| min | 100.000 | 1430.000 | 100.000 | 2340.000 | 100.000 | 5480.000 |
| max | 109224.000 | 16600.000 | 109224.000 | 69200.000 | 109224.000 | 33700.000 |
| n (Samp) | 518 | 5 | 518 | 9 | 518 | 7 |
| n (Pat) | 199 | 5 | 199 | 9 | 199 | 7 |

UO only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 17400.000 | 22550.000 | 17400.000 | 21300.000 | 17400.000 | 14150.000 |
| average | 25840.389 | 30314.462 | 25840.389 | 24006.667 | 25840.389 | 20496.875 |
| stdev | 23890.322 | 23301.176 | 23890.322 | 16764.426 | 23890.322 | 22291.579 |
| p (t-test) | | 0.357 | | 0.681 | | 0.381 |
| min | 100.000 | 1850.000 | 100.000 | 3410.000 | 100.000 | 2610.000 |
| max | 109224.000 | 88986.000 | 109224.000 | 73300.000 | 109224.000 | 91500.000 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
| --- | --- | --- | --- | --- | --- |
| 0 hours | 0.55 | 0.059 | 419 | 27 | 0.438 |
| 24 hours | 0.48 | 0.050 | 419 | 36 | 0.697 |
| 48 hours | 0.41 | 0.064 | 419 | 18 | 0.139 |

sCr only

| Time prior AKI stage | AUC | SE | nCohort 1 | nCohort 2 | p |
| --- | --- | --- | --- | --- | --- |
| 0 hours | 0.22 | 0.079 | 518 | 5 | 0.000 |
| 24 hours | 0.38 | 0.086 | 518 | 9 | 0.146 |
| 48 hours | 0.37 | 0.096 | 518 | 7 | 0.182 |

UO only

| Time prior AKI stage | AUC | SE | nCohort 1 | nCohort 2 | p |
| --- | --- | --- | --- | --- | --- |
| 0 hours | 0.58 | 0.060 | 352 | 26 | 0.191 |
| 24 hours | 0.53 | 0.056 | 352 | 30 | 0.558 |
| 48 hours | 0.42 | 0.069 | 352 | 16 | 0.280 |

sCr or UO

| Time prior AKI stage | Cutoff value | sens | spec | Quartile | OR | 95% CI of OR | |
| --- | --- | --- | --- | --- | --- | --- | --- |
| 0 hours | 15100 | 70% | 42% | 1 | | | |
| | 11500 | 81% | 35% | 2 | 2.1 | 1.0 | 4.5 |
| | 4110 | 93% | 9% | 3 | 1.8 | 0.8 | 4.0 |
| | 33100 | 33% | 70% | 4 | 2.1 | 1.0 | 4.5 |
| | 43300 | 22% | 80% | | | | |
| | 65100 | 11% | 90% | | | | |
| 24 hours | 12300 | 72% | 37% | 1 | | | |
| | 9050 | 81% | 27% | 2 | 2.1 | 1.2 | 3.6 |
| | 3330 | 92% | 7% | 3 | 1.9 | 1.1 | 3.3 |
| | 33100 | 19% | 70% | 4 | 1.2 | 0.6 | 2.3 |

## FIG. 2 - 4

| | 43300 | 11% | 80% | | | | |
|---|---|---|---|---|---|---|---|
| | 65100 | 8% | 90% | | | | |
| 48 hours | 6690 | 72% | 20% | 1 | | | |
| | 5400 | 83% | 13% | 2 | 2.1 | 0.5 | 9.3 |
| | 3000 | 94% | 6% | 3 | 3.1 | 0.8 | 12.1 |
| | 33100 | 11% | 70% | 4 | 3.1 | 0.8 | 12.1 |
| | 43300 | 11% | 80% | | | | |
| | 65100 | 6% | 90% | | | | |

sCr only

| Time prior AKI stage | Cutoff value | sens | spec | Quartile | OR | 95% CI of OR | |
|---|---|---|---|---|---|---|---|
| 0 hours | 4110 | 80% | 9% | 1 | | | |
| | 4110 | 80% | 9% | 2 | na | na | na |
| | 1410 | 100% | 2% | 3 | na | na | na |
| | 32500 | 0% | 70% | 4 | na | na | na |
| | 43000 | 0% | 80% | | | | |
| | 65300 | 0% | 90% | | | | |
| 24 hours | 2500 | 78% | 4% | 1 | | | |
| | 2340 | 89% | 4% | 2 | 0.0 | 0.0 | na |
| | 2230 | 100% | 4% | 3 | 1.5 | 0.3 | 8.0 |
| | 32500 | 22% | 70% | 4 | 2.0 | 0.5 | 9.2 |
| | 43000 | 22% | 80% | | | | |
| | 65300 | 11% | 90% | | | | |
| 48 hours | 9950 | 71% | 28% | 1 | | | |
| | 6690 | 86% | 19% | 2 | na | na | na |
| | 5460 | 100% | 14% | 3 | na | na | na |
| | 32500 | 14% | 70% | 4 | na | na | na |
| | 43000 | 0% | 80% | | | | |
| | 65300 | 0% | 90% | | | | |

UO only

| Time prior AKI stage | Cutoff value | sens | spec | Quartile | OR | 95% CI of OR | |
|---|---|---|---|---|---|---|---|
| 0 hours | 15100 | 73% | 44% | 1 | | | |
| | 12900 | 81% | 39% | 2 | 1.3 | 0.5 | 3.2 |
| | 6340 | 92% | 18% | 3 | 2.4 | 1.1 | 5.1 |
| | 30900 | 35% | 70% | 4 | 2.1 | 0.9 | 4.5 |
| | 41100 | 27% | 80% | | | | |
| | 62200 | 12% | 90% | | | | |
| 24 hours | 13100 | 70% | 40% | 1 | | | |
| | 11100 | 80% | 35% | 2 | 2.1 | 0.9 | 4.5 |
| | 6340 | 90% | 18% | 3 | 3.3 | 1.6 | 6.6 |
| | 30900 | 27% | 70% | 4 | 1.5 | 0.6 | 3.6 |
| | 41100 | 10% | 80% | | | | |
| | 62200 | 7% | 90% | | | | |
| 48 hours | 5400 | 75% | 13% | 1 | | | |
| | 3930 | 81% | 9% | 2 | 2.0 | 0.5 | 9.3 |
| | 2810 | 94% | 5% | 3 | 2.6 | 0.6 | 10.7 |
| | 30900 | 13% | 70% | 4 | 2.6 | 0.6 | 10.7 |
| | 41100 | 13% | 80% | | | | |
| | 62200 | 6% | 90% | | | | |

**Interleukin-1 alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.002 | 0.001 | 0.002 | 0.001 | 0.002 | 0.001 |
| average | 0.002 | 0.002 | 0.002 | 0.002 | 0.002 | 0.002 |
| stdev | 0.003 | 0.002 | 0.003 | 0.005 | 0.003 | 0.003 |
| p (t-test) | | 0.411 | | 0.839 | | 0.811 |
| min | 0.000 | 0.000 | 0.000 | 0.000 | 0.000 | 0.000 |
| max | 0.038 | 0.007 | 0.038 | 0.027 | 0.038 | 0.012 |
| n (Samp) | 419 | 27 | 419 | 36 | 419 | 18 |
| n (Pat) | 164 | 27 | 164 | 36 | 164 | 18 |

## FIG. 2 - 5

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.001 | 0.003 | 0.001 | 0.002 | 0.001 | 0.003 |
| average | 0.002 | 0.004 | 0.002 | 0.002 | 0.002 | 0.004 |
| stdev | 0.003 | 0.005 | 0.003 | 0.002 | 0.003 | 0.004 |
| p (t-test) | | 0.193 | | 0.948 | | 0.205 |
| min | 0.000 | 0.000 | 0.000 | 0.000 | 0.000 | 0.000 |
| max | 0.038 | 0.012 | 0.038 | 0.007 | 0.038 | 0.012 |
| n (Samp) | 518 | 5 | 518 | 9 | 518 | 7 |
| n (Pat) | 199 | 5 | 199 | 9 | 199 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.002 | 0.001 | 0.002 | 0.000 | 0.002 | 0.001 |
| average | 0.002 | 0.002 | 0.002 | 0.002 | 0.002 | 0.001 |
| stdev | 0.003 | 0.002 | 0.003 | 0.005 | 0.003 | 0.001 |
| p (t-test) | | 0.505 | | 0.714 | | 0.356 |
| min | 0.000 | 0.000 | 0.000 | 0.000 | 0.000 | 0.000 |
| max | 0.033 | 0.007 | 0.033 | 0.027 | 0.033 | 0.004 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.46 | 0.056 | 419 | 27 | 0.445 |
| 24 hours | 0.45 | 0.048 | 419 | 36 | 0.284 |
| 48 hours | 0.46 | 0.068 | 419 | 18 | 0.587 |

sCr only

| Time prior AKI stage | AUC | SE | nCohort 1 | nCohort 2 | p |
|---|---|---|---|---|---|
| 0 hours | 0.67 | 0.133 | 518 | 5 | 0.191 |
| 24 hours | 0.52 | 0.099 | 518 | 9 | 0.804 |
| 48 hours | 0.68 | 0.113 | 518 | 7 | 0.118 |

UO only

| Time prior AKI stage | AUC | SE | nCohort 1 | nCohort 2 | p |
|---|---|---|---|---|---|
| 0 hours | 0.47 | 0.057 | 352 | 26 | 0.557 |
| 24 hours | 0.45 | 0.053 | 352 | 30 | 0.304 |
| 48 hours | 0.44 | 0.071 | 352 | 16 | 0.431 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.4 | 0.1 | 0.9 |
| | 0 | 100% | 0% | 3 | 1.0 | 0.6 | 1.7 |
| | 0.0024 | 33% | 70% | 4 | 1.0 | 0.6 | 1.7 |
| | 0.00308 | 11% | 80% | | | | |
| | 0.00423 | 7% | 90% | | | | |
| 24 hours | 0 | 100% | 0% | 1 | | | |
| | 0 | 100% | 0% | 2 | 0.6 | 0.4 | 1.2 |
| | 0 | 100% | 0% | 3 | 0.9 | 0.5 | 1.5 |
| | 0.0024 | 28% | 70% | 4 | 1.5 | 1.0 | 2.3 |
| | 0.00308 | 17% | 80% | | | | |
| | 0.00423 | 8% | 90% | | | | |
| 48 hours | 0 | 100% | 0% | 1 | | | |
| | 0 | 100% | 0% | 2 | 0.5 | 0.1 | 2.2 |
| | 0 | 100% | 0% | 3 | 2.7 | 1.3 | 5.5 |
| | 0.0024 | 28% | 70% | 4 | 0.5 | 0.1 | 2.2 |
| | 0.00308 | 22% | 80% | | | | |
| | 0.00423 | 6% | 90% | | | | |

sCr only

| Time prior AKI stage | Cutoff value | sens | spec | Quartile | OR | 95% CI of OR | |
|---|---|---|---|---|---|---|---|
| 0 hours | 0.00201 | 80% | 63% | 1 | | | |

## FIG. 2 - 6

| | Cutoff value | sens | spec | Quartile | OR | 95% CI of OR | |
|---|---|---|---|---|---|---|---|
| | 0.00201 | 80% | 63% | 2 | na | na | na |
| | 0 | 100% | 0% | 3 | na | na | na |
| | 0.00231 | 60% | 70% | 4 | na | na | na |
| | 0.00294 | 40% | 80% | | | | |
| | 0.00416 | 20% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 0.00231 | 33% | 70% | 4 | na | na | na |
| | 0.00294 | 22% | 80% | | | | |
| | 0.00416 | 11% | 90% | | | | |
| 48 hours | 0.00212 | 71% | 66% | 1 | | | |
| | 0.00098 | 86% | 43% | 2 | 1.0 | 0.0 | 51.9 |
| | 0 | 100% | 0% | 3 | 2.0 | 0.1 | 39.3 |
| | 0.00231 | 57% | 70% | 4 | 3.0 | 0.2 | 42.5 |
| | 0.00294 | 43% | 80% | | | | |
| | 0.00416 | 14% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.2 | 0.1 | 0.7 |
| | 0 | 100% | 0% | 3 | 0.3 | 0.1 | 0.8 |
| | 0.00231 | 38% | 71% | 4 | 1.4 | 0.9 | 2.1 |
| | 0.00288 | 23% | 80% | | | | |
| | 0.00389 | 8% | 90% | | | | |
| 24 hours | 0 | 100% | 0% | 1 | | | |
| | 0 | 100% | 0% | 2 | 0.5 | 0.2 | 1.1 |
| | 0 | 100% | 0% | 3 | 2.2 | 1.5 | 3.3 |
| | 0.00231 | 27% | 71% | 4 | 0.2 | 0.1 | 0.8 |
| | 0.00288 | 20% | 80% | | | | |
| | 0.00389 | 10% | 90% | | | | |
| 48 hours | 0 | 100% | 0% | 1 | | | |
| | 0 | 100% | 0% | 2 | 0.2 | 0.0 | 2.9 |
| | 0 | 100% | 0% | 3 | 1.3 | 0.5 | 3.2 |
| | 0.00231 | 25% | 71% | 4 | 1.5 | 0.6 | 3.6 |
| | 0.00288 | 19% | 80% | | | | |
| | 0.00389 | 6% | 90% | | | | |

**Interleukin-4**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 25.200 | 25.200 | 25.200 | 22.750 | 25.200 | 24.850 |
| average | 24.595 | 24.147 | 24.595 | 22.464 | 24.595 | 24.585 |
| stdev | 11.809 | 13.033 | 11.809 | 12.920 | 11.809 | 15.993 |
| p (t-test) | | 0.849 | | 0.303 | | 0.997 |
| min | 0.416 | 0.416 | 0.416 | 0.416 | 0.416 | 0.416 |
| max | 62.400 | 52.700 | 62.400 | 48.600 | 62.400 | 56.700 |
| n (Samp) | 419 | 27 | 419 | 36 | 419 | 18 |
| n (Pat) | 164 | 27 | 164 | 36 | 164 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 25.000 | 25.500 | 25.000 | 23.200 | 25.000 | 33.400 |
| average | 24.270 | 28.620 | 24.270 | 21.913 | 24.270 | 30.114 |
| stdev | 11.992 | 10.637 | 11.992 | 13.025 | 11.992 | 16.540 |
| p (t-test) | | 0.420 | | 0.560 | | 0.203 |
| min | 0.416 | 15.800 | 0.416 | 0.416 | 0.416 | 3.800 |
| max | 62.400 | 44.600 | 62.400 | 40.600 | 62.400 | 48.600 |
| n (Samp) | 518 | 5 | 518 | 9 | 518 | 7 |
| n (Pat) | 199 | 5 | 199 | 9 | 199 | 7 |

UO only

| 0 hr prior toAKI stage | 24 hr prior toAKI stage | 48 hr prior toAKI stage |
|---|---|---|

## FIG. 2 - 7

|  | Cohort 1 | Cohort 2 | Cohort 1 | Cohort 2 | Cohort 1 | Cohort 2 |
|---|---|---|---|---|---|---|
| median | 25.400 | 24.100 | 25.400 | 23.750 | 25.400 | 25.300 |
| average | 24.861 | 24.233 | 24.861 | 23.803 | 24.861 | 26.021 |
| stdev | 12.554 | 13.682 | 12.554 | 13.219 | 12.554 | 15.468 |
| p (t-test) |  | 0.807 |  | 0.659 |  | 0.721 |
| min | 0.416 | 0.416 | 0.416 | 0.416 | 0.416 | 0.416 |
| max | 66.200 | 52.700 | 66.200 | 48.600 | 66.200 | 56.700 |
| n (Samp) | 352 | 26 | 352 | 30 | 352 | 16 |
| n (Pat) | 133 | 26 | 133 | 30 | 133 | 16 |

sCr or UO

| Time prior AKI stage | AUC | SE | nCohort 1 | nCohort 2 | p |
|---|---|---|---|---|---|
| 0 hours | 0.50 | 0.057 | 419 | 27 | 0.948 |
| 24 hours | 0.45 | 0.049 | 419 | 36 | 0.354 |
| 48 hours | 0.51 | 0.070 | 419 | 18 | 0.923 |

sCr only

| Time prior AKI stage | AUC | SE | nCohort 1 | nCohort 2 | p |
|---|---|---|---|---|---|
| 0 hours | 0.60 | 0.135 | 518 | 5 | 0.472 |
| 24 hours | 0.46 | 0.094 | 518 | 9 | 0.633 |
| 48 hours | 0.66 | 0.114 | 518 | 7 | 0.161 |

UO only

| Time prior AKI stage | AUC | SE | nCohort 1 | nCohort 2 | p |
|---|---|---|---|---|---|
| 0 hours | 0.49 | 0.058 | 352 | 26 | 0.859 |
| 24 hours | 0.48 | 0.054 | 352 | 30 | 0.718 |
| 48 hours | 0.53 | 0.075 | 352 | 16 | 0.721 |

sCr or UO

| Time prior AKI stage | Cutoff value | sens | spec | Quartile | OR | 95% CI of OR | |
|---|---|---|---|---|---|---|---|
| 0 hours | 20.1 | 74% | 32% | 1 |  |  |  |
|  | 12.6 | 81% | 17% | 2 | 0.6 | 0.3 | 1.2 |
|  | 0 | 100% | 0% | 3 | 0.9 | 0.5 | 1.5 |
|  | 30.6 | 30% | 71% | 4 | 0.9 | 0.5 | 1.5 |
|  | 33.4 | 19% | 81% |  |  |  |  |
|  | 39.6 | 7% | 90% |  |  |  |  |
| 24 hours | 16.4 | 72% | 23% | 1 |  |  |  |
|  | 12.6 | 81% | 17% | 2 | 0.4 | 0.2 | 0.8 |
|  | 0 | 100% | 0% | 3 | 1.1 | 0.7 | 1.7 |
|  | 30.6 | 28% | 71% | 4 | 1.1 | 0.7 | 1.7 |
|  | 33.4 | 22% | 81% |  |  |  |  |
|  | 39.6 | 11% | 90% |  |  |  |  |
| 48 hours | 20.1 | 72% | 32% | 1 |  |  |  |
|  | 0.416 | 89% | 4% | 2 | 0.8 | 0.3 | 2.0 |
|  | 0 | 100% | 0% | 3 | 0.6 | 0.2 | 1.7 |
|  | 30.6 | 39% | 71% | 4 | 1.2 | 0.6 | 2.6 |
|  | 33.4 | 28% | 81% |  |  |  |  |
|  | 39.6 | 17% | 90% |  |  |  |  |

sCr only

| Time prior AKI stage | Cutoff value | sens | spec | Quartile | OR | 95% CI of OR | |
|---|---|---|---|---|---|---|---|
| 0 hours | 25.1 | 80% | 51% | 1 |  |  |  |
|  | 25.1 | 80% | 51% | 2 | 0.0 | 0.0 | na |
|  | 15.7 | 100% | 22% | 3 | 2.0 | 0.1 | 39.0 |
|  | 30.4 | 40% | 70% | 4 | 2.0 | 0.1 | 39.0 |
|  | 33.4 | 20% | 81% |  |  |  |  |
|  | 39.6 | 20% | 90% |  |  |  |  |
| 24 hours | 12.6 | 78% | 17% | 1 |  |  |  |
|  | 8.12 | 89% | 10% | 2 | 0.5 | 0.0 | 9.7 |
|  | 0 | 100% | 0% | 3 | 1.5 | 0.3 | 8.0 |
|  | 30.4 | 33% | 70% | 4 | 1.5 | 0.3 | 8.0 |
|  | 33.4 | 22% | 81% |  |  |  |  |
|  | 39.6 | 11% | 90% |  |  |  |  |
| 48 hours | 32.9 | 71% | 79% | 1 |  |  |  |
|  | 11.1 | 86% | 15% | 2 | 0.0 | 0.0 | na |

# FIG. 2 - 8

|  | 0.416 | 100% | 5% | 3 | 0.0 | 0.0 | na |
|  | 30.4 | 71% | 70% | 4 | 2.5 | 0.6 | 10.3 |
|  | 33.4 | 43% | 81% |  |  |  |  |
|  | 39.6 | 29% | 90% |  |  |  |  |

UO only

| Time prior AKI stage | Cutoff value | sens | spec | Quartile | OR | 95% CI of OR | |
|---|---|---|---|---|---|---|---|
| 0 hours | 20.1 | 73% | 32% | 1 |  |  |  |
|  | 12.5 | 81% | 18% | 2 | 0.7 | 0.3 | 1.4 |
|  | 0 | 100% | 0% | 3 | 1.0 | 0.5 | 1.8 |
|  | 30.6 | 27% | 70% | 4 | 1.0 | 0.6 | 1.9 |
|  | 33.6 | 19% | 80% |  |  |  |  |
|  | 40.1 | 8% | 90% |  |  |  |  |
| 24 hours | 18 | 70% | 26% | 1 |  |  |  |
|  | 12.5 | 83% | 18% | 2 | 0.4 | 0.2 | 0.9 |
|  | 5.81 | 90% | 7% | 3 | 1.0 | 0.6 | 1.6 |
|  | 30.6 | 30% | 70% | 4 | 0.9 | 0.5 | 1.5 |
|  | 33.6 | 23% | 80% |  |  |  |  |
|  | 40.1 | 13% | 90% |  |  |  |  |
| 48 hours | 21.7 | 75% | 38% | 1 |  |  |  |
|  | 20.1 | 81% | 32% | 2 | 1.7 | 0.6 | 5.1 |
|  | 0 | 100% | 0% | 3 | 1.0 | 0.3 | 3.9 |
|  | 30.6 | 38% | 70% | 4 | 1.7 | 0.6 | 5.1 |
|  | 33.6 | 25% | 80% |  |  |  |  |
|  | 40.1 | 19% | 90% |  |  |  |  |

**Transforming growth factor beta-1**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 13.225 | 21.012 | 13.225 | 107.639 | 13.225 | 40.253 |
| average | 29.987 | 40.904 | 29.987 | 75.637 | 29.987 | 40.253 |
| stdev | 44.245 | 46.289 | 44.245 | 58.461 | 44.245 | 56.015 |
| p (t-test) |  | 0.510 |  | 0.086 |  | 0.748 |
| min | 0.036 | 0.036 | 0.036 | 8.162 | 0.036 | 0.644 |
| max | 270.067 | 111.111 | 270.067 | 111.111 | 270.067 | 79.861 |
| n (Samp) | 78 | 8 | 78 | 3 | 78 | 2 |
| n (Pat) | 56 | 8 | 56 | 3 | 56 | 2 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 13.225 | 0.036 | 13.225 | 0.036 | 13.225 | 53.373 |
| average | 31.603 | 111.111 | 31.603 | 111.111 | 31.603 | 53.373 |
| stdev | 44.147 | na | 44.147 | na | 44.147 | 37.459 |
| p (t-test) |  | na |  | na |  | 0.491 |
| min | 0.036 | 111.111 | 0.036 | 111.111 | 0.036 | 26.885 |
| max | 270.067 | 111.111 | 270.067 | 111.111 | 270.067 | 79.861 |
| n (Samp) | 96 | 1 | 96 | 1 | 96 | 2 |
| n (Pat) | 69 | 1 | 69 | 1 | 69 | 2 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 11.173 | 21.012 | 11.173 | 57.900 | 11.173 | 0.036 |
| average | 28.646 | 40.904 | 28.646 | 57.900 | 28.646 | 0.644 |
| stdev | 47.943 | 46.289 | 47.943 | 70.341 | 47.943 | na |
| p (t-test) |  | 0.497 |  | 0.404 |  | na |
| min | 0.036 | 0.036 | 0.036 | 8.162 | 0.036 | 0.644 |
| max | 270.067 | 111.111 | 270.067 | 107.639 | 270.067 | 0.644 |
| n (Samp) | 61 | 8 | 61 | 2 | 61 | 1 |
| n (Pat) | 43 | 8 | 43 | 2 | 43 | 1 |

sCr or UO

| Time prior AKI stage | AUC | SE | nCohort 1 | nCohort 2 | p |
|---|---|---|---|---|---|
| 0 hours | 0.55 | 0.110 | 78 | 8 | 0.621 |
| 24 hours | 0.78 | 0.160 | 78 | 3 | 0.075 |

**FIG. 2 - 9**

| 48 hours | 0.53 | 0.211 | 78 | 2 | 0.903 |

sCr only

| Time prior AKI stage | AUC | SE | nCohort 1 | nCohort 2 | p |
|---|---|---|---|---|---|
| 0 hours | 0.97 | 0.112 | 96 | 1 | 0.000 |
| 24 hours | 0.97 | 0.112 | 96 | 1 | 0.000 |
| 48 hours | 0.76 | 0.201 | 96 | 2 | 0.199 |

UO only

| Time prior AKI stage | AUC | SE | nCohort 1 | nCohort 2 | p |
|---|---|---|---|---|---|
| 0 hours | 0.57 | 0.112 | 61 | 8 | 0.533 |
| 24 hours | 0.71 | 0.210 | 61 | 2 | 0.320 |
| 48 hours | 0.12 | 0.116 | 61 | 1 | 0.001 |

sCr or UO

| Time prior AKI stage | Cutoff value | sens | spec | Quartile | OR | 95% CI of OR | |
|---|---|---|---|---|---|---|---|
| 0 hours | 6.0137457 | 75% | 35% | 1 | | | |
| | 0 | 100% | 0% | 2 | 0.5 | 0.0 | 10.4 |
| | 0 | 100% | 0% | 3 | 1.0 | 0.1 | 8.7 |
| | 35.227273 | 38% | 71% | 4 | 1.5 | 0.2 | 9.4 |
| | 54.487179 | 38% | 81% | | | | |
| | 74.652778 | 25% | 91% | | | | |
| 24 hours | 7.6815642 | 100% | 40% | 1 | | | |
| | 7.6815642 | 100% | 40% | 2 | na | na | na |
| | 7.6815642 | 100% | 40% | 3 | na | na | na |
| | 35.227273 | 67% | 71% | 4 | na | na | na |
| | 54.487179 | 67% | 81% | | | | |
| | 74.652778 | 67% | 91% | | | | |
| 48 hours | 0.0360285 | 100% | 12% | 1 | | | |
| | 0.0360285 | 100% | 12% | 2 | 0.0 | 0.0 | na |
| | 0.0360285 | 100% | 12% | 3 | 0.0 | 0.0 | na |
| | 35.227273 | 50% | 71% | 4 | 1.0 | 0.0 | 61.9 |
| | 54.487179 | 50% | 81% | | | | |
| | 74.652778 | 50% | 91% | | | | |

UO only

| Time prior AKI stage | Cutoff value | sens | spec | Quartile | OR | 95% CI of OR | |
|---|---|---|---|---|---|---|---|
| 0 hours | 6.0137457 | 75% | 38% | 1 | | | |
| | 0 | 100% | 0% | 2 | 0.5 | 0.0 | 11.4 |
| | 0 | 100% | 0% | 3 | 1.0 | 0.1 | 9.2 |
| | 30.377095 | 38% | 72% | 4 | 1.5 | 0.2 | 10.0 |
| | 41.666667 | 38% | 80% | | | | |
| | 74.652778 | 25% | 90% | | | | |
| 24 hours | 7.6815642 | 100% | 44% | 1 | | | |
| | 7.6815642 | 100% | 44% | 2 | na | na | na |
| | 7.6815642 | 100% | 44% | 3 | na | na | na |
| | 30.377095 | 50% | 72% | 4 | na | na | na |
| | 41.666667 | 50% | 80% | | | | |
| | 74.652778 | 50% | 90% | | | | |
| 48 hours | 0.0360285 | 100% | 11% | 1 | | | |
| | 0.0360285 | 100% | 11% | 2 | na | na | na |
| | 0.0360285 | 100% | 11% | 3 | na | na | na |
| | 30.377095 | 0% | 72% | 4 | na | na | na |
| | 41.666667 | 0% | 80% | | | | |
| | 74.652778 | 0% | 90% | | | | |

**Tubulointerstitial nephritis antigen**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.225 | 0.308 | 0.225 | 0.721 | 0.225 | 0.688 |
| average | 4.669 | 1.844 | 4.669 | 1.720 | 4.669 | 3.017 |
| stdev | 39.078 | 4.980 | 39.078 | 3.307 | 39.078 | 5.952 |

## FIG. 2 - 10

| p (t-test) | | 0.767 | | 0.713 | | 0.879 |
|---|---|---|---|---|---|---|
| min | 0.000 | 0.004 | 0.000 | 0.104 | 0.000 | 0.225 |
| max | 480.918 | 20.934 | 480.918 | 14.608 | 480.918 | 19.127 |
| n (Samp) | 165 | 17 | 165 | 24 | 165 | 13 |
| n (Pat) | 106 | 17 | 106 | 24 | 106 | 13 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.241 | 0.156 | 0.241 | 0.835 | 0.241 | 2.170 |
| average | 4.142 | 0.156 | 4.142 | 0.765 | 4.142 | 3.641 |
| stdev | 34.385 | 0.215 | 34.385 | 0.443 | 34.385 | 5.398 |
| p (t-test) | | 0.870 | | 0.827 | | 0.974 |
| min | 0.000 | 0.004 | 0.000 | 0.270 | 0.000 | 0.189 |
| max | 480.918 | 0.308 | 480.918 | 1.410 | 480.918 | 13.102 |
| n (Samp) | 214 | 2 | 214 | 5 | 214 | 5 |
| n (Pat) | 125 | 2 | 125 | 5 | 125 | 5 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.225 | 0.458 | 0.225 | 0.638 | 0.225 | 0.688 |
| average | 5.288 | 1.884 | 5.288 | 1.808 | 5.288 | 2.101 |
| stdev | 42.256 | 4.969 | 42.256 | 3.448 | 42.256 | 5.135 |
| p (t-test) | | 0.741 | | 0.701 | | 0.787 |
| min | 0.000 | 0.118 | 0.000 | 0.104 | 0.000 | 0.004 |
| max | 480.918 | 20.934 | 480.918 | 14.608 | 480.918 | 19.127 |
| n (Samp) | 141 | 17 | 141 | 22 | 141 | 13 |
| n (Pat) | 85 | 17 | 85 | 22 | 85 | 13 |

sCr or UO

| Time prior AKI stage | AUC | SE | nCohort 1 | nCohort 2 | p |
|---|---|---|---|---|---|
| 0 hours | 0.57 | 0.076 | 165 | 17 | 0.337 |
| 24 hours | 0.62 | 0.065 | 165 | 24 | 0.061 |
| 48 hours | 0.69 | 0.084 | 165 | 13 | 0.026 |

sCr only

| Time prior AKI stage | AUC | SE | nCohort 1 | nCohort 2 | p |
|---|---|---|---|---|---|
| 0 hours | 0.27 | 0.145 | 214 | 2 | 0.108 |
| 24 hours | 0.69 | 0.133 | 214 | 5 | 0.157 |
| 48 hours | 0.65 | 0.135 | 214 | 5 | 0.281 |

UO only

| Time prior AKI stage | AUC | SE | nCohort 1 | nCohort 2 | p |
|---|---|---|---|---|---|
| 0 hours | 0.59 | 0.076 | 141 | 17 | 0.215 |
| 24 hours | 0.62 | 0.068 | 141 | 22 | 0.088 |
| 48 hours | 0.66 | 0.085 | 141 | 13 | 0.057 |

sCr or UO

| Time prior AKI stage | Cutoff value | sens | spec | Quartile | OR | 95% CI of OR | |
|---|---|---|---|---|---|---|---|
| 0 hours | 0.2077793 | 94% | 15% | 1 | | | |
| | 0.2077793 | 94% | 15% | 2 | 0.0 | 0.0 | na |
| | 0.2077793 | 94% | 15% | 3 | 0.5 | 0.2 | 1.3 |
| | 0.5926724 | 35% | 70% | 4 | 0.8 | 0.4 | 1.7 |
| | 0.9698276 | 24% | 81% | | | | |
| | 1.3135453 | 18% | 90% | | | | |
| 24 hours | 0.2077793 | 88% | 15% | 1 | | | |
| | 0.2077793 | 88% | 15% | 2 | 0.1 | 0.0 | 1.3 |
| | 0.1814851 | 92% | 13% | 3 | 1.0 | 0.5 | 1.9 |
| | 0.5926724 | 58% | 70% | 4 | 1.3 | 0.7 | 2.4 |
| | 0.9698276 | 33% | 81% | | | | |
| | 1.3135453 | 25% | 90% | | | | |
| 48 hours | 0.4580337 | 77% | 64% | 1 | | | |
| | 0.2077793 | 100% | 15% | 2 | 2.0 | 0.1 | 41.4 |
| | 0.2077793 | 100% | 15% | 3 | 6.8 | 0.6 | 73.6 |
| | 0.5926724 | 62% | 70% | 4 | 4.2 | 0.3 | 53.4 |
| | 0.9698276 | 23% | 81% | | | | |
| | 1.3135453 | 23% | 90% | | | | |

## FIG. 2 - 11

sCr only

| Time prior AKI stage | Cutoff value | sens | spec | Quartile | OR | 95% CI of OR | |
|---|---|---|---|---|---|---|---|
| 0 hours | 2.884E-16 | 100% | 0% | 1 | | | |
| | 2.884E-16 | 100% | 0% | 2 | na | na | na |
| | 2.884E-16 | 100% | 0% | 3 | na | na | na |
| | 0.8021594 | 0% | 71% | 4 | na | na | na |
| | 1.0302616 | 0% | 80% | | | | |
| | 1.5625 | 0% | 91% | | | | |
| 24 hours | 0.434988 | 80% | 57% | 1 | | | |
| | 0.434988 | 80% | 57% | 2 | na | na | na |
| | 0.2693966 | 100% | 51% | 3 | na | na | na |
| | 0.8021594 | 60% | 71% | 4 | na | na | na |
| | 1.0302616 | 20% | 80% | | | | |
| | 1.5625 | 0% | 91% | | | | |
| 48 hours | 0.2077793 | 80% | 13% | 1 | | | |
| | 0.2077793 | 80% | 13% | 2 | 1.0 | 0.0 | 53.2 |
| | 0.1870013 | 100% | 12% | 3 | 0.0 | 0.0 | na |
| | 0.8021594 | 60% | 71% | 4 | 3.1 | 0.2 | 45.0 |
| | 1.0302616 | 60% | 80% | | | | |
| | 1.5625 | 60% | 91% | | | | |

UO only

| Time prior AKI stage | Cutoff value | sens | spec | Quartile | OR | 95% CI of OR | |
|---|---|---|---|---|---|---|---|
| 0 hours | 0.1870013 | 94% | 16% | 1 | | | |
| | 0.1870013 | 94% | 16% | 2 | 2.6 | 0.6 | 11.6 |
| | 0.1870013 | 94% | 16% | 3 | 2.1 | 0.4 | 10.3 |
| | 0.5402261 | 47% | 71% | 4 | 3.3 | 0.8 | 13.5 |
| | 0.9698276 | 24% | 81% | | | | |
| | 1.2997909 | 18% | 90% | | | | |
| 24 hours | 0.1870013 | 86% | 16% | 1 | | | |
| | 0.1870013 | 86% | 16% | 2 | 1.7 | 0.5 | 5.4 |
| | 0.1814851 | 91% | 15% | 3 | 2.1 | 0.7 | 6.3 |
| | 0.5402261 | 55% | 71% | 4 | 3.0 | 1.1 | 8.2 |
| | 0.9698276 | 36% | 81% | | | | |
| | 1.2997909 | 27% | 90% | | | | |
| 48 hours | 0.4580337 | 77% | 66% | 1 | | | |
| | 0.1870013 | 92% | 16% | 2 | 0.0 | 0.0 | na |
| | 0.1870013 | 92% | 16% | 3 | 1.8 | 0.6 | 5.6 |
| | 0.5402261 | 62% | 71% | 4 | 1.7 | 0.5 | 5.5 |
| | 0.9698276 | 23% | 81% | | | | |
| | 1.2997909 | 23% | 90% | | | | |

# FIG. 2 - 12

**Netrin-1**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.211 | 3.593 | 0.211 | 3.593 | 0.211 | 3.593 |
| average | 3.264 | 6.068 | 3.264 | 6.068 | 3.264 | 6.068 |
| stdev | 5.300 | 6.484 | 5.300 | 6.484 | 5.300 | 6.484 |
| p (t-test) |  | 0.053 |  | 0.053 |  | 0.053 |
| min | 0.211 | 0.211 | 0.211 | 0.211 | 0.211 | 0.211 |
| max | 21.067 | 17.556 | 21.067 | 17.556 | 21.067 | 17.556 |
| n (Samp) | 55 | 22 | 55 | 22 | 55 | 22 |
| n (Pat) | 55 | 22 | 55 | 22 | 55 | 22 |

sCr only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 2.802 | 0.228 | 2.802 | 0.228 | 2.802 | 0.228 |
| stdev | 4.433 | 0.035 | 4.433 | 0.035 | 4.433 | 0.035 |
| p (t-test) |  | 0.266 |  | 0.266 |  | 0.266 |
| min | 0.211 | 0.211 | 0.211 | 0.211 | 0.211 | 0.211 |
| max | 15.734 | 0.281 | 15.734 | 0.281 | 15.734 | 0.281 |
| n (Samp) | 21 | 4 | 21 | 4 | 21 | 4 |
| n (Pat) | 21 | 4 | 21 | 4 | 21 | 4 |

UO only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.211 | 4.100 | 0.211 | 4.100 | 0.211 | 4.100 |
| average | 4.180 | 5.915 | 4.180 | 5.915 | 4.180 | 5.915 |
| stdev | 6.530 | 5.913 | 6.530 | 5.913 | 6.530 | 5.913 |
| p (t-test) |  | 0.344 |  | 0.344 |  | 0.344 |
| min | 0.211 | 0.211 | 0.211 | 0.211 | 0.211 | 0.211 |
| max | 21.067 | 16.108 | 21.067 | 16.108 | 21.067 | 16.108 |
| n (Samp) | 44 | 17 | 44 | 17 | 44 | 17 |
| n (Pat) | 44 | 17 | 44 | 17 | 44 | 17 |

sCr or UO

| Time prior AKI stage | AUC | SE | nCohort 1 | nCohort 2 | p |
|---|---|---|---|---|---|
| 0 hours | 0.65 | 0.072 | 55 | 22 | 0.038 |
| 24 hours | 0.65 | 0.072 | 55 | 22 | 0.038 |
| 48 hours | 0.65 | 0.072 | 55 | 22 | 0.038 |

sCr only

| Time prior AKI stage | AUC | SE | nCohort 1 | nCohort 2 | p |
|---|---|---|---|---|---|
| 0 hours | 0.36 | 0.144 | 21 | 4 | 0.341 |
| 24 hours | 0.36 | 0.144 | 21 | 4 | 0.341 |
| 48 hours | 0.36 | 0.144 | 21 | 4 | 0.341 |

UO only

| Time prior AKI stage | AUC | SE | nCohort 1 | nCohort 2 | p |
|---|---|---|---|---|---|
| 0 hours | 0.63 | 0.083 | 44 | 17 | 0.113 |
| 24 hours | 0.63 | 0.083 | 44 | 17 | 0.113 |
| 48 hours | 0.63 | 0.083 | 44 | 17 | 0.113 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 3.0 | 0.6 | 15.5 |
|  | 0 | 100% | 0% | 3 | 3.9 | 0.8 | 18.9 |
|  | 3.0857271 | 50% | 71% | 4 | 7.0 | 1.6 | 30.9 |
|  | 4.4883303 | 45% | 80% |  |  |  |  |
|  | 12.289326 | 23% | 91% |  |  |  |  |
| 24 hours | 0 | 100% | 0% | 1 |  |  |  |
|  | 0 | 100% | 0% | 2 | 3.0 | 0.6 | 15.5 |
|  | 0 | 100% | 0% | 3 | 3.9 | 0.8 | 18.9 |
|  | 3.0857271 | 50% | 71% | 4 | 7.0 | 1.6 | 30.9 |
|  | 4.4883303 | 45% | 80% |  |  |  |  |

## FIG. 3 - 1

|  | 12.289326 | 23% | 91% |  |  |  |  |
|---|---|---|---|---|---|---|---|
| 48 hours | 0 | 100% | 0% | 1 |  |  |  |
|  | 0 | 100% | 0% | 2 | 3.0 | 0.6 | 15.5 |
|  | 0 | 100% | 0% | 3 | 3.9 | 0.8 | 18.9 |
|  | 3.0857271 | 50% | 71% | 4 | 7.0 | 1.6 | 30.9 |
|  | 4.4883303 | 45% | 80% |  |  |  |  |
|  | 12.289326 | 23% | 91% |  |  |  |  |

UO only

| Time prior AKI stage | Cutoff value | sens | spec | Quartile | OR | 95% CI of OR | |
|---|---|---|---|---|---|---|---|
| 0 hours | 0.2805206 | 71% | 61% | 1 |  |  |  |
|  | 0 | 100% | 0% | 2 | 0.6 | 0.1 | 4.3 |
|  | 0 | 100% | 0% | 3 | 2.7 | 0.7 | 10.4 |
|  | 4.0998542 | 47% | 73% | 4 | 2.4 | 0.6 | 9.2 |
|  | 10.30816 | 24% | 82% |  |  |  |  |
|  | 15.800562 | 6% | 93% |  |  |  |  |
| 24 hours | 0.2805206 | 71% | 61% | 1 |  |  |  |
|  | 0 | 100% | 0% | 2 | 0.6 | 0.1 | 4.3 |
|  | 0 | 100% | 0% | 3 | 2.7 | 0.7 | 10.4 |
|  | 4.0998542 | 47% | 73% | 4 | 2.4 | 0.6 | 9.2 |
|  | 10.30816 | 24% | 82% |  |  |  |  |
|  | 15.800562 | 6% | 93% |  |  |  |  |
| 48 hours | 0.2805206 | 71% | 61% | 1 |  |  |  |
|  | 0 | 100% | 0% | 2 | 0.6 | 0.1 | 4.3 |
|  | 0 | 100% | 0% | 3 | 2.7 | 0.7 | 10.4 |
|  | 4.0998542 | 47% | 73% | 4 | 2.4 | 0.6 | 9.2 |
|  | 10.30816 | 24% | 82% |  |  |  |  |
|  | 15.800562 | 6% | 93% |  |  |  |  |

# FIG. 3 - 2

**Growth-regulated alpha protein**

sCr or UO

| | 0 hr prior to AKI stage | | 24 hr prior to AKI stage | | 48 hr prior to AKI stage | |
| --- | --- | --- | --- | --- | --- | --- |
| | Cohort 1 | Cohort 2 | Cohort 1 | Cohort 2 | Cohort 1 | Cohort 2 |
| median | 2.181 | 1.141 | 2.181 | 3.744 | 2.181 | 3.744 |
| average | 55.603 | 6.449 | 55.603 | 8.476 | 55.603 | 8.476 |
| stdev | 291.484 | 10.560 | 291.484 | 11.679 | 291.484 | 11.679 |
| p (t-test) | | 0.639 | | 0.697 | | 0.697 |
| min | 0.368 | 0.368 | 0.368 | 0.368 | 0.368 | 0.368 |
| max | 1754.386 | 30.516 | 1754.386 | 30.516 | 1754.386 | 30.516 |
| n (Samp) | 36 | 8 | 36 | 6 | 36 | 6 |
| n (Pat) | 36 | 8 | 36 | 6 | 36 | 6 |

sCr only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 0.368 | 0.368 | 0.368 | 1.141 | 0.368 | 1.141 |
| average | 36.842 | 0.754 | 36.842 | 1.141 | 36.842 | 1.141 |
| stdev | 226.235 | 0.773 | 226.235 | 1.093 | 226.235 | 1.093 |
| p (t-test) | | 0.753 | | 0.826 | | 0.826 |
| min | 0.368 | 0.368 | 0.368 | 0.368 | 0.368 | 0.368 |
| max | 1754.386 | 1.913 | 1754.386 | 1.913 | 1754.386 | 1.913 |
| n (Samp) | 60 | 4 | 60 | 2 | 60 | 2 |
| n (Pat) | 60 | 4 | 60 | 2 | 60 | 2 |

UO only

| | 0 hr prior toAKI stage | | 24 hr prior toAKI stage | | 48 hr prior toAKI stage | |
| --- | --- | --- | --- | --- | --- | --- |
| | Cohort 1 | Cohort 2 | Cohort 1 | Cohort 2 | Cohort 1 | Cohort 2 |
| median | 3.189 | 8.846 | 3.189 | 8.846 | 3.189 | 8.846 |
| average | 74.235 | 12.144 | 74.235 | 12.144 | 74.235 | 12.144 |
| stdev | 342.889 | 13.158 | 342.889 | 13.158 | 342.889 | 13.158 |
| p (t-test) | | 0.724 | | 0.724 | | 0.724 |
| min | 0.368 | 0.368 | 0.368 | 0.368 | 0.368 | 0.368 |
| max | 1754.386 | 30.516 | 1754.386 | 30.516 | 1754.386 | 30.516 |
| n (Samp) | 26 | 4 | 26 | 4 | 26 | 4 |
| n (Pat) | 26 | 4 | 26 | 4 | 26 | 4 |

sCr or UO

| Time prior AKI stage | AUC | SE | nCohort 1 | nCohort 2 | p |
| --- | --- | --- | --- | --- | --- |
| 0 hours | 0.48 | 0.113 | 36 | 8 | 0.866 |
| 24 hours | 0.56 | 0.131 | 36 | 6 | 0.634 |
| 48 hours | 0.56 | 0.131 | 36 | 6 | 0.634 |

sCr only

| Time prior AKI stage | AUC | SE | nCohort 1 | nCohort 2 | p |
| --- | --- | --- | --- | --- | --- |
| 0 hours | 0.34 | 0.127 | 60 | 4 | 0.220 |
| 24 hours | 0.43 | 0.197 | 60 | 2 | 0.720 |
| 48 hours | 0.43 | 0.197 | 60 | 2 | 0.720 |

UO only

| Time prior AKI stage | AUC | SE | nCohort 1 | nCohort 2 | p |
| --- | --- | --- | --- | --- | --- |
| 0 hours | 0.64 | 0.160 | 26 | 4 | 0.367 |
| 24 hours | 0.64 | 0.160 | 26 | 4 | 0.367 |
| 48 hours | 0.64 | 0.160 | 26 | 4 | 0.367 |

sCr or UO

| Time prior AKI stage | Cutoff value | sens | spec | Quartile | OR | 95% CI of OR | |
| --- | --- | --- | --- | --- | --- | --- | --- |
| 0 hours | 0 | 100% | 0% | 1 | | | |
| | 0 | 100% | 0% | 2 | 1.0 | 0.1 | 11.0 |
| | 0 | 100% | 0% | 3 | 0.0 | 0.0 | na |
| | 7.0422535 | 25% | 72% | 4 | 2.6 | 0.4 | 18.4 |
| | 10.841837 | 25% | 81% | | | | |
| | 36.847015 | 0% | 92% | | | | |
| 24 hours | 0 | 100% | 0% | 1 | | | |
| | 0 | 100% | 0% | 2 | 0.4 | 0.0 | 11.8 |
| | 0 | 100% | 0% | 3 | 0.4 | 0.0 | 13.4 |
| | 7.0422535 | 33% | 72% | 4 | 0.9 | 0.1 | 10.0 |

# FIG. 4 - 1

| | | 10.841837 | 33% | 81% | | | | |
|---|---|---|---|---|---|---|---|---|
| | | 36.847015 | 0% | 92% | | | | |
| 48 hours | | 0 | 100% | 0% | 1 | | | |
| | | 0 | 100% | 0% | 2 | 0.4 | 0.0 | 11.8 |
| | | 0 | 100% | 0% | 3 | 0.4 | 0.0 | 13.4 |
| | | 7.0422535 | 33% | 72% | 4 | 0.9 | 0.1 | 10.0 |
| | | 10.841837 | 33% | 81% | | | | |
| | | 36.847015 | 0% | 92% | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 19.902 | 78.251 | 19.902 | 67.262 | 19.902 | 53.373 |
| average | 34.968 | 94.697 | 34.968 | 100.418 | 34.968 | 94.135 |
| stdev | 42.968 | 112.170 | 42.968 | 130.087 | 42.968 | 131.094 |
| p (t-test) | | 0.015 | | 0.020 | | 0.035 |
| min | 0.036 | 0.036 | 0.036 | 2.792 | 0.036 | 0.644 |
| max | 221.429 | 346.990 | 221.429 | 346.990 | 221.429 | 346.990 |
| n (Samp) | 36 | 8 | 36 | 6 | 36 | 6 |
| n (Pat) | 36 | 8 | 36 | 6 | 36 | 6 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 16.045 | 111.111 | 16.045 | 229.051 | 16.045 | 213.426 |
| average | 35.530 | 142.312 | 35.530 | 229.051 | 35.530 | 213.426 |
| stdev | 50.240 | 146.153 | 50.240 | 166.792 | 50.240 | 188.889 |
| p (t-test) | | 0.001 | | 0.000 | | 0.000 |
| min | 0.036 | 0.036 | 0.036 | 111.111 | 0.036 | 79.861 |
| max | 270.067 | 346.990 | 270.067 | 346.990 | 270.067 | 346.990 |
| n (Samp) | 60 | 4 | 60 | 2 | 60 | 2 |
| n (Pat) | 60 | 4 | 60 | 2 | 60 | 2 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 18.156 | 37.875 | 18.156 | 16.987 | 18.156 | 14.839 |
| average | 35.686 | 47.082 | 35.686 | 36.101 | 35.686 | 34.490 |
| stdev | 47.364 | 44.175 | 47.364 | 48.833 | 47.364 | 50.196 |
| p (t-test) | | 0.655 | | 0.987 | | 0.963 |
| min | 0.036 | 4.940 | 0.036 | 2.792 | 0.036 | 0.644 |
| max | 221.429 | 107.639 | 221.429 | 107.639 | 221.429 | 107.639 |
| n (Samp) | 26 | 4 | 26 | 4 | 26 | 4 |
| n (Pat) | 26 | 4 | 26 | 4 | 26 | 4 |

sCr or UO

| Time prior AKI stage | AUC | SE | nCohort 1 | nCohort 2 | p |
|---|---|---|---|---|---|
| 0 hours | 0.69 | 0.112 | 36 | 8 | 0.099 |
| 24 hours | 0.67 | 0.129 | 36 | 6 | 0.191 |
| 48 hours | 0.63 | 0.131 | 36 | 6 | 0.340 |

sCr only

| Time prior AKI stage | AUC | SE | nCohort 1 | nCohort 2 | p |
|---|---|---|---|---|---|
| 0 hours | 0.74 | 0.146 | 60 | 4 | 0.098 |
| 24 hours | 0.98 | 0.072 | 60 | 2 | 0.000 |
| 48 hours | 0.93 | 0.130 | 60 | 2 | 0.001 |

UO only

| Time prior AKI stage | AUC | SE | nCohort 1 | nCohort 2 | p |
|---|---|---|---|---|---|
| 0 hours | 0.63 | 0.160 | 26 | 4 | 0.401 |
| 24 hours | 0.51 | 0.158 | 26 | 4 | 0.952 |
| 48 hours | 0.45 | 0.153 | 26 | 4 | 0.754 |

sCr or UO

| Time prior AKI stage | Cutoff value | sens | spec | Quartile | OR | 95% CI of OR | |
|---|---|---|---|---|---|---|---|
| 0 hours | 23.393855 | 75% | 58% | 1 | | | |

**FIG. 4 - 2**

sCr only

| | 2.7920962 | 88% | 22% | 2 | 0.0 | 0.0 | na |
|---|---|---|---|---|---|---|---|
| | 0 | 100% | 0% | 3 | 1.0 | 0.1 | 11.0 |
| | 44.318182 | 63% | 72% | 4 | 2.6 | 0.4 | 18.4 |
| | 60.897436 | 50% | 81% | | | | |
| | 81.597222 | 50% | 92% | | | | |
| 24 hours | 6.0137457 | 83% | 28% | 1 | | | |
| | 6.0137457 | 83% | 28% | 2 | 0.9 | 0.0 | 68.6 |
| | 2.4441341 | 100% | 19% | 3 | 1.0 | 0.0 | 77.9 |
| | 44.318182 | 50% | 72% | 4 | 3.4 | 0.2 | 73.1 |
| | 60.897436 | 50% | 81% | | | | |
| | 81.597222 | 50% | 92% | | | | |
| 48 hours | 2.4441341 | 83% | 19% | 1 | | | |
| | 2.4441341 | 83% | 19% | 2 | 0.0 | 0.0 | na |
| | 0.0360285 | 100% | 8% | 3 | 0.4 | 0.0 | 13.4 |
| | 44.318182 | 50% | 72% | 4 | 1.5 | 0.2 | 12.5 |
| | 60.897436 | 50% | 81% | | | | |
| | 81.597222 | 33% | 92% | | | | |

sCr only

| Time prior AKI stage | Cutoff value | sens | spec | Quartile | OR | 95% CI of OR | |
|---|---|---|---|---|---|---|---|
| 0 hours | 107.63889 | 75% | 95% | 1 | | | |
| | 0 | 100% | 0% | 2 | 0.0 | 0.0 | na |
| | 0 | 100% | 0% | 3 | 0.0 | 0.0 | na |
| | 37.5 | 75% | 70% | 4 | 3.5 | 0.2 | 62.6 |
| | 60.897436 | 75% | 80% | | | | |
| | 83.333333 | 75% | 90% | | | | |
| 24 hours | 107.63889 | 100% | 95% | 1 | | | |
| | 107.63889 | 100% | 95% | 2 | na | na | na |
| | 107.63889 | 100% | 95% | 3 | na | na | na |
| | 37.5 | 100% | 70% | 4 | na | na | na |
| | 60.897436 | 100% | 80% | | | | |
| | 83.333333 | 100% | 90% | | | | |
| 48 hours | 76.388889 | 100% | 85% | 1 | | | |
| | 76.388889 | 100% | 85% | 2 | na | na | na |
| | 76.388889 | 100% | 85% | 3 | na | na | na |
| | 37.5 | 100% | 70% | 4 | na | na | na |
| | 60.897436 | 100% | 80% | | | | |
| | 83.333333 | 50% | 90% | | | | |

UO only

| Time prior AKI stage | Cutoff value | sens | spec | Quartile | OR | 95% CI of OR | |
|---|---|---|---|---|---|---|---|
| 0 hours | 23.393855 | 75% | 62% | 1 | | | |
| | 2.7920962 | 100% | 23% | 2 | 0.0 | 0.0 | na |
| | 2.7920962 | 100% | 23% | 3 | 2.4 | 0.1 | 93.1 |
| | 41.666667 | 50% | 73% | 4 | 0.9 | 0.0 | 79.2 |
| | 60.897436 | 25% | 81% | | | | |
| | 81.597222 | 25% | 92% | | | | |
| 24 hours | 6.0137457 | 75% | 27% | 1 | | | |
| | 2.4441341 | 100% | 15% | 2 | 0.9 | 0.0 | 79.2 |
| | 2.4441341 | 100% | 15% | 3 | 1.0 | 0.0 | 96.9 |
| | 41.666667 | 25% | 73% | 4 | 0.9 | 0.0 | 79.2 |
| | 60.897436 | 25% | 81% | | | | |
| | 81.597222 | 25% | 92% | | | | |
| 48 hours | 2.4441341 | 75% | 15% | 1 | | | |
| | 0.0360285 | 100% | 4% | 2 | 1.2 | 0.0 | 107.9 |
| | 0.0360285 | 100% | 4% | 3 | 0.0 | 0.0 | na |
| | 41.666667 | 25% | 73% | 4 | 2.8 | 0.1 | 103.7 |
| | 60.897436 | 25% | 81% | | | | |
| | 81.597222 | 25% | 92% | | | | |

**Tubulointerstitial nephritis antigen**

sCr or
UO

# FIG. 4 - 3

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.431 | 3.314 | 0.431 | 2.170 | 0.431 | 3.314 |
| average | 9.166 | 20.058 | 9.166 | 7.413 | 9.166 | 10.517 |
| stdev | 61.004 | 41.398 | 61.004 | 10.032 | 61.004 | 11.651 |
| p (t-test) |  | 0.572 |  | 0.925 |  | 0.954 |
| min | 0.000 | 0.436 | 0.000 | 0.436 | 0.000 | 0.673 |
| max | 480.918 | 141.270 | 480.918 | 27.778 | 480.918 | 27.778 |
| n (Samp) | 62 | 11 | 62 | 11 | 62 | 7 |
| n (Pat) | 62 | 11 | 62 | 11 | 62 | 7 |

sCr only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.553 | 11.172 | 0.553 | 11.172 | 0.553 | 11.552 |
| average | 7.757 | 32.110 | 7.757 | 32.110 | 7.757 | 12.929 |
| stdev | 48.692 | 54.742 | 48.692 | 54.742 | 48.692 | 13.498 |
| p (t-test) |  | 0.239 |  | 0.239 |  | 0.833 |
| min | 0.000 | 0.436 | 0.000 | 0.436 | 0.000 | 0.835 |
| max | 480.918 | 141.270 | 480.918 | 141.270 | 480.918 | 27.778 |
| n (Samp) | 106 | 6 | 106 | 6 | 106 | 4 |
| n (Pat) | 106 | 6 | 106 | 6 | 106 | 4 |

UO only

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.481 | 3.906 | 0.481 | 3.314 | 0.481 | 3.314 |
| average | 11.094 | 27.169 | 11.094 | 7.297 | 11.094 | 9.001 |
| stdev | 67.911 | 51.026 | 67.911 | 8.790 | 67.911 | 10.141 |
| p (t-test) |  | 0.550 |  | 0.884 |  | 0.946 |
| min | 0.000 | 0.673 | 0.000 | 0.673 | 0.000 | 0.673 |
| max | 480.918 | 141.270 | 480.918 | 20.934 | 480.918 | 20.934 |
| n (Samp) | 50 | 7 | 50 | 7 | 50 | 5 |
| n (Pat) | 50 | 7 | 50 | 7 | 50 | 5 |

sCr or UO

| Time prior AKI stage | AUC | SE | nCohort 1 | nCohort 2 | p |
|---|---|---|---|---|---|
| 0 hours | 0.82 | 0.081 | 62 | 11 | 0.000 |
| 24 hours | 0.81 | 0.082 | 62 | 11 | 0.000 |
| 48 hours | 0.81 | 0.101 | 62 | 7 | 0.002 |

sCr only

| Time prior AKI stage | AUC | SE | nCohort 1 | nCohort 2 | p |
|---|---|---|---|---|---|
| 0 hours | 0.80 | 0.111 | 106 | 6 | 0.007 |
| 24 hours | 0.80 | 0.111 | 106 | 6 | 0.007 |
| 48 hours | 0.85 | 0.123 | 106 | 4 | 0.005 |

UO only

| Time prior AKI stage | AUC | SE | nCohort 1 | nCohort 2 | p |
|---|---|---|---|---|---|
| 0 hours | 0.84 | 0.096 | 50 | 7 | 0.000 |
| 24 hours | 0.83 | 0.098 | 50 | 7 | 0.001 |
| 48 hours | 0.80 | 0.122 | 50 | 5 | 0.012 |

# FIG. 4 - 4

**Epidermal growth factor**

sCr or UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 86.700 | 62.300 | 86.700 | 58.500 | 86.700 | 69.700 |
| average | 140.511 | 94.066 | 140.511 | 91.869 | 140.511 | 102.827 |
| stdev | 162.049 | 96.009 | 162.049 | 118.520 | 162.049 | 120.782 |
| p (t-test) |  | 0.040 |  | 0.028 |  | 0.250 |
| min | 0.074 | 0.074 | 0.074 | 0.074 | 0.074 | 6.500 |
| max | 1330.000 | 477.000 | 1330.000 | 847.000 | 1330.000 | 577.000 |
| n (Samp) | 255 | 56 | 255 | 61 | 255 | 26 |
| n (Pat) | 111 | 56 | 111 | 61 | 111 | 26 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 75.700 | 68.200 | 75.700 | 68.050 | 75.700 | 78.850 |
| average | 128.800 | 111.272 | 128.800 | 91.880 | 128.800 | 96.193 |
| stdev | 152.832 | 138.412 | 152.832 | 72.955 | 152.832 | 55.422 |
| p (t-test) |  | 0.590 |  | 0.222 |  | 0.426 |
| min | 0.074 | 0.074 | 0.074 | 0.074 | 0.074 | 34.700 |
| max | 1330.000 | 601.000 | 1330.000 | 239.000 | 1330.000 | 219.000 |
| n (Samp) | 457 | 23 | 457 | 26 | 457 | 14 |
| n (Pat) | 179 | 23 | 179 | 26 | 179 | 14 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 88.100 | 61.300 | 88.100 | 67.800 | 88.100 | 55.100 |
| average | 138.633 | 85.987 | 138.633 | 107.000 | 138.633 | 100.617 |
| stdev | 161.635 | 86.129 | 161.635 | 139.082 | 161.635 | 129.099 |
| p (t-test) |  | 0.025 |  | 0.192 |  | 0.277 |
| min | 7.440 | 0.074 | 7.440 | 0.074 | 7.440 | 6.500 |
| max | 1330.000 | 477.000 | 1330.000 | 847.000 | 1330.000 | 577.000 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | nCohort 1 | nCohort 2 | p |
|---|---|---|---|---|---|
| 0 hours | 0.40 | 0.040 | 255 | 56 | 0.013 |
| 24 hours | 0.39 | 0.038 | 255 | 61 | 0.003 |
| 48 hours | 0.42 | 0.056 | 255 | 26 | 0.133 |

sCr only

| Time prior AKI stage | AUC | SE | nCohort 1 | nCohort 2 | p |
|---|---|---|---|---|---|
| 0 hours | 0.47 | 0.060 | 457 | 23 | 0.568 |
| 24 hours | 0.46 | 0.057 | 457 | 26 | 0.509 |
| 48 hours | 0.53 | 0.080 | 457 | 14 | 0.742 |

UO only

| Time prior AKI stage | AUC | SE | nCohort 1 | nCohort 2 | p |
|---|---|---|---|---|---|
| 0 hours | 0.38 | 0.041 | 213 | 51 | 0.003 |
| 24 hours | 0.40 | 0.042 | 213 | 53 | 0.020 |
| 48 hours | 0.38 | 0.057 | 213 | 23 | 0.030 |

sCr or UO

| Time prior AKI stage | Cutoff value | sens | spec | Quartile | OR | 95% CI of OR | |
|---|---|---|---|---|---|---|---|
| 0 hours | 33.8 | 71% | 20% | 1 |  |  |  |
|  | 27.1 | 80% | 14% | 2 | 1.8 | 1.2 | 2.7 |
|  | 12 | 91% | 5% | 3 | 1.5 | 1.0 | 2.4 |
|  | 157 | 21% | 70% | 4 | 2.5 | 1.7 | 3.7 |
|  | 216 | 9% | 80% |  |  |  |  |
|  | 298 | 4% | 90% |  |  |  |  |
| 24 hours | 36.7 | 70% | 22% | 1 |  |  |  |
|  | 25.2 | 80% | 12% | 2 | 1.5 | 1.0 | 2.3 |
|  | 17 | 90% | 10% | 3 | 2.3 | 1.6 | 3.4 |
|  | 157 | 18% | 70% | 4 | 2.8 | 1.9 | 4.1 |

# FIG. 5 - 1

| Time prior AKI stage | Cutoff value | sens | spec | Quartile | OR | 95% CI of OR | |
|---|---|---|---|---|---|---|---|
| | 216 | 8% | 80% | | | | |
| | 298 | 2% | 90% | | | | |
| 48 hours | 34.6 | 73% | 20% | 1 | | | |
| | 32.2 | 81% | 18% | 2 | 2.5 | 0.9 | 6.8 |
| | 18.7 | 92% | 11% | 3 | 2.1 | 0.8 | 6.0 |
| | 157 | 12% | 70% | 4 | 3.8 | 1.5 | 9.4 |
| | 216 | 12% | 80% | | | | |
| | 298 | 8% | 90% | | | | |

sCr only

| Time prior AKI stage | Cutoff value | sens | spec | Quartile | OR | 95% CI of OR | |
|---|---|---|---|---|---|---|---|
| 0 hours | 36.7 | 74% | 26% | 1 | | | |
| | 31.2 | 83% | 21% | 2 | 2.8 | 1.1 | 7.1 |
| | 27.2 | 91% | 17% | 3 | 2.1 | 0.7 | 5.7 |
| | 141 | 17% | 70% | 4 | 2.1 | 0.7 | 5.7 |
| | 201 | 13% | 80% | | | | |
| | 295 | 9% | 90% | | | | |
| 24 hours | 38.1 | 73% | 27% | 1 | | | |
| | 37.2 | 81% | 26% | 2 | 1.2 | 0.6 | 2.2 |
| | 18.4 | 92% | 12% | 3 | 1.4 | 0.7 | 2.5 |
| | 141 | 27% | 70% | 4 | 0.8 | 0.4 | 1.8 |
| | 201 | 12% | 80% | | | | |
| | 295 | 0% | 90% | | | | |
| 48 hours | 67.9 | 71% | 46% | 1 | | | |
| | 48 | 86% | 34% | 2 | 5.1 | 0.5 | 55.8 |
| | 38.5 | 93% | 27% | 3 | 6.2 | 0.6 | 63.3 |
| | 141 | 21% | 70% | 4 | 2.0 | 0.1 | 39.1 |
| | 201 | 7% | 80% | | | | |
| | 295 | 0% | 90% | | | | |

UO only

| Time prior AKI stage | Cutoff value | sens | spec | Quartile | OR | 95% CI of OR | |
|---|---|---|---|---|---|---|---|
| 0 hours | 33.8 | 71% | 17% | 1 | | | |
| | 20.5 | 80% | 10% | 2 | 2.1 | 1.3 | 3.4 |
| | 12.7 | 90% | 4% | 3 | 1.9 | 1.1 | 3.1 |
| | 154 | 18% | 70% | 4 | 3.4 | 2.2 | 5.4 |
| | 202 | 8% | 80% | | | | |
| | 295 | 2% | 90% | | | | |
| 24 hours | 37.6 | 72% | 20% | 1 | | | |
| | 25.2 | 81% | 11% | 2 | 1.1 | 0.7 | 1.8 |
| | 17.3 | 91% | 8% | 3 | 1.5 | 1.0 | 2.3 |
| | 154 | 21% | 70% | 4 | 2.1 | 1.5 | 3.1 |
| | 202 | 13% | 80% | | | | |
| | 295 | 4% | 90% | | | | |
| 48 hours | 32.2 | 74% | 16% | 1 | | | |
| | 30.2 | 83% | 15% | 2 | 1.4 | 0.4 | 4.6 |
| | 18.7 | 91% | 9% | 3 | 2.1 | 0.7 | 6.1 |
| | 154 | 22% | 70% | 4 | 3.8 | 1.5 | 9.6 |
| | 202 | 13% | 80% | | | | |
| | 295 | 9% | 90% | | | | |

**Interleukin-1 alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.002 | 0.000 | 0.002 | 0.000 | 0.002 | 0.000 |
| average | 0.003 | 0.002 | 0.003 | 0.002 | 0.003 | 0.001 |
| stdev | 0.004 | 0.003 | 0.004 | 0.003 | 0.004 | 0.002 |
| p (t-test) | | 0.005 | | 0.142 | | 0.009 |
| min | 0.000 | 0.000 | 0.000 | 0.000 | 0.000 | 0.000 |
| max | 0.020 | 0.010 | 0.020 | 0.009 | 0.020 | 0.007 |
| n (Samp) | 255 | 56 | 255 | 61 | 255 | 26 |
| n (Pat) | 111 | 56 | 111 | 61 | 111 | 26 |

## FIG. 5 - 2

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.001 | 0.000 | 0.001 | 0.001 | 0.001 | 0.000 |
| average | 0.003 | 0.002 | 0.003 | 0.002 | 0.003 | 0.002 |
| stdev | 0.003 | 0.003 | 0.003 | 0.003 | 0.003 | 0.002 |
| p (t-test) | | 0.086 | | 0.520 | | 0.220 |
| min | 0.000 | 0.000 | 0.000 | 0.000 | 0.000 | 0.000 |
| max | 0.020 | 0.011 | 0.020 | 0.009 | 0.020 | 0.007 |
| n (Samp) | 457 | 23 | 457 | 26 | 457 | 14 |
| n (Pat) | 179 | 23 | 179 | 26 | 179 | 14 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.003 | 0.000 | 0.003 | 0.000 | 0.003 | 0.000 |
| average | 0.003 | 0.002 | 0.003 | 0.002 | 0.003 | 0.001 |
| stdev | 0.004 | 0.003 | 0.004 | 0.003 | 0.004 | 0.002 |
| p (t-test) | | 0.022 | | 0.161 | | 0.007 |
| min | 0.000 | 0.000 | 0.000 | 0.000 | 0.000 | 0.000 |
| max | 0.020 | 0.009 | 0.020 | 0.009 | 0.020 | 0.006 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | nCohort 1 | nCohort 2 | p |
|---|---|---|---|---|---|
| 0 hours | 0.39 | 0.040 | 255 | 56 | 0.007 |
| 24 hours | 0.46 | 0.040 | 255 | 61 | 0.293 |
| 48 hours | 0.35 | 0.052 | 255 | 26 | 0.005 |

sCr only

| Time prior AKI stage | AUC | SE | nCohort 1 | nCohort 2 | p |
|---|---|---|---|---|---|
| 0 hours | 0.37 | 0.054 | 457 | 23 | 0.020 |
| 24 hours | 0.48 | 0.057 | 457 | 26 | 0.674 |
| 48 hours | 0.42 | 0.073 | 457 | 14 | 0.293 |

UO only

| Time prior AKI stage | AUC | SE | nCohort 1 | nCohort 2 | p |
|---|---|---|---|---|---|
| 0 hours | 0.41 | 0.043 | 213 | 51 | 0.034 |
| 24 hours | 0.46 | 0.043 | 213 | 53 | 0.311 |
| 48 hours | 0.33 | 0.053 | 213 | 23 | 0.001 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.4 | 0.9 | 2.2 |
| | 0 | 100% | 0% | 3 | 2.8 | 1.9 | 4.1 |
| | 0.0045 | 16% | 70% | 4 | 1.7 | 1.1 | 2.6 |
| | 0.00581 | 9% | 80% | | | | |
| | 0.00749 | 4% | 90% | | | | |
| 24 hours | 0 | 100% | 0% | 1 | | | |
| | 0 | 100% | 0% | 2 | 1.6 | 1.1 | 2.3 |
| | 0 | 100% | 0% | 3 | 2.4 | 1.7 | 3.3 |
| | 0.0045 | 25% | 70% | 4 | 1.1 | 0.7 | 1.7 |
| | 0.00581 | 15% | 80% | | | | |
| | 0.00749 | 5% | 90% | | | | |
| 48 hours | 0 | 100% | 0% | 1 | | | |
| | 0 | 100% | 0% | 2 | 2.7 | 0.6 | 11.1 |
| | 0 | 100% | 0% | 3 | 12.9 | 4.1 | 40.6 |
| | 0.0045 | 8% | 70% | 4 | 0.0 | 0.0 | na |
| | 0.00581 | 8% | 80% | | | | |
| | 0.00749 | 0% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |

**FIG. 5 - 3**

| | Cutoff value | sens | spec | Quartile | OR | 95% CI of OR | |
|---|---|---|---|---|---|---|---|
| | 0 | 100% | 0% | 2 | 0.7 | 0.2 | 2.4 |
| | 0 | 100% | 0% | 3 | 4.5 | 2.3 | 8.5 |
| | 0.00432 | 17% | 70% | 4 | 0.0 | 0.0 | na |
| | 0.00552 | 13% | 80% | | | | |
| | 0.00689 | 9% | 90% | | | | |
| 24 hours | 0 | 100% | 0% | 1 | | | |
| | 0 | 100% | 0% | 2 | 1.6 | 0.8 | 3.2 |
| | 0 | 100% | 0% | 3 | 2.8 | 1.6 | 5.0 |
| | 0.00432 | 27% | 70% | 4 | 0.0 | 0.0 | na |
| | 0.00552 | 12% | 80% | | | | |
| | 0.00689 | 8% | 90% | | | | |
| 48 hours | 0 | 100% | 0% | 1 | | | |
| | 0 | 100% | 0% | 2 | 5.2 | 0.5 | 56.3 |
| | 0 | 100% | 0% | 3 | 8.5 | 0.9 | 79.9 |
| | 0.00432 | 7% | 70% | 4 | 0.0 | 0.0 | na |
| | 0.00552 | 7% | 80% | | | | |
| | 0.00689 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.1 | 0.7 | 1.8 |
| | 0 | 100% | 0% | 3 | 1.4 | 0.9 | 2.2 |
| | 0.00463 | 18% | 71% | 4 | 2.8 | 1.9 | 4.1 |
| | 0.00565 | 12% | 80% | | | | |
| | 0.00716 | 4% | 90% | | | | |
| 24 hours | 0 | 100% | 0% | 1 | | | |
| | 0 | 100% | 0% | 2 | 0.9 | 0.6 | 1.4 |
| | 0 | 100% | 0% | 3 | 3.7 | 2.7 | 5.1 |
| | 0.00463 | 26% | 71% | 4 | 0.0 | 0.0 | na |
| | 0.00565 | 17% | 80% | | | | |
| | 0.00716 | 4% | 90% | | | | |
| 48 hours | 0 | 100% | 0% | 1 | | | |
| | 0 | 100% | 0% | 2 | 1.0 | 0.1 | 7.6 |
| | 0 | 100% | 0% | 3 | 13.5 | 4.2 | 43.5 |
| | 0.00463 | 9% | 71% | 4 | 0.0 | 0.0 | na |
| | 0.00565 | 9% | 80% | | | | |
| | 0.00716 | 0% | 90% | | | | |

**Interleukin-4**

sCr or UO

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 46.700 | 34.500 | 46.700 | 40.700 | 46.700 | 46.900 |
| average | 49.356 | 32.492 | 49.356 | 41.603 | 49.356 | 42.916 |
| stdev | 36.161 | 26.650 | 36.161 | 25.553 | 36.161 | 21.763 |
| p (t-test) | | 0.001 | | 0.115 | | 0.374 |
| min | 1.040 | 1.040 | 1.040 | 1.040 | 1.040 | 1.040 |
| max | 370.000 | 106.000 | 370.000 | 93.500 | 370.000 | 83.800 |
| n (Samp) | 255 | 56 | 255 | 61 | 255 | 26 |
| n (Pat) | 111 | 56 | 111 | 61 | 111 | 26 |

sCr only

| | 0 hr prior to AKI stage | | 24 hr prior to AKI stage | | 48 hr prior to AKI stage | |
| | Cohort 1 | Cohort 2 | Cohort 1 | Cohort 2 | Cohort 1 | Cohort 2 |
|---|---|---|---|---|---|---|
| median | 46.900 | 38.000 | 46.900 | 38.250 | 46.900 | 33.900 |
| average | 48.883 | 39.743 | 48.883 | 40.913 | 48.883 | 33.516 |
| stdev | 39.541 | 34.308 | 39.541 | 25.682 | 39.541 | 21.309 |
| p (t-test) | | 0.277 | | 0.311 | | 0.149 |
| min | 1.040 | 1.040 | 1.040 | 1.040 | 1.040 | 1.040 |
| max | 451.000 | 140.000 | 451.000 | 93.500 | 451.000 | 69.100 |
| n (Samp) | 457 | 23 | 457 | 26 | 457 | 14 |
| n (Pat) | 179 | 23 | 179 | 26 | 179 | 14 |

UO only

| 0 hr prior to AKI stage | 24 hr prior to AKI stage | 48 hr prior to AKI stage |
|---|---|---|

# FIG. 5 - 4

|  | Cohort 1 | Cohort 2 | Cohort 1 | Cohort 2 | Cohort 1 | Cohort 2 |
|---|---|---|---|---|---|---|
| median | 45.900 | 33.000 | 45.900 | 49.400 | 45.900 | 48.100 |
| average | 47.955 | 33.498 | 47.955 | 42.768 | 47.955 | 47.997 |
| stdev | 38.165 | 26.840 | 38.165 | 28.493 | 38.165 | 17.540 |
| p (t-test) |  | 0.011 |  | 0.355 |  | 0.996 |
| min | 1.040 | 1.040 | 1.040 | 1.040 | 1.040 | 1.040 |
| max | 370.000 | 106.000 | 370.000 | 109.000 | 370.000 | 83.800 |
| n (Samp) | 213 | 51 | 213 | 53 | 213 | 23 |
| n (Pat) | 89 | 51 | 89 | 53 | 89 | 23 |

sCr or UO

| Time prior AKI stage | AUC | SE | nCohort 1 | nCohort 2 | p |
|---|---|---|---|---|---|
| 0 hours | 0.35 | 0.038 | 255 | 56 | 0.000 |
| 24 hours | 0.44 | 0.040 | 255 | 61 | 0.122 |
| 48 hours | 0.45 | 0.058 | 255 | 26 | 0.416 |

sCr only

| Time prior AKI stage | AUC | SE | nCohort 1 | nCohort 2 | p |
|---|---|---|---|---|---|
| 0 hours | 0.42 | 0.058 | 457 | 23 | 0.174 |
| 24 hours | 0.44 | 0.056 | 457 | 26 | 0.291 |
| 48 hours | 0.37 | 0.068 | 457 | 14 | 0.048 |

UO only

| Time prior AKI stage | AUC | SE | nCohort 1 | nCohort 2 | p |
|---|---|---|---|---|---|
| 0 hours | 0.38 | 0.041 | 213 | 51 | 0.003 |
| 24 hours | 0.47 | 0.044 | 213 | 53 | 0.501 |
| 48 hours | 0.53 | 0.064 | 213 | 23 | 0.659 |

sCr or UO

| Time prior AKI stage | Cutoff value | sens | spec | Quartile | OR | 95% CI of OR | |
|---|---|---|---|---|---|---|---|
| 0 hours | 10.9 | 71% | 15% | 1 |  |  |  |
|  | 0 | 100% | 0% | 2 | 3.8 | 2.1 | 6.7 |
|  | 0 | 100% | 0% | 3 | 2.9 | 1.6 | 5.3 |
|  | 64.2 | 9% | 70% | 4 | 5.8 | 3.4 | 10.1 |
|  | 73.3 | 4% | 80% |  |  |  |  |
|  | 86.2 | 4% | 91% |  |  |  |  |
| 24 hours | 31 | 72% | 26% | 1 |  |  |  |
|  | 10.9 | 80% | 15% | 2 | 2.3 | 1.6 | 3.3 |
|  | 0 | 100% | 0% | 3 | 1.6 | 1.1 | 2.4 |
|  | 64.2 | 20% | 70% | 4 | 1.8 | 1.2 | 2.6 |
|  | 73.3 | 7% | 80% |  |  |  |  |
|  | 86.2 | 2% | 91% |  |  |  |  |
| 48 hours | 32.5 | 77% | 29% | 1 |  |  |  |
|  | 31 | 81% | 26% | 2 | 2.8 | 1.3 | 5.9 |
|  | 0 | 100% | 0% | 3 | 1.9 | 0.8 | 4.3 |
|  | 64.2 | 15% | 70% | 4 | 1.3 | 0.5 | 3.3 |
|  | 73.3 | 8% | 80% |  |  |  |  |
|  | 86.2 | 0% | 91% |  |  |  |  |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 0.8 | 0.3 | 2.0 |
|  | 0 | 100% | 0% | 3 | 1.4 | 0.7 | 2.9 |
|  | 63.2 | 22% | 70% | 4 | 1.4 | 0.7 | 2.9 |
|  | 70.5 | 13% | 80% |  |  |  |  |
|  | 86.2 | 4% | 90% |  |  |  |  |
| 24 hours | 31 | 73% | 28% | 1 |  |  |  |
|  | 22.7 | 81% | 23% | 2 | 0.7 | 0.3 | 1.5 |
|  | 0 | 100% | 0% | 3 | 1.7 | 1.0 | 3.0 |
|  | 63.2 | 23% | 70% | 4 | 1.0 | 0.5 | 2.0 |
|  | 70.5 | 12% | 80% |  |  |  |  |
|  | 86.2 | 4% | 90% |  |  |  |  |
| 48 hours | 31 | 71% | 28% | 1 |  |  |  |
|  | 0 | 100% | 0% | 2 | 3.1 | 0.2 | 43.1 |

# FIG. 5 - 5

| | | | | | | |
|---|---|---|---|---|---|---|
| 0 | 100% | 0% | 3 | 6.3 | 0.6 | 63.8 |
| 63.2 | 7% | 70% | 4 | 4.1 | 0.3 | 49.7 |
| 70.5 | 0% | 80% | | | | |
| 86.2 | 0% | 90% | | | | |

UO only

| Time prior AKI stage | Cutoff value | sens | spec | Quartile | OR | 95% CI of OR | |
|---|---|---|---|---|---|---|---|
| 0 hours | 13.4 | 71% | 21% | 1 | | | |
| | 0 | 100% | 0% | 2 | 3.3 | 1.8 | 6.0 |
| | 0 | 100% | 0% | 3 | 3.0 | 1.6 | 5.5 |
| | 63 | 16% | 70% | 4 | 4.9 | 2.8 | 8.7 |
| | 72.2 | 8% | 80% | | | | |
| | 84.3 | 4% | 90% | | | | |
| 24 hours | 28.2 | 72% | 27% | 1 | | | |
| | 9.1 | 81% | 17% | 2 | 1.8 | 1.2 | 2.6 |
| | 0 | 100% | 0% | 3 | 1.0 | 0.7 | 1.5 |
| | 63 | 26% | 70% | 4 | 1.4 | 0.9 | 2.0 |
| | 72.2 | 15% | 80% | | | | |
| | 84.3 | 6% | 90% | | | | |
| 48 hours | 45.9 | 74% | 52% | 1 | | | |
| | 33 | 83% | 30% | 2 | 2.6 | 0.6 | 11.2 |
| | 31 | 91% | 28% | 3 | 8.1 | 2.4 | 26.9 |
| | 63 | 17% | 70% | 4 | 1.5 | 0.3 | 8.4 |
| | 72.2 | 9% | 80% | | | | |
| | 84.3 | 0% | 90% | | | | |

**Netrin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 4.105 | 1.386 | 4.105 | 16.012 | 4.105 | 1.494 |
| stdev | 9.254 | 2.678 | 9.254 | 61.390 | 9.254 | 2.798 |
| p (t-test) | | 0.225 | | 0.161 | | 0.434 |
| min | 0.211 | 0.211 | 0.211 | 0.211 | 0.211 | 0.211 |
| max | 54.172 | 8.915 | 54.172 | 315.934 | 54.172 | 8.107 |
| n (Samp) | 55 | 18 | 55 | 26 | 55 | 8 |
| n (Pat) | 55 | 18 | 55 | 26 | 55 | 8 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 3.401 | 0.211 | 158.072 |
| average | 3.089 | 0.524 | 3.089 | 5.353 | 3.089 | 158.072 |
| stdev | 7.132 | 0.628 | 7.132 | 5.751 | 7.132 | 223.250 |
| p (t-test) | | 0.345 | | 0.447 | | 0.000 |
| min | 0.211 | 0.211 | 0.211 | 0.211 | 0.211 | 0.211 |
| max | 54.172 | 1.877 | 54.172 | 13.635 | 54.172 | 315.934 |
| n (Samp) | 107 | 7 | 107 | 6 | 107 | 2 |
| n (Pat) | 92 | 7 | 92 | 6 | 92 | 2 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 2.840 | 1.670 | 2.840 | 18.101 | 2.840 | 1.351 |
| stdev | 5.898 | 3.110 | 5.898 | 65.215 | 5.898 | 2.652 |
| p (t-test) | | 0.495 | | 0.106 | | 0.463 |
| min | 0.211 | 0.211 | 0.211 | 0.211 | 0.211 | 0.211 |
| max | 32.376 | 8.915 | 32.376 | 315.934 | 32.376 | 8.107 |
| n (Samp) | 49 | 13 | 49 | 23 | 49 | 9 |
| n (Pat) | 47 | 13 | 47 | 23 | 47 | 9 |

sCr or UO

| Time prior AKI stage | AUC | SE | nCohort 1 | nCohort 2 | p |
|---|---|---|---|---|---|
| 0 hours | 0.42 | 0.075 | 55 | 18 | 0.278 |
| 24 hours | 0.55 | 0.070 | 55 | 26 | 0.478 |

## FIG. 5 - 6

sCr only

| 48 hours | 0.42 | 0.104 | 55 | 8 | 0.417 |
|---|---|---|---|---|---|

| Time prior AKI stage | AUC | SE | nCohort 1 | nCohort 2 | p |
|---|---|---|---|---|---|
| 0 hours | 0.43 | 0.107 | 107 | 7 | 0.521 |
| 24 hours | 0.67 | 0.124 | 107 | 6 | 0.159 |
| 48 hours | 0.66 | 0.213 | 107 | 2 | 0.443 |

UO only

| Time prior AKI stage | AUC | SE | nCohort 1 | nCohort 2 | p |
|---|---|---|---|---|---|
| 0 hours | 0.45 | 0.088 | 49 | 13 | 0.539 |
| 24 hours | 0.58 | 0.074 | 49 | 23 | 0.301 |
| 48 hours | 0.44 | 0.101 | 49 | 9 | 0.532 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.5 | 0.4 | 6.2 |
| | 0 | 100% | 0% | 3 | 1.5 | 0.4 | 6.2 |
| | 1.8768769 | 17% | 71% | 4 | 3.4 | 1.0 | 11.7 |
| | 4.4221698 | 11% | 80% | | | | |
| | 11.792453 | 0% | 91% | | | | |
| 24 hours | 0 | 100% | 0% | 1 | | | |
| | 0 | 100% | 0% | 2 | 0.8 | 0.3 | 2.0 |
| | 0 | 100% | 0% | 3 | 0.5 | 0.2 | 1.3 |
| | 1.8768769 | 46% | 71% | 4 | 1.4 | 0.6 | 3.1 |
| | 4.4221698 | 31% | 80% | | | | |
| | 11.792453 | 19% | 91% | | | | |
| 48 hours | 0 | 100% | 0% | 1 | | | |
| | 0 | 100% | 0% | 2 | 3.5 | 0.2 | 62.6 |
| | 0 | 100% | 0% | 3 | 3.5 | 0.2 | 62.6 |
| | 1.8768769 | 25% | 71% | 4 | 1.1 | 0.0 | 70.8 |
| | 4.4221698 | 13% | 80% | | | | |
| | 11.792453 | 0% | 91% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.3 | 0.0 | 5.6 |
| | 0 | 100% | 0% | 3 | 1.4 | 0.3 | 6.2 |
| | 0.7370283 | 23% | 71% | 4 | 2.2 | 0.5 | 8.7 |
| | 4.4221698 | 15% | 82% | | | | |
| | 11.261261 | 0% | 92% | | | | |
| 24 hours | 0 | 100% | 0% | 1 | | | |
| | 0 | 100% | 0% | 2 | 0.8 | 0.3 | 2.0 |
| | 0 | 100% | 0% | 3 | 0.2 | 0.0 | 0.9 |
| | 0.7370283 | 43% | 71% | 4 | 1.3 | 0.5 | 3.1 |
| | 4.4221698 | 35% | 82% | | | | |
| | 11.261261 | 22% | 92% | | | | |
| 48 hours | 0 | 100% | 0% | 1 | | | |
| | 0 | 100% | 0% | 2 | 0.0 | 0.0 | na |
| | 0 | 100% | 0% | 3 | 1.6 | 0.2 | 11.4 |
| | 0.7370283 | 22% | 71% | 4 | 2.6 | 0.4 | 16.0 |
| | 4.4221698 | 11% | 82% | | | | |
| | 11.261261 | 0% | 92% | | | | |

**Osteopontin**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 82732.956 | 148298.000 | 82732.956 | 126476.357 | 82732.956 | na |
| average | 97936.896 | 146269.169 | 97936.896 | 114809.657 | 97936.896 | na |
| stdev | 47673.593 | 40964.688 | 47673.593 | 51308.820 | 47673.593 | na |

## FIG. 5 - 7

| | | | | | |
|---|---|---|---|---|---|
| p (t-test) | | 0.015 | | 0.277 | na |
| min | 48003.207 | 86632.026 | 48003.207 | 41626.547 | 48003.207 | na |
| max | 198386.623 | 199267.052 | 198386.623 | 199459.230 | 198386.623 | na |
| n (Samp) | 26 | 8 | 26 | 17 | 26 | 0 |
| n (Pat) | 25 | 8 | 25 | 17 | 25 | 0 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 88676.926 | 152264.239 | 88676.926 | 126476.357 | 88676.926 | 41626.547 |
| average | 105077.742 | 152264.239 | 105077.742 | 118147.752 | 105077.742 | 105459.492 |
| stdev | 48274.555 | 45283.240 | 48274.555 | 50526.597 | 48274.555 | na |
| p (t-test) | | 0.182 | | 0.463 | | na |
| min | 41626.547 | 120244.154 | 41626.547 | 42968.358 | 41626.547 | 105459.492 |
| max | 199267.052 | 184284.325 | 199267.052 | 199459.230 | 199267.052 | 105459.492 |
| n (Samp) | 47 | 2 | 47 | 9 | 47 | 1 |
| n (Pat) | 44 | 2 | 44 | 9 | 44 | 1 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 102075.984 | 136473.394 | 102075.984 | 132828.668 | 102075.984 | 48003.207 |
| average | 108201.322 | 140838.433 | 108201.322 | 118728.451 | 108201.322 | 218222.247 |
| stdev | 52403.038 | 41018.568 | 52403.038 | 48865.063 | 52403.038 | na |
| p (t-test) | | 0.137 | | 0.571 | | na |
| min | 48003.207 | 86632.026 | 48003.207 | 41626.547 | 48003.207 | 218222.247 |
| max | 199459.230 | 199267.052 | 199459.230 | 180359.177 | 199459.230 | 218222.247 |
| n (Samp) | 27 | 7 | 27 | 11 | 27 | 1 |
| n (Pat) | 25 | 7 | 25 | 11 | 25 | 1 |

sCr or UO

| Time prior AKI stage | AUC | SE | nCohort 1 | nCohort 2 | p |
|---|---|---|---|---|---|
| 0 hours | 0.80 | 0.101 | 26 | 8 | 0.003 |
| 24 hours | 0.59 | 0.090 | 26 | 17 | 0.304 |
| 48 hours | nd | nd | 26 | 0 | nd |

sCr only

| Time prior AKI stage | AUC | SE | nCohort 1 | nCohort 2 | p |
|---|---|---|---|---|---|
| 0 hours | 0.82 | 0.186 | 47 | 2 | 0.086 |
| 24 hours | 0.57 | 0.108 | 47 | 9 | 0.504 |
| 48 hours | 0.62 | 0.305 | 47 | 1 | 0.702 |

UO only

| Time prior AKI stage | AUC | SE | nCohort 1 | nCohort 2 | p |
|---|---|---|---|---|---|
| 0 hours | 0.70 | 0.121 | 27 | 7 | 0.100 |
| 24 hours | 0.55 | 0.105 | 27 | 11 | 0.620 |
| 48 hours | 1.00 | 0.000 | 27 | 1 | nd |

sCr or UO

| Time prior AKI stage | Cutoff value | sens | spec | Quartile | OR | 95% CI of OR | |
|---|---|---|---|---|---|---|---|
| 0 hours | 113148.86 | 75% | 77% | 1 | | | |
| | 103188.14 | 88% | 62% | 2 | na | na | na |
| | 83136.946 | 100% | 54% | 3 | na | na | na |
| | 110437.36 | 75% | 73% | 4 | na | na | na |
| | 129350.4 | 63% | 81% | | | | |
| | 180841.68 | 25% | 92% | | | | |
| 24 hours | 83136.946 | 71% | 54% | 1 | | | |
| | 55731.945 | 82% | 27% | 2 | 1.3 | 0.2 | 7.3 |
| | 41626.547 | 94% | 0% | 3 | 1.3 | 0.2 | 7.3 |
| | 110437.36 | 53% | 73% | 4 | 2.8 | 0.5 | 14.6 |
| | 129350.4 | 47% | 81% | | | | |
| | 180841.68 | 6% | 92% | | | | |
| 48 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |

# FIG. 5 - 8

sCr only

| Time prior AKI stage | Cutoff value | sens | spec | Quartile | OR | 95% CI of OR | |
|---|---|---|---|---|---|---|---|
| 0 hours | 113148.86 | 100% | 68% | 1 | | | |
| | 113148.86 | 100% | 68% | 2 | na | na | na |
| | 113148.86 | 100% | 68% | 3 | na | na | na |
| | 129350.4 | 50% | 70% | 4 | na | na | na |
| | 160480.15 | 50% | 81% | | | | |
| | 180359.18 | 50% | 91% | | | | |
| 24 hours | 83136.946 | 78% | 45% | 1 | | | |
| | 49396.377 | 89% | 13% | 2 | 0.5 | 0.0 | 12.0 |
| | 41626.547 | 100% | 2% | 3 | 2.4 | 0.4 | 15.0 |
| | 129350.4 | 44% | 70% | 4 | 1.0 | 0.1 | 9.8 |
| | 160480.15 | 11% | 81% | | | | |
| | 180359.18 | 11% | 91% | | | | |
| 48 hours | 104509.04 | 100% | 62% | 1 | | | |
| | 104509.04 | 100% | 62% | 2 | na | na | na |
| | 104509.04 | 100% | 62% | 3 | na | na | na |
| | 129350.4 | 0% | 70% | 4 | na | na | na |
| | 160480.15 | 0% | 81% | | | | |
| | 180359.18 | 0% | 91% | | | | |

UO only

| Time prior AKI stage | Cutoff value | sens | spec | Quartile | OR | 95% CI of OR | |
|---|---|---|---|---|---|---|---|
| 0 hours | 110437.36 | 71% | 67% | 1 | | | |
| | 103188.14 | 86% | 56% | 2 | na | na | na |
| | 83136.946 | 100% | 44% | 3 | na | na | na |
| | 129350.4 | 57% | 70% | 4 | na | na | na |
| | 178151.08 | 29% | 81% | | | | |
| | 184284.33 | 14% | 93% | | | | |
| 24 hours | 83136.946 | 73% | 44% | 1 | | | |
| | 78055.258 | 82% | 37% | 2 | 1.5 | 0.2 | 13.4 |
| | 55731.945 | 91% | 26% | 3 | 1.8 | 0.2 | 16.4 |
| | 129350.4 | 55% | 70% | 4 | 1.5 | 0.2 | 13.4 |
| | 178151.08 | 9% | 81% | | | | |
| | 184284.33 | 0% | 93% | | | | |
| 48 hours | 199459.23 | 100% | 100% | 1 | | | |
| | 199459.23 | 100% | 100% | 2 | na | na | na |
| | 199459.23 | 100% | 100% | 3 | na | na | na |
| | 129350.4 | 100% | 70% | 4 | na | na | na |
| | 178151.08 | 100% | 81% | | | | |
| | 184284.33 | 100% | 93% | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 21.535 | 12.228 | 21.535 | 2.717 | 21.535 | 0.036 |
| average | 33.087 | 14.917 | 33.087 | 2.717 | 33.087 | 0.036 |
| stdev | 33.822 | 13.757 | 33.822 | 1.921 | 33.822 | na |
| p (t-test) | | 0.063 | | 0.213 | | na |
| min | 0.036 | 0.486 | 0.036 | 1.359 | 0.036 | 0.036 |
| max | 166.667 | 52.557 | 166.667 | 4.076 | 166.667 | 0.036 |
| n (Samp) | 57 | 13 | 57 | 2 | 57 | 1 |
| n (Pat) | 37 | 13 | 37 | 2 | 37 | 1 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 20.380 | 9.046 | 20.380 | 3.221 | 20.380 | 11.820 |
| average | 29.375 | 9.629 | 29.375 | 2.969 | 29.375 | 11.820 |
| stdev | 31.017 | 4.325 | 31.017 | 1.218 | 31.017 | 11.449 |
| p (t-test) | | 0.124 | | 0.094 | | 0.428 |
| min | 0.036 | 5.343 | 0.036 | 1.359 | 0.036 | 3.724 |
| max | 166.667 | 16.677 | 166.667 | 4.076 | 166.667 | 19.916 |

**FIG. 5 - 9**

| n (Samp) | 89 | 6 | 89 | 4 | 89 | 2 |
| n (Pat) | 61 | 6 | 61 | 4 | 61 | 2 |

UO only

| | 0 hr prior toAKI stage | | 24 hr prior toAKI stage | | 48 hr prior toAKI stage | |
| | Cohort 1 | Cohort 2 | Cohort 1 | Cohort 2 | Cohort 1 | Cohort 2 |
|---|---|---|---|---|---|---|
| median | 22.447 | 8.832 | 22.447 | 40.910 | 22.447 | 0.036 |
| average | 37.376 | 13.071 | 37.376 | 40.910 | 37.376 | 0.036 |
| stdev | 36.083 | 14.115 | 36.083 | 52.091 | 36.083 | na |
| p (t-test) | | 0.018 | | 0.894 | | na |
| min | 0.036 | 0.036 | 0.036 | 4.076 | 0.036 | 0.036 |
| max | 166.667 | 52.557 | 166.667 | 77.744 | 166.667 | 0.036 |
| n (Samp) | 42 | 14 | 42 | 2 | 42 | 1 |
| n (Pat) | 27 | 14 | 27 | 2 | 27 | 1 |

sCr or UO

| Time prior AKI stage | AUC | SE | nCohort 1 | nCohort 2 | p |
|---|---|---|---|---|---|
| 0 hours | 0.32 | 0.076 | 57 | 13 | 0.018 |
| 24 hours | 0.09 | 0.065 | 57 | 2 | 0.000 |
| 48 hours | 0.02 | 0.024 | 57 | 1 | 0.000 |

sCr only

| Time prior AKI stage | AUC | SE | nCohort 1 | nCohort 2 | p |
|---|---|---|---|---|---|
| 0 hours | 0.28 | 0.092 | 89 | 6 | 0.016 |
| 24 hours | 0.13 | 0.064 | 89 | 4 | 0.000 |
| 48 hours | 0.32 | 0.167 | 89 | 2 | 0.281 |

UO only

| Time prior AKI stage | AUC | SE | nCohort 1 | nCohort 2 | p |
|---|---|---|---|---|---|
| 0 hours | 0.23 | 0.066 | 42 | 14 | 0.000 |
| 24 hours | 0.44 | 0.202 | 42 | 2 | 0.768 |
| 48 hours | 0.01 | 0.020 | 42 | 1 | 0.000 |

sCr or UO

| Time prior AKI stage | Cutoff value | sens | spec | Quartile | OR | 95% CI of OR | |
|---|---|---|---|---|---|---|---|
| 0 hours | 3.7240933 | 77% | 12% | 1 | | | |
| | 2.7173913 | 85% | 12% | 2 | 3.6 | 0.2 | 64.2 |
| | 1.3586957 | 92% | 7% | 3 | 6.5 | 0.5 | 89.6 |
| | 38.352273 | 8% | 70% | 4 | 5.2 | 0.3 | 79.1 |
| | 53.977273 | 0% | 82% | | | | |
| | 82.417582 | 0% | 91% | | | | |
| 24 hours | 0.0360285 | 100% | 4% | 1 | | | |
| | 0.0360285 | 100% | 4% | 2 | na | na | na |
| | 0.0360285 | 100% | 4% | 3 | na | na | na |
| | 38.352273 | 0% | 70% | 4 | na | na | na |
| | 53.977273 | 0% | 82% | | | | |
| | 82.417582 | 0% | 91% | | | | |
| 48 hours | 0 | 100% | 0% | 1 | | | |
| | 0 | 100% | 0% | 2 | na | na | na |
| | 0 | 100% | 0% | 3 | na | na | na |
| | 38.352273 | 0% | 70% | 4 | na | na | na |
| | 53.977273 | 0% | 82% | | | | |
| | 82.417582 | 0% | 91% | | | | |

UO only

| Time prior AKI stage | Cutoff value | sens | spec | Quartile | OR | 95% CI of OR | |
|---|---|---|---|---|---|---|---|
| 0 hours | 3.7240933 | 71% | 5% | 1 | | | |
| | 1.3586957 | 86% | 5% | 2 | 3.5 | 0.2 | 67.2 |
| | 0.0360285 | 93% | 2% | 3 | 3.5 | 0.2 | 67.2 |
| | 42.613636 | 7% | 71% | 4 | 13.0 | 0.9 | 187.9 |
| | 53.977273 | 0% | 81% | | | | |
| | 99.085366 | 0% | 93% | | | | |
| 24 hours | 1.3586957 | 100% | 5% | 1 | | | |
| | 1.3586957 | 100% | 5% | 2 | 0.0 | 0.0 | na |
| | 1.3586957 | 100% | 5% | 3 | 0.0 | 0.0 | na |
| | 42.613636 | 50% | 71% | 4 | 1.0 | 0.0 | 74.6 |
| | 53.977273 | 50% | 81% | | | | |

# FIG. 5 - 10

| | 99.085366 | 0% | 93% | | | | |
|---|---|---|---|---|---|---|---|
| 48 hours | 0 | 100% | 0% | 1 | | | |
| | 0 | 100% | 0% | 2 | na | na | na |
| | 0 | 100% | 0% | 3 | na | na | na |
| | 42.613636 | 0% | 71% | 4 | na | na | na |
| | 53.977273 | 0% | 81% | | | | |
| | 99.085366 | 0% | 93% | | | | |

# FIG. 5 - 11

**Bone morphogenetic protein 7**

sCr or
UO

|  | 0 hr prior to AKI stage | | 24 hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| median | 0.163 | 0.163 | 0.163 | 1.508 | 0.163 | 0.163 |
| average | 2.814 | 1.063 | 2.814 | 3.505 | 2.814 | 0.163 |
| stdev | 8.264 | 2.113 | 8.264 | 4.983 | 8.264 | 0.000 |
| p (t-test) |  | 0.530 |  | 0.869 |  | 0.653 |
| min | 0.163 | 0.163 | 0.163 | 0.163 | 0.163 | 0.163 |
| max | 64.356 | 6.616 | 64.356 | 10.839 | 64.356 | 0.163 |
| n (Samp) | 80 | 9 | 80 | 4 | 80 | 2 |
| n (Pat) | 58 | 9 | 58 | 4 | 58 | 2 |

sCr only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.623 | 4.505 | 0.623 | 5.731 | 0.623 | 8.316 |
| average | 2.564 | 4.505 | 2.564 | 5.731 | 2.564 | 8.316 |
| stdev | 7.521 | 2.986 | 7.521 | 7.224 | 7.521 | 11.531 |
| p (t-test) |  | 0.717 |  | 0.557 |  | 0.290 |
| min | 0.163 | 2.393 | 0.163 | 0.623 | 0.163 | 0.163 |
| max | 64.356 | 6.616 | 64.356 | 10.839 | 64.356 | 16.470 |
| n (Samp) | 98 | 2 | 98 | 2 | 98 | 2 |
| n (Pat) | 70 | 2 | 70 | 2 | 70 | 2 |

UO only

|  | 0 hr prior toAKI stage | | 24 hr prior toAKI stage | | 48 hr prior toAKI stage | |
|---|---|---|---|---|---|---|
|  | Cohort 1 | Cohort 2 | Cohort 1 | Cohort 2 | Cohort 1 | Cohort 2 |
| median | 0.623 | 0.163 | 0.623 | 1.278 | 0.623 | 0.163 |
| average | 3.333 | 1.063 | 3.333 | 1.278 | 3.333 | 0.163 |
| stdev | 9.312 | 2.113 | 9.312 | 1.577 | 9.312 | na |
| p (t-test) |  | 0.471 |  | 0.758 |  | na |
| min | 0.163 | 0.163 | 0.163 | 0.163 | 0.163 | 0.163 |
| max | 64.356 | 6.616 | 64.356 | 2.393 | 64.356 | 0.163 |
| n (Samp) | 62 | 9 | 62 | 2 | 62 | 1 |
| n (Pat) | 45 | 9 | 45 | 2 | 45 | 1 |

sCr or UO

| Time prior AKI stage | AUC | SE | nCohort 1 | nCohort 2 | p |
|---|---|---|---|---|---|
| 0 hours | 0.41 | 0.095 | 80 | 9 | 0.355 |
| 24 hours | 0.63 | 0.154 | 80 | 4 | 0.416 |
| 48 hours | 0.26 | 0.145 | 80 | 2 | 0.092 |

sCr only

| Time prior AKI stage | AUC | SE | nCohort 1 | nCohort 2 | p |
|---|---|---|---|---|---|
| 0 hours | 0.85 | 0.172 | 98 | 2 | 0.040 |
| 24 hours | 0.74 | 0.204 | 98 | 2 | 0.234 |
| 48 hours | 0.61 | 0.215 | 98 | 2 | 0.618 |

UO only

| Time prior AKI stage | AUC | SE | nCohort 1 | nCohort 2 | p |
|---|---|---|---|---|---|
| 0 hours | 0.39 | 0.095 | 62 | 9 | 0.246 |
| 24 hours | 0.49 | 0.208 | 62 | 2 | 0.969 |
| 48 hours | 0.23 | 0.191 | 62 | 1 | 0.162 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 |  |  |  |
|  | 0 | 100% | 0% | 2 | 2.2 | 0.1 | 50.2 |
|  | 0 | 100% | 0% | 3 | 4.9 | 0.3 | 69.1 |
|  | 1.3354701 | 11% | 70% | 4 | 2.2 | 0.1 | 50.2 |
|  | 2.7599715 | 11% | 80% |  |  |  |  |
|  | 5.2083333 | 11% | 93% |  |  |  |  |
| 24 hours | 0.1629274 | 75% | 51% | 1 |  |  |  |
|  | 0 | 100% | 0% | 2 | na | na | na |
|  | 0 | 100% | 0% | 3 | na | na | na |

# FIG. 6 - 1

| | Cutoff value | sens | spec | Quartile | OR | 95% CI of OR | |
|---|---|---|---|---|---|---|---|
| | 1.3354701 | 50% | 70% | 4 | na | na | na |
| | 2.7599715 | 25% | 80% | | | | |
| | 5.2083333 | 25% | 93% | | | | |
| 48 hours | 0 | 100% | 0% | 1 | | | |
| | 0 | 100% | 0% | 2 | na | na | na |
| | 0 | 100% | 0% | 3 | na | na | na |
| | 1.3354701 | 0% | 70% | 4 | na | na | na |
| | 2.7599715 | 0% | 80% | | | | |
| | 5.2083333 | 0% | 93% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 2.1 | 0.1 | 51.0 |
| | 0 | 100% | 0% | 3 | 1.0 | 0.0 | 63.5 |
| | 2.0477208 | 11% | 73% | 4 | 7.1 | 0.5 | 98.3 |
| | 3.8006757 | 11% | 82% | | | | |
| | 5.2083333 | 11% | 90% | | | | |
| 24 hours | 0 | 100% | 0% | 1 | | | |
| | 0 | 100% | 0% | 2 | na | na | na |
| | 0 | 100% | 0% | 3 | na | na | na |
| | 2.0477208 | 50% | 73% | 4 | na | na | na |
| | 3.8006757 | 0% | 82% | | | | |
| | 5.2083333 | 0% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 2.0477208 | 0% | 73% | 4 | na | na | na |
| | 3.8006757 | 0% | 82% | | | | |
| | 5.2083333 | 0% | 90% | | | | |

**Complement C3**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.804 | 0.821 | 0.804 | 0.788 | 0.804 | 0.848 |
| average | 0.819 | 0.812 | 0.819 | 0.785 | 0.819 | 0.858 |
| stdev | 0.255 | 0.252 | 0.255 | 0.271 | 0.255 | 0.310 |
| p (t-test) | | 0.889 | | 0.445 | | 0.527 |
| min | 0.186 | 0.402 | 0.186 | 0.269 | 0.186 | 0.290 |
| max | 1.720 | 1.580 | 1.720 | 1.470 | 1.720 | 1.580 |
| n (Samp) | 434 | 28 | 434 | 36 | 434 | 18 |
| n (Pat) | 173 | 28 | 173 | 36 | 173 | 18 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.798 | 0.606 | 0.798 | 0.850 | 0.798 | 0.845 |
| average | 0.810 | 0.638 | 0.810 | 0.825 | 0.810 | 0.860 |
| stdev | 0.262 | 0.116 | 0.262 | 0.273 | 0.262 | 0.131 |
| p (t-test) | | 0.110 | | 0.860 | | 0.616 |
| min | 0.186 | 0.538 | 0.186 | 0.466 | 0.186 | 0.633 |
| max | 1.730 | 0.864 | 1.730 | 1.220 | 1.730 | 1.030 |
| n (Samp) | 542 | 6 | 542 | 10 | 542 | 7 |
| n (Pat) | 208 | 6 | 208 | 10 | 208 | 7 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.794 | 0.867 | 0.794 | 0.768 | 0.794 | 0.845 |
| average | 0.803 | 0.832 | 0.803 | 0.766 | 0.803 | 0.831 |
| stdev | 0.241 | 0.249 | 0.241 | 0.251 | 0.241 | 0.334 |
| p (t-test) | | 0.542 | | 0.414 | | 0.659 |
| min | 0.216 | 0.402 | 0.216 | 0.269 | 0.216 | 0.290 |
| max | 1.680 | 1.580 | 1.680 | 1.470 | 1.680 | 1.580 |
| n (Samp) | 356 | 27 | 356 | 32 | 356 | 16 |
| n (Pat) | 138 | 27 | 138 | 32 | 138 | 16 |

sCr or UO

# FIG. 6 - 2

| Time prior AKI stage | AUC | SE | nCohort 1 | nCohort 2 | p |
|---|---|---|---|---|---|
| 0 hours | 0.49 | 0.056 | 434 | 28 | 0.832 |
| 24 hours | 0.47 | 0.049 | 434 | 36 | 0.480 |
| 48 hours | 0.54 | 0.071 | 434 | 18 | 0.615 |

sCr only

| Time prior AKI stage | AUC | SE | nCohort 1 | nCohort 2 | p |
|---|---|---|---|---|---|
| 0 hours | 0.27 | 0.085 | 542 | 6 | 0.007 |
| 24 hours | 0.52 | 0.093 | 542 | 10 | 0.842 |
| 48 hours | 0.59 | 0.114 | 542 | 7 | 0.416 |

UO only

| Time prior AKI stage | AUC | SE | nCohort 1 | nCohort 2 | p |
|---|---|---|---|---|---|
| 0 hours | 0.54 | 0.059 | 356 | 27 | 0.515 |
| 24 hours | 0.46 | 0.052 | 356 | 32 | 0.432 |
| 48 hours | 0.51 | 0.074 | 356 | 16 | 0.845 |

sCr or UO

| Time prior AKI stage | Cutoff value | sens | spec | Quartile | OR | 95% CI of OR | |
|---|---|---|---|---|---|---|---|
| 0 hours | 0.652 | 71% | 25% | 1 | | | |
| | 0.602 | 82% | 19% | 2 | 3.0 | 1.5 | 6.0 |
| | 0.518 | 93% | 11% | 3 | 1.5 | 0.7 | 3.6 |
| | 0.931 | 32% | 70% | 4 | 1.8 | 0.8 | 4.1 |
| | 1.03 | 11% | 82% | | | | |
| | 1.16 | 7% | 90% | | | | |
| 24 hours | 0.637 | 72% | 23% | 1 | | | |
| | 0.508 | 81% | 10% | 2 | 1.1 | 0.7 | 1.9 |
| | 0.453 | 92% | 7% | 3 | 1.0 | 0.6 | 1.7 |
| | 0.931 | 28% | 70% | 4 | 1.4 | 0.9 | 2.3 |
| | 1.03 | 17% | 82% | | | | |
| | 1.16 | 8% | 90% | | | | |
| 48 hours | 0.717 | 72% | 35% | 1 | | | |
| | 0.632 | 83% | 23% | 2 | 0.7 | 0.2 | 2.4 |
| | 0.446 | 94% | 6% | 3 | 1.8 | 0.8 | 4.0 |
| | 0.931 | 39% | 70% | 4 | 1.0 | 0.4 | 2.8 |
| | 1.03 | 11% | 82% | | | | |
| | 1.16 | 11% | 90% | | | | |

sCr only

| Time prior AKI stage | Cutoff value | sens | spec | Quartile | OR | 95% CI of OR | |
|---|---|---|---|---|---|---|---|
| 0 hours | 0.568 | 83% | 18% | 1 | | | |
| | 0.568 | 83% | 18% | 2 | na | na | na |
| | 0.536 | 100% | 15% | 3 | na | na | na |
| | 0.931 | 0% | 70% | 4 | na | na | na |
| | 1.01 | 0% | 80% | | | | |
| | 1.15 | 0% | 91% | | | | |
| 24 hours | 0.65 | 70% | 27% | 1 | | | |
| | 0.568 | 80% | 18% | 2 | 0.3 | 0.0 | 4.6 |
| | 0.491 | 90% | 11% | 3 | 1.0 | 0.3 | 3.8 |
| | 0.931 | 30% | 70% | 4 | 1.0 | 0.3 | 3.8 |
| | 1.01 | 30% | 80% | | | | |
| | 1.15 | 10% | 91% | | | | |
| 48 hours | 0.831 | 71% | 57% | 1 | | | |
| | 0.799 | 86% | 51% | 2 | 0.0 | 0.0 | na |
| | 0.632 | 100% | 25% | 3 | 4.1 | 0.3 | 48.8 |
| | 0.931 | 29% | 70% | 4 | 2.0 | 0.1 | 38.9 |
| | 1.01 | 14% | 80% | | | | |
| | 1.15 | 0% | 91% | | | | |

UO only

| Time prior AKI stage | Cutoff value | sens | spec | Quartile | OR | 95% CI of OR | |
|---|---|---|---|---|---|---|---|
| 0 hours | 0.684 | 70% | 33% | 1 | | | |
| | 0.65 | 81% | 26% | 2 | 1.0 | 0.4 | 2.3 |

## FIG. 6 - 3

| | | | | | | | |
|---|---|---|---|---|---|---|---|
| | 0.518 | 93% | 11% | 3 | 2.3 | 1.3 | 4.3 |
| | 0.913 | 33% | 70% | 4 | 1.2 | 0.6 | 2.6 |
| | 1.01 | 11% | 81% | | | | |
| | 1.13 | 7% | 91% | | | | |
| 24 hours | 0.678 | 72% | 32% | 1 | | | |
| | 0.531 | 81% | 12% | 2 | 1.4 | 0.7 | 2.5 |
| | 0.453 | 91% | 6% | 3 | 1.6 | 0.9 | 2.8 |
| | 0.913 | 25% | 70% | 4 | 1.6 | 0.9 | 2.8 |
| | 1.01 | 16% | 81% | | | | |
| | 1.13 | 6% | 91% | | | | |
| 48 hours | 0.704 | 75% | 35% | 1 | | | |
| | 0.544 | 81% | 13% | 2 | 0.7 | 0.2 | 2.4 |
| | 0.435 | 94% | 6% | 3 | 1.3 | 0.5 | 3.2 |
| | 0.913 | 38% | 70% | 4 | 1.0 | 0.4 | 2.8 |
| | 1.01 | 13% | 81% | | | | |
| | 1.13 | 13% | 91% | | | | |

**Epidermal growth factor**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 79.000 | 67.350 | 79.000 | 59.650 | 79.000 | 48.700 |
| average | 130.455 | 142.071 | 130.455 | 71.301 | 130.455 | 89.184 |
| stdev | 152.140 | 170.033 | 152.140 | 58.897 | 152.140 | 87.376 |
| p (t-test) | | 0.698 | | 0.021 | | 0.254 |
| min | 0.074 | 12.700 | 0.074 | 8.880 | 0.074 | 4.320 |
| max | 1330.000 | 604.000 | 1330.000 | 227.000 | 1330.000 | 327.000 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 74.500 | 98.900 | 74.500 | 51.350 | 74.500 | 99.900 |
| average | 124.554 | 187.462 | 124.554 | 75.975 | 124.554 | 98.303 |
| stdev | 145.722 | 230.488 | 145.722 | 80.525 | 145.722 | 62.748 |
| p (t-test) | | 0.297 | | 0.294 | | 0.634 |
| min | 0.074 | 0.074 | 0.074 | 0.074 | 0.074 | 4.320 |
| max | 1330.000 | 601.000 | 1330.000 | 227.000 | 1330.000 | 188.000 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 79.000 | 59.200 | 79.000 | 63.050 | 79.000 | 48.700 |
| average | 129.447 | 117.570 | 129.447 | 85.658 | 129.447 | 116.750 |
| stdev | 155.109 | 142.993 | 155.109 | 95.616 | 155.109 | 154.509 |
| p (t-test) | | 0.700 | | 0.117 | | 0.749 |
| min | 0.074 | 10.900 | 0.074 | 9.550 | 0.074 | 6.500 |
| max | 1330.000 | 604.000 | 1330.000 | 513.000 | 1330.000 | 601.000 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.48 | 0.056 | 434 | 28 | 0.727 |
| 24 hours | 0.38 | 0.045 | 434 | 36 | 0.008 |
| 48 hours | 0.41 | 0.064 | 434 | 18 | 0.157 |

sCr only

| Time prior AKI stage | AUC | SE | nCohort 1 | nCohort 2 | p |
|---|---|---|---|---|---|
| 0 hours | 0.52 | 0.120 | 542 | 6 | 0.841 |
| 24 hours | 0.38 | 0.082 | 542 | 10 | 0.145 |
| 48 hours | 0.52 | 0.111 | 542 | 7 | 0.859 |

UO only

| Time prior | AUC | SE | nCohort 1 | nCohort 2 | p |
|---|---|---|---|---|---|

**FIG. 6 - 4**

| AKI stage | | | | | |
|---|---|---|---|---|---|
| 0 hours | 0.45 | 0.056 | 356 | 27 | 0.358 |
| 24 hours | 0.41 | 0.049 | 356 | 32 | 0.058 |
| 48 hours | 0.43 | 0.070 | 356 | 16 | 0.308 |

sCr or UO

| Time prior AKI stage | Cutoff value | sens | spec | Quartile | OR | 95% CI of OR | |
|---|---|---|---|---|---|---|---|
| 0 hours | 32.2 | 71% | 21% | 1 | | | |
| | 27.1 | 82% | 16% | 2 | 0.4 | 0.2 | 0.9 |
| | 20.5 | 93% | 14% | 3 | 0.5 | 0.3 | 1.0 |
| | 149 | 32% | 70% | 4 | 1.1 | 0.7 | 1.8 |
| | 205 | 25% | 80% | | | | |
| | 295 | 14% | 90% | | | | |
| 24 hours | 32 | 72% | 21% | 1 | | | |
| | 30.7 | 81% | 19% | 2 | 2.1 | 0.8 | 5.7 |
| | 15.9 | 92% | 10% | 3 | 5.6 | 2.5 | 12.7 |
| | 149 | 17% | 70% | 4 | 4.4 | 1.9 | 10.3 |
| | 205 | 8% | 80% | | | | |
| | 295 | 0% | 90% | | | | |
| 48 hours | 22.4 | 72% | 14% | 1 | | | |
| | 19.4 | 83% | 13% | 2 | 1.0 | 0.4 | 2.8 |
| | 5.37 | 94% | 2% | 3 | 0.7 | 0.2 | 2.4 |
| | 149 | 28% | 70% | 4 | 1.8 | 0.8 | 4.0 |
| | 205 | 6% | 80% | | | | |
| | 295 | 6% | 90% | | | | |

sCr only

| Time prior AKI stage | Cutoff value | sens | spec | Quartile | OR | 95% CI of OR | |
|---|---|---|---|---|---|---|---|
| 0 hours | 22.4 | 83% | 14% | 1 | | | |
| | 22.4 | 83% | 14% | 2 | 0.5 | 0.0 | 9.7 |
| | 0 | 100% | 0% | 3 | 0.5 | 0.0 | 9.7 |
| | 137 | 50% | 70% | 4 | 1.0 | 0.1 | 7.3 |
| | 189 | 33% | 80% | | | | |
| | 275 | 33% | 90% | | | | |
| 24 hours | 32.7 | 70% | 23% | 1 | | | |
| | 15.9 | 80% | 9% | 2 | 1.0 | 0.1 | 7.3 |
| | 8.64 | 90% | 3% | 3 | 1.0 | 0.1 | 7.3 |
| | 137 | 20% | 70% | 4 | 2.0 | 0.5 | 9.1 |
| | 189 | 20% | 80% | | | | |
| | 275 | 0% | 90% | | | | |
| 48 hours | 92.1 | 71% | 59% | 1 | | | |
| | 38.1 | 86% | 27% | 2 | 1.0 | 0.0 | 51.9 |
| | 0.0735 | 100% | 1% | 3 | 4.1 | 0.3 | 48.8 |
| | 137 | 29% | 70% | 4 | 1.0 | 0.0 | 51.5 |
| | 189 | 0% | 80% | | | | |
| | 275 | 0% | 90% | | | | |

UO only

| Time prior AKI stage | Cutoff value | sens | spec | Quartile | OR | 95% CI of OR | |
|---|---|---|---|---|---|---|---|
| 0 hours | 32.2 | 70% | 20% | 1 | | | |
| | 27.1 | 81% | 16% | 2 | 0.7 | 0.3 | 1.4 |
| | 17.5 | 93% | 11% | 3 | 0.7 | 0.3 | 1.4 |
| | 143 | 26% | 70% | 4 | 1.5 | 0.9 | 2.5 |
| | 188 | 19% | 80% | | | | |
| | 284 | 7% | 90% | | | | |
| 24 hours | 32 | 72% | 19% | 1 | | | |
| | 30.2 | 81% | 18% | 2 | 0.8 | 0.3 | 2.0 |
| | 22.4 | 91% | 14% | 3 | 2.8 | 1.6 | 5.1 |
| | 143 | 19% | 70% | 4 | 2.1 | 1.1 | 4.0 |
| | 188 | 9% | 80% | | | | |
| | 284 | 3% | 90% | | | | |
| 48 hours | 22.4 | 75% | 14% | 1 | | | |
| | 20.5 | 81% | 14% | 2 | 0.7 | 0.2 | 2.4 |
| | 14.1 | 94% | 7% | 3 | 0.7 | 0.2 | 2.4 |

**FIG. 6 - 5**

| | | | | | | |
|---|---|---|---|---|---|---|
| 143 | 25% | 70% | 4 | 1.5 | 0.6 | 3.6 |
| 188 | 13% | 80% | | | | |
| 284 | 13% | 90% | | | | |

**Interleukin-1 alpha**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.002 | 0.000 | 0.002 | 0.000 | 0.002 | 0.000 |
| average | 0.003 | 0.002 | 0.003 | 0.002 | 0.003 | 0.001 |
| stdev | 0.003 | 0.003 | 0.003 | 0.002 | 0.003 | 0.003 |
| p (t-test) | | 0.100 | | 0.202 | | 0.101 |
| min | 0.000 | 0.000 | 0.000 | 0.000 | 0.000 | 0.000 |
| max | 0.020 | 0.009 | 0.020 | 0.007 | 0.020 | 0.012 |
| n (Samp) | 434 | 28 | 434 | 36 | 434 | 18 |
| n (Pat) | 173 | 28 | 173 | 36 | 173 | 18 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.001 | 0.000 | 0.001 | 0.000 | 0.001 | 0.000 |
| average | 0.003 | 0.000 | 0.003 | 0.001 | 0.003 | 0.000 |
| stdev | 0.003 | 0.000 | 0.003 | 0.002 | 0.003 | 0.000 |
| p (t-test) | | 0.049 | | 0.105 | | 0.034 |
| min | 0.000 | 0.000 | 0.000 | 0.000 | 0.000 | 0.000 |
| max | 0.020 | 0.000 | 0.020 | 0.005 | 0.020 | 0.000 |
| n (Samp) | 542 | 6 | 542 | 10 | 542 | 7 |
| n (Pat) | 208 | 6 | 208 | 10 | 208 | 7 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.002 | 0.000 | 0.002 | 0.001 | 0.002 | 0.000 |
| average | 0.003 | 0.002 | 0.003 | 0.002 | 0.003 | 0.002 |
| stdev | 0.003 | 0.003 | 0.003 | 0.003 | 0.003 | 0.003 |
| p (t-test) | | 0.195 | | 0.421 | | 0.183 |
| min | 0.000 | 0.000 | 0.000 | 0.000 | 0.000 | 0.000 |
| max | 0.020 | 0.009 | 0.020 | 0.007 | 0.020 | 0.012 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.42 | 0.053 | 434 | 28 | 0.116 |
| 24 hours | 0.45 | 0.049 | 434 | 36 | 0.340 |
| 48 hours | 0.37 | 0.061 | 434 | 18 | 0.038 |

sCr only

| Time prior AKI stage | AUC | SE | nCohort 1 | nCohort 2 | p |
|---|---|---|---|---|---|
| 0 hours | 0.25 | 0.080 | 542 | 6 | 0.002 |
| 24 hours | 0.36 | 0.079 | 542 | 10 | 0.075 |
| 48 hours | 0.25 | 0.074 | 542 | 7 | 0.001 |

UO only

| Time prior AKI stage | AUC | SE | nCohort 1 | nCohort 2 | p |
|---|---|---|---|---|---|
| 0 hours | 0.44 | 0.055 | 356 | 27 | 0.251 |
| 24 hours | 0.47 | 0.052 | 356 | 32 | 0.610 |
| 48 hours | 0.39 | 0.066 | 356 | 16 | 0.090 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.2 | 0.6 | 2.6 |
| | 0 | 100% | 0% | 3 | 3.8 | 2.2 | 6.6 |
| | 0.00446 | 18% | 70% | 4 | 0.0 | 0.0 | na |
| | 0.00577 | 7% | 80% | | | | |
| | 0.00716 | 4% | 90% | | | | |

## FIG. 6 - 6

| | Cutoff value | sens | spec | Quartile | OR | 95% CI of OR | |
|---|---|---|---|---|---|---|---|
| 24 hours | 0 | 100% | 0% | 1 | | | |
| | 0 | 100% | 0% | 2 | 2.6 | 1.4 | 4.7 |
| | 0 | 100% | 0% | 3 | 4.3 | 2.5 | 7.4 |
| | 0.00446 | 22% | 70% | 4 | 0.0 | 0.0 | na |
| | 0.00577 | 8% | 80% | | | | |
| | 0.00716 | 0% | 90% | | | | |
| 48 hours | 0 | 100% | 0% | 1 | | | |
| | 0 | 100% | 0% | 2 | 1.5 | 0.3 | 8.0 |
| | 0 | 100% | 0% | 3 | 7.2 | 2.2 | 23.2 |
| | 0.00446 | 11% | 70% | 4 | 0.0 | 0.0 | na |
| | 0.00577 | 11% | 80% | | | | |
| | 0.00716 | 6% | 90% | | | | |

sCr only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | na | na | na |
| | 0 | 100% | 0% | 3 | na | na | na |
| | 0.00436 | 0% | 70% | 4 | na | na | na |
| | 0.00558 | 0% | 80% | | | | |
| | 0.00705 | 0% | 90% | | | | |
| 24 hours | 0 | 100% | 0% | 1 | | | |
| | 0 | 100% | 0% | 2 | 2.0 | 0.1 | 39.2 |
| | 0 | 100% | 0% | 3 | 0.0 | 0.0 | na |
| | 0.00436 | 10% | 70% | 4 | 7.3 | 0.8 | 70.8 |
| | 0.00558 | 0% | 80% | | | | |
| | 0.00705 | 0% | 90% | | | | |
| 48 hours | 0 | 100% | 0% | 1 | | | |
| | 0 | 100% | 0% | 2 | na | na | na |
| | 0 | 100% | 0% | 3 | na | na | na |
| | 0.00436 | 0% | 70% | 4 | na | na | na |
| | 0.00558 | 0% | 80% | | | | |
| | 0.00705 | 0% | 90% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 1.2 | 0.6 | 2.6 |
| | 0 | 100% | 0% | 3 | 1.4 | 0.7 | 2.9 |
| | 0.00446 | 19% | 71% | 4 | 1.9 | 1.0 | 3.7 |
| | 0.00552 | 11% | 80% | | | | |
| | 0.0068 | 4% | 90% | | | | |
| 24 hours | 0 | 100% | 0% | 1 | | | |
| | 0 | 100% | 0% | 2 | 1.7 | 1.0 | 3.1 |
| | 0 | 100% | 0% | 3 | 0.8 | 0.4 | 1.8 |
| | 0.00446 | 25% | 71% | 4 | 1.9 | 1.1 | 3.4 |
| | 0.00552 | 16% | 80% | | | | |
| | 0.0068 | 6% | 90% | | | | |
| 48 hours | 0 | 100% | 0% | 1 | | | |
| | 0 | 100% | 0% | 2 | 1.0 | 0.1 | 7.4 |
| | 0 | 100% | 0% | 3 | 6.7 | 2.1 | 22.1 |
| | 0.00446 | 13% | 71% | 4 | 0.0 | 0.0 | na |
| | 0.00552 | 13% | 80% | | | | |
| | 0.0068 | 6% | 90% | | | | |

**Interleukin-4**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 46.000 | 34.500 | 46.000 | 43.300 | 46.000 | 36.050 |
| average | 48.456 | 37.966 | 48.456 | 37.711 | 48.456 | 38.428 |
| stdev | 40.230 | 26.209 | 40.230 | 25.558 | 40.230 | 25.454 |
| p (t-test) | | 0.174 | | 0.116 | | 0.295 |

## FIG. 6 - 7

| | | | | | | |
|---|---|---|---|---|---|---|
| min | 1.040 | 1.040 | 1.040 | 1.040 | 1.040 | 1.040 |
| max | 451.000 | 108.000 | 451.000 | 72.800 | 451.000 | 96.000 |
| n (Samp) | 434 | 28 | 434 | 36 | 434 | 18 |
| n (Pat) | 173 | 28 | 173 | 36 | 173 | 18 |

sCr only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 45.900 | 37.250 | 45.900 | 34.200 | 45.900 | 28.100 |
| average | 47.210 | 37.097 | 47.210 | 25.741 | 47.210 | 31.234 |
| stdev | 38.771 | 34.954 | 38.771 | 22.139 | 38.771 | 19.648 |
| p (t-test) | | 0.525 | | 0.082 | | 0.277 |
| min | 1.040 | 1.040 | 1.040 | 1.040 | 1.040 | 1.040 |
| max | 451.000 | 78.900 | 451.000 | 67.400 | 451.000 | 58.400 |
| n (Samp) | 542 | 6 | 542 | 10 | 542 | 7 |
| n (Pat) | 208 | 6 | 208 | 10 | 208 | 7 |

UO only

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 45.950 | 33.000 | 45.950 | 48.750 | 45.950 | 48.100 |
| average | 48.810 | 36.227 | 48.810 | 39.293 | 48.810 | 47.429 |
| stdev | 42.530 | 25.100 | 42.530 | 25.932 | 42.530 | 26.122 |
| p (t-test) | | 0.130 | | 0.214 | | 0.898 |
| min | 1.040 | 1.040 | 1.040 | 1.040 | 1.040 | 1.040 |
| max | 451.000 | 108.000 | 451.000 | 72.800 | 451.000 | 96.000 |
| n (Samp) | 356 | 27 | 356 | 32 | 356 | 16 |
| n (Pat) | 138 | 27 | 138 | 32 | 138 | 16 |

sCr or UO

| Time prior AKI stage | AUC | SE | nCohort 1 | nCohort 2 | p |
|---|---|---|---|---|---|
| 0 hours | 0.41 | 0.052 | 434 | 28 | 0.096 |
| 24 hours | 0.43 | 0.048 | 434 | 36 | 0.141 |
| 48 hours | 0.42 | 0.065 | 434 | 18 | 0.213 |

sCr only

| Time prior AKI stage | AUC | SE | nCohort 1 | nCohort 2 | p |
|---|---|---|---|---|---|
| 0 hours | 0.44 | 0.113 | 542 | 6 | 0.615 |
| 24 hours | 0.30 | 0.071 | 542 | 10 | 0.004 |
| 48 hours | 0.36 | 0.094 | 542 | 7 | 0.125 |

UO only

| Time prior AKI stage | AUC | SE | nCohort 1 | nCohort 2 | p |
|---|---|---|---|---|---|
| 0 hours | 0.40 | 0.053 | 356 | 27 | 0.056 |
| 24 hours | 0.45 | 0.052 | 356 | 32 | 0.375 |
| 48 hours | 0.52 | 0.075 | 356 | 16 | 0.790 |

sCr or UO

| Time prior AKI stage | Cutoff value | sens | spec | Quartile | OR | 95% CI of OR | |
|---|---|---|---|---|---|---|---|
| 0 hours | 17.9 | 71% | 23% | 1 | | | |
| | 10.9 | 86% | 17% | 2 | 2.8 | 1.1 | 7.2 |
| | 0 | 100% | 0% | 3 | 2.8 | 1.1 | 7.1 |
| | 63.7 | 14% | 71% | 4 | 3.2 | 1.3 | 7.9 |
| | 72.2 | 7% | 81% | | | | |
| | 86.2 | 4% | 91% | | | | |
| 24 hours | 13.4 | 72% | 20% | 1 | | | |
| | 0 | 100% | 0% | 2 | 1.9 | 1.1 | 3.3 |
| | 0 | 100% | 0% | 3 | 1.4 | 0.7 | 2.5 |
| | 63.7 | 19% | 71% | 4 | 1.9 | 1.1 | 3.3 |
| | 72.2 | 6% | 81% | | | | |
| | 86.2 | 0% | 91% | | | | |
| 48 hours | 22.7 | 72% | 24% | 1 | | | |
| | 13.4 | 83% | 20% | 2 | 3.1 | 0.8 | 11.9 |
| | 0 | 100% | 0% | 3 | 2.0 | 0.5 | 9.2 |
| | 63.7 | 11% | 71% | 4 | 3.1 | 0.8 | 11.9 |
| | 72.2 | 11% | 81% | | | | |
| | 86.2 | 6% | 91% | | | | |

sCr only

## FIG. 6 - 8

112

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.5 | 0.0 | 9.7 |
| | 0 | 100% | 0% | 3 | 0.0 | 0.0 | na |
| | 62 | 33% | 71% | 4 | 1.5 | 0.3 | 8.0 |
| | 69.8 | 17% | 80% | | | | |
| | 85 | 0% | 90% | | | | |
| 24 hours | 1.04 | 70% | 16% | 1 | | | |
| | 0 | 100% | 0% | 2 | 0.0 | 0.0 | na |
| | 0 | 100% | 0% | 3 | 5.2 | 0.5 | 55.7 |
| | 62 | 10% | 71% | 4 | 4.1 | 0.3 | 48.8 |
| | 69.8 | 0% | 80% | | | | |
| | 85 | 0% | 90% | | | | |
| 48 hours | 22.7 | 71% | 25% | 1 | | | |
| | 20 | 86% | 24% | 2 | na | na | na |
| | 0 | 100% | 0% | 3 | na | na | na |
| | 62 | 0% | 71% | 4 | na | na | na |
| | 69.8 | 0% | 80% | | | | |
| | 85 | 0% | 90% | | | | |

UO only

| Time prior AKI stage | Cutoff value | sens | spec | Quartile | OR | 95% CI of OR | |
|---|---|---|---|---|---|---|---|
| 0 hours | 17.9 | 70% | 23% | 1 | | | |
| | 10.9 | 85% | 18% | 2 | 9.8 | 1.1 | 90.6 |
| | 0 | 100% | 0% | 3 | 8.6 | 0.9 | 81.8 |
| | 63 | 15% | 71% | 4 | 9.9 | 1.1 | 91.6 |
| | 72.8 | 4% | 81% | | | | |
| | 86.2 | 4% | 90% | | | | |
| 24 hours | 18.8 | 72% | 23% | 1 | | | |
| | 0 | 100% | 0% | 2 | 1.9 | 1.1 | 3.4 |
| | 0 | 100% | 0% | 3 | 0.8 | 0.4 | 1.8 |
| | 63 | 22% | 71% | 4 | 1.7 | 1.0 | 3.1 |
| | 72.8 | 0% | 81% | | | | |
| | 86.2 | 0% | 90% | | | | |
| 48 hours | 32.5 | 75% | 31% | 1 | | | |
| | 25.8 | 81% | 26% | 2 | 1.0 | 0.3 | 3.9 |
| | 9.1 | 94% | 18% | 3 | 2.4 | 0.9 | 6.5 |
| | 63 | 31% | 71% | 4 | 1.0 | 0.3 | 3.9 |
| | 72.8 | 19% | 81% | | | | |
| | 86.2 | 6% | 90% | | | | |

**Transforming growth factor beta-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 19.916 | 6.962 | 19.916 | 6.329 | 19.916 | 5.938 |
| average | 30.020 | 13.519 | 30.020 | 8.888 | 30.020 | 5.938 |
| stdev | 32.147 | 16.183 | 32.147 | 8.942 | 32.147 | 3.131 |
| p (t-test) | | 0.134 | | 0.195 | | 0.295 |
| min | 0.036 | 0.036 | 0.036 | 1.359 | 0.036 | 3.724 |
| max | 166.667 | 52.557 | 166.667 | 21.535 | 166.667 | 8.152 |
| n (Samp) | 82 | 9 | 82 | 4 | 82 | 2 |
| n (Pat) | 59 | 9 | 59 | 4 | 59 | 2 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 18.297 | 11.010 | 18.297 | 4.970 | 18.297 | 3.724 |
| average | 28.305 | 11.010 | 28.305 | 4.970 | 28.305 | 3.724 |
| stdev | 30.341 | 8.014 | 30.341 | 5.107 | 30.341 | 0.000 |
| p (t-test) | | 0.424 | | 0.282 | | 0.257 |
| min | 0.036 | 5.343 | 0.036 | 1.359 | 0.036 | 3.724 |
| max | 166.667 | 16.677 | 166.667 | 8.582 | 166.667 | 3.724 |
| n (Samp) | 101 | 2 | 101 | 2 | 101 | 2 |

# FIG. 6 - 9

| n (Pat) | 71 | 2 | 71 | 2 | 71 | 2 |
|---|---|---|---|---|---|---|

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 20.380 | 6.962 | 20.380 | 12.806 | 20.380 | 0.036 |
| average | 32.928 | 13.519 | 32.928 | 12.806 | 32.928 | 8.152 |
| stdev | 34.109 | 16.183 | 34.109 | 12.345 | 34.109 | na |
| p (t-test) | | 0.099 | | 0.411 | | na |
| min | 0.036 | 0.036 | 0.036 | 4.076 | 0.036 | 8.152 |
| max | 166.667 | 52.557 | 166.667 | 21.535 | 166.667 | 8.152 |
| n (Samp) | 64 | 9 | 64 | 2 | 64 | 1 |
| n (Pat) | 46 | 9 | 46 | 2 | 46 | 1 |

sCr or UO

| Time prior AKI stage | AUC | SE | nCohort 1 | nCohort 2 | p |
|---|---|---|---|---|---|
| 0 hours | 0.32 | 0.084 | 82 | 9 | 0.031 |
| 24 hours | 0.27 | 0.108 | 82 | 4 | 0.033 |
| 48 hours | 0.20 | 0.123 | 82 | 2 | 0.016 |

sCr only

| Time prior AKI stage | AUC | SE | nCohort 1 | nCohort 2 | p |
|---|---|---|---|---|---|
| 0 hours | 0.33 | 0.169 | 101 | 2 | 0.313 |
| 24 hours | 0.19 | 0.114 | 101 | 2 | 0.006 |
| 48 hours | 0.16 | 0.102 | 101 | 2 | 0.001 |

UO only

| Time prior AKI stage | AUC | SE | nCohort 1 | nCohort 2 | p |
|---|---|---|---|---|---|
| 0 hours | 0.29 | 0.081 | 64 | 9 | 0.008 |
| 24 hours | 0.33 | 0.171 | 64 | 2 | 0.315 |
| 48 hours | 0.21 | 0.176 | 64 | 1 | 0.101 |

sCr or UO

| Time prior AKI stage | Cutoff value | sens | spec | Quartile | OR | 95% CI of OR | |
|---|---|---|---|---|---|---|---|
| 0 hours | 3.7240933 | 78% | 16% | 1 | | | |
| | 2.7173913 | 89% | 16% | 2 | 1.0 | 0.0 | 60.2 |
| | 0 | 100% | 0% | 3 | 3.3 | 0.2 | 54.3 |
| | 32.670455 | 11% | 71% | 4 | 4.9 | 0.3 | 69.1 |
| | 52.556818 | 0% | 80% | | | | |
| | 79.268293 | 0% | 90% | | | | |
| 24 hours | 2.7173913 | 75% | 16% | 1 | | | |
| | 0.4857513 | 100% | 9% | 2 | na | na | na |
| | 0.4857513 | 100% | 9% | 3 | na | na | na |
| | 32.670455 | 0% | 71% | 4 | na | na | na |
| | 52.556818 | 0% | 80% | | | | |
| | 79.268293 | 0% | 90% | | | | |
| 48 hours | 2.7173913 | 100% | 16% | 1 | | | |
| | 2.7173913 | 100% | 16% | 2 | na | na | na |
| | 2.7173913 | 100% | 16% | 3 | na | na | na |
| | 32.670455 | 0% | 71% | 4 | na | na | na |
| | 52.556818 | 0% | 80% | | | | |
| | 79.268293 | 0% | 90% | | | | |

UO only

| Time prior AKI stage | Cutoff value | sens | spec | Quartile | OR | 95% CI of OR | |
|---|---|---|---|---|---|---|---|
| 0 hours | 3.7240933 | 78% | 13% | 1 | | | |
| | 2.1049223 | 89% | 13% | 2 | 1.1 | 0.0 | 66.8 |
| | 0 | 100% | 0% | 3 | 2.3 | 0.1 | 53.6 |
| | 38.602941 | 11% | 70% | 4 | 6.9 | 0.5 | 94.3 |
| | 53.977273 | 0% | 83% | | | | |
| | 82.417582 | 0% | 91% | | | | |
| 24 hours | 2.1049223 | 100% | 13% | 1 | | | |
| | 2.1049223 | 100% | 13% | 2 | na | na | na |
| | 2.1049223 | 100% | 13% | 3 | na | na | na |
| | 38.602941 | 0% | 70% | 4 | na | na | na |
| | 53.977273 | 0% | 83% | | | | |

## FIG. 6 - 10

| | 82.417582 | 0% | 91% | | | | |
|---|---|---|---|---|---|---|---|
| 48 hours | 6.9624352 | 100% | 20% | 1 | | | |
| | 6.9624352 | 100% | 20% | 2 | na | na | na |
| | 6.9624352 | 100% | 20% | 3 | na | na | na |
| | 38.602941 | 0% | 70% | 4 | na | na | na |
| | 53.977273 | 0% | 83% | | | | |
| | 82.417582 | 0% | 91% | | | | |

**Tubulointerstitial nephritis antigen**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.927 | na | 0.927 | 0.630 | 0.927 | 0.087 |
| average | 47.568 | na | 47.568 | 77.232 | 47.568 | 0.779 |
| stdev | 111.317 | na | 111.317 | 151.335 | 111.317 | na |
| p (t-test) | | na | | 0.438 | | na |
| min | 0.087 | na | 0.087 | 0.111 | 0.087 | 0.779 |
| max | 408.188 | na | 408.188 | 411.095 | 408.188 | 0.779 |
| n (Samp) | 46 | 0 | 46 | 13 | 46 | 1 |
| n (Pat) | 43 | 0 | 43 | 13 | 43 | 1 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.952 | na | 0.952 | 0.457 | 0.952 | na |
| average | 54.223 | na | 54.223 | 2.208 | 54.223 | na |
| stdev | 120.392 | na | 120.392 | 3.314 | 120.392 | na |
| p (t-test) | | na | | 0.461 | | na |
| min | 0.087 | na | 0.087 | 0.136 | 0.087 | na |
| max | 411.095 | na | 411.095 | 6.029 | 411.095 | na |
| n (Samp) | 59 | 0 | 59 | 3 | 59 | 0 |
| n (Pat) | 54 | 0 | 54 | 3 | 54 | 0 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.853 | na | 0.853 | 0.630 | 0.853 | 0.087 |
| average | 42.123 | na | 42.123 | 77.238 | 42.123 | 0.779 |
| stdev | 111.818 | na | 111.818 | 151.332 | 111.818 | na |
| p (t-test) | | na | | 0.373 | | na |
| min | 0.087 | na | 0.087 | 0.111 | 0.087 | 0.779 |
| max | 408.188 | na | 408.188 | 411.095 | 408.188 | 0.779 |
| n (Samp) | 40 | 0 | 40 | 13 | 40 | 1 |
| n (Pat) | 37 | 0 | 37 | 13 | 37 | 1 |

sCr or UO

| Time prior AKI stage | AUC | SE | nCohort 1 | nCohort 2 | p |
|---|---|---|---|---|---|
| 0 hours | nd | nd | 46 | 0 | nd |
| 24 hours | 0.52 | 0.092 | 46 | 13 | 0.813 |
| 48 hours | 0.41 | 0.273 | 46 | 1 | 0.750 |

sCr only

| Time prior AKI stage | AUC | SE | nCohort 1 | nCohort 2 | p |
|---|---|---|---|---|---|
| 0 hours | nd | nd | 59 | 0 | nd |
| 24 hours | 0.34 | 0.145 | 59 | 3 | 0.276 |
| 48 hours | nd | nd | 59 | 0 | nd |

UO only

| Time prior AKI stage | AUC | SE | nCohort 1 | nCohort 2 | p |
|---|---|---|---|---|---|
| 0 hours | nd | nd | 40 | 0 | nd |
| 24 hours | 0.54 | 0.094 | 40 | 13 | 0.638 |
| 48 hours | 0.45 | 0.285 | 40 | 1 | 0.861 |

sCr or UO

| Time prior AKI stage | Cutoff value | sens | spec | Quartile | OR | 95% CI of OR | |
|---|---|---|---|---|---|---|---|
| 0 hours | na | na | na | 1 | | | |
| | na | na | na | 2 | na | na | na |

# FIG. 6 - 11

| | na | na | na | 3 | na | na | na |
|---|---|---|---|---|---|---|---|
| | na | na | na | 4 | na | na | na |
| | na | na | na | | | | |
| | na | na | na | | | | |
| 24 hours | 0.4821005 | 77% | 35% | 1 | | | |
| | 0.4079312 | 85% | 26% | 2 | 3.0 | 0.5 | 16.9 |
| | 0.111254 | 92% | 4% | 3 | 1.5 | 0.2 | 10.6 |
| | 2.8722426 | 38% | 72% | 4 | 1.5 | 0.2 | 10.6 |
| | 25.091241 | 23% | 80% | | | | |
| | 216.32752 | 15% | 91% | | | | |
| 48 hours | 0.7046084 | 100% | 41% | 1 | | | |
| | 0.7046084 | 100% | 41% | 2 | na | na | na |
| | 0.7046084 | 100% | 41% | 3 | na | na | na |
| | 2.8722426 | 0% | 72% | 4 | na | na | na |
| | 25.091241 | 0% | 80% | | | | |
| | 216.32752 | 0% | 91% | | | | |

UO only

| Time prior AKI stage | Cutoff value | sens | spec | Quartile | OR | 95% CI of OR | |
|---|---|---|---|---|---|---|---|
| 0 hours | na | na | na | 1 | | | |
| | na | na | na | 2 | na | na | na |
| | na | na | na | 3 | na | na | na |
| | na | na | na | 4 | na | na | na |
| | na | na | na | | | | |
| | na | na | na | | | | |
| 24 hours | 0.4821005 | 77% | 38% | 1 | | | |
| | 0.4079312 | 85% | 30% | 2 | 3.4 | 0.6 | 20.7 |
| | 0.111254 | 92% | 5% | 3 | 1.0 | 0.1 | 10.1 |
| | 1.8446181 | 38% | 70% | 4 | 2.2 | 0.3 | 13.9 |
| | 7.5577446 | 23% | 80% | | | | |
| | 125.72674 | 23% | 90% | | | | |
| 48 hours | 0.7046084 | 100% | 45% | 1 | | | |
| | 0.7046084 | 100% | 45% | 2 | na | na | na |
| | 0.7046084 | 100% | 45% | 3 | na | na | na |
| | 1.8446181 | 0% | 70% | 4 | na | na | na |
| | 7.5577446 | 0% | 80% | | | | |
| | 125.72674 | 0% | 90% | | | | |

# FIG. 6 - 12

**Netrin-1**

sCr or UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 2.013 | 0.913 | 2.013 | 0.913 | 2.013 | 0.913 |
| stdev | 4.612 | 1.719 | 4.612 | 1.719 | 4.612 | 1.719 |
| p (t-test) | | 0.574 | | 0.574 | | 0.574 |
| min | 0.211 | 0.211 | 0.211 | 0.211 | 0.211 | 0.211 |
| max | 20.646 | 4.422 | 20.646 | 4.422 | 20.646 | 4.422 |
| n (Samp) | 24 | 6 | 24 | 6 | 24 | 6 |
| n (Pat) | 24 | 6 | 24 | 6 | 24 | 6 |

sCr only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.211 | na | 0.211 | na | 0.211 | na |
| average | 2.473 | na | 2.473 | na | 2.473 | na |
| stdev | 6.407 | na | 6.407 | na | 6.407 | na |
| p (t-test) | | na | | na | | na |
| min | 0.211 | na | 0.211 | na | 0.211 | na |
| max | 20.646 | na | 20.646 | na | 20.646 | na |
| n (Samp) | 10 | 0 | 10 | 0 | 10 | 0 |
| n (Pat) | 10 | 0 | 10 | 0 | 10 | 0 |

UO only

| | 0 hr prior toAKI stage | | 24 hr prior toAKI stage | | 48 hr prior toAKI stage | |
|---|---|---|---|---|---|---|
| | Cohort 1 | Cohort 2 | Cohort 1 | Cohort 2 | Cohort 1 | Cohort 2 |
| median | 0.211 | 0.211 | 0.211 | 0.211 | 0.211 | 0.211 |
| average | 1.425 | 1.053 | 1.425 | 1.053 | 1.425 | 1.053 |
| stdev | 2.755 | 1.883 | 2.755 | 1.883 | 2.755 | 1.883 |
| p (t-test) | | 0.782 | | 0.782 | | 0.782 |
| min | 0.211 | 0.211 | 0.211 | 0.211 | 0.211 | 0.211 |
| max | 8.915 | 4.422 | 8.915 | 4.422 | 8.915 | 4.422 |
| n (Samp) | 17 | 5 | 17 | 5 | 17 | 5 |
| n (Pat) | 17 | 5 | 17 | 5 | 17 | 5 |

sCr or UO

| Time prior AKI stage | AUC | SE | nCohort 1 | nCohort 2 | p |
|---|---|---|---|---|---|
| 0 hours | 0.44 | 0.130 | 24 | 6 | 0.649 |
| 24 hours | 0.44 | 0.130 | 24 | 6 | 0.649 |
| 48 hours | 0.44 | 0.130 | 24 | 6 | 0.649 |

sCr only

| Time prior AKI stage | AUC | SE | nCohort 1 | nCohort 2 | p |
|---|---|---|---|---|---|
| 0 hours | nd | nd | 10 | 0 | nd |
| 24 hours | nd | nd | 10 | 0 | nd |
| 48 hours | nd | nd | 10 | 0 | nd |

UO only

| Time prior AKI stage | AUC | SE | nCohort 1 | nCohort 2 | p |
|---|---|---|---|---|---|
| 0 hours | 0.48 | 0.149 | 17 | 5 | 0.906 |
| 24 hours | 0.48 | 0.149 | 17 | 5 | 0.906 |
| 48 hours | 0.48 | 0.149 | 17 | 5 | 0.906 |

sCr or UO

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 11.7 | 0.4 | 311.7 |
| | 0.2106742 | 17% | 71% | 4 | 0.0 | 0.0 | na |
| | 1.8768769 | 17% | 83% | | | | |
| | 8.1073113 | 0% | 92% | | | | |
| 24 hours | 0 | 100% | 0% | 1 | | | |
| | 0 | 100% | 0% | 2 | 0.0 | 0.0 | na |
| | 0 | 100% | 0% | 3 | 11.7 | 0.4 | 311.7 |
| | 0.2106742 | 17% | 71% | 4 | 0.0 | 0.0 | na |
| | 1.8768769 | 17% | 83% | | | | |

## FIG. 7 - 1

117

| | 8.1073113 | 0% | 92% | | | | |
|---|---|---|---|---|---|---|---|
| 48 hours | 0 | 100% | 0% | 1 | | | |
| | 0 | 100% | 0% | 2 | 0.0 | 0.0 | na |
| | 0 | 100% | 0% | 3 | 11.7 | 0.4 | 311.7 |
| | 0.2106742 | 17% | 71% | 4 | 0.0 | 0.0 | na |
| | 1.8768769 | 17% | 83% | | | | |
| | 8.1073113 | 0% | 92% | | | | |

UO only

| Time prior AKI stage | Cutoff value | sens | spec | Quartile | OR | 95% CI of OR | |
|---|---|---|---|---|---|---|---|
| 0 hours | 0 | 100% | 0% | 1 | | | |
| | 0 | 100% | 0% | 2 | 0.0 | 0.0 | na |
| | 0 | 100% | 0% | 3 | 10.0 | 0.2 | 457.0 |
| | 0.2106742 | 20% | 76% | 4 | 0.0 | 0.0 | na |
| | 1.8768769 | 20% | 82% | | | | |
| | 8.1073113 | 0% | 94% | | | | |
| 24 hours | 0 | 100% | 0% | 1 | | | |
| | 0 | 100% | 0% | 2 | 0.0 | 0.0 | na |
| | 0 | 100% | 0% | 3 | 10.0 | 0.2 | 457.0 |
| | 0.2106742 | 20% | 76% | 4 | 0.0 | 0.0 | na |
| | 1.8768769 | 20% | 82% | | | | |
| | 8.1073113 | 0% | 94% | | | | |
| 48 hours | 0 | 100% | 0% | 1 | | | |
| | 0 | 100% | 0% | 2 | 0.0 | 0.0 | na |
| | 0 | 100% | 0% | 3 | 10.0 | 0.2 | 457.0 |
| | 0.2106742 | 20% | 76% | 4 | 0.0 | 0.0 | na |
| | 1.8768769 | 20% | 82% | | | | |
| | 8.1073113 | 0% | 94% | | | | |

**FIG. 7 - 2**

**Growth-regulated alpha protein**

sCr or
UO

|  | 0 hr prior to AKI stage | | 24 hr prior to AKI stage | | 48 hr prior to AKI stage | |
| --- | --- | --- | --- | --- | --- | --- |
|  | Cohort 1 | Cohort 2 | Cohort 1 | Cohort 2 | Cohort 1 | Cohort 2 |
| median | 330.612 | 122.944 | 330.612 | 97.074 | 330.612 | 74.379 |
| average | 722.768 | 227.094 | 722.768 | 161.749 | 722.768 | 165.744 |
| stdev | 1191.020 | 230.399 | 1191.020 | 211.282 | 1191.020 | 232.872 |
| p (t-test) |  | 0.200 |  | 0.225 |  | 0.265 |
| min | 0.368 | 15.427 | 0.368 | 19.383 | 0.368 | 19.383 |
| max | 4966.418 | 627.680 | 4966.418 | 625.731 | 4966.418 | 625.731 |
| n (Samp) | 37 | 10 | 37 | 7 | 37 | 6 |
| n (Pat) | 37 | 10 | 37 | 7 | 37 | 6 |

sCr only

|  | 0 hr prior toAKI stage | | 24 hr prior toAKI stage | | 48 hr prior toAKI stage | |
| --- | --- | --- | --- | --- | --- | --- |
|  | Cohort 1 | Cohort 2 | Cohort 1 | Cohort 2 | Cohort 1 | Cohort 2 |
| median | 306.452 | 97.074 | 306.452 | 97.074 | 306.452 | 60.207 |
| average | 648.994 | 171.434 | 648.994 | 82.168 | 648.994 | 60.207 |
| stdev | 1001.062 | 240.431 | 1001.062 | 41.082 | 1001.062 | 52.139 |
| p (t-test) |  | 0.295 |  | 0.334 |  | 0.413 |
| min | 0.368 | 15.427 | 0.368 | 35.714 | 0.368 | 23.339 |
| max | 4966.418 | 595.238 | 4966.418 | 113.715 | 4966.418 | 97.074 |
| n (Samp) | 60 | 5 | 60 | 3 | 60 | 2 |
| n (Pat) | 60 | 5 | 60 | 3 | 60 | 2 |

UO only

|  | 0 hr prior toAKI stage | | 24 hr prior toAKI stage | | 48 hr prior toAKI stage | |
| --- | --- | --- | --- | --- | --- | --- |
|  | Cohort 1 | Cohort 2 | Cohort 1 | Cohort 2 | Cohort 1 | Cohort 2 |
| median | 332.111 | 177.254 | 332.111 | 120.311 | 332.111 | 114.468 |
| average | 867.224 | 282.754 | 867.224 | 221.434 | 867.224 | 218.513 |
| stdev | 1357.150 | 232.134 | 1357.150 | 277.619 | 1357.150 | 279.889 |
| p (t-test) |  | 0.351 |  | 0.357 |  | 0.355 |
| min | 0.368 | 66.840 | 0.368 | 19.383 | 0.368 | 19.383 |
| max | 4966.418 | 627.680 | 4966.418 | 625.731 | 4966.418 | 625.731 |
| n (Samp) | 27 | 5 | 27 | 4 | 27 | 4 |
| n (Pat) | 27 | 5 | 27 | 4 | 27 | 4 |

sCr or UO

| Time prior AKI stage | AUC | SE | nCohort 1 | nCohort 2 | p |
| --- | --- | --- | --- | --- | --- |
| 0 hours | 0.35 | 0.092 | 37 | 10 | 0.101 |
| 24 hours | 0.26 | 0.090 | 37 | 7 | 0.007 |
| 48 hours | 0.26 | 0.095 | 37 | 6 | 0.010 |

sCr only

| Time prior AKI stage | AUC | SE | nCohort 1 | nCohort 2 | p |
| --- | --- | --- | --- | --- | --- |
| 0 hours | 0.24 | 0.093 | 60 | 5 | 0.006 |
| 24 hours | 0.14 | 0.079 | 60 | 3 | 0.000 |
| 48 hours | 0.11 | 0.079 | 60 | 2 | 0.000 |

UO only

| Time prior AKI stage | AUC | SE | nCohort 1 | nCohort 2 | p |
| --- | --- | --- | --- | --- | --- |
| 0 hours | 0.40 | 0.133 | 27 | 5 | 0.470 |
| 24 hours | 0.31 | 0.130 | 27 | 4 | 0.154 |
| 48 hours | 0.31 | 0.128 | 27 | 4 | 0.128 |

sCr or UO

| Time prior AKI stage | Cutoff value | sens | spec | Quartile | OR | 95% CI of OR | |
| --- | --- | --- | --- | --- | --- | --- | --- |
| 0 hours | 77.256944 | 70% | 19% | 1 |  |  |  |
|  | 53.571429 | 80% | 14% | 2 | 0.5 | 0.0 | 12.5 |
|  | 15.427215 | 90% | 5% | 3 | 1.7 | 0.2 | 12.9 |
|  | 442.85714 | 20% | 70% | 4 | 2.9 | 0.4 | 20.0 |
|  | 677.94486 | 0% | 81% |  |  |  |  |
|  | 3418.0139 | 0% | 92% |  |  |  |  |
| 24 hours | 53.571429 | 71% | 14% | 1 |  |  |  |
|  | 19.382911 | 86% | 5% | 2 | 0.0 | 0.0 | na |
|  | 3.5601266 | 100% | 5% | 3 | 2.2 | 0.1 | 63.6 |

# FIG. 8 - 1

| | Cutoff value | sens | spec | Quartile | OR | 95% CI of OR | |
|---|---|---|---|---|---|---|---|
| | 442.85714 | 14% | 70% | 4 | 5.7 | 0.3 | 106.6 |
| | 677.94486 | 0% | 81% | | | | |
| | 3418.0139 | 0% | 92% | | | | |
| 48 hours | 19.382911 | 83% | 5% | 1 | | | |
| | 19.382911 | 83% | 5% | 2 | 0.0 | 0.0 | na |
| | 3.5601266 | 100% | 5% | 3 | 2.2 | 0.1 | 63.6 |
| | 442.85714 | 17% | 70% | 4 | 4.3 | 0.2 | 94.1 |
| | 677.94486 | 0% | 81% | | | | |
| | 3418.0139 | 0% | 92% | | | | |

sCr only

| Time prior AKI stage | Cutoff value | sens | spec | Quartile | OR | 95% CI of OR | |
|---|---|---|---|---|---|---|---|
| 0 hours | 23.338608 | 80% | 7% | 1 | | | |
| | 23.338608 | 80% | 7% | 2 | 0.0 | 0.0 | na |
| | 3.5601266 | 100% | 3% | 3 | 0.0 | 0.0 | na |
| | 477.55102 | 20% | 70% | 4 | 5.3 | 0.3 | 82.3 |
| | 677.94486 | 0% | 80% | | | | |
| | 1787.5289 | 0% | 90% | | | | |
| 24 hours | 23.338608 | 100% | 7% | 1 | | | |
| | 23.338608 | 100% | 7% | 2 | na | na | na |
| | 23.338608 | 100% | 7% | 3 | na | na | na |
| | 477.55102 | 0% | 70% | 4 | na | na | na |
| | 677.94486 | 0% | 80% | | | | |
| | 1787.5289 | 0% | 90% | | | | |
| 48 hours | 19.382911 | 100% | 5% | 1 | | | |
| | 19.382911 | 100% | 5% | 2 | na | na | na |
| | 19.382911 | 100% | 5% | 3 | na | na | na |
| | 477.55102 | 0% | 70% | 4 | na | na | na |
| | 677.94486 | 0% | 80% | | | | |
| | 1787.5289 | 0% | 90% | | | | |

UO only

| Time prior AKI stage | Cutoff value | sens | spec | Quartile | OR | 95% CI of OR | |
|---|---|---|---|---|---|---|---|
| 0 hours | 130.30303 | 80% | 19% | 1 | | | |
| | 130.30303 | 80% | 19% | 2 | 1.0 | 0.0 | 88.2 |
| | 53.571429 | 100% | 11% | 3 | 1.0 | 0.0 | 88.2 |
| | 447.94721 | 20% | 70% | 4 | 2.3 | 0.1 | 81.0 |
| | 647.86967 | 0% | 81% | | | | |
| | 3464.2032 | 0% | 93% | | | | |
| 24 hours | 53.571429 | 75% | 11% | 1 | | | |
| | 0.3677885 | 100% | 4% | 2 | 0.0 | 0.0 | na |
| | 0.3677885 | 100% | 4% | 3 | 1.0 | 0.0 | 88.2 |
| | 447.94721 | 25% | 70% | 4 | 2.8 | 0.1 | 103.7 |
| | 647.86967 | 0% | 81% | | | | |
| | 3464.2032 | 0% | 93% | | | | |
| 48 hours | 39.663462 | 75% | 7% | 1 | | | |
| | 0.3677885 | 100% | 4% | 2 | 0.0 | 0.0 | na |
| | 0.3677885 | 100% | 4% | 3 | 1.0 | 0.0 | 88.2 |
| | 447.94721 | 25% | 70% | 4 | 2.8 | 0.1 | 103.7 |
| | 647.86967 | 0% | 81% | | | | |
| | 3464.2032 | 0% | 93% | | | | |

**Transforming growth factor beta-1**

sCr or
UO

| | 0 hr prior to AKI stage | | 24 hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| median | 23.154 | 8.582 | 23.154 | 3.724 | 23.154 | 3.724 |
| average | 36.931 | 11.770 | 36.931 | 5.725 | 36.931 | 5.644 |
| stdev | 35.835 | 9.784 | 35.835 | 3.815 | 35.835 | 3.579 |
| p (t-test) | | 0.034 | | 0.028 | | 0.040 |
| min | 0.036 | 1.359 | 0.036 | 1.359 | 0.036 | 2.717 |
| max | 166.667 | 34.091 | 166.667 | 11.820 | 166.667 | 11.820 |

# FIG. 8 - 2

| n (Samp) | 37 | 10 | 37 | 7 | 37 | 6 |
| n (Pat) | 37 | 10 | 37 | 7 | 37 | 6 |

sCr only

|  | 0 hr prior toAKI stage | | 24 hr prior toAKI stage | | 48 hr prior toAKI stage | |
|  | Cohort 1 | Cohort 2 | Cohort 1 | Cohort 2 | Cohort 1 | Cohort 2 |
|---|---|---|---|---|---|---|
| median | 21.739 | 8.582 | 21.739 | 8.582 | 21.739 | 7.772 |
| average | 33.191 | 7.137 | 33.191 | 7.253 | 33.191 | 7.772 |
| stdev | 32.494 | 3.959 | 32.494 | 5.356 | 32.494 | 5.725 |
| p (t-test) |  | 0.080 |  | 0.175 |  | 0.277 |
| min | 0.036 | 1.359 | 0.036 | 1.359 | 0.036 | 3.724 |
| max | 166.667 | 11.820 | 166.667 | 11.820 | 166.667 | 11.820 |
| n (Samp) | 61 | 5 | 61 | 3 | 61 | 2 |
| n (Pat) | 61 | 5 | 61 | 3 | 61 | 2 |

UO only

|  | 0 hr prior toAKI stage | | 24 hr prior toAKI stage | | 48 hr prior toAKI stage | |
|  | Cohort 1 | Cohort 2 | Cohort 1 | Cohort 2 | Cohort 1 | Cohort 2 |
|---|---|---|---|---|---|---|
| median | 24.457 | 16.677 | 24.457 | 3.724 | 24.457 | 3.724 |
| average | 41.340 | 16.404 | 41.340 | 4.579 | 41.340 | 4.579 |
| stdev | 38.205 | 12.084 | 38.205 | 2.429 | 38.205 | 2.429 |
| p (t-test) |  | 0.163 |  | 0.068 |  | 0.068 |
| min | 1.359 | 2.717 | 1.359 | 2.717 | 1.359 | 2.717 |
| max | 166.667 | 34.091 | 166.667 | 8.152 | 166.667 | 8.152 |
| n (Samp) | 27 | 5 | 27 | 4 | 27 | 4 |
| n (Pat) | 27 | 5 | 27 | 4 | 27 | 4 |

sCr or UO

| Time prior AKI stage | AUC | SE | nCohort 1 | nCohort 2 | p |
|---|---|---|---|---|---|
| 0 hours | 0.22 | 0.073 | 37 | 10 | 0.000 |
| 24 hours | 0.13 | 0.058 | 37 | 7 | 0.000 |
| 48 hours | 0.13 | 0.063 | 37 | 6 | 0.000 |

sCr only

| Time prior AKI stage | AUC | SE | nCohort 1 | nCohort 2 | p |
|---|---|---|---|---|---|
| 0 hours | 0.17 | 0.072 | 61 | 5 | 0.000 |
| 24 hours | 0.17 | 0.090 | 61 | 3 | 0.000 |
| 48 hours | 0.18 | 0.115 | 61 | 2 | 0.006 |

UO only

| Time prior AKI stage | AUC | SE | nCohort 1 | nCohort 2 | p |
|---|---|---|---|---|---|
| 0 hours | 0.25 | 0.105 | 27 | 5 | 0.018 |
| 24 hours | 0.05 | 0.038 | 27 | 4 | 0.000 |
| 48 hours | 0.05 | 0.038 | 27 | 4 | 0.000 |

**Tubulointerstitial nephritis antigen**

sCr or UO

|  | 0 hr prior to AKI stage | | 24 hr prior to AKI stage | | 48 hr prior to AKI stage | |
|  | Cohort 1 | Cohort 2 | Cohort 1 | Cohort 2 | Cohort 1 | Cohort 2 |
|---|---|---|---|---|---|---|
| median | 0.532 | 0.618 | 0.532 | 0.618 | 0.532 | 0.618 |
| average | 49.191 | 1.514 | 49.191 | 1.514 | 49.191 | 0.649 |
| stdev | 117.958 | 2.240 | 117.958 | 2.240 | 117.958 | 0.447 |
| p (t-test) |  | 0.337 |  | 0.337 |  | 0.425 |
| min | 0.087 | 0.136 | 0.087 | 0.136 | 0.087 | 0.136 |
| max | 408.188 | 6.029 | 408.188 | 6.029 | 408.188 | 1.224 |
| n (Samp) | 25 | 6 | 25 | 6 | 25 | 4 |
| n (Pat) | 25 | 6 | 25 | 6 | 25 | 4 |

sCr only

|  | 0 hr prior toAKI stage | | 24 hr prior toAKI stage | | 48 hr prior toAKI stage | |
|  | Cohort 1 | Cohort 2 | Cohort 1 | Cohort 2 | Cohort 1 | Cohort 2 |
|---|---|---|---|---|---|---|
| median | 0.754 | 0.457 | 0.754 | 0.457 | 0.754 | 0.358 |
| average | 60.042 | 0.391 | 60.042 | 0.391 | 60.042 | 0.358 |
| stdev | 131.942 | 0.230 | 131.942 | 0.230 | 131.942 | 0.315 |
| p (t-test) |  | 0.442 |  | 0.442 |  | 0.530 |
| min | 0.087 | 0.136 | 0.087 | 0.136 | 0.087 | 0.136 |
| max | 411.095 | 0.581 | 411.095 | 0.581 | 411.095 | 0.581 |
| n (Samp) | 44 | 3 | 44 | 3 | 44 | 2 |

# FIG. 8 - 3

| n (Pat) | 44 | 3 | 44 | 3 | 44 | 2 |
|---------|-----|---|-----|---|-----|---|

UO only

| | 0 hr prior toAKI stage | | 24 hr prior toAKI stage | | 48 hr prior toAKI stage | |
|---------|----------|----------|----------|----------|----------|----------|
| | Cohort 1 | Cohort 2 | Cohort 1 | Cohort 2 | Cohort 1 | Cohort 2 |
| median | 0.804 | 0.939 | 0.804 | 0.939 | 0.804 | 0.655 |
| average | 49.793 | 2.122 | 49.793 | 2.122 | 49.793 | 0.820 |
| stdev | 118.251 | 2.620 | 118.251 | 2.620 | 118.251 | 0.352 |
| p (t-test) | | 0.434 | | 0.434 | | 0.487 |
| min | 0.087 | 0.581 | 0.087 | 0.581 | 0.087 | 0.581 |
| max | 408.188 | 6.029 | 408.188 | 6.029 | 408.188 | 1.224 |
| n (Samp) | 25 | 4 | 25 | 4 | 25 | 3 |
| n (Pat) | 25 | 4 | 25 | 4 | 25 | 3 |

sCr or UO

| Time prior AKI stage | AUC | SE | nCohort 1 | nCohort 2 | p |
|---------|-----|-----|-----|-----|-----|
| 0 hours | 0.49 | 0.133 | 25 | 6 | 0.940 |
| 24 hours | 0.49 | 0.133 | 25 | 6 | 0.940 |
| 48 hours | 0.44 | 0.152 | 25 | 4 | 0.694 |

sCr only

| Time prior AKI stage | AUC | SE | nCohort 1 | nCohort 2 | p |
|---------|-----|-----|-----|-----|-----|
| 0 hours | 0.31 | 0.139 | 44 | 3 | 0.163 |
| 24 hours | 0.31 | 0.139 | 44 | 3 | 0.163 |
| 48 hours | 0.27 | 0.155 | 44 | 2 | 0.142 |

UO only

| Time prior AKI stage | AUC | SE | nCohort 1 | nCohort 2 | p |
|---------|-----|-----|-----|-----|-----|
| 0 hours | 0.57 | 0.161 | 25 | 4 | 0.664 |
| 24 hours | 0.57 | 0.161 | 25 | 4 | 0.664 |
| 48 hours | 0.52 | 0.181 | 25 | 3 | 0.912 |

sCr or UO

| Time prior AKI stage | Cutoff value | sens | spec | Quartile | OR | 95% CI of OR | |
|---------|---------|------|------|----------|-----|------|------|
| 0 hours | 0.4079312 | 83% | 40% | 1 | | | |
| | 0.4079312 | 83% | 40% | 2 | 4.2 | 0.2 | 112.2 |
| | 0.0865309 | 100% | 4% | 3 | 1.0 | 0.0 | 88.2 |
| | 1.9259983 | 17% | 72% | 4 | 1.2 | 0.0 | 107.9 |
| | 22.125912 | 0% | 80% | | | | |
| | 209.06008 | 0% | 92% | | | | |
| 24 hours | 0.4079312 | 83% | 40% | 1 | | | |
| | 0.4079312 | 83% | 40% | 2 | 4.2 | 0.2 | 112.2 |
| | 0.0865309 | 100% | 4% | 3 | 1.0 | 0.0 | 88.2 |
| | 1.9259983 | 17% | 72% | 4 | 1.2 | 0.0 | 107.9 |
| | 22.125912 | 0% | 80% | | | | |
| | 209.06008 | 0% | 92% | | | | |
| 48 hours | 0.5315467 | 75% | 52% | 1 | | | |
| | 0.0865309 | 100% | 4% | 2 | na | na | na |
| | 0.0865309 | 100% | 4% | 3 | na | na | na |
| | 1.9259983 | 0% | 72% | 4 | na | na | na |
| | 22.125912 | 0% | 80% | | | | |
| | 209.06008 | 0% | 92% | | | | |

# FIG. 8 - 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- PRAUGHT ; SHLIPAK. *Curr Opin Nephrol Hypertens,* 2005, vol. 14, 265-270
- CHERTOW et al. *J Am Soc Nephrol,* 2005, vol. 16, 3365-3370
- LASSNIGG. *J Am Soc Nephrol,* 2004, vol. 15, 1597-1605
- BELLOMO et al. *Crit Care.,* 2004, vol. 8 (4), R204-12
- KELLUM. *Crit. Care Med.,* 2008, vol. 36, S141-45
- RICCI et al. *Kidney Int.,* 2008, vol. 73, 538-546
- MEHTA et al. *Crit. Care,* 2007, vol. 11, R31
- MCCOLLOUGH et al. *Rev Cardiovasc Med.,* 2006, vol. 7 (4), 177-197
- The Immunoassay Handbook. Stockton Press, 1994
- Fundamental Immunology. Raven Press, 1993
- WILSON. *J. Immunol. Methods,* 1994, vol. 175, 267-273
- YARMUSH. *J. Biochem. Biophys. Methods,* 1992, vol. 25, 85-97
- WARD et al. *Nature,* 1989, vol. 341, 544-546
- VAN ERP et al. *J. Immunoassay,* 1991, vol. 12, 425-43
- NELSON ; GRISWOLD. *Comput. Methods Programs Biomed.,* 1988, vol. 27, 65-8
- CWIRLA et al. *Proc. Natl. Acad. Sci. USA,* 1990, vol. 87, 6378-82
- DEVLIN et al. *Science,* 1990, vol. 249, 404-6
- SCOTT ; SMITH. *Science,* 1990, vol. 249, 386-88
- FISCHER et al. *Intensive Care Med.,* 2003, vol. 29, 1043-51
- BAGSHAW et al. *Nephrol. Dial. Transplant.,* 2008, vol. 23, 1203-1210
- Merck Manual of Diagnosis and Therapy. Merck Research Laboratories, 1999
- WIJEYSUNDERA et al. *JAMA,* 2007, vol. 297, 1801-9
- HANLEY, J. A. ; MCNEIL, B.J. The meaning and use of the area under a receiver operating characteristic (ROC) curve. *Radiology,* 1982, vol. 143, 29-36